# EUROPEAN PATENT APPLICATION

(11) **EP 3 822 974 A1**
(43) Date of publication of application: **19.05.2021**
(21) Application number: 20164353.3
(22) Date of filing: 19.03.2020
(51) Int. Cl.: G16B 20/00, G16H 20/10

(54) **COMPUTATIONAL PLATFORM TO IDENTIFY THERAPEUTIC TREATMENTS FOR NEURODEVELOPMENTAL CONDITIONS**

(30) Priority: 15.11.2019 EP 19383010
(71) Applicant: Stalicla S.A., 1202 Geneva (CH)
(72) Inventor: DURHAM, Lynn, 1202 Geneva (CH)
(74) Representative: Stolmár & Partner Intellectual Property GmbH

(57) **Abstract**

Systems and techniques can determine candidate treatments for individuals diagnosed with a biological condition. The biological condition can include a neurodevelopmental condition and the candidate treatments can include one or more therapeutics. In one or more implementations, data, such as genetic data, morphological data, and levels of analytes, can be used to group individuals that have been diagnosed with a neurodevelopmental condition based on behavioral classifications. Biological pathways that are disrupted by the neurodevelopmental condition can also be identified along with one or more genes of the biological pathways whose expression is impacted by the neurodevelopmental condition. Therapeutics can be identified to treat individuals diagnosed with the neurodevelopmental condition based on genetic profiles of the therapeutics and genetic profiles of the individuals in which the neurodevelopmental condition is present.

## Description

### BACKGROUND

Neurodevelopmental conditions include a group of conditions in which the growth and development of the brain and the central nervous system of individuals is disturbed. These conditions can include autism spectrum disorder, dyslexia, attention deficit hyperactivity disorder (ADHD), schizophrenia, and the like. Typically, neurodevelopmental conditions are characterized based on behavioral indicators. Conventional treatments for neurodevelopmental conditions are often broadly applied to individuals based on a general diagnosis by a clinician according to various behaviors that individuals exhibit. The effectiveness of conventional approaches for the treatment of neurodevelopmental conditions is frequently limited, inconsistent, and difficult to predict.

### BRIEF DESCRIPTION OF DRAWINGS

The present disclosure is illustrated by way of example and not limitation in the figures of the accompanying drawings, in which like references indicate similar elements.
Figure 1 is a diagram illustrating an example framework to determine one or more candidate treatments for individuals in which one or more neurodevelopmental conditions are present, in accordance with one or more example implementations.
Figure 2 is a diagram illustrating an example framework to generate a number of curated databases using information obtained from a number of data sources in accordance with one or more example implementations.
Figure 3 is a diagram illustrating an example framework to determine characteristics of different subgroups of individuals in which a neurodevelopmental condition is present, in accordance with one or more example implementations.
Figure 4 is a diagram of an example framework to analyze a number of types of genetic data to determine confidence levels that various genes are related to risk for at least one biological condition, in accordance with one or more example implementations.
Figure 5 is a diagram of an example framework to remove redundant biological pathways from a set of biological pathways that are related to a neurodevelopmental condition, in accordance with one or more example implementations.
Figure 6 is a diagram of an example framework to analyze a number of types of data to determine subsets of a group of individuals in which a neurodevelopmental condition is present, in accordance with one or more example implementations.
Figure 7 is an example framework to evaluate groupings of individuals that have been identified according to different types of information and determining a level of preservation among the groupings associated with the respective types of information, in accordance with one or more example implementations.
Figure 8 is a diagram of an example framework to determine one or more biological conditions that characterize a group of individuals in which a neurodevelopmental condition is present and to determine a group of genes that is common to the one or more biological conditions, in accordance with one or more example implementations.
Figure 9 is a diagram of an example framework to determine genes of individuals in which autism spectrum disorder is present, where the genes are common to individuals having biological conditions that include seizures, inflammation, and regulation of lipid metabolism by PPAR alpha, in accordance with one or more example implementations.
Figure 10 is a diagram of an example framework to determine gene expression profiles indicating up-regulation and down-regulation of a number of genes for a plurality of therapeutics, in accordance with one or more example implementations.
Figure 11 is a diagram illustrating an example framework to analyze characteristics of groups of individuals in which a neurodevelopmental condition is present and characteristics of a number of therapeutics to determine one or more therapeutics that can be used to treat the neurodevelopmental condition in one or more of the groups of individuals, in accordance with one or more example implementations.
Figure 12 is a diagram of an example framework to determine one or more therapeutics to treat different sub-groups of individuals in which a neurodevelopmental condition is present based on gene expression profiles of the therapeutics and gene expression profiles of the individuals, in accordance with one or more example implementations.
Figure 13 is a diagram of an example framework to determine genetic profiles of individuals that have at least a threshold probability of being responsive to a therapeutic for the treatment of a neurodevelopmental condition, in accordance with one or more example implementations.
Figure 14 is a diagram of an example framework to determine therapeutics that can be candidate treatments for subgroups of individuals based on biological pathways affected by the therapeutics and biological pathways impacted in individuals in which a neurodevelopmental condition is present, in accordance with one or more example implementations.
Figure 15 is a flow diagram of an example process 1500 to determine a candidate treatment for individuals in which a neurodevelopmental condition is present, in accordance with one or more example implementations.
Figure 16 is a flow diagram of an example process 1600 to determine one or more biological pathways that can be used to determine subgroups of individuals in which a neurodevelopmental condition is present, in accordance with one or more example implementations.
Figure 17 is a flow diagram of a first example process 1700 to determine features that can be used to identify subgroups of individuals in which a neurodevelopmental condition is present, in accordance with one or more example implementations.
Figure 18 is a flow diagram of a second example process 1800 to determine features that can be used to identify subgroups of individuals in which a neurodevelopmental condition is present, in accordance with one or more example implementations.
Figure 19 is a flow diagram of an example process 1900 to generate a gene expression profile distance network, in accordance with one or more example implementations.
Figure 20 is a flow diagram of an example process 2000 to determine one or more therapeutics that can be candidate treatments for one or more subgroups of individuals in which a neurodevelopmental condition is present, in accordance with one or more example implementations.
Figure 21 is a flow diagram of an example process 2100 to determine features of a group of individuals that have at least a threshold probability of being responsive to a therapeutic in relation to a neurodevelopmental condition, in accordance with one or more example implementations.
Figure 22 is a block diagram illustrating an example architecture of software, which can be installed on any one or more of the devices described herein, in accordance with one or more example implementations.
Figure 23 illustrates a diagrammatic representation of an example machine in the form of a computer system within which a set of instructions may be executed for causing the machine to perform any one or more of the methodologies discussed herein, in accordance with one or more example implementations.

### DETAILED DESCRIPTION

Although neurodevelopmental conditions are typically diagnosed in individuals based on behavioral indicators, the behavioral indicators are often symptomatic of underlying causes. In many instances, neurodevelopmental conditions can be rooted, at least in part, in genetic causes. However, genetic features are not usually considered in the diagnosis and treatment of individuals with respect to neurodevelopmental conditions because the genetic information is not known by the clinicians making the diagnosis and prescribing the treatment. Additionally, individuals diagnosed with neurodevelopmental conditions can exhibit various morphological features that are not often considered in the diagnosis and treatment of neurodevelopmental conditions because the focus of the diagnosis and treatment is on the behavioral indicators.

The diagnosis and treatment of individuals with neurodevelopmental conditions can also differ from the diagnosis and treatment of individuals with other biological conditions in that the pathology used to diagnose and treat many biological conditions can be based on the analysis of tissue samples. For example, the diagnosis and treatment of cancer often takes place after the analysis of a tissue sample obtained from a biopsy. In these situations, the biological conditions being diagnosed and treated are often not developmental in nature, but have an onset that does not necessarily affect the development of an individual and/or that does not manifest in a manner that affects the development of the individual. Additionally, genetic information can be obtained from the tissue samples and be used in determining the treatment of the individuals from which the tissue samples were taken.

The conventional diagnosis and treatment of neurodevelopmental conditions fails to take into consideration the heterogeneity of individuals diagnosed with the neurodevelopmental conditions. That is, individuals can be diagnosed with a neurodevelopmental condition based on a subset of common behavioral traits of the individuals. However, some groups of individuals diagnosed with a neurodevelopmental condition can have additional characteristics that differ from characteristics of another group of individuals diagnosed with the neurodevelopmental condition. For example, one group of individuals diagnosed with autism spectrum disorder can have morphological features and/or genetic characteristics that are different from those of another group of individuals diagnosed with autism spectrum disorder.

Conventional treatments for neurodevelopmental conditions do not take into account the heterogeneity of the individuals diagnosed with respective neurodevelopmental conditions. As a result, although treatments provided to some individuals diagnosed with a neurodevelopmental condition can be effective, the same treatments provided to additional individuals diagnosed with the neurodevelopmental condition are not effective. Thus, the conventional constructs for diagnosing individuals with neurodevelopmental conditions and prescribing treatments are limited in the relief that individuals can obtain from their symptoms. In particular, the frameworks used to diagnose individuals with neurodevelopmental conditions are not predictive of the response of individuals to treatments. Accordingly, the symptoms of many individuals diagnosed with neurodevelopmental conditions remain untreated or, in some cases, become more severe.

The implementations described herein are directed to developing frameworks, architectures, systems, methods, and techniques for characterizing individuals diagnosed with neurodevelopmental conditions according to characteristics that are not merely behavioral characteristics. The implementations described herein can include a computational biology system that identifies subgroups of individuals in which a neurodevelopmental condition is present. The individuals included in each sub-group can have a common set of features that can include at least one or more morphological features, one or more genetic features, one or more co-occurring conditions, disruption of one or more biological pathways, one or more metabolic features, levels of one or more analytes, or one or more combinations thereof. The characteristics of the individuals included in each sub-group can also be predictive of the responsiveness of the individuals to one or more treatments for a given neurodevelopmental condition.

In various implementations, the techniques and systems described herein can analyze medical records of individuals, genetic information of individuals, medical and biological literature, or one or more combinations thereof, to determine similarities between groups of individuals diagnosed with a neurodevelopmental condition. The analysis of data used to determine the groups of individuals diagnosed with a neurodevelopmental condition can include at least one of one or more machine learning techniques or one or more statistical techniques. In one or more implementations, a computational biology system can determine common genetic features of groups of individuals in which a neurodevelopmental condition is present by identifying biological pathways of the individuals that are disrupted in the individuals. The identification of the disrupted biological pathways can be used by the computational biology system to determine genetic features that are common to various groups of individuals in which a neurodevelopmental condition is present. Additionally, the computational biology system can determine one or more genetic features that are common to groups of individuals diagnosed with a neurodevelopmental condition based one or more morphological characteristics of the individuals included in a respective group. Further, the computational biology system can determine one or more genetic features that are common to groups of individuals diagnosed with a neurodevelopmental condition based on one or more co-occurring conditions of the individuals included in a respective group. In this way, the computational biology system can determine different genetic profiles for the individuals included in respective groups in which a neurodevelopmental condition is present.

In addition to determining genetic profiles for individuals in which a neurodevelopmental condition is present, the computational biology system can also determine genetic profiles for therapeutics that can be used to treat the neurodevelopmental condition. To illustrate, the computational biology system can identify biological pathways that are affected by the therapeutics and determine the genetic features that are associated with the biological pathways to determine at least a portion of the genetic profiles for the therapeutics. The computational biology system can also determine at least a portion of the genetic profile for a therapeutic by analyzing proteins that bind to the therapeutics and genes regulated by the therapeutics in relation to biological pathways disrupted in individuals in which a neurodevelopmental condition is present.

The computational biology system can analyze the genetic profiles of therapeutics in relation to genetic profiles of individuals in which a neurodevelopmental condition is present to identify one or more therapeutics that can be prescribed as treatment for the neurodevelopmental condition. In example implementations, the genetic profiles of individuals in which a neurodevelopmental condition is present can indicate one or more genes that are up-regulated and one or more genes that are down-regulated. The genetic profiles of the therapeutics can also indicate one or more genes that are up-regulated and one or more genes that are down-regulated by the therapeutics. The computational biology system can analyze the genetic profiles of therapeutics with respect to the genetic profiles of individuals to determine correlations between the up-regulated genes and down-regulated genes of therapeutics in relation to the up-regulated genes and down-regulated genes of individuals in which a neurodevelopmental condition is present. Based on the analysis by the computational biology system and the correlations between the genetic profiles of individuals and therapeutics, the computational biology system can determine one or more therapeutics that have at least a threshold probability of relieving symptoms of a group of individuals in which a neurodevelopmental condition is present. Thus, the one or more therapeutics can candidate treatments for the neurodevelopmental condition in the group of individuals. In various implementations, one or more first therapeutics can be identified by the computational biology system as candidate treatments for a first group of individuals with respect to a neurodevelopmental condition that can be different from one or more second therapeutics determined by the computational biology system as candidate treatments for a second, different group of individuals with respect to the neurodevelopmental condition.

By analyzing characteristics of individuals in which a neurodevelopmental condition is present that are different from behavioral characteristics, the computational biology system described herein can place the individuals into subgroups that are defined by the computational biology system rather than analyzing information about individuals that are placed into broader groups according to behavioral characteristics. The subgroups can be determined based on genetic characteristics of the individuals, morphological characteristics of the individuals, comorbid conditions of the individuals, levels of analytes of the individuals, electrical activity data of the individuals, internal structural features of the individuals, or one or more combinations thereof. Additionally, by grouping the individuals in which a neurodevelopmental condition is present according to implementations described herein, the computational biology system can have a higher probability of identifying therapeutics that can be used to effectively treat the neurodevelopmental condition. In particular, the computational biology system can determine subgroups for individuals in which a neurodevelopmental condition is present based on the predisposition of the individuals to respond to one or more therapeutics. Accordingly, the recommendations for candidate treatments determined by the computational biology system for individuals within a particular grouping can have a greater probability of decreasing the symptoms of the individuals with respect to the neurodevelopmental condition than the treatments of the neurodevelopmental condition that are determined according to conventional methods and techniques.

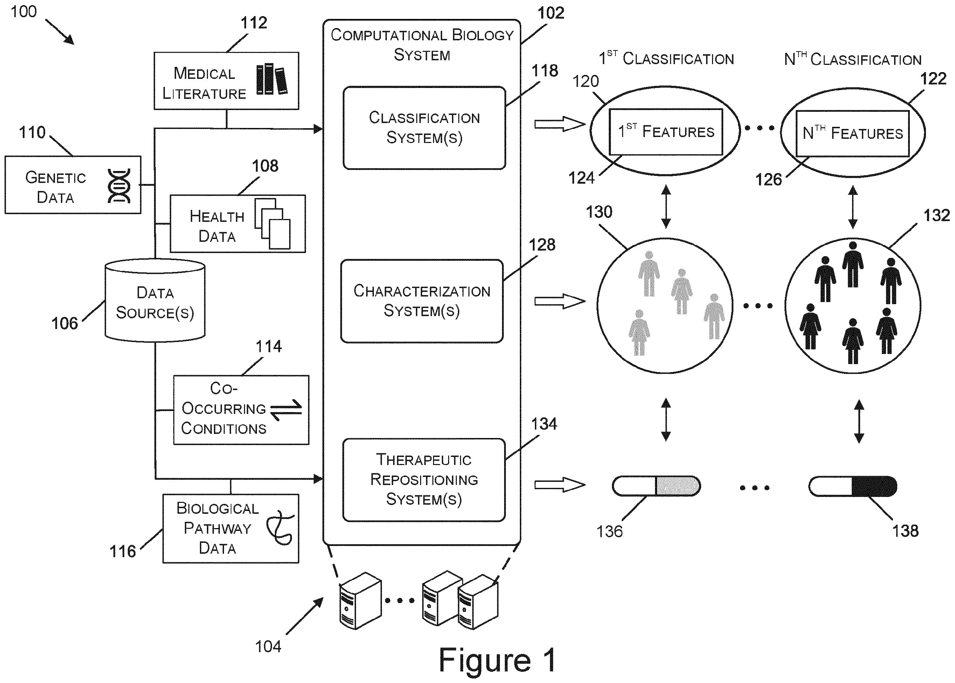

Figure 1 is a diagram illustrating an example framework 100 to determine one or more candidate treatments for individuals in which one or more neurodevelopmental conditions are present, in accordance with one or more example implementations. The framework 100 can include a computational biology system 102 that can analyze information about a number of individuals in which a neurodevelopmental condition is present and determine recommendations for treatment of the neurodevelopmental condition. The computational biology system 102 can be implemented by one or more computing devices 104. The one or more computing devices 104 can include one or more server computing devices, one or more desktop computing devices, one or more laptop computing devices, one or more tablet computing devices, one or more mobile computing devices, or combinations thereof. In one or more implementations, at least a portion of the one or more computing devices 104 can be arranged in a distributed computing environment. For example, at least a portion of the one or more computing devices 104 can be implemented in a cloud computing architecture.

The computational biology system 102 can be coupled to, or otherwise have access to one or more data sources 106. The one or more data sources 106 can include publicly accessible databases, privately accessible databases, websites, one or more combinations thereof, and the like. In one or more implementations, at least a portion of the data obtained by the computational biology system 102 from the one or more data sources 106 can be sent from the one or more data sources 106 to the computational biology system 102. In addition, at least a portion of the data obtained by the computational biology system 102 from the one or more data sources 106 can be requested by the computational biology system 102. In various implementations, the computational biology system 102 can access the one or more data sources 106 using calls of one or more application programming interfaces. In situations where the one or more data sources 106 include websites, the computational biology system 102 can use web crawlers and/or other data scraping tools to obtain data from the websites. In additional implementations, the one or more data sources 106 can include electronic medical records of individuals.

The computational biology system 102 can obtain health data 108 from the one or more data sources 106. The health data 108 can include observations made by healthcare practitioners. In one or more implementations, the health data 108 can include diagnoses of individuals with one or more biological conditions. As used herein, a biological condition can refer to an abnormality of function and/or structure in an individual to such a degree as to produce or threaten to produce a detectable feature of the abnormality. A biological condition can be characterized by external and/or internal characteristics, signs, and/or symptoms that indicate a deviation from a biological norm in one or more populations. A biological condition can include at least one of one or more diseases, one or more disorders, one or more injuries, one or more syndromes, one or more disabilities, one or more infections, one or more isolated symptoms, or other atypical variations of biological structure and/or function of individuals. In one or more illustrative examples, the one or more biological conditions can include one or more neurodevelopmental conditions. The health data 108 can also include laboratory test results for individuals. The laboratory test results can include levels of one or more analytes present in individuals. The analytes can include substances that can be detected through the blood of individuals. In addition, the health data 108 can include results of diagnostic tests of individuals. The diagnostic test results can include imaging information obtained via one or more medical imaging technologies. The diagnostic test results can also include results from one or more assays that have been performed with respect to fluids and/or tissue of individuals. Further, the health data 108 can include electrical activity data, such as electroencephalogram (EEG) data and electrocardiogram (ECG) data, obtained from individuals.

The computational biology system 102 can also obtain genetic data 110 related to a number of individuals from the one or more data sources 106. The genetic data 110 can include deoxyribonucleic acid (DNA) information of individuals, ribonucleic acid (RNA) information of individuals, or combinations thereof. The genetic data 110 can include whole genome sequencing with respect to the number of individuals. In additional implementations, the genetic data 110 can indicate the presence or absence of one or more genetic alterations within the number of individuals. The genetic data 110 can also include the expression levels of one or more genes of the individuals. In various implementations, the expression level of a gene can correspond to a level of one or more proteins that are expressed in relation to the gene.

Additionally, the computational biology system 102 can obtain medical literature information 112 from the one or more data sources 106. The medical literature information 112 can include research papers, medical books, journal articles, treatises, clinical studies, databases and knowledge-based web sites, or one or more combinations thereof. The medical literature information 112 can indicate genetic features of individuals in which one or more biological conditions are present. The medical literature information 112 can also indicate behavioral characteristics of individuals in which a neurodevelopmental condition is present. In addition, the medical literature information 112 can indicate other characteristics of individuals in which one or more biological conditions are present, such as morphological features of individuals in which one or more biological conditions are present, levels of analytes of individuals in which one or more biological conditions are present, diagnostic test results of individuals in which one or more biological conditions are present, one or more combinations thereof, and the like.

Further, the medical literature information 112 can include information about treatments for biological conditions. In various examples, the medical literature information 112 can include information about therapeutics used to treat one or more biological conditions. In illustrative examples, the medical literature information 112 can indicate the effectiveness of treatments for various biological conditions. For example, the medical literature information 112 can indicate the effect that therapeutics have on the symptoms of individuals in which one or more biological conditions are present. The medical literature information 112 can also indicate genetic information related to therapeutics, such as at least one of the up-regulation of one or more genes or the down-regulation of one or more genes by one or more therapeutics. In one or more implementations, the medical literature information 112 can indicate therapeutics used to treat one or more neurological disorders and the effectiveness of the therapeutics in the treatment of the neurological disorders.

The computational biology system 102 can obtain co-occurring conditions information 114 from the one or more data sources 106. The co-occurring conditions information 114 can indicate one or more biological conditions that are present in individuals that have also been diagnosed with one or more additional biological conditions. In illustrative examples, the co-occurring conditions information 114 can indicate one or more biological conditions that are present in individuals diagnosed with a neurodevelopmental condition. For example, the co-occurring conditions information 114 can indicate that individuals diagnosed with autism spectrum disorder can also be diagnosed with one or more digestive conditions, one or more biological conditions related to inflammation, or both. In various examples, co-occurring conditions can also be referred to herein as co-morbidities.

In addition, the computational biology system 102 can obtain biological pathway data 116 from the one or more data sources 106. The biological pathway data 116 can indicate one or more biological pathways that are disrupted in individuals in which one or more biological conditions are present. The biological pathway data 116 can also indicate one or more biological pathways that are impacted by therapeutics that are used to treat one or more biological conditions. In one or more illustrative examples, the biological pathway data 116 can indicate one or more biological pathways that are disrupted by one or more neurodevelopmental conditions. In various implementations, the biological pathway data 116 can indicate the up-regulation or the down-regulation of genes that can be expressed to cause the production of one or more proteins in conjunction with one or more biological pathways. In one or more implementations, the biological pathway data 116 can be obtained by screening one or more therapeutics in relation to one or more cell lines.

The computational biology system 102 can include one or more classification systems 118. The one or more classification systems 118 can determine classifications in which to group individuals in which a neurodevelopmental condition is present. The one or more classification systems 118 can analyze data obtained from the one or more data sources 106 and determine features of individuals in which a neurodevelopmental condition is present that can be used in the classification of the individuals. In one or more implementations, the one or more classification systems 118 can implement at least one of one or more machine learning techniques or one or more statistical techniques to determine amounts of correlation between features of individuals in which a neurodevelopmental condition is present. To illustrate, the one or more classification systems 118 can determine features related to classification of individuals in which a neurodevelopmental condition is present based on the features having at least a threshold amount of correlation with respect to one another. The threshold amount of correlation can be based on a number of the individuals in which a neurodevelopmental condition is present that exhibit the features in relation to a broader group of individuals in which the neurodevelopmental condition is present. In various examples, the one or more classification systems 118 can implement one or more combinations of multiple machine learning techniques and/or one or more combinations of statistical techniques to determine features to include in each of a number of classifications of individuals in which a neurodevelopmental condition is present.

The features corresponding to each classification can be different from conventional classification systems that group individuals according to behavioral characteristics. For example, the one or more classification systems 118 can determine at least one of genetic features, morphological features, co-occurring conditions, levels of analytes, electrical activity features, or metabolic features, that can be used to classify individuals in which a neurodevelopmental condition is present. In this way, the amount of heterogeneity of individuals diagnosed with a same neurodevelopmental condition can be captured in the different features associated with each classification determined by the one or more classification systems 118. In various examples, the one or more classification systems 118 can determine different features for individual classifications for different subgroups of individuals in which one or more neurodevelopmental conditions are present. To illustrate, the one or more classification systems 118 can determine a first set of classifications for a first subgroup of individuals in which a neurodevelopmental condition is present, such as autism spectrum disorder, and a second set of classifications for a second subgroup of individuals in which a neurodevelopmental condition is present. In these scenarios, individual classifications of the first set of classifications can be associated with respective sets of features that are different from the sets of features associated with the individual classifications of the second set of classifications.

In various implementations, the one or more classification systems 118 can determine a first classification 120 up to an Nth classification 122 for a neurodevelopmental condition. Each classification corresponds to a different subgroup of individuals in which the neurodevelopmental condition is present. The first classification 120 can include first features 124 and the Nth classification can include Nth features 126, where at least one of the Nth features 126 is different from at least one of the first features 124. In one or more illustrative examples, the neurodevelopmental condition can include autism spectrum disorder. In these scenarios, the one or more classification systems 118 can analyze data obtained from the data sources 106 related to individuals in which autism spectrum disorder is present to determine the first features 124 of the first classification 120 and the Nth features 126 of the Nth classification 122. In one or more illustrative examples, the one or more classification systems 118 can determine that the first features 124 can include at least one of hearing impairment, loss of functional mutations on the chromodomain-helicase-DNA-binding protein 8 (CHD8) gene, altered chromatin remodeling resulting in altered gene expression, spacing between eyes that is greater than a threshold distance, or relatively short nose having a relatively flat nasal bridge in relation to individuals of a control population. Additionally, the one or more classification systems 102 can determine that the Nth features 126 can include at least one of severe muscular hypotonia with feeding difficulties in infancy, loss of functional mutations on the additional sex combs like 3 (ASXL3) gene, a facial length that is greater than a threshold length, downward slanted palpebral fissures, hypoglycemia, or lipogenesis greater than a threshold level due to an overactivation of thyroid hormone receptor beta.

Additionally, the computational biology system 102 can include one or more characterization systems 128. The one or more characterization systems 128 can characterize individuals by determining respective classifications in which to place the individuals. The one or more characterization systems 128 can determine features associated with a number of individuals and determine respective classifications for the plurality of individuals based on the features associated with each classification and the features of the individuals. In various implementations, the one or more characterization systems 128 can determine an amount of similarity between features associated with an individual and features related to one or more of the classifications determined by the one or more classification systems 118. The one or more characterization systems 128 can determine a pairing between a set of features of an individual and a set of features of a classification that has a highest level of similarity and characterize the individual as being included in the classification. The one or more characterization systems 128 can determine amounts of similarity between individuals and classifications based on at least one of commonalities between genetic features, morphological features, levels of analytes, co-occurring conditions, electrical activity features, features included in images, or clinical observations.

The one or more characterization systems 128 can determine one or more profiles for individuals. In one or more implementations, the one or more profiles can be used to determine amounts of similarity between features of the individuals and features associated with one or more classifications. The one or more profiles can include genetic profiles, profiles of morphological features of individuals, profiles of levels of analytes present in individuals, profiles of electrical activity data of individuals, profiles of imaging data of individuals, or one or more combinations thereof. The one or more profiles determined by the one or more characterization systems 128 can be generated using information obtained from the one or more data sources 106.

In situations where the one or more characterization systems 128 determine genetic profiles for individuals, the genetic profiles can indicate respective genes of individuals that are up-regulated and respective genes of individuals that are down-regulated. The up-regulation of a gene can correspond to increased production of one or more proteins generated by the expression of the gene in relation to a specified range of production for the protein, such as a baseline range of production for the protein. For example, the one or more characterization systems 128 can determine a distribution for the amount of one or more RNA transcripts or proteins produced by a number of individuals in relation to the expression of a gene or to the abundance of a protein, respectively. The one or more characterization systems 128 can determine a range of amounts of RNA transcript or protein production that are characterized as a normal amount and ranges of amounts of RNA transcript or protein production that are characterized as being above normal and below normal. In various examples, the one or more characterization systems 128 can determine one or more degrees of above normal RNA transcript or protein production and/or one or more degrees of below normal RNA transcript or protein production based on the one or more statistical analyses of the distribution of protein production by individuals. In this way, the genetic profiles of an individual can indicate the genes that are up-regulated and/or down-regulated with respect to the individual and a degree to which the genes are up-regulated and/or down-regulated. The degree to which one or more genes are up-regulated and/or down-regulated can be analyzed with respect to features included in the classifications to determine a classification for an individual.

In one or more illustrative examples, the one or more characterization systems 128 can determine a normal distribution for the production of a protein or one or more RNA transcripts in relation to the expression of a gene that includes a mean and a number of standard deviations above the mean and a number of standard deviations below the mean. The standard deviations above the mean can indicate production of the protein or the one or more RNA transcripts greater than the mean and standard deviations below the mean can indicate production of the protein or the one or more RNA transcripts less than the mean. In various examples, up-regulation of the gene can correspond to production of the protein or RNA transcript that is at least a threshold number of standard deviations above the mean and down-regulation of the gene can correspond to production of the protein or RNA transcript that is no greater than a threshold number of standard deviations below the mean. In one or more implementations, the threshold number of standard deviations above the mean for RNA transcript or protein production to indicate up-regulation of the gene can be at least 1.5 standard deviations, at least 2 standard deviations, at least 2.5 standard deviations, at least 3 standard deviations, at least 3.5 standard deviations, or at least 4 standard deviations. Additionally, the threshold number of standard deviations below the mean for RNA transcript or protein production to indicate down-regulation of the gene can be at least 1.5 standard deviations, at least 2 standard deviations, at least 2.5 standard deviations, at least 3 standard deviations, at least 3.5 standard deviations, or at least 4 standard deviations. In one or more illustrative examples, the threshold number of standard deviations above the mean to indicate up-regulation of a gene and the threshold number of standard deviations below the mean to indicate down-regulation of a gene can include at least one standard deviation indicating relatively low confidence genes, at least 1.5 standard deviations for relatively medium confidence genes, or at least 2 standard deviations for relatively high confidence genes.

In additional implementations, an amount of up-regulation and/or an amount of down-regulation for a gene can be determined by performing a differential level change analysis to determine changes in expression levels of a gene between a group of individuals in which a biological condition is present and individuals included in a control group. The control group can include individuals in which the biological condition is not present. In various examples, pooled variance can be implemented in conjunction with the differential level change analysis to determine the variance in the means of the expression levels of genes with respect to the group of individuals in which the biological condition is present and the group of individuals in which the biological condition is not present. In one or more implementations, the expression levels of a gene can be determined based on at least one of levels of one or more proteins related to the gene or levels of one or more RNA transcripts related to the gene. Further, in one or more examples, the differential gene expression analysis can fit a linear model that indicates an amount of correlation of observed RNA or protein expression levels from one or more additional individuals in relation to the RNA or protein expression levels of the group of individuals in which the biological condition is present and in relation to the RNA or protein expression levels of the group of individuals in which the biological condition is not present. In addition, deficient or excessive (dimensionless) levels of a gene can be determined by analyzing DNA sequencing data from the group of individuals in which the biological condition is present and identifying mutations predicted to cause deficiency or excess of the corresponding protein, such as protein truncating variants (PTVs).

In one or more illustrative examples, the one or more characterization systems 128 can determine that a first group of individuals 130 is characterized by the first features 124 included in the first classification 120. For example, the one or more characterization systems 128 can determine that a level of similarity between features of the first group of individuals 130 and the first features 124 of the first classification 120 is greater than a level of similarity between features of the first group of individuals 130 with respect to features of other classifications, such as the Nth features 126 of the Nth classification 122. Additionally, the one or more characterization systems 128 can determine that an Nth second group of individuals 132 is characterized by the Nth features 126 of the Nth classification 122. To illustrate, the one or more characterization systems 128 can determine that a level of similarity between features of the Nth group of individuals 132 and the Nth features 126 of the Nth classification 122 is greater than a level of similarity between features of the Nth group of individuals 132 with respect to features of other classifications, such as the first features 124 of the first classification 120.

Further, the computational biology system 102 can include one or more therapeutic repositioning systems 134. The one or more therapeutic repositioning systems 134 can determine one or more recommendations for therapeutics that can be candidate treatments for neurodevelopmental conditions of individuals. The one or more therapeutic repositioning systems 134 can determine genetic profiles for a number of therapeutics based on analyzing data obtained from the one or more data sources 106 associated with the number of therapeutics. The genetic profiles for the therapeutics can indicate the up-regulation and/or down-regulation of one or more genes by the respective therapeutics. The one or more therapeutic repositioning systems 134 can determine an amount of up-regulation of genes and/or an amount of down-regulation of genes based on levels of RNA transcripts or proteins related to the genes that are expressed in cells of individuals that interact with or regulated by the therapeutic.

The therapeutics evaluated by the one or more therapeutic repositioning systems 134 can include substances that are provided to individuals in the treatment of the individuals with respect to a biological condition. The therapeutics can include small molecules that have molecular weights that are less than a threshold molecular weight, such as about 900 daltons (Da). In various examples, the small molecules can include pharmaceutical products that are provided for the treatment of one or more biological conditions. The therapeutics can also include large molecules that have molecular weights greater than the molecular weights of small molecules. In an illustrative example, large molecules can have molecular weights that are greater than about 3000 Da, such as from about 3000 Da to about 150,000 Da. The large molecules can include proteins, such as antibodies and/or enzymes. The therapeutics evaluated by the one or more therapeutic repositioning systems 134 can be used to treat one or more biological conditions that are different from one or more neurodevelopmental conditions for which the one or more classification systems 118 have determined classifications for individuals. Thus, the use of therapeutics evaluated by the one or more therapeutic repositioning systems 134 to treat individuals diagnosed with at least one neurodevelopmental condition classified by the one or more classification systems 118 can be considered a new or different use of the therapeutics. Cells can interact with a respective therapeutic by being placed in an environment in which conditions are present for one or more molecules of the respective therapeutic to be physically proximate to the cells and/or for one or more molecules of the respective therapeutic to chemically react with the cells.

The one or more therapeutic repositioning systems 134 can analyze information obtained from the one or more data sources 106 with respect to one or more therapeutics in relation to the features associated with the individuals characterized according to one or more classifications, such as the first classification 120 and/or the Nth classification 122. The one or more therapeutic repositioning systems 134 can determine a probability that a therapeutic can be a candidate treatment for one or more individuals in which a neurodevelopmental condition is present. In various implementations, the probability that a therapeutic can be a candidate therapeutic to treat an individual can be based on analyzing a genetic profile of the individual with respect to a genetic profile of the therapeutic. For example, the one or more therapeutic repositioning systems 134 can determine correlations between a genetic profile of an individual in which a neurodevelopmental condition is present and a genetic profile of a therapeutic. In various implementations, the one or more therapeutic repositioning systems 134 can analyze a genetic profile of an individual in which a neurodevelopmental condition is present with respect to an inverse of a genetic profile of a therapeutic. That is, the one or more therapeutic repositioning systems 134 can determine a measure of similarity between genes that are up-regulated in an individual diagnosed with a neurodevelopmental condition and genes that are down-regulated by a therapeutic. In addition, the one or more therapeutic repositioning systems 134 can determine a measure of similarity between genes that are down-regulated in the individuals in which a neurodevelopmental condition is present and genes that are up-regulated by the therapeutic. In this way, the one or more therapeutic repositioning systems 134 can determine one or more therapeutics that offset the up-regulation of one or more genes of an individual diagnosed with a neurodevelopmental condition due to the down-regulation of the one or more genes by the therapeutic. The one or more therapeutics determined by the one or more therapeutic repositioning systems 134 to treat individuals in which a neurodevelopmental condition is present can also offset the down regulation of one or more genes of the individuals due to the up-regulation of the one or more genes by the therapeutic.

In the illustrative example of Figure 1, the one or more therapeutic repositioning systems 134 can determine that at least a first therapeutic 136 can be a recommendation to treat a neurodevelopmental condition with respect to the first group of individuals 130. Additionally, the one or more therapeutic repositioning systems 134 can determine that at least an Nth therapeutic 138 can be a recommendation to treat a neurodevelopmental condition with respect to the Nth group of individuals 132. In various examples, the first therapeutic 136 and the Nth therapeutic 138 can be recommended as treatments for the same neurodevelopmental condition. However, due to different features being associated with the first group of individuals 130 and the Nth group of individuals 132, the first therapeutic 136 can have a greater probability of reducing symptoms of the neurodevelopmental condition with respect to the first group of individuals 130 than the Nth therapeutic 138. Additionally, the Nth therapeutic 138 can have a greater probability of reducing symptoms of the neurodevelopmental condition with respect to the second group of individuals 132 than the first therapeutic 136 based on differences between the features of the Nth group of individuals 132 and the first group of individuals 130.

Although the illustrative example of Figure 1 indicates the first therapeutic 136 being recommended to treat the first group of individuals 130 diagnosed with a neurodevelopmental condition and the Nth therapeutic 138 being recommended to treat the Nth group of individuals 132 for the neurodevelopmental condition, one or more additional therapeutics can be recommended to treat at least one of the first group of individuals 130 or the Nth group of individuals 132 for the neurodevelopmental condition. For example, the one or more therapeutic repositioning systems 134 can determine one or more additional therapeutics as recommendations to treat a neurodevelopmental condition with respect to the first group of individuals 130 and one or more further therapeutics as recommendations to treat the neurodevelopmental condition with respect to the Nth group of individuals 132. In this way, the one or more therapeutic repositioning systems 134 can be used to determine different combinations of therapeutics as candidate treatments for a neurodevelopmental condition with respect to different groups of individuals.

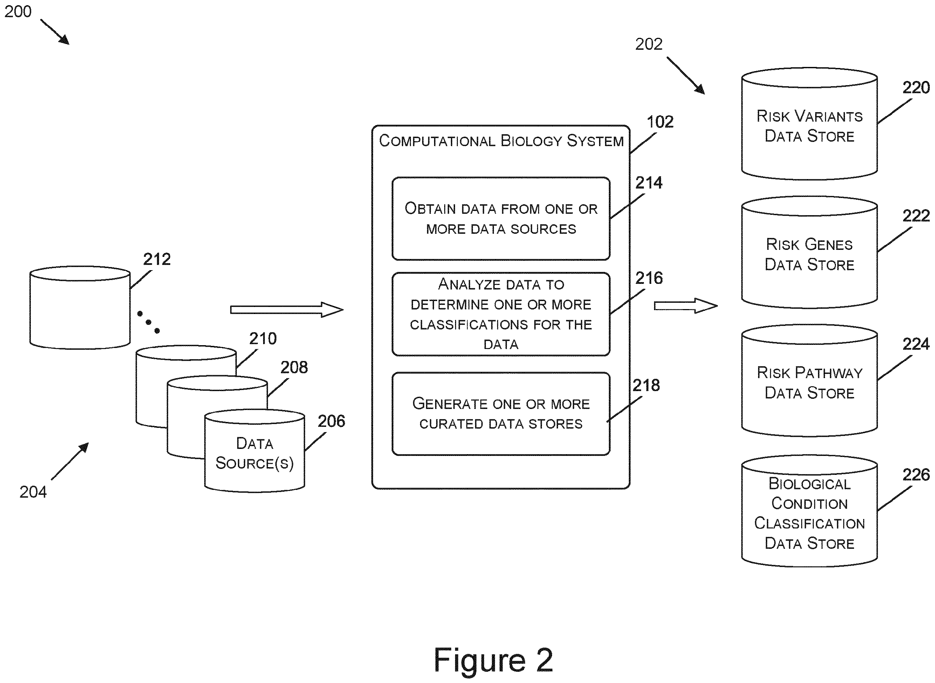

Figure 2 is a diagram illustrating an example framework 200 to generate a number of curated databases 202 using information obtained from a number of data sources 204. At least a portion of the number of data sources 204 can include publicly accessible information. Information can be publicly accessible when in can be obtained without a specified form of authentication and/or credentials. In various examples, publicly accessible information can be obtained via one or more websites and/or via a physical repository of information that is accessible to the public. Publicly accessible information can be included in publications, such as research articles, medical literature, clinical trials information, one or more combinations thereof, and the like. In addition, at least a portion of the number of data sources 204 can store privately accessible information. In one or more implementations, the privately accessible information can be obtained using specified authentication information and/or credentials. Privately accessible information can be obtained electronically or from a physical location that has restricted access.

The number of data sources 204 can include a first data source 206, a second data source 208, a third data source 210, and up to at least a fourth data source 212. The number of data sources 204 can store genetic information. The genetic information can include deoxyribonucleic acid (DNA) sequence information, ribonucleic acid (RNA) information, or combinations thereof. In one or more illustrative examples, the genetic information stored by at least a portion of the number of data sources 204 can be related to single nucleotide variants (SNVs), nucleic acid insertions with respect to one or more genes or genomic regions, nucleic acid duplications with respect to one or more genes or genomic regions, nucleic acid deletions with respect to one or more genes or genomic regions, common genetic variants, rare genetic variants, very rare genetic variants, ultrarare genetic variants, or one or more combinations thereof. Common genetic variants can have an allele frequency greater than 0.05. Rare genetic variants can have an allele frequency of no greater than 0.01. Very rare genetic variants can have an allele frequency of no greater than 0.0001. Ultrarare genetic variants can be absent from the control population. In one or more implementations, the genetic information stored by at least a portion of the number of data sources 204 can correspond to genetic variants having an allele frequency from 0.0001 to 0.05. As used herein, the allele frequency can correspond to the proportion of a specific allele with respect to all possible alleles at a specific locus in a human population in which one or more biological conditions are not present. In various examples, the genetic information can indicate a number of genes that correspond to one or more biological conditions, such as one or more neurodevelopmental conditions. In one or more examples, the genetic information can indicate a probability that one or more genes correspond to one or more neurodevelopmental conditions. The genetic information included in the one or more data sources 204 can also include locations of genes that correspond to a neurodevelopmental condition.

The number of data sources 204 can also store information that indicates biological pathways and genetic information related to the biological pathways. For example, the number of data sources 204 can include information corresponding to genes that are involved in one or more biological pathways. Additionally, the number of data sources 204 can store information that indicates molecules produced in conjunction with the expression of one or more genes. To illustrate, the number of data sources 204 can store information indicating proteins that are produced with respect to the expression of one or more genes. Further, the number of data sources 204 can include information derived from the analysis of genetic information. In one or more implementations, the number of data sources 204 can include information derived from one or more statistical analyses of genetic information. For example, the number of data sources 204 can store false discovery rate (FDR) information for one or more genes with respect to its/their association with at least one neurodevelopmental condition. In various examples, the number of data sources 204 can store information derived from transmission and de novo association (TADA) analyses related to genetic information that corresponds to one or more neurodevelopmental conditions.

In one or more illustrative examples, at least a portion of the one or more data sources 204 can include information corresponding to autism spectrum disorder. In one or more implementations, the number of data sources 204 can comprise information included in the Autism Sequencing Consortium (ASC) cohort. Additionally, the number of data sources 204 can comprise information included in the Simons Simplex Collection (SSC) cohort. Further, the number of data sources 204 can comprise information included in the Autism Genome Project (AGP) cohort. In various implementation, the number of data sources 204 can comprise information included in the Hartwell Foundation Initiative (iHART) cohort.

In one or more implementations, the number of data sources 204 can include one or more classification systems and/or one or more categorization systems for grouping individuals in relation to one or more biological conditions. For example, the number of data sources 204 can include one or more classification systems for individuals based on observable traits and/or observable characteristics, such as one or more phenotypes. The one or more classification systems and/or the one or more categorization systems can include genetic information related to one or more biological conditions. In various examples, the one or more classification systems and/or the one or more categorization systems can include genetic information related to at least one of one or more traits or one or more characteristics that correspond to one or more biological conditions. In an illustrative example, the number of data sources 204 can include at least a portion of the information included in the Human Phenotype Ontology (HPO) database.

The framework 200 can include the computational biology system 102. The computational biology system 102 can perform operations to obtain data from the number of data sources 204 and to generate the number of curated databases 202. For example, at operation 214, the computational biology system 102 can obtain data from the one or more data sources 204. To illustrate, the computational biology system 102 can utilize one or more web crawlers to obtain data from one or more websites included in the number of data sources 204. Additionally, the computational biology system 102 can extract text from one or more documents. The one or more documents can include electronic documents. The one or more documents can also include physical documents. In various examples, the computational biology system 102 can perform at least one of optical character recognition (OCR) operations or natural language process operations to extract text information from documents included in the number of data sources 204. In one or more implementations, information can be extracted from the one or more data sources 204 in relation to one or more search criteria provided by the computational biology system 102, such as at least one of one or more keywords, one or more categories, or one or more classifications. Information can be sent from at least one of the data sources 206, 208, 210, 212 to the computational biology system 102 in response to one or more requests for information provided by the computational biology system 102. Information can also be obtained from the one or more data sources 204 through manual entry of the information via at least one of one or more user interfaces or one or more input devices of one or more computing devices that are coupled to and/or in electronic communication with the computational biology system 102.

At operation 216, the computational biology system 102 can analyze data to determine one or more classifications for the data obtained from the one or more data sources 204. The computational biology system 102 can perform one or more statistical analyses of information included in at least a portion of the number of data sources 204. Additionally, the computational biology system 102 can identify portions of the information obtained from the one or more data sources 204 according to one or more criteria. To illustrate, the computational biology system 102 can obtain information from at least a portion of the number of data sources 204 that corresponds to one or more classifications of genes. In one or more illustrative examples, the computational biology system 102 can identify variants of genes based on one or more threshold allele frequencies. In various examples, the computational biology system 102 can determine common variants from information included in at least a portion of the number of data sources 204 according to a threshold allele frequency, such as an allele frequency greater than about 0.001, an allele frequency greater than about 0.005, an allele frequency greater than about 0.01, an allele frequency greater than about 0.05, or an allele frequency greater than about 0.1. The computational biology system 102 can also determine rare variants from information included in at least a portion of the number of data sources 204 according to an additional threshold allele frequency, such as no greater than about 0.005, no greater than about 0.001, no greater than about 0.01, no greater than about 0.0005, or no greater than about 0.0001. Further, the computational biology system 102 can identify information included in at least a portion of the number of data sources 204 that corresponds to variants with different predicted consequences, including protein truncating variants (PTVs). The computational biology system 102 can determine genetic variants that have an allele frequency of 0. An allele frequency of 0 can indicate that the genetic variants are absent from a control population. In one or more implementations, the computational biology system 102 can determine mutations of genes that have not been recorded as being present in the parents of individuals in which the biological condition is present. In these situations, the gene mutations can be referred to as "de novo" variants.

The computational biology system 102 can also identify genes that have at least a threshold probability of being associated with a biological condition, such as a neurodevelopmental condition. In one or more implementations, the computational biology system 102 can determine one or more genes that have at least a threshold probability of being associated with autism spectrum disorder. In various examples, the computational biology system 102 can analyze information included in at least a portion of the number of data sources 204 in conjunction with one or more threshold false discovery rate (FDR) values. To illustrate, the computational biology system 102 can determine one or more genes included in at least a portion of the number of data sources 204 that have FDR values in relation to the neurodevelopmental condition that are no greater than about 0.30, no greater than about 0.25, no greater than about 0.20, no greater than about 0.15, no greater than about 0.10, or no greater than about 0.05.

The computational biology system 102 can also determine genes that have at least a threshold probability of being associated with one or more neurodevelopmental conditions by using one or more software tools. In these situations, the one or more software tools can analyze information associated with at least one gene and one or more neurodevelopmental disorders to determine a level of confidence that the at least one gene is predictive of an individual in which one or more neurodevelopmental conditions are present. In various examples, the computational biology system 102 can filter genes with respect to the one or more neurodevelopmental disorders according to the confidence levels determined by the one or more software tools for the respective genes.

In one or more implementations, the computational biology system 102 can, at operation 218, generate one or more curated data stores. For example, the computational biology system 102 can generate the number of curated databases 202. The plurality of curated databases 202 can include a risk variants data store 220, a risk genes data store 222, a risk pathway data store 224, and a biological condition classification data store 226. In various implementations, the computational biology system 102 can use the one or more data analyses performed with respect to operation 216 to generate at least a portion of the number of curated databases 202. In addition to generating the number of curated databases 202 based on information obtained from at least a portion of the number of data sources 204, the computational biology system 102 can analyze information from at least a portion of the number of data sources 204 to determine various types of information obtained from at least a portion of the number of data sources 204. The computational biology system 102 can also store the data corresponding to the various types of information in relation to one or more identifiers associated with individual types of information. For example, the computational biology system 102 can identify information corresponding to genetic variants related to a neurodevelopmental condition, such as autism spectrum disorder, and store the information according to one or more identifiers associated with genetic variants and/or one or more identifiers associated with the neurodevelopmental condition. In additional examples, the computational biology system 102 can identify additional information corresponding to the neurodevelopmental condition, such as at least one of information related to biological pathways impacted by the neurodevelopmental condition, information related to genes associated with the neurodevelopmental condition, information related to clinical features and/or morphological features associated with the neurodevelopmental condition, or information related to co-occurring conditions associated with the neurodevelopmental condition. The computational biology system 102 can then classify the additional information and store the information in conjunction with one or more identifiers corresponding to the individual types of the additional information.

In one or more illustrative examples, the risk variants data store 220 can include information that corresponds to a number of variants of genes that correspond to one or more neurodevelopmental conditions. For example, the risk variants data store 220 can include information that corresponds to a number of variants of genes that correspond to autism spectrum disorder. In various examples, the risk variants data store 220 can include information related to copy number variants (CNVs) related to one or more neurodevelopmental conditions. Additionally, the risk variants data store 220 can include information corresponding to single nucleotide variants (SNVs) and small insertions and deletions (INDELs) related to one or more neurodevelopmental conditions. Further, the variants data store 220 can include information corresponding to common variants and/or rare variants. The risk variants data store 220 can store information indicating values for strength of association between variants and a neurodevelopmental condition. In various examples, one or more values for strength of association between one or more variants and a neurodevelopmental condition can be extracted from information obtained from at least one data source of the number of data sources 204. In additional implementations, the computational biology system 102 can analyze a plurality of different values indicating an association between one or more variants and a neurodevelopmental condition to determine a measure of significance between the one or more variants and the neurodevelopmental condition. To illustrate, the computational biology system 102 can determine a *p*-value to indicate a measure of significance of one or more variants in relation to the neurodevelopmental disorder.

In one or more implementations, the computational biology system 102 can conduct a Fisher's exact test to determine a *p*-value indicating a strength of association between a genetic region and a neurodevelopmental condition. The computational biology system 102 can also use one or more multiple-testing correction techniques to determine the strengths of association between a genetic region and a neurodevelopmental condition, by modifying one or more *p*-values generated using Fisher's exact test to produce one or more adjusted *p-*values. In one or more illustrative examples, the computational biology system 102 can perform a Bonferroni one-step correction to determine *p*-values (associations) that pass this multiple-test correction. Additionally, the computational biology system 102 can perform a Šidák one-step correction to generate one or more adjusted *p*-vales from one or more *p*-values produced using the Fisher's exact test. Further, the computational biology system 102 can produce one or more adjusted *p*-values using a Holm- Šidák step-down method with Šidák adjustments based on one or more *p*-values generated using the Fisher's exact test. The computational biology system 102 can also perform a Holm step-down method using Bonferroni adjustments with respect to one or more *p*-values to generate one or more adjusted *p*-values. In various implementations, the computational biology system 102 can perform a Simes-Hochberg step-up method to produce one or more adjusted *p*-values based on one or more adjusted *p*-values generated by the computational biology system 102 using Fisher's exact test. In one or more additional implementations, the computational biology system 102 can generate one or more adjusted *p*-values using a Hommel closed method based on Simes tests with respect to one or more *p*-values generated by the computational biology system 102 using Fisher's exact test. In one or more further implementations, the computational biology system 102 can use a one stage Benjamini-Hochberg procedure for false discovery rate correction or a two stage Benjamini-Hochberg false discovery rate correction to produce one or more adjusted *p*-values with respect to one or more *p*-values determined by the computational biology system 102 using Fisher's exact test. In one or more illustrative examples, the computational biology system 102 can implement a one stage Benjamini-Yekutieli procedure for false discovery rate correction or a two stage Benjamini-Yekutieli false discovery rate correction to generate one or more adjusted *p*-values from one or more *p*-values produced by the computational biology system 102 using Fisher's exact test.

The number of curated databases 202 can also include a risk genes data store 222. The risk genes data store 222 can include a plurality of genes that have at least a threshold amount of correspondence with at least one neurodevelopmental condition. In various implementations, the risk genes data store 222 can include a plurality of genes that have at least a threshold amount of correspondence with autism spectrum disorder. In one or more illustrative examples, the risk genes data store 222 can include a plurality of genes that have a false discovery rate of no greater than about 0.30 with respect to a neurodevelopmental condition, no greater than about 0.25 with respect to a neurodevelopmental condition, no greater than about 0.20 with respect to a neurodevelopmental condition, no greater than about 0.15 with respect to a neurodevelopmental condition, no greater than about 0.10 with respect to a neurodevelopmental condition, or no greater than about 0.05 with respect to a neurodevelopmental condition. In one or more implementations, the genes included in the risk genes data store 222 can be determined based on analyzing information included in at least one data source of the plurality of data sources 204 to identify genes having at least a threshold amount of correspondence with respect to at least one neurodevelopmental condition.

Additionally, a number of versions of the risk genes data store 222 can be generated by the computational biology system 102. For example, the computational biology system 102 can generate different versions of the risk genes data store 222 based on sources of information used to generate the respective versions of the risk genes data store 222. To illustrate, a first version of the risk genes data store 222 can be generated using information obtained from the first data source 206 and a second version of the risk genes data store 222 can be generated using information from the second data source 208. In these situations, the information included in the first data source 206 and the second data source 208 can include information obtained from at least one large scale sequencing study and/or information obtained from a software tool that determines measures of correspondence between genetic regions and one or more biological conditions. In one or more additional illustrative examples, a first version of the risk genes data store 222 can be generated using information obtained from at least one of the number of data sources 204 in relation to a first threshold amount of correspondence between a genetic region and one or more neurodevelopmental conditions, such as a first false discovery rate value, and a second version of the risk genes data store 222 can be generated using information obtained from at least one of the number of data sources 204 in relation to a second threshold amount of correspondence between a genetic region and one or more neurodevelopmental conditions, such as a second false discovery rate value.

Further, the number of curated databases 202 can include a risk pathway data store 224. The risk pathway data store 224 can include information indicating biological pathways that have at least a threshold amount of association with one or more neurodevelopmental conditions. The biological pathways can include one or more interactions between molecules that cause at least one analyte to be produced or consumed and/or that cause a change in a cell. The biological pathways having at least a threshold amount of association with one or more neurodevelopmental conditions can be determined by the computational biology system 102 by determining an amount of overlap between one or more genes associated with one or more biological pathways and one or more genes associated with at least one neurodevelopmental condition. In one or more implementations, the genes associated with the biological pathways and the genes associated with the neurodevelopmental condition can be determined based on information obtained from different data sources of the number of data sources 204. For example, the information used by the computational biology system 102 to determine the biological pathway related genes can be obtained from at least the third data source 210 and the information used by the computational biology system 102 to determine genes related to a neurodevelopmental condition can be obtained from at least the fourth data source 212.

In various examples, the biological pathways included in the risk pathway data store 224 can be associated with more genes that overlap with genes corresponding to a neurodevelopmental condition than the overlap that would be obtained by any other randomly selected group of genes (expected by chance, with a nominal or adjusted *p-value* greater than 0.05). In one or more implementations the overlap expected by chance can be determined by randomly selecting the same number of genes from the human genome as the number of genes involved in a given biological pathway and checking how many of the selected genes would overlap with the neurodevelopmental condition in consideration, and repeating this process multiple times to ensure that estimates satisfy one or more statistical criteria. In addition, one or more different threshold amounts of significance can be used with respect to different data sources included in the number of data sources 204 to identify genes to include in the risk pathway data store 224. To illustrate, the computational biology system 102 can identify genes having at least a threshold amount of correspondence with a neurodevelopmental condition by analyzing information obtained from the first data source 206 with respect to a first statistical measure, such as the false discovery rate. The computational biology system 102 can also identify genes having at least a threshold amount of correspondence with the neurodevelopmental condition by analyzing information obtained from the second data source 208 with respect to a second statistical measure, such as one or more g:SCS (Set Counts and Sizes) significance cutoffs. In various additional examples, the second statistical measure can correspond to p-values that satisfy Bonferroni or FDR correction tests at one or more cutoff values.

The biological pathways included in the risk pathway data store 224 for a respective neurodevelopmental condition can also be determined by eliminating redundant pathways with a large proportion of genes that overlap between multiple pathways. For example, the computational biology system 102 can determine at least one pair of biological pathways that have at least a threshold amount of overlap between the genes associated with the individual pathways included in the pair of biological pathways. To illustrate, a first biological pathway can be associated with a first number of genes that has at least a threshold amount of overlap with a second number of genes of a second biological pathway. In these scenarios, the computational biology system 102 can determine that the first biological pathway or the second biological pathway has a greater number of genes and the computational biology system 102 can add the genes associated with the biological pathway having the lesser number of genes to the risk pathway data store 224. A threshold amount of overlap between genes of at least two pathways can correspond to an overlap coefficient of at least 0.60, at least 0.70, at least 0.80, or at least 0.90.

The number of curated databases 202 can include a biological condition classification data store 226. The biological condition classification data store 226 can indicate classifications of a biological condition, such as a neurodevelopmental condition, in a system of biological condition classifications that are associated with at least a threshold number of genes and that are also associated with the biological condition. For example, the biological condition classification data store 226 can include phenotypes that are associated with at least a threshold number of genes that are also associated with the neurodevelopmental condition. In one or more illustrative examples, the biological condition classification data store 226 can be generated using information from the Human Phenotype Ontology (HPO) database. In these situations, the computational biology system 102 can analyze genes associated with individual phenotypes included in the HPO with respect to genes associated with a neurodevelopmental condition, such as autism spectrum disorder. Based on this analysis, the computational biology system 102 can determine phenotypes of the HPO that are associated with at least a threshold number of genes that are also associated with the neurodevelopmental condition and include the phenotypes and their respective genes in the biological condition classification data store 226.

Additionally, the biological condition classification data store 226 can be generated by the computational biology system 102 by eliminating phenotypes obtained from the HPO that have at least a threshold number of redundant genes. To illustrate, the computational biology system 102 can determine at least one pair of phenotypes that have at least a threshold amount of overlap between the genes associated with the individual phenotypes included in the pair of phenotypes. In various examples, a first phenotype can be associated with a first number of genes that has at least a threshold amount of overlap with a second number of genes of a second phenotype. In these scenarios, the computational biology system 102 can determine that the first phenotype or the second phenotype has a greater number of genes and the computational biology system 102 can add the genes associated with the phenotype having the lesser number of genes to the biological condition classification data store 226.

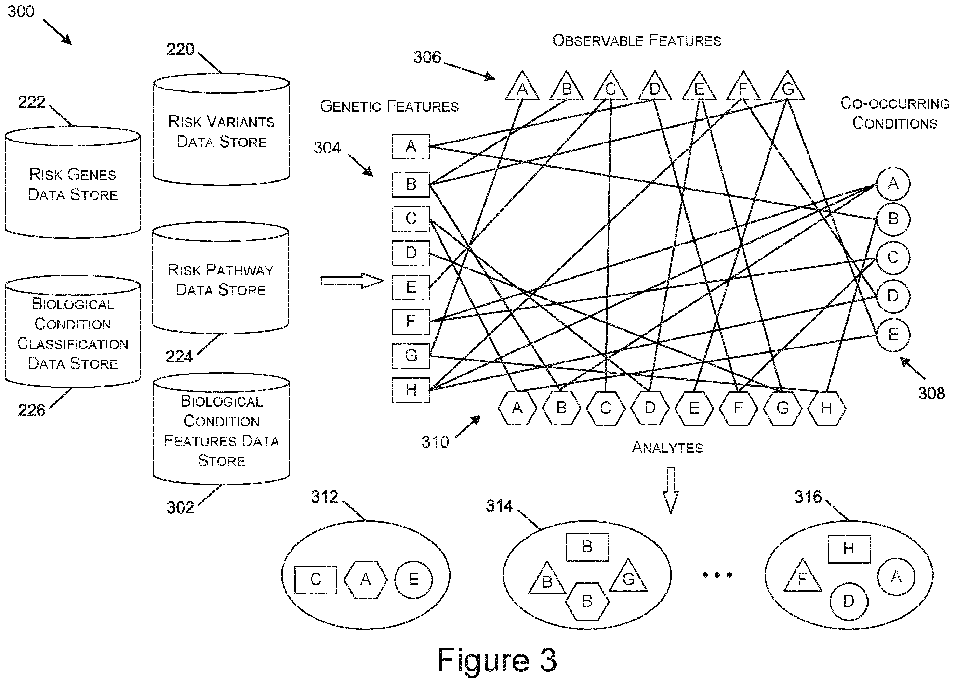

Figure 3 is a diagram illustrating an example framework 300 to determine characteristics of different subgroups of individuals in which a neurodevelopmental condition is present, in accordance with one or more example implementations. Information stored by at least one of the risk variants data store 220, the risk genes data store 222, the risk pathway data store 224, or the biological condition classification data store 226 can be used to determine characteristics of different subgroups of individuals in which a neurodevelopmental condition is present. Additionally, information stored by a biological condition features data store 302 can be used to determine characteristics of subgroups of individuals in which a neurodevelopmental condition is present. The biological condition features data store 302 can store information of individuals that have been diagnosed with the neurodevelopmental condition. In various examples, the biological condition features data store 302 can include information corresponding to clinical features of individuals in which a neurodevelopmental condition is present, co-occurring conditions present in individuals in which a neurodevelopmental condition is present, and/or levels of analytes present in individuals in which a neurodevelopmental condition is present.

The biological condition features data store 302 can be generated by the computational biology system 102 of Figure 1 and Figure 2 based on analyzing information obtained by the computational biology system 102 from one or more data sources. The biological condition features data store 302 can include information obtained from medical records of individuals in which a neurodevelopmental condition is present. The biological condition features data store 302 can also include information obtained from at least one of medical literature, journal articles, books, databases, or clinical trials data indicating features of individuals in which a neurodevelopmental condition is present.

In the illustrative example of Figure 3, the framework 300 can include genetic features 304 having at least a threshold amount of significance with respect to a neurodevelopmental condition. The genetic features 304 can be obtained from at least one of the risk variants data store 220, the risk genes data store 222, the risk pathway data store 224, the biological condition classification data store 226, or the biological condition features data store 302. The genetic features 304 can be detected by analyzing genetic data 110 from individuals in which a neurodevelopmental condition is present. The genetic features 304 can include genes that have at least a threshold level of correspondence with the neurodevelopmental condition. In one or more illustrative examples, the genetic features 304 can be represented as an identifier of a gene. In one or more additional illustrative examples, the genetic features 304 can be represented as nucleic acid sequences and locations of the nucleic acid sequences in chromosomes of individuals.

The framework 300 can also include observable features 306 having at least a threshold amount of significance in relation to the neurodevelopmental condition. In various examples, the observable features 306 can be obtained from the biological condition features data store 302. The observable features 306 can include clinical features that are visually detectable by a clinician, such as a healthcare practitioner. In one or more examples, the observable features 306 can include morphological features. The observable features 306 can also be detected using one or more diagnostic tests. To illustrate, the observable features 306 can be detected using one or more imaging techniques. The one or more imaging techniques can include magnetic resonance imaging (MRI), computed tomography (CT) imaging, X-ray imaging, ultrasound imaging, or one or more combinations thereof. The observable features 306 can also be detected using techniques that measure electrical activity. For example, one or more observable features can be detected by an electroencephalogram (EEG) or an electrocardiogram (ECG). In various examples, the observable features 306 can also include at least one of age, gender, or ethnicity of individuals.

Additionally, the framework 300 can include co-occurring conditions 308 that have at least a threshold amount of significance with respect to the neurodevelopmental condition. In one or more implementations, the co-occurring conditions 308 can be obtained from the biological condition features data store 302. The co-occurring conditions 308 can include biological conditions that are present in conjunction with one or more neurodevelopmental conditions. In one or more implementations, the co-occurring conditions 308 can include one or more phenotypes that are present in individuals when at least one neurodevelopmental condition is also present in the individuals. The one or more phenotypes included in the co-occurring conditions 308 can include non-behavioral phenotypes. In one or more illustrative examples, in situations where an individual is diagnosed with autism spectrum disorder, at least one of the co-occurring conditions 308 can include seizures or epilepsy. The identification of non-behavioral phenotypes included in the co-occurring conditions 308 can be performed by the computational biology system 102 filtering at least a portion of the co-occurring conditions 308 according to one or more terms included in a phenotype classification system, such as the unified medical language system (UMLS).

Further, the framework 300 can include analytes 310 having at least a threshold amount of significance in relation to the neurodevelopmental condition. In one or more implementations, the analytes 310 can also be obtained from the biological condition features data store 302. The analytes 310 can include substances found in individuals that can be detected using one or more laboratory tests. In various examples, the analytes 310 can include a number of molecules. The number of molecules included in the analytes 310 can include proteins. For example, the number of molecules included in the analytes 310 can include antibodies and/or enzymes. Additionally, the number of molecules included in the analytes 310 can include vitamins and/or minerals. Further, the number of molecules included in the analytes 310 can include one or more molecules produced with respect to at least one biological pathway. To illustrate, the analytes 310 can include nutritional molecules and/or one or more molecules produced via at least one metabolic pathway. In one or more illustrative examples, the analytes 310 can include one or more carbohydrates and/or one or more lipids.

In various implementations, an amount of significance between features stored in the biological condition features data store 302 and the neurodevelopmental condition can be determined using information obtained from health information of individuals that have not been diagnosed with the neurodevelopmental condition. For example, the computational biology system 102 can determine features with occurrences that are overrepresented in individuals in which a neurodevelopmental condition is present in relation to occurrences of the features in individuals in which the neurodevelopmental condition is not present. In one or more illustrative examples, the computational biology system 102 can determine a first number of occurrences of a clinical feature in a first number of individuals in which a neurodevelopmental condition is present and a second number of occurrences of the clinical feature in a second number of individuals in which the neurodevelopmental condition is not present. Continuing with this example, the computational biology system 102 can perform an analysis of the first number of occurrences of the clinical feature in the first number of individuals with respect to the second number of occurrences of the clinical feature in the second number of individuals. The clinical feature being analyzed can be included in the clinical features 306 in situations where the analysis indicates that the first number of occurrences of the clinical feature in the first number of individuals has at least a threshold amount of statistical difference with respect to the second number of occurrences of the clinical feature in the second number of individuals. The co-occurring conditions 308 can also be determined based on analyzing the number of individuals in which a neurodevelopmental condition is present that are also diagnosed with one or more additional biological conditions in relation to the number of individuals in which the neurodevelopmental condition is not present and in which the one or more additional biological conditions are present. Further, the analytes 310 can be determined based on analyzing the number of individuals in which a neurodevelopmental condition is present that have threshold amounts of one or more analytes with respect to the number of individuals in which the neurodevelopmental condition is not present.

The framework 300 can include determining a number of groups of features that can be used to determine subgroups of individuals in which a neurodevelopmental condition is present. In the illustrative example of Figure 3, the framework 300 can include at least a first group of features 312, a second group of features 314, and a third group of features 316. Each group 312, 314, 316 can include at least one of one or more genetic features, one or more observable features, one or more co-occurring conditions, or one or more analytes. For example, the first group of features 312 can include a genetic feature, C, an analyte, A, and a co-occurring condition, E. Additionally, the second group of features 314 can include a genetic feature, B, an analyte, B, and two observable features, B and G. Further, the third group of features 316 can include a genetic feature, H, an observable feature, F, and two co-occurring conditions, A and D. In one or more illustrative examples, individuals can be included in a subgroup of individuals that each have the features included in the first set of features 312: genetic feature, C, threshold levels of the analyte, A, and have been diagnosed with the co-occurring condition, E. In one or more additional illustrative examples, additional individuals can be included in an additional subgroup of individuals that each have the features included in the second group of features 314: a genetic feature, B, threshold levels of the analyte, G, and observable features B and G.

The features included in the individual groups of features 312, 314, 316 can be determined by the computational biology system 102 by analyzing the information stored by at least one of the risk variants data store 220, the risk genes data store 222, the risk pathway data store 224, the biological condition classification data store 226, or the biological condition features data store 302. For example, the computational biology system 102 can determine levels of significance between individuals in which a neurodevelopmental condition is present and at least one of one or more genetic features 304, one or more observable features 306, one or more co-occurring conditions 308, or one or more analytes 310. To illustrate, the computational biology system 102 can implement one or more hierarchical-based clustering techniques to determine a number of groups of features to use to identify subgroups of individuals in which the same neurodevelopmental condition is present. The computational biology system 102 can also implement one or more partitioning-based clustering techniques such as k-means clustering techniques to determine a number of groups of features to use to identify subgroups of individuals in which the same neurodevelopmental condition is present. The computational biology system 102 can also implement one or more density-based clustering techniques to determine a number of groups of features to use to identify subgroups of individuals in which the same neurodevelopmental condition is present. In addition, the computational biology system 102 can implement one or more model-based clustering techniques such as latent class analysis techniques to determine a number of groups of features to use to identify subgroups of individuals in which a neurodevelopmental condition is present. Further, the computational biology system 102 can implement one or more graph-based clustering techniques to determine a number of groups of features to identify sub-groups of individuals in which a neurodevelopmental condition is present.

In one or more implementations, the computational biology system 102 can analyze information corresponding to a number of individuals in which a neurodevelopmental condition is present to determine subgroups for the individuals. For example, the computational biology system 102 can analyze at least one of genetic features information, observable features information, co-occurring conditions information, or analytes information of a number of individuals in which a neurodevelopmental condition is present to determine subgroups for the number of individuals. In various examples, the computational biology system 102 can implement at least one of a hierarchical-based clustering technique, partitioning-based clustering technique, density-based clustering technique, model-based clustering technique such as a latent class analysis technique, or a graph-based clustering technique to determine the sub-groups of individuals. After determining the sub-groups of individuals, the computational biology system 102 can determine groups of features that correspond to the respective subgroups of individuals. For example, the computational biology system 102 can determine that a first subgroup of individuals in which a neurodevelopmental condition is present can have the first group of features 312, a second subgroup of individuals in which the neurodevelopmental condition is present can have the second group of features 314, and a third subgroup of individuals in which the neurodevelopmental condition is present can have the third group of features 316. After determining the groups of features 312, 314, 316 that can be used to determine subgroups of individuals in which a neurodevelopmental condition is present, the computational biology system 102 can use the groups of features 312, 314, 316 to determine subgroups to place additional individuals for which the computational biology system 102 receives information and in which the neurodevelopmental condition is present. In one or more examples, the groups of features 314, 314, 316 can be determined by using domain knowledge and/or by using biomedical literature in regards to the neurodevelopmental condition. In one or more other examples, statistical analysis such as analysis of variance and pairwise correlation of the values of the features corresponding to their relevance for the neurodevelopmental condition can be used to determine the groups of features.

The reliability of the groups of features 312, 314, 316 and the subgroups of individuals in which a neurodevelopmental condition is present that are identified according to the groups of features 312, 314, 316 can be evaluated by the computational biology system 102. In various implementations, for an example subgrouping of individuals, the overall similarity within subgroups compared to the similarity within other subgroups can be measured using one or more validity indices, such as Dunn Index, Davies-Boulding index and/or Silhouette index. For example, the groups of features 312, 314, 316 and/or the subgroups of individuals generated using the groups of features 312, 314, 316 can be evaluated using a Davies-Bouldin index, assessing how close the members in a cluster are to the centroid of the cluster and how distant the centroids of different clusters are. In various examples, the features included in the group of features 312, 314, 316 can be identified based on Davies-Bouldin index values for the respective groups of features 312, 314, 316 being at most threshold Davies-Bouldin index values. Additionally, the groups of features 312, 314, 316 and/or the sub-groups of individuals generated using the groups of features 312, 314, 316 can be evaluated using a Silhouette index, assessing how close the members of the same cluster are to each other and how close they are to the members of other clusters. The features included in a respective group of features 312, 314, 316 can have Silhouette values that correspond to at least threshold Silhouette values. In one or more illustrative examples, subgroups can be produced that have Dunn index values, Davies-Bouldin index values, and/or Silhouette index values such that a variance within individuals of the subgroup in terms of distance is relatively small and that centers of different subgroups are sufficiently far apart in relation to the variance within the subgroups. In one or more implementations, the computational biology system 102 can determine the reliability of the subgrouping using the overall similarity within subgroups compared to the similarity within other subgroups assessed by Dunn index values, Davies-Bouldin index values and/or Silhouette index values.

In one or more implementations, groups of features used to determine subgroups of individuals in which a neurodevelopmental condition is present can be determined using specified types of information. In this way, the computational biology system 102 can determine different subgroups of individuals based on analyzing different types of information. For example, a first number of groups of features for use in determining subgroups of individuals in which a neurodevelopmental condition is present can be determined based on observable features data that indicates observable features of individuals diagnosed with a neurodevelopmental condition, such as the observable features 306. The observable features 306 can be identified from phenotypic data and/or clinical data. In one or more additional examples, a second number of groups of features for use in determining subgroups of individuals in which a neurodevelopmental condition is present can be determined based on genetic data 110 that indicates genetic features 304 of individuals in which a neurodevelopmental condition is present. In one or more further examples, a third number of groups can be determined based on molecular data that indicates information related to biological pathways that can be disrupted in individuals in which a neurodevelopmental condition is present. In various examples, the molecular data can indicate levels of one or more of the analytes 310 in relation to threshold levels of at least one of the analytes 310.

In one or more illustrative examples, the computational biology system 102 can determine a plurality of groupings of features used to determine subgroups of individuals in which a neurodevelopmental condition is present according to one or more techniques. The computational biology system 102 can then evaluate the plurality of groupings of features and/or evaluate the subgroups of individuals to determine one or more measures of reliability of one or more of the groups of features included in the plurality of groupings of features and/or to determine one or more measures of reliability for one or more of the subgroups of individuals identified by the computational biology system 102. In situations where the respective measures of reliability of at least one of the groupings of features is less than a threshold measure of reliability, the computational biology system 102 can determine different groupings of the features used to determine the subgroups of individuals. The computational biology system 102 can then evaluate the different groupings of the features and determine additional measures of reliability for the different groupings of features. In this way, the computational biology system 102 can iteratively evaluate different groupings of features until the measures of reliability of the groupings of features used to determine subgroups of individuals in which a neurodevelopmental condition is present in order to satisfy one or more threshold criteria.

In various implementations, the computational biology system 102 can also evaluate groupings of individuals that were determined using information obtained from different data sources and/or using a group of genetic and molecular features and biological conditions within the data sources. For example, the computational biology system 102 can evaluate groupings of individuals based on one or more groupings of observable features 306 determined using observable features data in relation to groupings of individuals based on one or more groupings of genetic features 304 determined using genetic features data. In one or more additional examples, the computational biology system 102 can evaluate groupings of individuals based on one or more groupings of molecular features determined using molecular data in relation to groupings of individuals based on one or more groupings of genetic features determined using genetic features data. In one or more further examples, the computational biology system 102 can evaluate groupings of individuals based on one or more groupings of observable features 306 determined using observable features data in relation to groupings of individuals based on one or more groupings of molecular features determined using molecular data. In other one or more examples, the computational biology system 102 can evaluate groupings of individuals based on one or more groupings of clinical features, determined using clinical features data. The evaluations performed by the computational biology system 102 with respect to the groupings of individuals determined using information from different pairs of data sources can produce measures of reliability for one or more of the groupings of individuals. The computational biology system 102 can iteratively modify groupings of individuals determined using information obtained from different data sources until one or more threshold criteria are satisfied for the measures of reliability determined by the computational biology system 102 for the respective groupings of individuals. In one or more implementations, the threshold criteria can be determined by the coherence of grouping of individuals obtained from different data sources based on Dunn index values, Davies-Bouldin index values and/or Silhouette index values. For example, a first grouping corresponding to a first, second, third and Nth group of individuals can be obtained by the computational biology system 102 and a second grouping corresponding to a fourth, fifth, sixth and Mth group of individuals. The computational biology system 102 can determine that the first grouping is better than the second grouping based on whether average coherence among the first, second, third and Nth group is higher than the average coherence among the fourth, fifth, sixth and Mth group assessed by the Dunn index values, Davies-Bouldin index values and/or Silhouette index values. In one or more other implementations, the threshold criteria can be determined by using a group of features using an overlap coefficient, Jaccard Index, S0rensen-Dice coefficient, Tversky index and/or mutual information. In one or more examples, the computational biology system 102 can determine that the first grouping is better than the second grouping based on whether an average similarity among the first, second, third, and Nth group is higher than the average similarity among the fourth, fifth, sixth, and Mth group assessed by the overlap coefficient, Jaccard Index, S0rensen-Dice coefficient, Tversky index and/or mutual information.

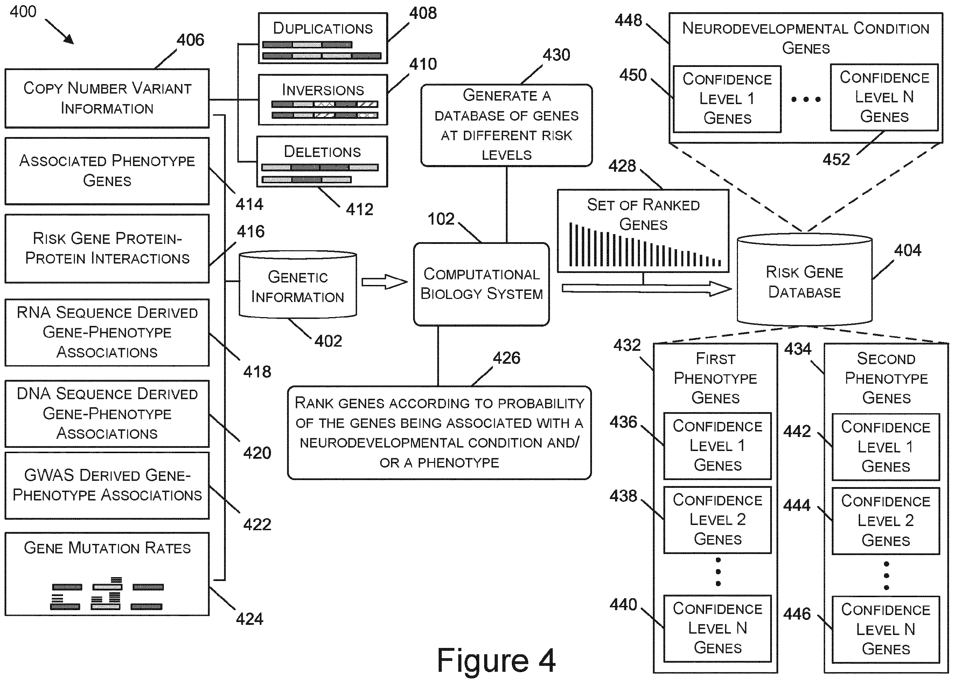

Figure 4 is a diagram of an example framework 400 to analyze a number of types of genetic data to determine confidence levels that various genes are related to risk for at least one biological condition, in accordance with one or more example implementations. The framework 400 can include genetic information 402 that is accessible by the computational biology system 102. At least a portion of the genetic information 402 can be stored by one or more of the curated databases 202 described with respect to Figure 2 and Figure 3. In addition, at least a portion of the genetic information 402 can be stored by one or more of the data sources 106 described with respect to Figure 1 and/or one or more of the data sources 204 described with respect to Figure 2. The computational biology system 102 can analyze the genetic information 402 to produce a risk gene database 404. The risk gene database 404 can indicate respective measures of probability that the presence of one or more genes in an individual corresponds to the individual being diagnosed with a biological condition. In one or more illustrative examples, the risk gene database 404 can indicate measures of probability that the presence of one or more genes in an individual corresponds to a biological condition being present in the individual being diagnosed with a neurodevelopmental condition, such as autism spectrum disorder. In various examples, at least a portion of the information stored by the risk gene database 404 can be included in one or more of the curated databases 202.

The genetic information 402 can include copy number variant information 406. The copy number variant information 406 can indicate differences in the number of copies of one or more genes or genomic regions present in a first group of individuals in relation to the number of copies of the one or more genes or genomic regions present in the second group of individuals. In one or more illustrative examples, the first group of individuals can include individuals in which a neurodevelopmental condition is present, such as autism spectrum disorder, and the second group of individuals can include individuals in which the neurodevelopmental condition is not present. The copy number variant information 406 can include duplications 408. The duplications 408 can indicate that the number of copies of one or more genes or genomic regions present in genomes of the first group of individuals is greater than the number of copies of the one or more genes or genomic regions present in genomes of the second group of individuals. The copy number variant information 406 can also include inversions 410. The inversions can indicate that locations of one or more genes or genomic regions included in genomes of the first group of individuals is switched with respect to the locations of one or more additional genes or genomic regions in the genomes of the second group of individuals. Additionally, the copy number variant information 406 can include deletions 412. The deletions 412 can indicate that the number of copies of one or more genes or genomic regions present in the genomes of the first group of individuals is less than the number of copies of the one or more genes or genomic regions present in the genomes of the second group of individuals.

The genetic information 402 can also include associated phenotype genes 414. The associated phenotype genes 414 can include genes that are present in individuals having one or more phenotypes that are co-occurring with respect to a neurodevelopmental condition. The one or more phenotypes can include one or more observable features. The one or more phenotypes can also be related to a biological condition that is co-occurring with respect to the neurodevelopmental condition. In one or more examples, the associated phenotype genes 414 can include genes present in individuals that have one or more phenotypes that can be co-occurring with respect to autism spectrum disorder. For example, the associated phenotype genes 414 can include one or more genes present in individuals that have been diagnosed with Williams syndrome. In one or more additional examples, the associated phenotype genes 414 can include one or more genes present in individuals that have been diagnosed with inflammatory bowel disease. In one or more further examples, the associated phenotype genes 414 can include one or more genes present in individuals that have been diagnosed with macrocephaly.

Additionally, the genetic information 402 can include risk gene protein-protein interactions 416. In various examples, protein-protein interactions can include the binding of proteins to each other through at least one of hydrophobic bonding, van der Waals forces, or salt bridges at binding sites on the proteins interacting with each other. The risk gene protein-protein interactions 416 can indicate interactions between at least one first protein and a second protein that is produced in association with the expression of a gene that has at least a threshold probability of being altered in genomes of individuals in which a neurodevelopmental condition is present. To illustrate, the risk gene protein-protein interactions 416 can indicate interaction with other proteins by a protein produced in conjunction with a gene that has at least a threshold probability of being altered in the genome of individuals diagnosed with autism spectrum disorder.

Further, the genetic information 402 can include RNA sequence derived gene-phenotype associations 418. The RNA sequence derived gene-phenotype associations 418 can indicate genes identified from RNA sequence information of individuals in which a neurodevelopmental condition is present in relation to RNA sequence information of individuals in which a neurodevelopmental condition is not present. In various examples, the RNA sequence derived gene-phenotype associations 418 can be determined by the computational biology system 102. In additional implementations, the RNA sequence derived gene-phenotype associations 418 can be obtained from one or more data sources that have predetermined the RNA sequence derived gene-phenotype associations 418.

The RNA sequence information used to determine the RNA sequence derived gene-phenotype associations 418 can include whole transcriptome sequencing information. The transcriptomic information can include nucleic acid sequences of messenger RNA (mRNA). Additionally, the transcriptomic information can include nucleic acid sequences of non-coding RNA. The non-coding RNA can include at least one of transfer RNA (tRNA), ribosomal RNA (rRNA), micro RNA (miRNA), small interfering RNA (siRNA), piwi interacting RNA (piRNA), small nucleolar RNA (snoRNA), small nuclear RNA (snRNA), extracellular RNA (exRNA), small cajal body-specific RNA (scaRNA), or long non-coding RNAs.

In one or more implementations, one or more genes that correspond to a neurodevelopmental condition can be identified by analyzing RNA sequencing information of individuals in which a neurodevelopmental condition is present in relation to RNA sequencing information of individuals in which the neurodevelopmental condition is not present. In one or more illustrative examples, the RNA sequence derived gene-phenotype associations 418 can be determined based on an analysis of RNA sequencing information indicating that a frequency of one or more genes that are present in individuals in which a neurodevelopmental condition is present is at least a threshold amount greater than a frequency of the one or more genes in individuals in which the neurodevelopmental condition is not present. In additional implementations, the absence of one or more genes in individuals in which a neurodevelopmental condition is present that are present in individuals in which the neurodevelopmental condition is not present can be determined based on an analysis of RNA sequencing information. For example, RNA sequencing information can be determined indicating that a frequency of one or more genes in individuals in which a neurodevelopmental condition is present is less than a threshold frequency, where the threshold frequency is determined based on the frequency that the one or more genes are present in individuals in which the neurodevelopmental condition is not present.

In various implementations, the genetic information 402 can include DNA sequence derived gene-phenotype associations 420. The DNA sequence derived gene-phenotype associations 420 can indicate one or more genetic modifications identified from DNA sequence information of individuals in which a neurodevelopmental condition is present in relation to DNA sequence information of individuals in which the neurodevelopmental condition is not present. The DNA sequencing information can indicate nucleic acid sequences of DNA molecules of individuals, determined either using one or more DNA sequencing technology and/or inferred by imputation across a cohort of individuals with DNA sequence information. In one or more illustrative implementations, the DNA sequence information can also include human leukocyte antigen (HLA) complex sequence information. In one or more examples, the DNA sequence derived gene-phenotype associations 420 can be determined by the computational biology system 102. In further implementations, the DNA sequence derived gene-phenotype associations 420 can be obtained from one or more data sources that have predetermined the DNA sequence derived gene-phenotype associations 420.

One or more genetic modifications that correspond to a neurodevelopmental condition can be identified by analyzing DNA sequencing information of individuals in which a neurodevelopmental condition is present in relation to DNA sequencing information of individuals in which the neurodevelopmental condition is not present. To illustrate, the DNA sequence derived gene-phenotype associations 420 can be determined based on an analysis of DNA sequencing information indicating that a frequency of one or more genetic modifications that are present in individuals in which a neurodevelopmental condition is present is at least a threshold amount greater than a frequency of the one or more genetic modifications in individuals in which the neurodevelopmental condition is not present. Additionally, the absence of one or more genetic modifications in individuals in which a neurodevelopmental condition is present that are present in individuals in which the neurodevelopmental condition is not present can be determined based on an analysis of DNA sequencing information. For example, DNA sequencing information can be determined indicating that a frequency of one or more genetic modifications in individuals in which the neurodevelopmental condition is present is less than a threshold frequency, where the threshold frequency is determined based on the frequency that the one or more genetic modifications are present in individuals in which the neurodevelopmental condition is not present.

In one or more implementations, the genetic information 402 can include genome-wide association study (GWAS) derived gene-phenotype associations 422. The GWAS derived gene-phenotype associations 422 can be determined based on the analysis of one or more genome-wide association studies. At least a portion of the GWAS derived gene-phenotype associations 422 can be determined based on an analysis performed by the computational biology system 102 of one or more genome-wide association studies. In additional implementations, at least a portion of the GWAS derived gene-phenotype associations 422 can be obtained from one or more data sources that indicated previously determined GWAS derived gene-phenotype associations 422.
The genome-wide association studies can include one or more genetic variants present in individuals with a neurodevelopmental condition and/or one or more biological conditions co-occurring with neurodevelopmental disorders at a higher rate than expected by chance (a.k.a. case individuals), at a higher/lower allele frequency than in those without the neurodevelopmental and/or one or more biological conditions (a.k.a, control individuals). The one or more genetic variants can be identified through an analysis of genomes of a number of case individuals and genomes of a number of control individuals.

Estimates of expected gene mutation rates 424 based on gene length and sequence context can also be included in the genetic information 402. The gene mutation rates 424 can be used to identify biologically relevant genes that are intolerant to carry some kind of mutations (such as PTVs) in the general population. To illustrate, the gene mutation rates 424 can be used to determine genes having an observed frequency of mutations in a control population that is lower than expected based on gene length and sequence content.

The computational biology system 102 can analyze at least portions of the genetic information 402, at operation 426, to rank genes according to risk associated with a phenotype and/or a neurodevelopmental condition. For example, the computational biology system 102 can determine a probability that the presence and/or absence of a gene corresponds to an individual having a respective phenotype and/or corresponds to a respective individual in which a neurodevelopmental condition is present. In various examples, a first genetic variant that is ranked more highly than a second genetic variant can have a greater probability of being included in the genomes of individuals expressing the phenotype and/or of being diagnosed with the neurodevelopmental condition than the second genetic variant.

In additional implementations, the computational biology system 102 can rank rare variants and identify rare variants that can be used to group individuals in which a neurodevelopmental condition is present. Rare variants can have a threshold allele frequency, such as no greater than about 0.001 with respect to individuals included in a population. The computational biology system 102 can rank rare variants based on one or more criteria. The one or more criteria can include modifications to the expression and/or functionality of a gene due to variants of the gene. For example, the computational biology system 102 can determine rankings for rare genetic variants based on whether or not the genetic variants are protein truncating variants (i.e. that result in non-functional protein products).

The computational biology system 102 can also determine rankings of rare genetic variants based on mutation rates of genes that can carry the mutations. To illustrate, the computational biology system 102 can rank a rare genetic variant by determining a measure of conservation of the gene related to the rare genetic variant. In one or more implementations, the computational biology system 102 can determine a ranking of a rare genetic variant with respect to the ranking of an additional rare genetic variant based on the measure of conservation of the gene of the rare genetic variant being higher than a measure of conservation of the gene of the additional rare genetic variant. In further implementations, the computational biology system 102 can determine a ranking of a rare genetic variant with respect to rankings of additional rare genetic variants based on the measure of conservation of the gene being less than the measure of conservation of a gene of the additional rare genetic variant.

The result of operation 426 can include one or more sets of ranked genes, such as the set of ranked genes 428. An individual set of ranked genes 428 can include a number of genes and the respective probabilities that the number of genes is associated with at least one neurodevelopmental condition and/or phenotype. At operation 430, the computational biology system 102 can use the sets of ranked genes 428 to generate the risk gene database 404. The risk gene database 404 can include a plurality of genes and probabilities that the respective genes are related to one or more phenotypes, where the one or more phenotypes can be present in individuals in which a neurodevelopmental condition is present. For example, the risk gene database 404 can include first phenotype genes 432 and second phenotype genes 434. The first phenotype genes 432 can include a first plurality of genes that can be present in first individuals in which a first phenotype is present and the second phenotype genes 434 can include a second plurality of genes that can be present in second individuals in which a second phenotype is present. In one or more implementations, the second plurality of genes can have one or more genes that are different from the first plurality of genes. Additionally, in various examples, at least one of the second individuals can be different from at least one of the first individuals.

The first phenotype genes 432 can be organized in the risk gene database 404 according to confidence levels that the individual genes are related to the first phenotype. The different confidence levels can correspond to various probabilities that the first phenotype genes 432 are related to the first phenotype. In the illustrative example of Figure 4, the first phenotype genes 432 can be arranged according to confidence level 1 genes 436, confidence level 2 genes 438, up to confidence level N genes 440. The confidence level 1 genes 436 can have a first range of probabilities of being present in individuals exhibiting the first phenotype. The confidence level 2 genes 438 can have a second range of probabilities of being present in individuals exhibiting the first phenotype and that are different from the first range of probabilities. Additionally, the confidence level N genes 440 can have a third range of probabilities of being present in individuals exhibiting the first phenotype and are different from the first range of probabilities and the second range of probabilities.

The second phenotype genes 434 can be arranged according to confidence level 1 genes 442, confidence level 2 genes 444, up to confidence level N genes 446. The different confidence levels can correspond to various probabilities that the second phenotype genes 434 are related to the second phenotype. In one or more implementations, the respective ranges of probabilities associated with the individual confidence level genes 442, 444, 446 can correspond to the respective ranges of probabilities associated with the individual confidence levels 436, 438, 440. For example, the confidence level 1 genes 436 can have a first range of probabilities of being present in individuals exhibiting the first phenotype that corresponds to a second range of probabilities of the confidence level 1 genes 442 being present in individuals exhibiting the second phenotype. Additionally, the confidence level 2 genes 438 can have a third range of probabilities of being present in individuals that exhibit the first phenotype that corresponds to a fourth range of probabilities of the confidence level 2 genes 444 being present in individuals exhibiting the second phenotype, where the third range of probabilities and the fourth range of probabilities are different from the first range of probabilities and the second range of probabilities.

One or more sets of ranked genes 428 can also be stored in the risk gene database 404 as neurodevelopmental condition genes 448. The neurodevelopmental condition genes 448 can include genes having at least a threshold probability of being altered in the genomes of individuals in which a neurodevelopmental condition is present (a.k.a. risk condition). In one or more illustrative examples, the neurodevelopmental condition genes 448 can include genes having at least a threshold probability of being altered in genomes of individuals in which autism spectrum disorder is present. The neurodevelopmental condition genes 448 can be arranged according to ranges of confidence levels of the respective genes being altered in genomes of individuals in which a neurodevelopmental condition is present. The confidence levels of genes being altered in the genomes of individuals in which a neurodevelopmental condition is present can correspond to the probabilities used to rank the neurodevelopmental condition genes 448 in the set of ranked genes 428. For example, the neurodevelopmental condition genes 448 can be arranged as confidence 1 level genes 450 up to confidence level N genes 452. The confidence level 1 genes can have a first range of probabilities of being altered in the genomes of individuals in which a neurodevelopmental condition is present, and the confidence level N genes can have an additional range of probabilities of being altered in the genomes of individuals in which a neurodevelopmental condition is present. In one or more implementations, the confidence level 1 genes 450 can have a greater range of probabilities of being altered in the genomes of individuals in which a neurodevelopmental condition is present than an additional range of probabilities of the confidence level N genes 452. In additional implementations, the confidence level 1 genes 450 can have a lower range of probabilities of being altered in the genomes of individuals in which a neurodevelopmental condition is present than an additional range of probabilities of the confidence level N genes 452.

In one or more implementations, the computational biology system 102 can access the risk gene database 404 to determine individuals in which a neurodevelopmental condition is present that can be grouped according to one or more phenotypes. Additionally, the computational biology system 402 can access the risk gene database 404 to determine biological pathways that have at least a threshold probability of being related to a phenotype and/or being related to a neurodevelopmental condition. In various examples, the computational biology system 402 can access the risk gene database 404 to determine biological pathways that have at least a threshold probability of being disrupted or modified in individuals having a phenotype and/or in individuals in which a neurodevelopmental condition is present.

In one or more illustrative examples, the computational biology system 102 can identify genetic variants as features that can be used to determine groupings of individuals in which a neurodevelopmental condition is present based on the respective rankings of the genetic variants. To illustrate, genetic variants that have at least a threshold probability of being related to the expression of a phenotype in individuals in which a neurodevelopmental condition is present can be used as features to determine subgroups of the individuals in which the neurodevelopmental condition is present. In one or more implementations, the computational biology system 102 can identify one or more genetic variants that are classified as common variants having at least a threshold allele frequency, such as at least 0.05, for a phenotype. The computational biology system 102 can determine polygenic risk scores for individuals based on the number of common variants related to the phenotype that are included in the respective genomes of the individuals. The polygenic risk scores can be determined using genome-wide association studies related to a neurodevelopmental condition, such as autism spectrum disorder. Further, the polygenic risk scores can be determined using genome-wide association studies related to autism spectrum disorder in addition to genome-wide association studies related to biological conditions that are co-occurring with respect to autism spectrum disorder and/or other neurodevelopmental conditions.

The polygenic risk scores for a given individual with respect to one or more phenotypes can be used by the computational biology system 102 to group the individual into one or more subgroups of individuals in which a neurodevelopmental condition is present. For example, first individuals diagnosed with a neurodevelopmental condition that have polygenic risk scores within a specified range for a first phenotype can be grouped together and second individuals diagnosed with the neurodevelopmental condition that have polygenic risk scores within an additional range for a second phenotype can be grouped together. In addition, third individuals diagnosed with a neurodevelopmental condition that have polygenic risk scores within a specified range for a first phenotype and polygenic risk scores within a specified range for a second phenotype can be grouped together. In one or more illustrative examples, first polygenic risk scores can be determined for individuals diagnosed with autism spectrum disorder with respect to genetic variations related to rheumatoid arthritis and second polygenic risk scores can be determined for the individuals with respect to genetic variations related to Alzheimer's disease. Continuing with this illustrative example, the computational biology system 102 can determine a first group of individuals that have first polygenic risk scores related to rheumatoid arthritis within a range of scores. The computational biology system 102 can also determine a second group of individuals that have second polygenic risk scores related to Alzheimer's disease within an additional range of scores. Further, the computational biology system 102 can also determine a third group of individuals that have a first polygenic risk scores related to rheumatoid arthritis within a range of scores and second polygenic risk scores related to Alzheimer's disease within an additional range of scores.

In various examples, the computational biology system 102 can determine groupings of individuals for a number of phenotypes based on rare genetic variants that have at least a threshold ranking. In these situations, the computational biology system 102 can determine rare genetic variants that also have at least a threshold probability of being implicated in one or more biological pathways, where the one or more biological pathways have at least a threshold probability of being associated with a neurodevelopmental condition. In one or more implementations, the computational biology system 102 can utilize these rare genetic variants that are included in specified biological pathways to identify groupings of individuals in which a neurodevelopmental condition is present. To illustrate, individuals in which the neurodevelopmental condition is present can be grouped according to biological pathways that are disrupted by rare genetic variants corresponding to the disrupted biological pathways.

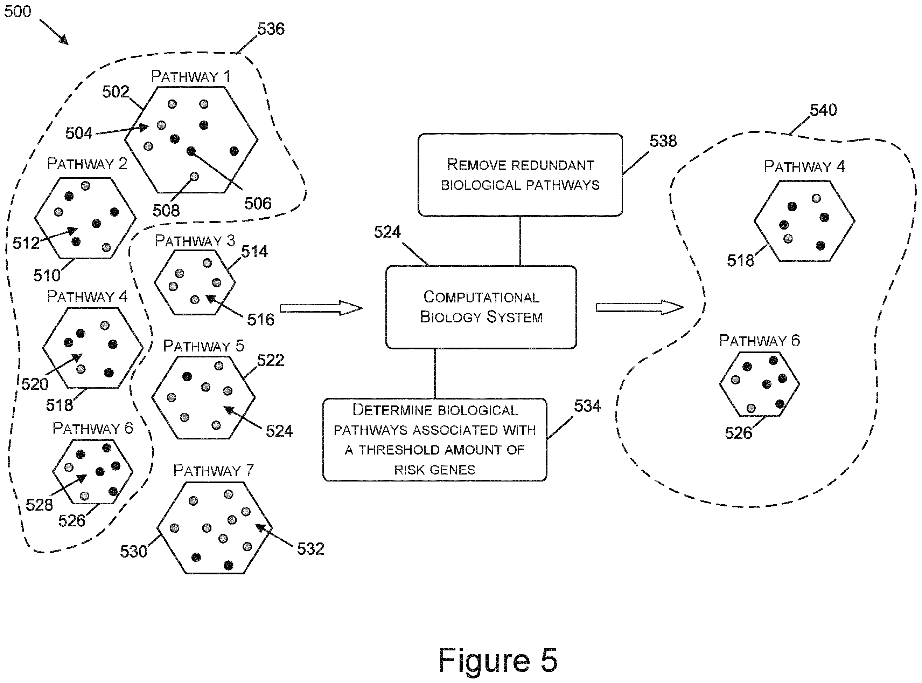

Figure 5 is a diagram of an example framework 500 to remove redundant biological pathways from a set of biological pathways that are related to a neurodevelopmental condition, in accordance with one or more example implementations. At least a portion of the number of genes of the individual biological pathways can be expressed during activation of the individual biological pathways. The activation of the individual biological pathways can cause a series of events to take place, such as one or more chemical reactions, movement of one or more molecules, structural modification of one or more molecules, combinations thereof, and the like. The series of events that take place during the activation of the individual biological pathways can be determined based on the genes associated with the respective biological pathways. The removal of redundant biological pathways can be used in the production of one or more of the curated databases 202 described with respect to Figure 2. For example, the removal of redundant biological pathways described in relation to Figure 5 can be used in generating the risk pathway data store 224 described in relation to Figure 2.

In the illustrative example of Figure 5, the framework 500 can include a first biological pathway 502 that is associated with a first set of genes 504. Individual genes included in the set of genes 504 can have a respective probability of being included in genomes of individuals in which a neurodevelopmental condition is present. The shading of the genes depicted in the illustrative example of Figure 5 can be indicative of the probability of the respective genes being altered in the genomes of individuals in which a neurodevelopmental condition is present. For example, the set of genes 504 can include a darker shaded first gene 506 that has a first probability of being altered in the genomes of individuals in which a neurodevelopmental condition is present and a lighter shaded second gene 508 that has a second probability of being altered in the genomes of individuals in which a neurodevelopmental condition is present. In one or more illustrative examples, the first gene 506 can have a higher probability of being altered in genomes of individuals in which a neurodevelopmental condition is present than a probability of the second gene 508 being altered in the genomes of individual in which the neurodevelopmental condition is present.

The framework 500 can also include a second biological pathway 510 that is associated with a second set of genes 512 and a third biological pathway 514 that is associated with a third set of genes 516. In addition, the framework 500 can include a fourth biological pathway 518 that is associated with a fourth set of genes 520 and a fifth biological pathway 522 that is associated with a fifth set of genes 524. Further, the framework 500 can include a sixth biological pathway 526 that is associated with a sixth set of genes 528 and a seventh biological pathway 530 that is associated with a seventh set of genes 532. The respective sets of genes 512, 516, 520, 524, 528, and 532 can each include a number of genes that have at least a threshold probability of being altered in genomes of individuals in which a neurodevelopmental condition is present and an additional number of genes that have less than the threshold probability of being altered in genomes of individuals in which a neurodevelopmental condition is present.

In addition, the framework 500 can include the computational biology system 102. The computational biology system 102 can analyze genes associated with a number of biological pathways to determine biological pathways that correspond to one or more neurodevelopmental conditions. For example, at operation 534, the computational biology system 102 can determine biological pathways associated with a threshold number of risk genes. In the illustrative example of Figure 5, the computational biology system 102 can determine a group of biological pathways 536 that have at least a threshold number of genes that have at least a threshold probability of being altered in the genomes of individuals in which a neurodevelopmental condition is present.

The risk genes can include genes having at least a threshold probability of being altered in the genomes of individuals in which a neurodevelopmental condition is present. In various examples, the computational biology system 102 can determine risk genes by accessing one or more curated databases that indicate probabilities of genes being altered in genomes of individuals in which a neurodevelopmental condition is present, such as the risk gene database 404 described with respect to Figure 4. Additionally, the computational biology system 102 can determine the threshold amount of risk genes by analyzing the number of risk genes associated with at least a portion of the biological pathways related to the neurodevelopmental condition. In one or more implementations, the computational biology system 102 can determine biological pathways that have more risk genes than a number of genes expected to be associated with biological pathways by chance. That is, the computational biology system 102 can determine that a threshold number of risk genes associated with a biological pathway has at least a threshold amount of statistical significance. In one or more implementations, the statistical significance can be determined using Fisher's exact test and the threshold amount of statistical significance after multiple hypothesis testing can correspond to one or more false discovery rate levels, such as no greater than about 0.3, no greater than about 0.25, no greater than about 0.2, no greater than about 0.1, no greater than about 0.05, or no greater than about 0.01. In one or more other implementations the threshold amount of statistical significance can also correspond to a threshold percentage of the biological pathways being included in the group of biological pathways 536 are statistically significant, such as at least about 20% of the biological pathways, at least about 30% of the biological pathways, at least about 40% of the biological pathways, at least about 50% of the biological pathways, at least about 60% of the biological pathways, at least about 70% of the biological pathways, at least about 80% of the biological pathways, at least about 90% of the biological pathways, at least about 95% of the biological pathways, or at least about 99% of the biological pathways.

The computational biology system 102 can also, at operation 538, removing redundant biological pathways from the group of biological pathways 536 to produce a modified group of biological pathways 540. For example, the computational biology system 102 can determine that a number of biological pathways are redundant with respect to one another based on the number of genes associated with the respective biological pathways being the same. In one or more illustrative examples, the computational biology system 102 can determine that a number of biological pathways are redundant with respect to one another based on a threshold percentage of genes associated with the number of biological pathways being the same, such as at least about 70% of the genes are common between the number of biological pathways, at least about 75% of the genes are common between the number of biological pathways, at least about 80% of the genes are common between the number of biological pathways, at least about 85% of the genes are common between the number of biological pathways, at least about 90% of the genes are common between the number of biological pathways, or at least about 95% of the genes are common between the number of biological pathways. In various other examples, the computational biology system 102 can determine the Jaccard index or overlap coefficient as the threshold value of genes associated with the number of biological pathways being the same given the total number of genes associated with the number of biological pathways.

In various implementations, the computational biology system 102 can generate the modified group of biological pathways 540 by determining a group of redundant biological pathways and removing one or more of the biological pathways that have a greater number of genes than at least one other biological pathway included in the group of redundant biological pathways. In one or more implementations, the computational biology system 102 can determine pairs of redundant biological pathways and remove the biological pathway from the pair that has a greater number of genes than the other biological pathway included in the pair of redundant biological pathways to produce the modified group of biological pathways 540. In one or more illustrative examples, the computational biology system 102 can determine that fourth biological pathway 518 is redundant with respect to the first biological pathway 502 and that the sixth biological pathway 526 is redundant with respect to the second biological pathway 510. In these situations, the computational biology system 102 can include the fourth biological pathway 518 in the modified group of biological pathways 540 based on determining that the fourth biological pathway 518 is associated with fewer genes than the first biological pathway 502. Additionally, the computational biology system 102 can include the sixth biological pathway 526 in the modified group of biological pathways 540 based on determining that the sixth biological pathway 526 is associated with fewer genes than the second biological pathway 510.

The computational biology system 102 can produce the modified group of biological pathways 540 by determining redundant biological pathways that have a parent-child relationship. A parent-child relationship between at least two biological pathways can indicate that the at least two biological pathways are included in a series of related biological pathways that occur in a sequence with one of the biological pathways being activated after an additional biological pathway that is included in the series of related biological pathways. In these situations, the parent biological pathway can be activated first in the series of related biological pathways and the child biological pathway can be activated after the parent biological pathway. In various examples, the series of related biological pathways can produce an effect, such as causing one or more changes to one or more molecules, causing one or more changes to one or more cells, causing one or more molecules to be produced, combinations thereof, and so forth. In one or more illustrative examples, a series of biological pathways can be related to glycolysis that causes the breakdown of glucose to pyruvate and hydrogen ions through a sequence of enzyme-catalyzed reactions that produce a number of intermediate molecules. Continuing with this example, a parent biological pathway can correspond to the production of a first intermediate molecule during glycolysis, such as the production of glucose-6-phosphate from glucose via hexokinase, with a child biological pathway corresponding to the production of fructose 6-phosphate via phosphoglucose isomerase. In one or more implementations where a pair of redundant biological pathways have a parent-child relationship, the computational biology system 102 can generate the modified group of biological pathways 540 by removing the biological pathway having the greater number of genes with respect to the other biological pathway included in the pair. In additional implementations where a pair of redundant biological pathways do not have a parent-child relationship, the computational biology system 102 can generate the modified group of biological pathways 540 by including both of the biological pathways. Thus, in one or more implementations, the modified group of biological pathways 540 produced by the computational biology system 102 can include redundant biological pathways that do not have a parent-child relationship and/or an individual biological pathway that is included in a redundant pair of biological pathways that have a parent-child relationship.

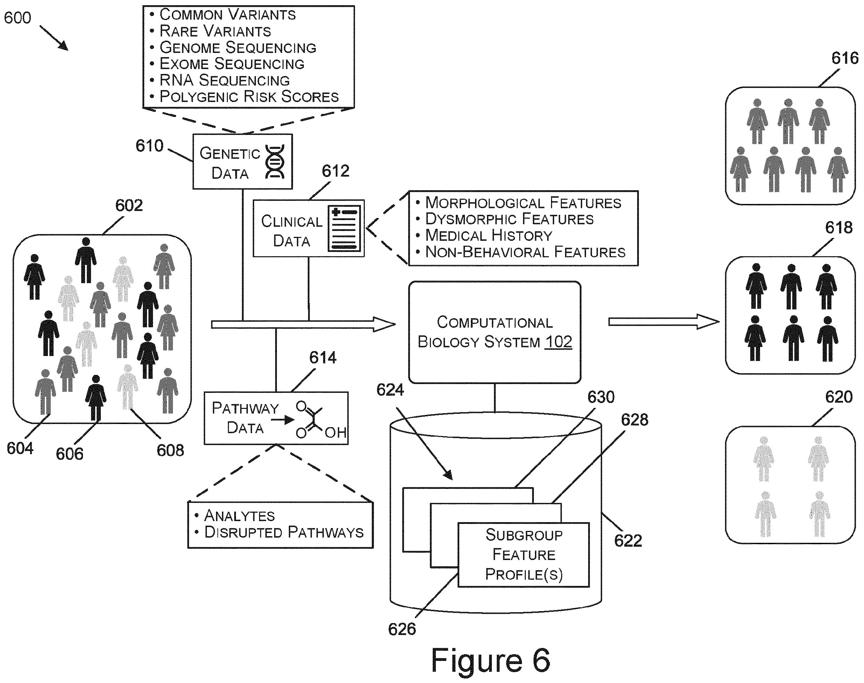

Figure 6 is a diagram of an example framework 600 to analyze a number of types of data to determine subsets of a group of individuals in which a neurodevelopmental condition is present, in accordance with one or more example implementations. The framework 600 can include individuals 602 that have been diagnosed with a neurodevelopmental condition. For example, the individuals 602 can be diagnosed with autism spectrum disorder. In various examples, the individuals 602 can be diagnosed with the neurodevelopmental condition according to clinical analyses by healthcare practitioners. In one or more implementations, the individuals 602 can be diagnosed with the neurodevelopmental condition according to one or more behavioral features of the individuals 602. In various implementations, the framework 600 can include the computational biology system 102 and the computational biology system 102 can determine subgroups of the individuals 602 based on features of the individuals 602 other than the one or more behavioral features.

The individuals 602 can include a number of individuals having different biological features, a number of different genetic features, a number of different morphological features, a number of different phenotypes, or one or more combinations thereof. In one or more illustrative examples, the individuals 602 can include a first individual 604 having a first set of features, a second individual 606 having a second set of features, and a third individual having a third set of features. The first set of features of the first individual 604 can be different from a second set of features of the second individual 606. Additionally, the third set of features of the third individual 608 can be different from both the first set of features of the first individual 604 and the second set of features of the second individual 606.

The computational biology system 102 can identify various subgroups of the individuals 602 by analyzing a number of types of data. For example, the computational biology system 102 can analyze genetic data 610 of the individuals 602 to determine a number of subgroups for the individuals 602. The genetic data 610 can indicate that at least a portion of the individuals 602 have one or more genetic mutations, such as one or more nucleotide substitutions, one or more insertions, one or more deletions, one or more inversions, or one or more combinations thereof. In one or more examples, the genetic features of at least a portion of the individuals 602 can include at least one of one or more rare variants or one or more common variants. In various examples, the genetic data 610 can include DNA sequencing information, such as at least a portion of the nucleic acid sequences of genomes of at least a portion of the individuals 602. Additionally, the genetic data 610 can include RNA sequencing information. In one or more examples, the RNA sequencing information can include transcriptomic information that can indicate nucleic acid sequences of RNA molecules of at least a portion of the individuals 602. In one or more illustrative examples, the RNA sequencing information can include nucleic acid sequences of messenger RNA (mRNA) molecules of at least a portion of the individuals 602. Further, the genetic data 610 can include polygenic risk scores. The polygenic risk scores can indicate a probability that an individual is at risk to develop a biological condition. The polygenic risk scores can be based on genome-wide association studies. The computational biology system 102 can generate at least a portion of the polygenic risk scores, in one or more implementations. In additional implementations, the computational biology system 102 can obtain at least a portion of the polygenic risk scores from one or more data sources.

The computational biology system 102 can also analyze clinical data 612 to determine a number of subgroups of the individuals 602. The clinical data 612 can include morphological features of at least a portion of the individuals 602. The morphological features can be observed by a healthcare practitioner. In one or more examples, the morphological features can be observed in a clinical setting. In additional examples, the morphological features can be identified using one or more diagnostic tests. In various examples, imaging techniques can be used to determine one or more morphological features of at least a portion of the individuals 602. In one or more illustrative examples, the morphological features can include facial features, dimensions of body parts (e.g., head circumference, finger length, etc.), and/or other anatomical features, such as features of internal organs. In one or more examples, the morphological features can indicate one or more dysmorphic features of at least a portion of the individuals. The one or more dysmorphic features can include physical features of at least a portion of the individuals 602 that are different from a control group of individuals that is separate from the individuals 602.

The clinical data 612 can also include medical history information of at least a portion of the individuals 602. The medical history information can be obtained from medical records of at least a portion of the individuals 602. In various examples, the medical history information can include notes from healthcare practitioners, laboratory test results, imaging procedure results, diagnostic test results, one or more combinations thereof, and so forth. Further, the clinical data 612 can indicate additional non-behavioral features. For example, the clinical data 612 can include information indicating electrical activity of at least a portion of the individuals 602. To illustrate, the clinical data 612 can include electroencephalogram (EEG) data and/or electrocardiogram (ECG) data.

In one or more implementations, the computational biology system 102 can analyze pathway data 614 to determine a number of subgroups of the individuals 602. The pathway data 614 can include levels of one or more molecules related to one or more biological pathways. For example, the pathway data 614 can indicate one or more analytes present in at least a portion of the individuals 602. The one or more analytes can include molecules that are measurable by one or more analytical techniques. In one or more illustrative examples, the one or more analytes can include one or more proteins. In additional examples, the one or more analytes can include one or more metabolites. The one or more analytes can also include at least one of one or more vitamins, one or more minerals, one or more hormones, one or more lipids, or one or more carbohydrates. The pathway data 614 can also indicate one or more biological pathways that are disrupted in at least a portion of the individuals 602. In various examples, the one or more disrupted biological pathways can be identified based on the expression of one or more genes associated with the one or more disrupted biological pathways. To illustrate, the expression of one or more genes can be determined based on levels of one or more proteins produced in conjunction with at least one biological pathway. A biological pathway can be disrupted in situations where the expression of one or more genes related to the biological pathway is up-regulated or down-regulated.

The computational biology system 102 can analyze at least one of the genetic data 610, the clinical data 612, or the pathway data 614 to determine a first subgroup 616 of the individuals 602, a second subgroup 618 of the individuals 602, and a third subgroup 620 of the individuals 602. Each respective subgroup 616, 618, 620 can include individuals having a respective set of features. The sets of features used to identify the individuals to include in the subgroups 616, 618, 620 can be determined by the computational biology system 102. For example, as explained previously with respect to Figure 3, the computational biology system 102 can analyze information stored in a number of databases to determine sets of features that can be used to determine subgroups of individuals in which a neurodevelopmental condition is present. The sets of features used by the computational biology system 102 to determine the subgroups 616, 618, 620 can include at least one of genetic features 304, observable features 306, co-occurring conditions 308, or analytes information 310. The computational biology system 102 can determine the individuals 602 to include in a respective subgroup 616, 618, 620 by analyzing the features of the individuals 602 with respect to the features associated with at least one of the subgroups 616, 618, 620. Features related to each subgroup 616, 618, 620 can be included in the subgroup feature profiles 624 generated by the computational biology system 102 for each respective subgroup 616, 618, 620.

In one or more implementations, the computational biology system 102 can be coupled to, or otherwise have access to information stored by a data store 622. The data store 622 can store a plurality of subgroup feature profiles 624. Each of the subgroup feature profiles 624 can indicate a set of features associated with a subgroup of individuals in which a neurodevelopmental condition is present. For example, the subgroup feature profiles 624 can include at least a first subgroup feature profile 626, a second subgroup feature profile 628, and a third subgroup feature profile 630. The set of features included in each respective subgroup feature profile 626, 628, 630 can be different from one another. To illustrate, a first set of features included in the first subgroup feature profile 626 can be different from a second set of features included in the second subgroup feature profile 628. Additionally, a third set of features included in the third subgroup feature profile 630 can be different from the first set of features included in the first subgroup feature profile 626 and the second set of features included in the second subgroup feature profile 628. In one or more illustrative examples, the respective subgroup feature profiles 624 can include at least one of genetic features, observable features, co-occurring conditions, or levels of analytes. In various examples, the features included in the subgroup feature profiles 624 can be determined by the computational biology system 102 for at least a portion of the individuals 602 based on information included in at least one of the genetic data 610, the clinical data 612, or the pathway data 614.

The computational biology system 102 can compare features of the individuals 602 with the features included in the respective subgroup features profiles 624 and determine a measure of similarity between each of the individuals 602 and at least one of the subgroup features profiles 624. The measure of similarity can correspond to a probability that an individual is included in a respective subgroup, such as the first subgroup 616, the second subgroup 618, or the third subgroup 620. For example, the computational biology system 102 can determine features of the first individual 604 from at least one of the genetic data 610, the clinical data 612, or the pathway data 614. The computational biology system 102 can perform one or more analyses of the features of the first individual 604 with respect to the features included in at least one of the first subgroup feature profile 626, the second subgroup feature profile 628, or the third subgroup feature profile 630. Based on the one or more analyses, the computational biology system 102 can determine at least one of a first measure of similarity between the features of the first individual 604 and the features of the first subgroup feature profile 626, a second measure of similarity between the features of the first individual 604 and the features of the second subgroup feature profile 628, or a third measure of similarity between the features of the first individual 604 and the features of the third subgroup feature profile 630. The computational biology system 102 can evaluate at least one of the first measure of similarity, the second measure of similarity, or the third measure of similarity with respect to one or more threshold levels of similarity to determine a subgroup for the first individual 604. In the illustrative example of Figure 6, the computational biology system 102 can determine that the first measure of similarity between the features of the first individual 604 and the features included in the first subgroup feature profile 626 is greater than a threshold level of similarity. In these scenarios, the computational biology system 102 can determine that the first individual 604 can be included in the first subgroup 616. Additionally, the computational biology system 102 can determine that the features of the second individual 606 have at least a threshold level of similarity with respect to the features included in the second subgroup feature profile 628 and the computational biology system 102 can also determine that the second individual 606 can be included in the second subgroup 618. Further, the computational biology system 102 can determine that the features of the third individual 608 can have at least a threshold level of similarity with respect to the features included in the third subgroup feature profile 630 and also determine that the third individual 608 can be included in the third subgroup 620. In one or more examples, the threshold levels of similarity can be determined as the maximum level of similarity between the individual and the subgroups. To illustrate, the computational biology system 102 can determine measures of similarity between features of the first individual 604 and the features of each subgroup 616, 618, 620. The computational biology system 102 can then determine the subgroup for which the first individual 604 has the maximum similarity metric, such as the first subgroup 616, with respect to the similarity metric of the other subgroups (e.g., the second subgroup 618 and the third subgroup 620) and determine that the first individual 604 is to be included in the first subgroup 616.

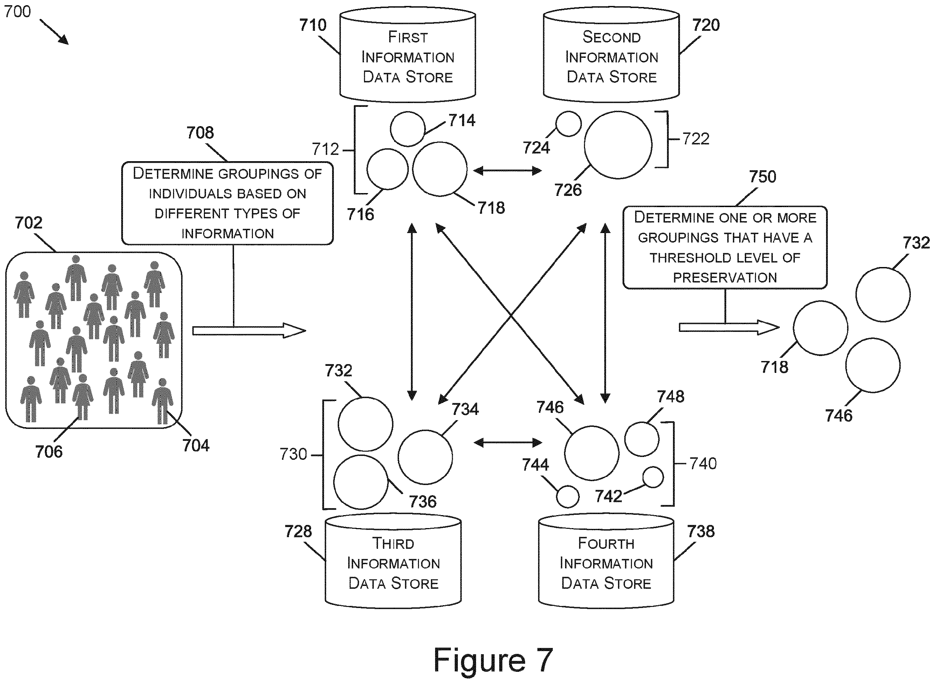

Figure 7 is an example framework to evaluate groupings of individuals that have been identified according to different types of information and determining a level of preservation among the groupings associated with the respective types of information, in accordance with one or more example implementations. In one or more implementations, the framework 700 can be implemented by the computational biology system 102 described herein. The framework 700 can include a number of individuals 702 that includes a first individual 704 and a second individual 706. The number of individuals 702 can have one or more features that correspond to different types of information. For example, the number of individuals 702 can have a number of genetic features and a number of phenotypic features. The number of individuals 702 can also have clinical features and biological pathway features. The framework 700 can include, at operation 708, determining groupings for the number of individuals 702 based on different types of information associated with the number of individuals 702. In one or more illustrative examples, the computational biology system 102 can obtain various types of information about the number of individuals 702 and determine one or more features of at least a portion of the number of individuals 702 based on the different types of information. The computational biology system 102 can then determine one or more groupings of the number of individuals 702 according to the respective types of information. In various examples, the groupings can be determined using one or more clustering techniques. To illustrate, the computational biology system 102 can determine groupings of the number of individuals 702 based on one or more types of information using at least one of a hierarchical-based clustering technique, partitioning-based clustering technique, density-based clustering technique, model-based clustering technique such as a latent class analysis technique, or a graph-based clustering technique.

In one or more illustrative examples, the framework 700 can include a first information data store 710 that can store a first type of information associated with the number of individuals 702. For example, the first information data store 710 can include genetic information related to at least a portion of the number of individuals 702. In these situations, the computational biology system 102 can determine a first set of groupings 712 for the number of individuals 702 based on the information stored by the first information data store 710. To illustrate, the computational biology system 102 can determine that the first set of groupings 712 includes a first grouping 714 having a first portion of the number of individuals 702, a second grouping 716 having a second portion of the number of individuals 702, and a third grouping 718 having a third portion of the number of individuals 702.

In situations where the first information data store 710 stores genetic information, the computational biology system 102 can determine that the first grouping 714 includes a first portion of the number individuals 702 having a first set of genetic features, that the second grouping 716 includes a second portion of the number of individuals 702 having a second set of genetic features, and that the third grouping 718 includes a third portion of the number of individuals 702 having a third set of genetic features. In various examples, the first set of groupings 712 can be determined based on DNA sequence information of at least a portion of the number of individuals 702. The DNA sequence information can indicate genetic variants of at least a portion of the number of individuals 702. In one or more implementations, the computational biology system 102 can determine the first set of groupings 712 based on at least one of common variants or rare variants of at least a portion of the number of individuals 702. Further, the first information data store 710 can store RNA sequence information of at least a portion of the number of individuals 702. In these scenarios, the computational biology system 102 can determine the first set of groupings 712 using RNA sequence information, such as transcriptomic information, mRNA sequence information, and the like.

The framework 700 can also include a second information data store 720 that can store a second type of information associated with at least a portion of the number of individuals 702. The second type of information can include biological pathway information. The biological pathway information can indicate levels of molecules produced in association with activation of one or more biological pathways. In addition, the biological pathway information can indicate levels of expression of genes associated with one or more biological pathways. Further, the biological pathway information can indicate one or more biological pathways that are disrupted in at least a portion of the number of individuals 702. The disruption of a biological pathway can be indicated by the levels of expression of one or more genes associated with the biological pathway or by the presence of rare and possibly damaging mutations in genes corresponding to the biological pathway. In various examples, the levels of expression of one or more genes associated with a biological pathway can be determined based on levels of one or more proteins produced by the expression of the one or more genes. The second information data store 720 can also store information indicating levels of one or more analytes for at least a portion of the number of individuals 702. The levels of the one or more analytes can be detected using one or more analytical techniques and/or one or more diagnostic techniques. For example, the second information data store 720 can store information indicating levels of one or more vitamins, levels of one or more minerals, levels of one or more metabolites, levels of one or more lipids, levels of one or more carbohydrates, levels of one or more proteins, or one or more combinations thereof.

The computational biology system 102 can determine a second set of groupings 722 for at least a portion of the number of individuals 702 based on the information stored by the second information data store 720. To illustrate, the computational biology system 102 can determine that the second set of groupings 722 includes a fourth grouping 724 having a fourth portion of the number of individuals 702 and a fifth grouping 726 having a fifth portion of the number of individuals 702. The computational biology system 102 can determine the second set of groupings 722 based on biological pathway information stored by the second information data store 720. For example, the computational biology system 102 can determine that the second set of groupings 722 is based on one or more disrupted pathways of at least a portion of the number of individuals 702. In these scenarios, the computational biology system 102 can determine that a portion of the number of individuals 702 included in the fourth grouping 724 has one or more first disrupted biological pathways and that an additional portion of the number of individuals 702 included in the fifth grouping 726 has one or more second disrupted biological pathways. Additionally, the computational biology system 102 can determine the second set of groupings 722 based on levels of one or more analytes stored by the second information data store 720. To illustrate, the computational biology system 102 can determine that a portion of the number of individuals 702 included in the fourth grouping 724 have first levels of one or more first analytes and that an additional portion of the number of individuals 702 included in the fifth grouping 726 have second levels of one or more second analytes.

Additionally, the framework 700 can include a third information data store 728 that can store a third type of information associated with the number of individuals 702. In one or more implementations, the third information data store 728 can store phenotypic information related to at least a portion of the number of individuals 702. The phenotypic information can indicate observable features of at least a portion of the number of individuals 702. For example, the phenotypic information can indicate visually observable features related to the appearance or behavior of at least a portion of the individuals. The phenotypic information can also indicate internal features of at least a portion of the number of individuals 702. In various examples, the phenotypic information can include classifications of one or more phenotypes included in a phenotype classification system. To illustrate, phenotypic information stored by the third information data store 728 can include classifications according to the Human Phenotype Ontology (HPO). The computational biology system 102 can determine a third set of groupings 730 of at least a portion of the number of individuals 702. The third set of groupings 730 can include a sixth grouping 732 that includes a sixth portion of the number of individuals 702, a seventh grouping 734 that includes a seventh portion of the number of individuals 702, and an eighth grouping 736 that includes an eighth portion of the number of individuals 702. In one or more implementations where the third information data store 728 includes phenotypic information, the computational biology system 102 can determine that the sixth grouping 732 includes a portion of the individuals 702 that have a first set of phenotypic features and that the seventh grouping 734 includes an additional portion of the individuals 702 that have a second set of phenotypic features. Further, the computational biology system 102 can determine that the eighth grouping 736 includes a portion of the individuals 702 that have a third set of phenotypic features. In one or more illustrative examples, the computational biology system 102 can determine that the sixth grouping 732 includes a portion of the number of individuals 702 associated with one or more first classifications of the HPO and that the seventh grouping 734 includes an additional portion of the number of individuals 702 associated with one or more second classifications of the HPO. The computational biology system 102 can also determine that the eighth grouping 736 includes a portion of the number of individuals 702 associated with one or more third classifications of the HPO.

Further, the framework 700 can include a fourth information data store 738 that can store a fourth type of information associated with at least a portion of the number of individuals 702. In one or more examples, the fourth type of information can include clinical data related to at least a portion of the number of individuals 702. The clinical data can include observations made by a healthcare practitioner. In one or more implementations, the clinical data can be obtained from medical records of at least a portion of the number of individuals 702. In various examples, the computational biology system 102 can determine a fourth set of groupings 740 for at least a portion of the number of individuals 702 based on clinical data stored by the fourth information data store 738. In the illustrative example of Figure 7, the computational biology system 102 can determine that the fourth set of groupings 740 can include a ninth grouping 742 that includes a ninth portion of the number of individuals 702, a tenth grouping 744 that includes a tenth portion of the number of individuals 702, an eleventh grouping 746 that includes an eleventh portion of the number of individuals 702, and a twelfth grouping 748 that includes a twelfth portion of the number of individuals 702. The fourth set of groupings 740 can each be associated with different sets of clinical features. To illustrate, the ninth grouping 742 can include a portion of the number of individuals 702 having a set of clinical features and the tenth grouping 744 can include a portion of the number of individuals 702 having an additional set of clinical features. Further, the eleventh grouping 746 can include a portion of the number of individuals 702 having another set of clinical features and the twelfth grouping 748 can comprise a portion of the number of individuals 702 having still a different set of clinical features.

In one or more implementations, the portion of the number of individuals 702 included in a grouping of a respective set of groupings can be different from the portion of the number of individuals 704 included in another grouping of the respective set of groupings. For example, the portion of the number of individuals 702 included in the first grouping 714 can be different from the portion of the number of individuals 702 included in the second grouping 716. Additionally, the portion of the number of individuals 702 included in the third grouping 718 can be different from the respective portions of the number of individuals 702 included in the first grouping 714 and the second grouping 716. In further examples, the portion of the number of individuals 702 included in the sixth grouping 732 can be different from the portion of the number of individuals 702 included in the seventh grouping 734 and the portion of the number of individuals 702 included in the eighth grouping 736 can be different from the respective portions of the number of individuals 702 included in the sixth grouping 732 and the seventh grouping 734. In one or more illustrative examples, the first individual 704 and the second individual 706 can be included in the first set of groupings 712 with the first individual 704 being in the first grouping 714 and the second individual 706 being in the third grouping 718.

In various examples, at least a portion of the number of individuals 702 included in one grouping of a respective set of groupings can be the same as at least a portion of the number of individuals 702 included in another grouping of a different set of groupings. To illustrate, at least one individual of the number of individuals 702 included in the fourth grouping 724 can also be included in the tenth grouping 744. In one or more illustrative examples, the first individual 704 can be included in the fourth grouping 724 and also in the tenth grouping 744. In one or more additional illustrative examples, the second individual 706 can be included in the second grouping 716, the fourth grouping 724, the seventh grouping 734, and the twelfth grouping 748.

The computational biology system 102 can determine preservation levels between the groupings determined based on information from at least a portion of the data stores 710, 720, 728, 738. For example, the computational biology system 102 can determine measures of correlation between the groupings included in at least a portion of the respective sets of groupings 712, 722, 730, 740. The measures of correlation can indicate a number of individuals that are common between the groupings included in at least a portion of the respective sets of groupings 712, 722, 730, 740. The measures of correlation can also indicate measures of similarity between individuals included within the groupings of the respective sets of groupings 712, 722, 730, 740. The computational biology system 102 can determine a level of preservation between at least a portion of the first set of groupings 712 and at least one of at least a portion of the second set of groupings 722, at least a portion of the third set of groupings 730, or at least a portion of the fourth set of groupings 740. Additionally, the computational biology system 102 can determine a level of preservation between at least a portion of the second set of groupings 722 and at least one of at least a portion of the first set of groupings 712, at least a portion of the third set of groupings 730, or at least a portion of the fourth set of groupings 740. Further, the computational biology system 102 can determine a level of preservation between at least a portion of the third set of groupings 730 and at least one of at least a portion of the first set of groupings 712, at least a portion of the second set of groupings 722, or at least a portion of the fourth set of groupings 740. The computational biology system 102 can also determine a level of preservation between at least a portion of the fourth set of groupings 740 and at least one of at least a portion of the first set of groupings 712, at least a portion of the second set of groupings 722, or at least a portion of the third set of groupings 730.

In various examples, the computational biology system 102 can determine a level of preservation for groupings determined based on information from different portions of the data stores by comparing the overlap of individuals in these groupings to the one expected by chance. The overlap expected by chance can be calculated by randomly selecting the same number of individuals and checking for the number of overlaps. This process is repeated multiple times to ensure an accurate estimate of the *p-value.*

The framework 700 can include, at operation 750, determining one or more groupings that have at least a threshold level of preservation across one or more types of information. In scenarios where a z statistic is calculated for a grouping, in one or more illustrative examples, a z statistic of less than 2 can indicate no preservation of the grouping across different data types, a z statistic between 2 and 10 can indicate relatively weak to moderate preservation of the grouping across different data types, and a z statistic greater than 10 can indicate a relatively strong preservation of the grouping across different data types. In various examples, the computational biology system 102 can determine one or more groupings that have a z statistic greater than 10. In the illustrative example of Figure 7, the third grouping 718, the sixth grouping 732 and the eleventh grouping 746 can be associated with the threshold level of preservation. For example, the respective portions of the number of individuals 702 included in the groupings 718, 732, and 746 can have at least a threshold amount of similarity. That is, the individuals included in the third grouping 718 determined using information stored by the first information data store 710 has at least a threshold number of the same individuals as the individuals included in the sixth grouping 732 determined using information stored by the third information data store 728 and at least a threshold number of the same individuals as the individuals included in the eleventh grouping 746 determined from the information stored by the fourth information data store 738.

In additional implementations, the computational biology system 102 can determine that a grouping has a threshold level of preservation by analyzing features shared between individuals included in a respective grouping in relation to the features shared with individuals included in additional groupings. In various examples, the computational biology system 102 can determine a p-value based on the analysis between features of individuals included in a respective grouping and features of individuals included in additional groupings. In one or more examples, the computational biology system 102 can perform an analysis using Fisher's exact test followed by one or more multiple test correction techniques. In one or more illustrative examples, the computational biology system 102 can determine a p-value for the individuals included in the first grouping 714 with respect to individuals included in at least one of the additional groupings 716, 718, 724, 726, 732, 734, 736, 742, 744, 746, 748. Additionally, the computational biology system 102 can determine a probability of individuals included in a respective grouping sharing one or more features with individuals included in a population, such as a control population. In one or more examples, the control population is defined by healthy individuals. In one or more further examples, the control population is defined by patients that do not have certain neurodevelopmental disorders, such as ASD and/or one or more co-occurring conditions. In these scenarios, the computational biology system 102 can also perform an analysis using Fisher's exact test followed by one or more multiple test correction techniques. For example, the computational biology system 102 can determine a probability that one or more features of individuals included in the first grouping 714 are also present in a population of individuals that does not include the number of individuals 702 in which a neurodevelopmental condition is present.

In various examples, the computational biology system 102 can determine that the probability of one or more features of individuals included in the first grouping are also present in a population of individuals in which the neurodevelopmental condition is not present is less than a threshold probability. In these scenarios, the computational biology system 102 can determine that a level of preservation of the first grouping 714 is greater than the threshold level of preservation. Further, the computational biology system 102 can determine that the probability of one or more features of individuals included in the first grouping are also present in a population of individuals in which the neurodevelopmental condition is not present is at least a threshold probability. In these situations, the computational biology system 102 can determine that a level of preservation of the first grouping 714 is less than the threshold level of preservation.

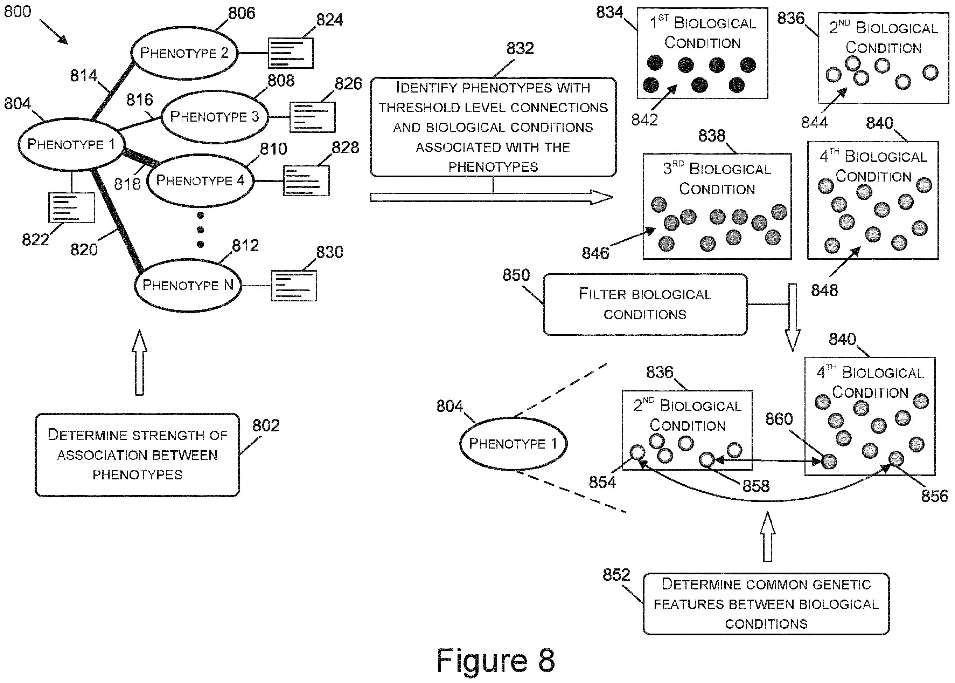

Figure 8 is a diagram of an example framework 800 to determine one or more biological conditions that characterize a group of individuals in which a neurodevelopmental condition is present and to determine a group of genes that is common to the one or more biological conditions, in accordance with one or more example implementations. The framework 800 can include determining a strength of association between phenotypes that can be present in individuals in which a neurodevelopmental condition is present. In one or more implementations, the computational biology system 102 described herein can perform at least a portion of the operations described in relation to the framework 800.

In the illustrative example of Figure 8, at operation 802, a strength of association can be determined between a number of phenotypes. For example, the framework 800 can include a first phenotype 804, a second phenotype 806, a third phenotype 808, a fourth phenotype 810, up to an Nth phenotype 812. The phenotypes 804, 806, 808, 810, 812 can each correspond to one or more indicators of at least one biological condition. The phenotypes 804, 806, 808, 810, 812 can be related to observable features of individuals for which a biological condition is present. To illustrate, the phenotypes 804, 806, 808, 810, 812 can correspond to symptoms, levels of analytes, biological features that can be detected using one or more diagnostic techniques, structural aspects of external and/or internal anatomical features, one or more combinations thereof, and so forth.

The illustrative example of Figure 8 indicates relative strengths of association between the first phenotype 804 and additional phenotypes, such as the second phenotype 806, the third phenotype 808, the fourth phenotype 810, up to the Nth phenotype 812. For example, the framework 800 includes a first strength of association 814 between the first phenotype 804 and the second phenotype 806. The framework 800 also includes a second strength of association 816 between the first phenotype 804 and the third phenotype 808. Additionally, the framework 800 includes a third strength of association 818 between the first phenotype 804 and the fourth phenotype 810 and a fourth strength of association 820 between the first phenotype 804 and the Nth phenotype 812. The thickness of the respective lines connecting the first phenotype 804 to the additional phenotypes 806, 808, 810, 812 can be an indicator of the strength of association between the first phenotype 804 and the additional phenotypes 806, 808, 810, 812. To illustrate, the third strength of association 818 between the first phenotype 804 and the fourth phenotype 810 can be greater than the second strength of association 816 between the first phenotype 804 and the third phenotype 808. In an additional illustrative example, the first strength of association 814 between the first phenotype 804 and the second phenotype 806 can be greater than the second strength of association 816 between the first phenotype 804 and the third phenotype 808.

The respective phenotypes 804, 806, 808, 810, 812 can each be associated with a number of items that can be used to determine the strength of association between a given phenotype 804, 806, 808, 810, 812 and at least one additional phenotype. The number of items that can be used by the computational biology system 102 to determine a strength of association between a phenotype and one or more additional phenotypes can include at least one of one or more genes, one or more genetic variants, one or more biological pathways, one or more symptoms, one or more anatomical features, one or more morphological features, one or more clinical observations, one or more clinical diagnoses, one or more publications, one or more individuals, or one or more molecules bound by the therapeutic used to treat a biological condition that is a target of a treatment of a biological condition. In the illustrative example of Figure 8, the first phenotype 804 can be related to a first set of items 822 and the second phenotype 806 can be related to a second set of items 824. Additionally, the third phenotype 808 can be related to a third set of items 826, the fourth phenotype 810 can be related to a fourth set of items 828, and the Nth phenotype 812 can be related to an Nth set of items 830.

The computational biology system 102 can analyze a set of items related to one phenotype with respect to an additional set of items related to an additional phenotype to determine the strength of association between the phenotypes. For example, the computational biology system 102 can determine a strength of association between the first phenotype 804 and the second phenotype 806 by analyzing the first set of items 822 with respect to the second set of items 824. In one or more illustrative examples, the computational biology system 102 can determine a first number of items included in the first set of items 822 that are not included in the second set of items 824. The computational biology system 102 can also determine a second number of items included in the second set of items 824 that are not included in the first set of items 822. Further, the computational biology system 102 can determine a third number of items that are not included in the first set of items 804 or in the second set of items 806, but are included in at least one additional set of items, such as the third set of items 826, the fourth set of items 828, up to the Nth set of items 830. In one or more implementations, the computational biology system 102 can determine the first strength of association 814 between the first phenotype 804 and the second phenotype 806 based on the first number of items, the second number of items, and the third number of items.

The framework 800 can also include, at operation 832, identifying phenotypes with threshold level connections and identifying biological conditions associated with the phenotypes. In one or more implementations, the computational biology system 102 can determine that the first phenotype 804 has at least a threshold strength of association with one or more additional phenotypes. The computational biology system 102 can also determine that the one or more additional phenotypes having at least a threshold strength of association with the first phenotype 804 are associated with one or more biological conditions. In the illustrative example of Figure 8, the computational biology system 102 can determine that a first biological condition 834, a second biological condition 836, a third biological condition 838, and a fourth biological condition 840 are associated with one or more phenotypes having at least a threshold strength of association with the first phenotype 804. The biological conditions 834, 836, 838, 840 can respectively correspond to at least one of a disease, a neurological condition, a physiological abnormality, a psychological condition, a developmental disability, or an anatomical abnormality. In one or more illustrative examples, the first phenotype 804 can be related to a neurodevelopmental condition, such as autism spectrum disorder, and the additional phenotypes 806, 808, 810, 812 can be related to one or more additional biological conditions. In these situations, by identifying biological conditions that are related to a phenotype corresponding to the neurodevelopmental condition, the computational biology system 102 can generate a network of biological conditions that are associated with the neurodevelopmental condition.

Additionally, the biological conditions 834, 836, 838, 840 can be related to one or more genetic features. To illustrate, a biological condition can be related to one or more genetic features that can be present in individuals in which the biological condition is present. The one or more genetic features can include at least one of a gene, a genetic variant, a single nucleotide variant, a copy number variant, a gene insertion, a gene deletion, or an inversion of a location of a gene. In one or more illustrative examples, the first biological condition 834 can be related to a first set of genetic features 842 and the second biological condition 836 can be related to a second set of genetic features 844. Further, the third biological condition 838 can be related to a third set of genetic features 846 and the fourth biological condition 840 can be related to a fourth set of genetic features 848. In various examples, the genetic features that correspond to the respective biological conditions 834, 836, 838, 840 can be related to one or more phenotypes present in individuals in which a respective biological condition 834, 836, 838, 840 is present.

In one or more implementations, the framework 800 can include an operation 850 that comprises filtering a number of biological conditions. In the illustrative example of Figure 8, the computational biology system 102 can filter the group of biological conditions that includes at least the first biological condition 834, the second biological condition 836, the third biological condition 838, and the fourth biological condition 840. The computational biology system 102 can filter the biological conditions 834, 836, 838, 840 according to one or more criteria. In one or more scenarios, the biological conditions 834, 836, 838, 840 can include one or more behavioral conditions. For example, at least one of the biological conditions 834, 836, 838, 840 can include depression, mania, drug addiction, schizophrenia, and so forth. In these situations, the computational biology system 102 can remove the biological conditions that correspond to behavioral conditions such that a group of non-behavioral conditions is identified by the computational biology system 102. In one or more illustrative examples, the computational biology system 102 can filter the biological conditions 834, 836, 838, 840 based on one or more criteria to identify a group of filtered biological conditions that includes the second biological condition 836 and the fourth biological condition 840. In various examples, the second biological condition 836 and the fourth biological condition 840 can include non-behavioral conditions related to a neurodevelopmental condition, such as autism spectrum disorder. In one or more examples, the second biological condition 836 and the fourth biological condition 840 can be related to seizures, epilepsy, speech delay, allodynia, hyperalgesia, and the like.

At operation 852, the framework 800 can include determining one or more common genetic features between a number of biological conditions. For example, the computational biology system 102 can determine one or more genetic features that are present with respect to both the second biological condition 836 and the fourth biological condition 840. To illustrate, the computational biology system 102 can determine that the second biological condition 836 includes a first genetic feature 854 that corresponds to a second genetic feature 856 of the fourth biological condition 840. Additionally, the computational biology system 102 can determine that the second biological condition 836 includes a third genetic feature 858 that corresponds to the fourth genetic feature 860. By determining the genetic features that are common between biological conditions that are related to a given phenotype, the computational biology system 102 can determine a genetic profile for the phenotype. In one or more illustrative examples, the computational biology system 102 can determine that a genetic profile of the first phenotype 804 can include first genetic features 854, 856 and second genetic features 858, 860 by determining that the second biological condition 836 and the fourth biological condition 840 are related to the first phenotype 804 based on the second biological condition 836 and the fourth biological condition 840 being associated with phenotypes that have at least a threshold strength of association with the first phenotype 804. In this way, at least a portion of the genetic profile of a phenotype can be determined using genetic data related to biological conditions associated with the phenotype. Thus, the computational biology system 102 can determine one or more portions of a genetic profile of a phenotype in the absence of direct information specifically indicating one or more genetic features related to the phenotype.

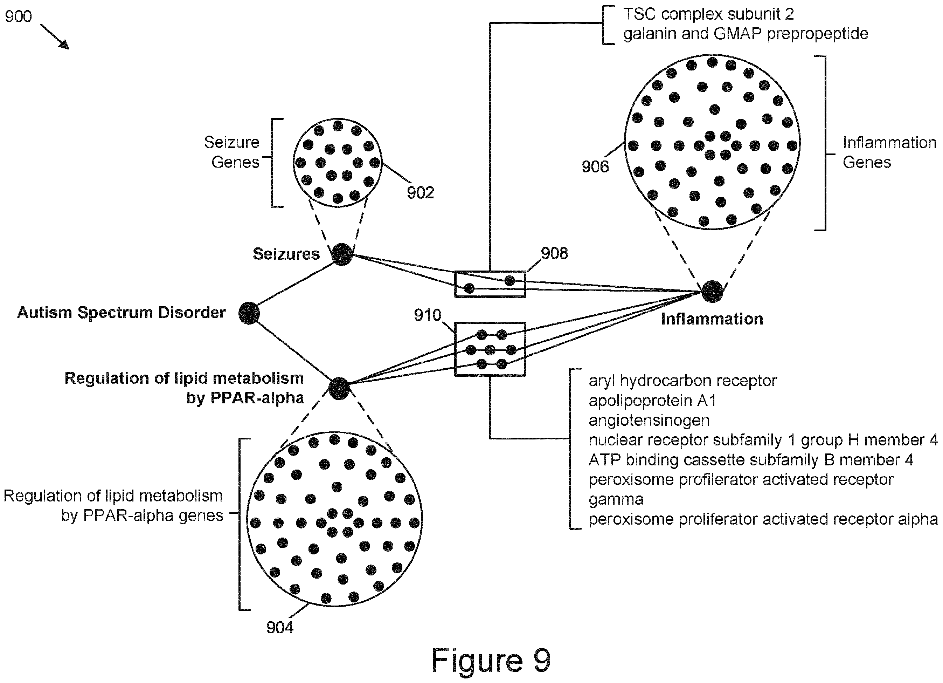

Figure 9 is a diagram of an example framework 900 to determine genes of individuals in which autism spectrum disorder is present, where the genes are common to individuals having biological conditions that include seizures, inflammation, and regulation of lipid metabolism by PPAR-alpha, in accordance with one or more example implementations. The framework 900 indicates that the biological condition autism spectrum disorder is related to the additional biological conditions of seizures and regulation of lipid metabolism by PPAR-alpha. The framework 900 also indicates that the biological condition of inflammation is related to autism spectrum disorder, seizures, and regulation of lipid metabolism by PPAR-alpha.

The framework 900 can include a first set of genetic features 902 that corresponds to the biological condition of seizures and a second set of genetic features 904 that corresponds to the biological condition regulation of lipid metabolism by PPAR-alpha. Additionally, the framework 900 can include a third set of genetic features 906 that corresponds to the biological condition of inflammation. In the illustrative example of Figure 9, the framework 900 can include a first common group of genetic features 908 that includes genetic features that are included in both the first set of genetic features 902 related to seizures and the third set of genetic features 906 related to inflammation. Further, the framework 900 can include a second common group of genetic features 910 that includes genetic features that are included in both the second set of genetic features 904 related to the regulation of lipid metabolism by PPAR-alpha and the third set of genetic features 906 related to inflammation.

At least a portion of a genetic profile of autism spectrum disorder can be produced by identifying the genetic features that are common to the biological conditions that are related to autism spectrum disorder. For example, both seizures and inflammation can be related to autism spectrum disorder and, in these scenarios, a genetic profile for autism spectrum disorder can include the first common group of genetic features 908 that includes genetic features that are associated with seizures and inflammation. Additionally, inflammation and regulation of lipid metabolism by PPAR-alpha can also be related to autism spectrum disorder, and the genetic profile for autism spectrum disorder can include the second common group of genetic features 910 that are associated with both inflammation and regulation of lipid metabolism by PPAR-alpha.

In one or more implementations, the identification of the first common group of genetic features 908 and the second common group of genetic features 910 in the genetic profile of autism spectrum disorder can be performed, at least in part, by one or more of the operations described with respect to the framework 800 of Figure 8. For example, the computational biology system 102 can determine that autism spectrum disorder is related to seizures by determining that one or more phenotypes of autism spectrum disorder and one or more phenotypes of seizures have at least a threshold strength of association. Additionally, the computational biology system 102 can determine that autism spectrum disorder and the regulation of lipid metabolism by PPAR-alpha can be related by determining that one or more phenotypes of autism spectrum disorder and one or more phenotypes of the regulation of lipid metabolism by PPAR-alpha have at least a threshold strength of association. Further, the computational biology system 102 can determine that autism spectrum disorder is related to inflammation based on one or more phenotypes related to seizures and one or more phenotypes related to regulation of lipid metabolism by PPAR-alpha have at least a threshold strength of association with one or more phenotypes of inflammation. In this way, the computational biology system 102 can determine a network of biological conditions that correspond to autism spectrum disorder that includes seizures, regulation of lipid metabolism by PPAR-alpha, and inflammation. Also, the computational biology system 102 can use the network of biological conditions related to autism spectrum disorder to identify the genetic features that are common to a number of the biological conditions included in the network of biological conditions to determine a number of genetic features that can correspond to autism spectrum disorder.

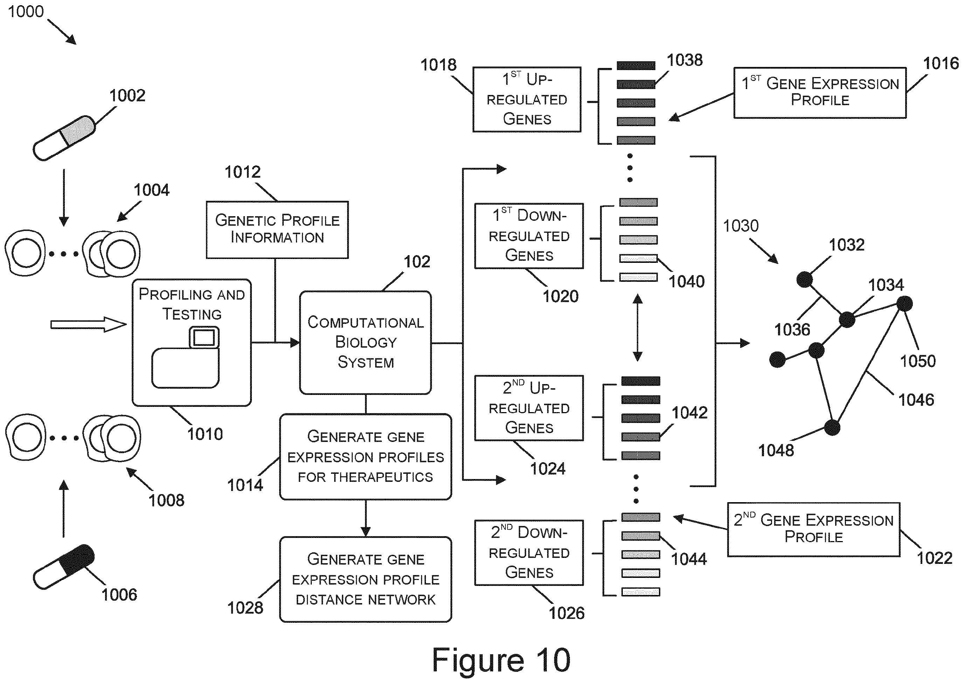

Figure 10 is a diagram of an example framework 1000 to determine gene expression profiles indicating up-regulation and down-regulation of a number of genes for a plurality of therapeutics, in accordance with one or more example implementations. The framework 1000 can include a first therapeutic 1002 that can interact with first cells 1004. The first cells 1004 can be derived from one or more cells obtained from individuals in which one or more first biological conditions is present. In various implementations, the first therapeutic 1002 can interact with a first cell line that includes the first cells 1004. Additionally, the framework 1000 can include a second therapeutic 1006 that can interact with second cells 1008. The second cells 1008 can be derived from one or more cells obtained from individuals in which one or more second biological conditions are present that are different from the one or more first biological conditions, or from individuals in which no biological conditions are determined (a.k.a healthy individuals). In one or more implementations, the second therapeutic 1006 can interact with a second cell line that includes the second cells 1008, where the second cell line is different from the first cell line. In one or more illustrative examples, the first cells 1004 can be included in a first human cancer cell line and the second cells 1008 can be included in a second human cancer cell lines. For example, the first cells 1004 and the second cells 1008 can be included in, respectively, a Michigan Center Foundation- 7 (MCF-7) cell line, a prostate cancer-3 (PC-3) cell line, a human leukemia-60 (HL-60) cell line, or an SK-MEL-5 cell line, a neural progenitor (NPC) cell line, or a lymphoblastoid (LCL) cell line. Although the illustrative example of Figure 10 includes a first therapeutic 1002 and a second therapeutic 1006, the framework 1000 can include more therapeutics, such as hundreds, up to thousands, or even up to millions of therapeutics. Further, although the illustrative example of Figure 10 includes first cells 1004 having first characteristics and second cells 1008 having second characteristics, the framework 1000 can include one hundred or more different type of cells or cell lines having different characteristics. Thus, the framework 1000 can include and/or be used to obtain information corresponding to the interaction of hundreds, up to thousands of therapeutics with hundreds, up to thousands of different types of cells and/or cell lines.

The framework 1000 can, at operation 1010, include profiling and testing of the first cells 1004 after interacting with the first therapeutic 1002 and profiling and testing of the second cells 1008 after interacting with the second therapeutic 1006. In one or more illustrative examples, the profiling and testing of operation 1010 can include determining the expression of a number of genes of the first cells 1004 after interacting with the first therapeutic 1002 and/or the expression of a number of genes of the second cells 1008 after interacting with the second therapeutic 1006 using one or more DNA microarray techniques. Additionally, the profiling and testing of operation 1010 can include performing one or more RNA sequencing operations to determine the expression of a number of genes of the first cells 1004 after interacting with the first therapeutic 1002 and/or the expression of a number of genes of the second cells 1008 after interacting with the second therapeutic 1006.

The profiling and testing of the first cells 1004 and the second cells 1008 can generate genetic profile information 1012. The genetic profile information 1012 can indicate nucleic acid sequences of genes of the first cells 1004 and the second cells 1008. In one or more examples, the genetic profile information 1012 can include one or more DNA sequences derived from at least one of the first cells 1004 after interacting with the first therapeutic 1002 or the second cells 1008 after interacting with the second therapeutic 1006. In various examples, the genetic profile information 1012 can include nucleic acid sequences of complementary DNA (cDNA). Additionally, the genetic profile information 1012 can include one or more RNA sequences derived from at least one of the first cells 1004 after interacting with the first therapeutic 1002 or the second cells 1008 after interacting with the second therapeutic 1006. For example, the genetic profile information 1012 can include transcriptomic data indicating nucleic acid sequences of mRNA. In various examples, the genetic profile information 1012 related to the first cells 1004 can indicate that the expression of one or more genes included in the first cells 1004 is modified by the first therapeutic 1002. Further, the genetic profile information related to the second cells 1008 can indicate that the expression of one or more genes included in the second cells 1008 is modified by the second therapeutic 1006.

The framework 1000 can include the computational biology system 102 and the computational biology system 102 can obtain the genetic profile information 1012 from one or more data sources. For example, the computational biology system 102 can obtain the genetic profile information 1012 from one or more databases that are in communication with, or otherwise electronically accessible to, the computational biology system 102. In various examples, at least a portion of the genetic profile information 1012 can be obtained from a database maintained by one or more third parties. To illustrate, the computational biology system 102 can obtain at least a portion of the genetic profile information 1012 from the Connectivity Map (cMap) database. In one or more additional illustrative examples, the computational biology system 102 can obtain at least a portion of the genetic profile information 1012 from Library of Integrated Network-Based Cellular Signatures (LINCS) L 1 000 database.

The framework 1000 can include, at operation 1014, the computational biology system 102 generating one or more gene expression profiles for one or more therapeutics. The gene expression profiles can indicate modifications to gene expression caused by interaction with a therapeutic. The modifications to gene expression caused by interaction with a therapeutic can be determined based on changes to the production of one or more molecules in cells that interact with and/or regulated by the therapeutic. For example, a measure of expression of a gene can be determined based on an amount of one or more proteins (encoded by the gene) produced by one or more cells. In one or more additional examples, a measure of expression of a gene can be determined based on an amount of RNA molecules corresponding to the gene produced by one or more cells. As used herein, the term "expression molecule" can correspond to a molecule that is being quantified to determine an amount of expression of a gene.

In one or more examples, the gene expression profiles can indicate measures of differences between the expression of genes in cells that have interacted with a therapeutic and the expression of genes in cells that have not interacted with (but regulated by) a therapeutic. In these scenarios, the differences between measures of expression of the genes can be determined by determining amounts of one or more expression molecules produced by cells that have interacted with the therapeutic in relation to the amounts of the one or more expression molecules produced by cells that have not interacted with the therapeutic. In one or more implementations, up-regulation of a gene with respect to a therapeutic can correspond to a greater number of expression molecules being produced by cells that have interacted with the therapeutic in relation to the number of expression molecules produced by cells that have not interacted with the therapeutic. Measures of up-regulation for genes can increase as the number of expression molecules increases with respect to genes of cells that have interacted with a therapeutic relative to production of expression molecules with respect to genes of cells that have not interacted with the therapeutic. Additionally, down-regulation of a gene can correspond to fewer expression molecules being produced by cells that have interacted with the therapeutic in relation to the number of expression molecules produced by cells that have not interacted with the therapeutic. Measures of down-regulation for genes can increase as the number of expression molecules decreases with respect to genes of cells that have interacted with a therapeutic relative to production of expression molecules with respect to genes of cells that have not interacted with the therapeutic.

In addition, the gene expression profiles can indicate measures of difference between the expression of genes in cells of individuals diagnosed with a biological condition that have interacted with a therapeutic and the expression of genes in cells of individuals that have not been diagnosed with the biological condition that have interacted with the therapeutic. In these situations, the differences between measures of expression of the genes can be determined by determining amounts of one or more expression molecules produced by cells of one or more individuals in which a biological condition is present and that have interacted with the therapeutic in relation to amounts of the one or more expression molecules produced by cells of one or more individuals in which the biological condition is not present and that have interacted with the therapeutic. In various examples, up-regulation of a gene with respect to a therapeutic can correspond to a greater number of expression molecules being produced by cells of one or more individuals in which a biological condition is present and that have interacted with the therapeutic in relation to the number of expression molecules produced by cells of one or more individuals in which the biological condition is not present and that have interacted with the therapeutic. Measures of up-regulation for genes can increase as the number of expression molecules increases with respect to genes of cells of one or more individuals in which a biological condition is present and that have interacted with a therapeutic relative to production of expression molecules with respect to genes of cells of one or more individuals in which the biological condition is not present and that have not interacted with the therapeutic. Further, down-regulation of a gene with respect to a therapeutic can correspond to fewer expression molecules being produced by cells of one or more individuals in which a biological condition is present and that have interacted with the therapeutic in relation to the number of expression molecules produced by cells of one or more individuals in which the biological condition is not present and that have interacted with the therapeutic. Measures of down-regulation for genes can increase as the number of expression molecules decreases with respect to genes of cells of one or more individuals in which a biological condition is present and that have interacted with a therapeutic relative to production of expression molecules with respect to genes of cells of one or more individuals in which the biological condition is not present and that have not interacted with the therapeutic.

In the illustrative example of Figure 10, the computational biology system 102 can produce a first gene expression profile 1016 for first therapeutic 1002. The first gene expression profile 1016 can indicate, for at least a portion of the genes included in the first cells 1004, a measure of up-regulation or a measure of down-regulation for respective genes of the first cells 1004 based on interaction with the first therapeutic 1002. In one or more implementations, the first gene expression profile 1016 can include a ranked list of genes included in the first cells 1004 based on the measures of expression for respective genes of the first cells 1004 in response to interaction with the first therapeutic 1002. In various examples, the first gene expression profile 1016 can indicate first up-regulated genes 1018 and first down-regulated genes 1020. In one or more examples, a number of genes included in the first up-regulated genes 1018 can include from about 10 genes to about 1000 genes, from about 50 genes to about 500 genes, from about 100 genes to about 300 genes, or from about 225 genes to about 275 genes. In one or more illustrative examples, the first up-regulated genes 1018 can include genes of the first cells 1004 having the highest 250 measures of up-regulation in response to interaction with the first therapeutic 1002. Additionally, a number of genes included in the first down-regulated genes 1020 can include from about 10 genes to about 1000 genes, from about 50 genes to about 500 genes, from about 100 genes to about 300 genes, or from about 225 genes to about 275 genes. The first down-regulated genes 1020 can include genes of the first cells 1004 having the highest 250 measures of down-regulation in response to interaction with the first therapeutic 1002. In this way, the first gene expression profile 1016 can indicate the genes of the first cells 1004 with expression levels that are most impacted by the first therapeutic 1002 with respect to either increases in the production of expression molecules by the first cells 1004 in response to interaction with the first therapeutic 1002 or decreases in the production of expression molecules by the first cells 1004 in response to interaction with the first therapeutic 1002.

The computational biology system 102 can also produce a second gene expression profile 1022 for the second therapeutic 1006. The second gene expression profile 1022 can indicate, for at least a portion of the second cells 1008, a measure of up-regulation or a measure of down-regulation for respective genes of the second cells 1008 based on interaction with the second therapeutic 1006. In one or more implementations, the second gene expression profile 1022 can include a ranked list of genes included in the second cells 1008 based on the measures of expression for respective genes of the second cells 1008 in response to interaction with the second therapeutic 1006. The second gene expression profile 1022 can include second up-regulated genes 1024 and second down-regulated genes 1026. A number of genes included in each of the second up-regulated genes 1024 and the second down-regulated genes 1026 can include from about 10 genes to about 1000 genes, from about 50 genes to about 500 genes, from about 100 genes to about 300 genes, or from about 225 genes to about 275 genes. In one or more illustrative examples, the second up-regulated genes 1024 can include genes of the second cells 1008 having the highest 250 measures of up-regulation in response to interaction with the second therapeutic 1006. The second down-regulated genes 1026 can include genes of the second cells 1008 having the highest 250 measures of down-regulation in response to interaction with the second therapeutic 1006.

The computational biology system 102 can also, at operation 1028, generate at least a portion of a gene expression profile distance network 1030. The gene expression profile distance network 1030 can include a plurality of nodes and a plurality of edges connecting the nodes. For example, the gene expression profile distance network 1030 can include a first node 1032 and a second node 1034 that are connected by an edge 1036. The respective nodes included in the gene expression profile distance network 1030 can correspond to a therapeutic. Additionally, the edges included in the gene expression profile distance network 1030 can indicate differences between the gene expression profiles of respective therapeutics. In one or more implementations, the greater a distance between a pair of nodes within the gene expression profile distance network 1030, the greater the difference between the gene expression profiles of the pair of nodes. Further, although the illustrative example of Figure 10 shows six nodes and six edges of the gene expression profile distance network 1030, the gene expression profile distance network 1030 can include hundreds, up to thousands of nodes and thousands up to millions of edges. In one or more illustrative examples, the first node 1032 can correspond to the first therapeutic 1002, the second node 1034 can correspond to the second therapeutic 1006, and the edge 1036 can correspond to a difference between the first gene expression profile 1016 and the second gene expression profile 1022.

The computational biology system 102 can generate the gene expression profile distance network 1030 by analyzing the gene expression profiles of a plurality of therapeutics in relation to one another. In various examples, for a given pair of therapeutics, the computational biology system 102 can determine a distance between the gene expression profiles of the respective therapeutics by analyzing measures of up-regulation and down-regulation for one or more group of genes affected by the respective therapeutics. In one or more illustrative examples, the computational biology system 102 can analyze the first up-regulated genes 1018 with respect to the second up-regulated genes 1024 and the computational biology system 102 can analyze the first down-regulated genes 1020 with respect to the second down-regulated genes 1026. For example, the computational biology system 102 can determine a first profile similarity metric indicating a similarity between the first gene expression profile 1016 and the second gene expression profile 1022 by identifying individual genes included in the first up-regulated genes 1018 that are also included in the second up-regulated genes. To illustrate, the computational biology system 102 can identify a first gene 1038 of the first up-regulated genes 1018 and determine whether the first gene 1038 is included in the second up-regulated genes 1024. In situations where the first gene 1038 is included in the second up-regulated genes 1024, the computational biology system 102 can adjust the first profile similarity metric. Additionally, the computational biology system 102 can determine the first profile similarity metric by identifying the second gene 1040 included in the first down-regulated genes 1020 and determining whether the second gene 1040 is included in the second down-regulated genes 1026. In scenarios where the second gene 1040 is included in the second down-regulated genes 1026, the computational biology system 102 can adjust the first profile similarity metric. In one or more implementations, the computational biology system 102 can determine the first profile similarity metric by analyzing each gene included in the first up-regulated genes 1018 with respect to the group of second up-regulated genes 1024 and by analyzing each gene included in the first down-regulated genes 1020 with respect to the group of second down-regulated genes 1026.

In one or more illustrative examples, for each gene of the first up-regulated genes 1018 included in the second up-regulated genes 1024 and for each gene of the first down-regulated genes 1020 included in the second down-regulated genes 1026, a value of the similarity metric can be increased. Also, for each gene of the first up-regulated genes 1018 not included in the second up-regulated genes 1024 and for each gene of the first down-regulated genes 1020 not included in the second down-regulated genes 1026, a value of the similarity metric can be decreased. In various examples, the computational biology system 102 can determine the similarity metric between the first gene expression profile 1016 and the second gene expression profile 1022 based on an amount of overlap between a top 250 up-regulated genes of the first gene expression profile 1016 with respect to a top 250 up-regulated genes of the second gene expression profile 1022. Additionally, the computational biology system 102 can determine the similarity metric between the first gene expression profile 1016 and the second gene expression profile 1022 based on an amount of overlap between a bottom 250 down-regulated genes of the first gene expression profile 1026 with respect to a bottom 250 down-regulated genes of the second gene expression profile 1022.

The computational biology system 102 can also determine a second profile similarity metric indicating a similarity between the second gene expression profile 1022 and the first gene expression profile 1016. The computational biology system 102 can determine the second profile similarity metric by analyzing the second up-regulated genes 1024 with respect to the first up-regulated genes 1018 and by analyzing the second down-regulated genes 1026 with respect to the first down-regulated genes 1020. In one or more illustrative examples, the computational biology system 102 can determine the second profile similarity metric by identifying individual genes included in the second up-regulated genes 1026 that are also included in the first up-regulated genes 1018. To illustrate, the computational biology system 102 can identify a third gene 1042 of the second up-regulated genes 1024 and determine whether the third gene 1042 is included in the first up-regulated genes 1018. In situations where the third gene 1042 is included in the first up-regulated genes 1018, the computational biology system 102 can adjust the second profile similarity metric. Additionally, the computational biology system 102 can determine the second profile similarity metric by identifying a fourth gene 1044 included in the second down-regulated genes 1026 and determining whether the fourth gene 1044 is included in the first down-regulated genes 1020. In scenarios where the fourth gene 1044 is included in the first down-regulated genes 1020, the computational biology system 102 can adjust the second profile similarity metric. In one or more implementations, the computational biology system 102 can determine the second profile similarity metric by analyzing each gene included in the second up-regulated genes 1024 with respect to the group of first up-regulated genes 1018 and by analyzing each gene included in the second down-regulated genes 1026 with respect to the group of first down-regulated genes 1020.

In various examples, for each gene of the second up-regulated genes 1024 included in the first up-regulated genes 1018 and for each gene of the second down-regulated genes 1026 included in the first down-regulated genes 1020, a value of the similarity metric can be increased. Also, for each gene of the second up-regulated genes 1024 not included in the first up-regulated genes 1018 and for each gene of the second down-regulated genes 1026 not included in the first down-regulated genes 1020, a value of the similarity metric can be decreased. In various examples, the computational biology system 102 can determine the similarity metric between the second gene expression profile 1022 and the first gene expression profile 1016 based on an amount of overlap between a top 250 up-regulated genes of the second gene expression profile 1022 with respect to a top 250 up-regulated genes of the first gene expression profile 1016. Additionally, the computational biology system 102 can determine the similarity metric between the second gene expression profile 1022 and the first gene expression profile 1016 based on an amount of overlap between a bottom 250 down-regulated genes of the second gene expression profile 1020 with respect to a bottom 250 down-regulated genes of the first gene expression profile 1016.

The computational biology system 102 can determine a composite profile similarity metric by combining the first profile similarity metric and the second profile similarity metric. For example, an average of the first profile similarity metric and the second profile similarity metric can be determined. The profile similarity score can then be used to generate an edge in the gene expression profile distance network 1030. The length of the edge between the gene expression profile distance network 1030 can be related to a magnitude of the profile similarity score. For example, as the magnitude of the profile similarity scores increase for respective pairs of therapeutics, the distance between the respective pairs of therapeutics in the gene expression profile distance network 1030 decreases. In the illustrative example of Figure 10, a length of the edge 1036 between the first node 1032 and the second node 1034 of the gene expression profile distance network 1030 can be shorter than the length of the edge 1046 between the third node 1048 and the fourth node 1050 because the profile similarity score between the first therapeutic 1002 and the second therapeutic 1006 can be greater than the profile similarity score between a third therapeutic represented by the third node 1048 and a fourth therapeutic represented by the fourth node 1050 in the gene expression profile distance network 1030.

The computational biology system 102 can generate multiple gene expression profile distance networks. For example, the computational biology system 102 can obtain information used to produce gene expression profiles from a number of data sources. In various examples, the computational biology system 102 can generate a first gene expression profile distance network based on information obtained from one or more first data sources and a second gene expression profile distance network based on information obtained from one or more second data sources. In one or more illustrative examples, the computational biology system 102 can generate a first gene expression profile distance network based on information obtained from a Connectivity Map dataset and a second gene expression profile distance network based on information obtained from a LINCS L1000 dataset.

Further, the computational biology system 102 can also generate gene expression profile distance networks for additional causes of genetic expression modification. For example, the computational biology system 102 can generate gene expression profile distance networks for conditions that modify the expression of genes other than therapeutics. To illustrate, the computational biology system 102 can generate one or more gene expression profile distance networks for a number of biological conditions that modify the expression of genes in individuals in which one or more biological conditions of the number of biological conditions is present. In one or more additional illustrative examples, the computational biology system 102 can generate one or more gene expression profile distance networks for a number of short hairpin ribonucleic acid (shRNA) molecules.

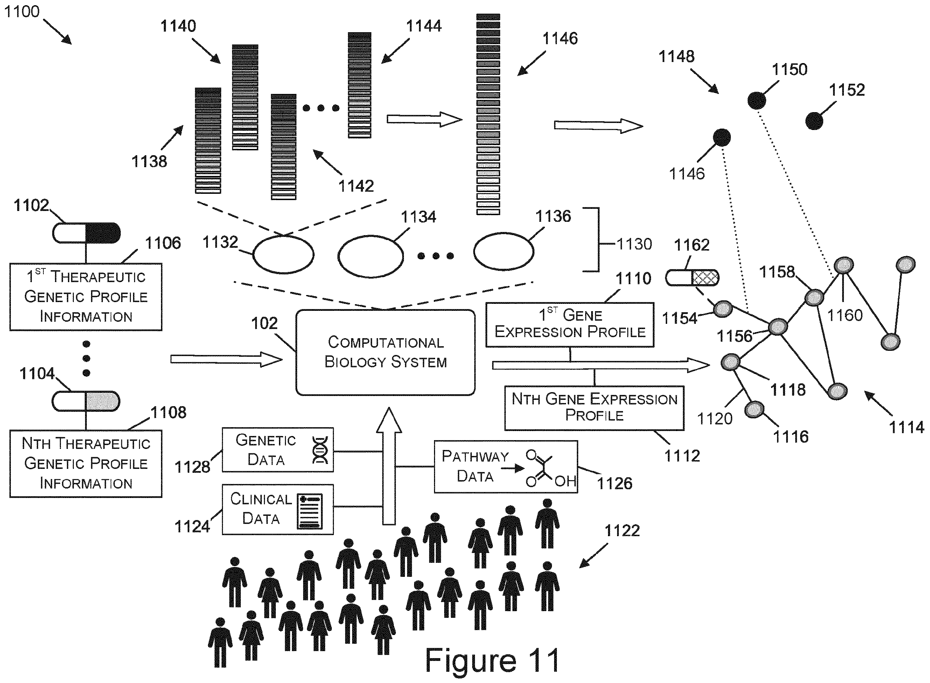

Figure 11 is a diagram illustrating an example framework 1100 to analyze characteristics of groups of individuals in which a neurodevelopmental condition is present and characteristics of a number of therapeutics to determine one or more of the therapeutics that can be used to treat the neurodevelopmental condition in one or more of the groups of individuals, in accordance with one or more example implementations. The framework 1100 can include the computational biology system 102 that can generate gene expression profiles for a number of groups of individuals in which a neurodevelopmental condition is present. Additionally, the computational biology system 102 can generate one or more gene expression profile distance networks indicating amounts of similarity between gene expression profiles of a number of therapeutics.

The framework 1100 can include a number of therapeutics that include a first therapeutic 1102 up to an Nth therapeutic 1104 with respective therapeutics being associated with genetic profile information. For example, the first therapeutic 1102 can be associated with first therapeutic genetic profile information 1106 and the Nth therapeutic 1104 can be associated with Nth therapeutic genetic profile information 1108. The genetic profile information can indicate measures of the expression of genes in one or more groups of cells that have interacted with a respective therapeutic. In one or more implementations, the genetic profile information can indicate measures of the expression of genes by one or more cells lines that have interacted with one or more therapeutics. The measures of expression of genes can be related to the production of one or more expression molecules by the groups of cells that have interacted with one or more therapeutics. The computational biology system 102 can obtain the genetic profile information from a number of data sources.

The computational biology system 102 can determine gene expression profiles for a number of therapeutics based on the genetic profile information. The gene expression profiles can indicate the up-regulation and/or the down-regulation of genes in cells that have interacted with one or more therapeutics. In various examples, the gene expression profiles can include a ranked list of genes included in cells that have interacted with respective therapeutics based on amounts of up-regulation and/or amounts of down-regulation of the genes in response to interaction with the respective therapeutics. In one or more illustrative examples, the gene expression profiles can include a group of genes that have a threshold amount of up-regulation in response to interaction with respective therapeutics and a threshold amount of down-regulation in response to interaction with the respective therapeutics. For example, the gene expression profiles can indicate a top 250 up-regulated genes and a bottom 250 down-regulated genes in relation to interaction with one or more therapeutics. In one or more examples, the computational biology system 102 can produce the gene expression profiles in a manner that is at least similar to the one or more implementations described with respect to Figure 10. In one or more illustrative examples, the computational biology system 102 can produce a first gene expression profile 1110 that indicates the up-regulation and/or down-regulation of genes included in a number of cells that have interacted with the first therapeutic 1102. Additionally, the computational biology system 102 can produce an Nth gene expression profile 1112 that indicates the up-regulation and/or the down-regulation of genes included in a number of cells that have interacted with the Nth therapeutic 1104.

The computational biology system 102 can generate one or more gene expression profile distance networks based on the gene expression profiles. A gene expression profile distance network can include a number of nodes with each node corresponding to at least one therapeutic. Additionally, a gene expression profile distance network can include a number of edges connecting the respective nodes. The length of the edges included in the gene expression profile distance network can indicate an amount of difference between the gene expression profiles of the respective therapeutics that are connected by the edges. In the illustrative example of Figure 11, the computational biology system 102 can generate a gene expression profile distance network 1114 that includes a number of nodes, such as the first node 1116 and a second node 1118 that are connected by an edge 1120. In one or more examples, the computational biology system 102 can generate the gene expression profile distance network 1114 according to one or more implementations described with respect to Figure 10.

The framework 1100 can also include a number of individuals 1122. A neurodevelopmental condition can be present in the number of individuals 1122. For example, the number of individuals 1122 can be diagnosed with autism spectrum disorder. The computational biology system 102 can analyze information corresponding to the group of individuals 1122 and determine a number of subgroups of the group of individuals 1122. For example, the computational biology system 102 can obtain at least one of clinical data 1124, pathway data 1126, or genetic data 1128 corresponding to each individual included in the group of individuals 1122. The computational biology system 102 can analyze the information related to the group of individuals to determine a number of subgroups 1130 of the group of individuals 1122. The number of subgroups 1130 can include at least a first subgroup 1132, a second subgroup 1134, up to an Nth subgroup 1136. In one or more examples, the computational biology system 102 can determine the number of subgroups according to one or more implementations described with respect to Figure 6 and/or Figure 7.

The computational biology system 102 can generate additional gene expression profiles corresponding to individuals included in the number of subgroups 1130. For example, the computational biology system 102 can generate an additional gene expression profile for each individual included in the first subgroup 1132 and an additional gene expression profile for each individual included in the second subgroup 1134. Further, the computational biology system 102 can generate an additional gene expression profile for each individual included in the Nth subgroup 1136. In one or more illustrative examples, the computational biology system 102 can generate at least a first additional gene expression profile 1138 for a first individual included in the first subgroup 1132, a second additional gene expression profile 1140 for a second individual included in the first subgroup 1132, a third additional gene expression profile 1142 for a third individual included in the first subgroup 1132, up to an Nth additional gene expression profile 1144 for an Nth individual included in the first subgroup 1132. The computational biology system 102 can generate the additional gene expression profiles of individuals included in the respective subgroups 1130 by analyzing and/or obtaining transcriptomic information of the individuals included in the respective subgroups. The transcriptomic information can include RNA sequencing information. To illustrate, the transcriptomic information used to generate the additional gene expression profiles of individuals included in the respective subgroups 1130 can include at least one of messenger RNA information, ribosomal RNA information, transfer RNA information, or microRNA information.

The computational biology system 102 can also generate a consensus gene expression profile for each respective subgroup included in the number of subgroups 1130 based on the additional gene expression profiles of individuals included in the respective subgroups of the number of subgroups. For example, the computational biology system 102 can generate a consensus gene expression profile 1146 for the first subgroup 1132 based on the additional gene expression profiles of the individuals included in the first subgroup 1132. The consensus gene expression profile 1146 can include a ranked list of genes that are included in the additional gene expression profiles of the individuals included in the first subgroup 1132. To illustrate, the computational biology system 102 can rank the genes included in the consensus gene expression profile 1146 based on measures of up-regulation and measures of down-regulation of genes included in the additional gene expression profiles of individuals included in the first subgroup 1132. In various examples, a subset of the total number of genes included in the additional gene expression profiles of individuals included in the first subgroup 1132 are included in the consensus gene expression profile 1146.

The consensus gene expression profile 1148 can be generated by determining a weighted average across the gene expression profiles 1138, 1140, 1142, 1144. In one or more illustrative examples, pairwise combinations of the gene expression profiles 1138, 1140, 1142, 1144 can be generated by the computational biology system 102 to determine a number of merged profiles. In addition, a profile similarity score can be determined for each of the merged profiles. Further, the computational biology system 102 can, based on the profile similarity scores, determine an amount of correlation between each of the gene expression profiles 1138, 1140, 1142, 1144 with respect to one another to determine an amount of contribution of the genes of the respective gene expression profiles 1138, 1140, 1142, 1144 to the consensus gene expression profile 1146. The respective gene expression profiles 1138, 1140, 1142, 1144 that are most strongly correlated with respect to other gene expression profiles 1138, 1140, 1142, 1144 can contribute a greater number of genes to the consensus gene expression profile 1146. In one or more implementations, the computational biology system 102 can use the correlations between the gene expression profiles 1138, 1140, 1142, 1144 to rank the genes included in the respective gene expression profiles 1138, 1140, 1142, 1144 to determine a top 250 up-regulated genes and a bottom 250 down-regulated genes to include in the consensus gene expression profile 1146.

The computational biology system 102 can analyze a number of consensus gene expression profiles 1148 with respect to the gene expression profile distance network 1114 to determine candidate therapeutics for individuals included in subgroups of individuals that correspond to the respective consensus gene expression profiles 1148. In one or more illustrative examples, the number of consensus gene expression profiles 1148 can include the consensus gene expression profile 1146. The number of consensus gene expression profiles 1148 can also include a number of additional composite gene expression profiles generated by the computational biology system 102, such as a first additional composite gene expression profile 1150 that corresponds to the second subgroup 1134 and a second additional composite gene expression profile 1152 that corresponds to the Nth subgroup 1136. The computational biology system 102 can analyze the inverse of the consensus gene expression profiles 1148 related to the respective subgroups included in the number of subgroups 1130 with respect to the gene expression profiles of the therapeutics included in the gene expression profile distance network 1114 to determine one or more therapeutics that up-regulate genes that are down-regulated in individuals included in the respective subgroups of the number of subgroups 1130 and that up-regulate genes that are down-regulated in individuals included in the respective subgroups of the number of subgroups 1130.

In one or more implementations, the computational biology system 102 can determine a distance between the respective one of the consensus gene expression profiles 1148 and a node of the gene expression profile distance network 1114 that corresponds to a therapeutic. The distances between a respective one of the consensus gene expression profiles 1148 and one or more nodes of the gene expression profile distance network 1114 can be used by the computational biology system 102 to determine one or more therapeutics that can be candidate treatments for individuals included in the respective subgroup of individuals corresponding to the respective one of the consensus gene expression profiles 1148.

In one or more implementations, the computational biology system 102 can determine one or more nodes of the gene expression profile distance network 1114 having a minimum distance with respect to an inverted consensus gene expression profile 1148 or having less than a threshold distance with respect to an inverted one of the consensus gene expression profiles 1148. The nodes having less than the threshold distance can correspond to a candidate therapeutic for individuals included in the subgroup corresponding to one of the consensus gene expression profiles 1148. In various examples, the computational biology system 102 can determine a distribution of pairwise distances, such as profile similarity scores, of the therapeutics included in the gene expression profile distance network 1114 to determine a mean of the distribution and a standard deviation for the distribution. The computational biology system 102 can determine one or more candidate therapeutics corresponding to a consensus gene expression profile 1148 based on a threshold number of standard deviations from the mean for a particular pair of therapeutics having nodes included in the gene expression profile distance network 1114 in relation to an inverted consensus gene expression profile 1148. The threshold number of standard deviations can include no greater than about 3 standard deviations, no greater than about 2,5 standard deviations, no greater than about 2 standard deviations, no greater than 1.5 standard deviations, no greater than about 1 standard deviation, or no greater than about 0.5 standard deviations.

The distances between respective gene expression profiles 1148 from nodes of the gene expression profile distance network 1114 can correspond to respective probabilities of the therapeutics corresponding to the nodes being candidate treatments for one or more neurodevelopmental conditions present in individuals in respective subgroups related to the respective gene expression profiles 1148. In various examples, the computational biology system 102 can determine distances of a number of consensus gene expression profiles with respect to a number of nodes of the gene expression profile distance network 1114 to determine therapeutics having at least a threshold probability of being candidate therapeutics to treat individuals included in subgroups corresponding to the respective consensus gene expression profiles.

In one or more illustrative examples, the consensus gene expression profile 1146 can correspond to the first subgroup 1132 and the computational biology system 102 can determine a distance between an inverse of the consensus gene expression profile 1146 and a third node 1154 and a fourth node 1156 of the gene expression profile distance network 1114. In these scenarios, the computational biology system 102 can determine that the distance between the consensus gene expression profile 1146 and the third node 1154 is less than the distance between the consensus gene expression profile 1146 and the fourth node 1156. The computational biology system 102 can then determine that the probability of a therapeutic related to the third node 1154 being a candidate therapeutic to treat the neurodevelopmental condition in the first subgroup 1132 is greater than a probability of a therapeutic related to the fourth node 1156 being a candidate therapeutic to treat the neurodevelopmental condition in the first subgroup 1132.

Additionally, the first additional composite gene expression profile 1150 can correspond to the second subgroup 1134 and the computational biology system 102 can determine a distance between an inverse of the first additional composite gene expression profile 1154 and a fifth node 1158 and a sixth node 1160 of the gene expression profile distance network 1114. In one or more implementations, the computational biology system 102 can determine that the distance between the first additional composite gene expression profile 1150 and the sixth node 1160 is less than the distance between the first additional composite gene expression profile 1150 and the fifth node 1158. In these situations, the computational biology system 102 can determine that the probability of a therapeutic related to the sixth node 1160 being a candidate therapeutic to treat the neurodevelopmental condition in the second subgroup 1134 is greater than a probability of a therapeutic related to the fifth node 1158 being a candidate therapeutic to treat the neurodevelopmental condition in the second subgroup 1134.

The computational biology system 102 can determine a distance between one of the consensus gene expression profiles 1148 and a node of a therapeutic in the gene expression profile distance network 1114 by determining one or more similarity metrics between a respective one of the consensus gene expression profiles 1148 and a gene expression profile that corresponds to a therapeutic included in the gene expression profile distance network 1114. In one or more illustrative examples, the computational biology system 102 can analyze a number of up-regulated genes and a number of down-regulated genes of the consensus gene expression profile 1146 in relation to a number of up-regulated genes and a number of down-regulated genes associated with the therapeutic that corresponds to the third node 1154. In the illustrative example of Figure 11, the third node 1156 can correspond to a second therapeutic 1162. In various examples, the computational biology system 102 can determine a subgroup of the up-regulated genes included in the consensus gene expression profile 1146 and a subgroup of down-regulated genes included in the consensus gene expression profile 1146. The computational biology system 102 can also determine a subgroup of up-regulated genes and a sub-group of down-regulated genes included in the gene expression profile of the second therapeutic 1162. In one or more implementations, the computational biology system 102 can determine a first profile similarity metric by analyzing each gene included in the subgroup of up-regulated genes of the consensus gene expression profile 1146 with respect to the subgroup of down-regulated genes of the second therapeutic 1162 and by analyzing each gene included in the subgroup of down-regulated genes included in the consensus gene expression profile 1146 with respect to the subgroup of up-regulated genes of the gene expression profile of the second therapeutic 1162.

In one or more illustrative examples, the computational biology system 102 can determine the 250 most up-regulated genes and the 250 most down-regulated genes included in the consensus gene expression profile 1146. The computational biology system 102 can also determine the 250 most up-regulated genes and the 250 most down-regulated genes included in the gene expression profile of the second therapeutic 1162. The computational biology system 102 can then determine a first similarity metric by identifying individual genes included in the 250 most up-regulated genes of the consensus gene expression profile 1146 that are also included in the 250 most down-regulated genes of the gene expression profile of the second therapeutic 1162. In addition, the computational biology system 102 can determine the first similarity metric by identifying individual genes included in the 250 most down-regulated genes of the consensus gene expression profile 1146 that are also included in the 250 most up-regulated genes of the gene expression profile of the second therapeutic 1162. In additional illustrative examples, additional numbers of the most up-regulated genes and the most down-regulated genes can be evaluated with respect to the consensus gene expression profile 1146 and the gene expression profile of the second therapeutic 1162, such as one or more values from 1 to 500 including 50, 100, 150, 200, 300, 350, 400, 450, or 500.

The computational biology system 102 can also determine a second profile similarity metric indicating a similarity between the gene expression profile of the second therapeutic 1162 and the consensus gene expression profile 1146. In one or more implementations, the computational biology system 102 can analyze a number of up-regulated genes and a number of down-regulated genes of the gene expression profile of the second therapeutic 1162 in relation to a number of up-regulated genes and a number of down-regulated genes consensus gene expression profile 1146. For example, the computational biology system 102 can determine a second profile similarity metric by analyzing each gene included in the subgroup of up-regulated genes of the gene expression profile of the second therapeutic 1162 with respect to the subgroup of down-regulated genes of the consensus gene expression profile 1146 and by analyzing each gene included in the subgroup of down-regulated genes included in the gene expression profile of the second therapeutic 1162 with respect to the subgroup of up-regulated genes of the consensus gene expression profile 1146.

The computational biology system 102 can determine a profile similarity score by combining the first profile similarity metric and the second profile similarity metric. The profile similarity score can then be used to determine a distance between the third node 1154 and the consensus gene expression profile 1146 within the gene expression profile distance network 1114. In turn, the distance between the third node 1154 and the consensus gene expression profile 1146 within the gene expression profile distance network 1114 can be used to determine a probability that the second therapeutic 1162 can be a candidate treatment for a subgroup of individuals in which a neurodevelopmental condition is present. For example, as the magnitude of the profile similarity score increases between the gene expression profile 1146 and the third node 1154 of the gene expression profile distance network 1114, the lower the probability of the second therapeutic 1162 being a candidate therapeutic for the first subgroup 1132 that corresponds to the consensus gene expression profile 1146.

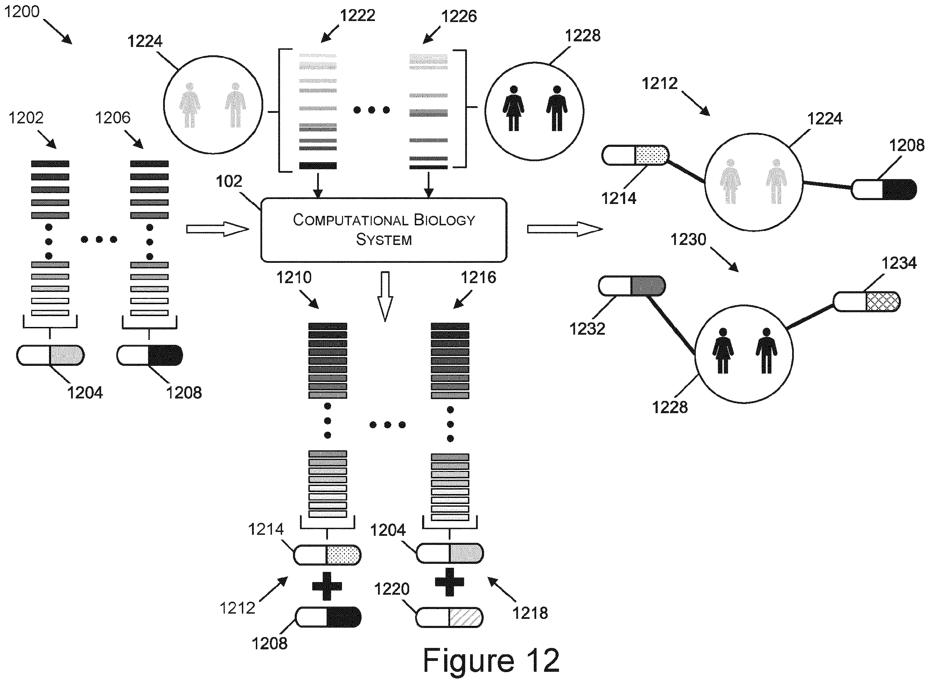

Figure 12 is a diagram of an example framework 1200 to determine one or more therapeutics to treat different sub-groups of individuals in which a neurodevelopmental condition is present based on gene expression profiles of the therapeutics and gene expression profiles of the individuals, in accordance with one or more example implementations. The framework 1200 can include the computational biology system 102 that obtains gene expression profiles of therapeutics, such as a first gene expression profile 1202 of a first therapeutic 1204 up to an Nth gene expression profile 1206 of an Nth therapeutic 1208. The gene expression profiles can indicate the up-regulation of genes and the down-regulation of genes included in cells, such as one or more cell lines, that have interacted with one or more therapeutics. For example, the first gene expression profile 1202 can indicate the up-regulation of a number of genes and the down-regulation of a number of genes included in cells that have interacted with the first therapeutic 1204. Additionally, the Nth gene expression profile 1206 can indicate the up-regulation and the down-regulation of a number of genes included in cells that have interacted with the Nth therapeutic 1208. In various examples, the gene expression profiles 1202, 1206 can be obtained from one or more data sources that store data indicating the up-regulation and down-regulation of genes in response to interactions between cells including the genes and a number of therapeutics.

The computational biology system 102 can generate merged gene expression profiles that indicate up-regulated genes and down-regulated genes of cells that have interacted with a combination of therapeutics. To illustrate, the computational biology system 102 can generate a first merged gene expression profile 1210 that indicates genes that are up-regulated and down-regulated in cells that have interacted with a first combination of therapeutics 1212, where the first combination of therapeutics 1212 can include the Nth therapeutic 1208 and a second therapeutic 1214. Additionally, the computational biology system 102 can generate an Nth merged gene expression profile 1216 that indicates genes that are up-regulated and down-regulated in cells that have interacted with an Nth combination of therapeutics 1218, where the Nth combination of therapeutics can include the first therapeutic 1204 and a third therapeutic 1220.

The computational biology system 102 can generate the merged gene expression profiles by combining the gene expression profiles for a plurality of therapeutics and generating a ranked list of genes based on measures of up-regulation and measures of down-regulation for genes included in the gene expression profiles of the plurality of therapeutics. For example, the first merged gene expression profile 1210 can include a ranked list of genes based on measures of up-regulation and measures of down-regulation of genes included in the Nth gene expression profile 1206 and in the gene expression profile for the second therapeutic 1214. In one or more examples, the computational biology system 102 can generate merged gene expression profiles by averaging the ranks of the genes in the gene expression profile for the first therapeutic and the genes in the gene expression profile for the second therapeutic. In one or more other examples, the computational biology system 102 can generate merged gene expression profiles by aggregating a list of genes included in the gene expression profiles of a plurality of therapeutics based on measures of up-regulation and measures of down-regulation of the respective genes. To illustrate, the computational biology system 102 can perform an analysis of a group of gene expression profiles for a number of therapeutics to determine genes that have at least a threshold amount of up-regulation or down-regulation with respect to expression profiles of vehicle (e.g., dimethyl sulfoxide) treated cell lines or with respect to at least a threshold number of therapeutics. The computational biology system 102 can then modify initial ranked lists of genes that are generated from a plurality of combinations of gene expression profiles for a number of therapeutics. In one or more illustrative examples, the computational biology system 102 can determine a number of over-expressed genes that have at least 20% up-regulation in response to interactions with at least 75% of the therapeutics included in a group of therapeutics for which the computational biology system 102 has obtained gene expression profiles. Continuing with this example, the computational biology system 102 can generate an initial version of a merged gene expression profile by combining the genes included in the individual gene expression profiles for a number of therapeutics, such as the Nth therapeutic 1208 and the second therapeutic 1214 and ranking the combined group of genes based on the measures of up-regulation and the measures of down-regulation of the respective genes included in the gene expression profiles. In various examples, the computational biology system 102 can then modify the initial version of the merged gene expression profile based on any genes included in the initial version of the merged gene expression profile that are also included in the number of over-expressed genes. For example, a gene having an initial ranking in the initial version of the merged gene expression profile that is also included in the number of over-expressed genes can be changed to a modified ranking that is higher than the initial ranking to produce a modified version of the merged gene expression profile. Additionally, the computational biology system 102 can also determine a number of under-expressed genes based on performing an analysis of gene expression profiles of a number of therapeutics. To illustrate, the computational biology system 102 can determine a number of under-expressed genes that have at least 20% down-regulation in response to interactions with at least 75% of the therapeutics included in a group of therapeutics for which the computational biology system 102 has obtained gene expression profiles. The computational biology system 102 can generate a modified version of an initial gene expression profile by modifying the rankings of genes that are included in the number of under-expressed genes. In one or more illustrative examples, the computational biology system 102 modify the initial version of a merged gene expression profile modifying the rankings of genes included in the initial version of the merged gene expression profile that are included in the number of under-expressed genes. In one or more implementations, the computational biology system 102 can lower the rankings of genes included in the initial version of the merged gene expression profile that are also included in the number of under-expressed genes.

Additionally, the computational biology system 102 can generate merged gene expression profiles using the gene expression profiles of a plurality of therapeutics by combining a number of up-regulated genes and a number of down-regulated genes from each gene expression profile used to generate the merged gene expression profile. For example, the computational biology system 102 can generate the first merged gene expression profile 1210 by combining a number of up-regulated genes and a number of down-regulated genes of the Nth therapeutic 1208 with a number of up-regulated genes and a number of down-regulated genes of the second therapeutic 1214. To illustrate, the computational biology system 102 can generate a merged gene expression profile by combining from about 50 to about 1000, from about 100 to about 500, or from about 125 to about 200 of the top up-regulated genes and from about 50 to about 1000, from about 100 to about 500, or from about 125 to about 200 of the bottom down-regulated genes included in the gene expression profiles of a number of therapeutics. In one or more illustrative examples, the Nth gene expression profile 1206 can include a ranked list of genes based on levels of expression of the respective genes in response to interacting with the Nth therapeutic 1208. The computational biology system 102 can determine the 125 genes at the top of the ranked list that correspond to the most up-regulated genes included in the Nth gene expression profile 1206 and the 125 genes at the bottom of the ranked list that correspond to the most down-regulated genes included in the Nth gene expression profile 1206. To generate the first merged gene expression profile 1210, the computational biology system 102 can combine the 125 genes at the top of the Nth gene expression profile 1206 and the 125 genes at the bottom of the Nth gene expression profile 1206 with the 125 genes at the top of the gene expression profile related to the second therapeutic 1214 and the 125 genes at the bottom of the gene expression profile related to the second therapeutic 1214 to generate the first merged gene expression profile 1210.

The computational biology system 102 can also obtain and/or produce gene expression profiles of groups of individuals in which a neurodevelopmental condition is present. For example, the computational biology system 102 can obtain and/or produce a first subgroup gene expression profile 1222 for a first group of individuals 1224 in which a neurodevelopmental condition is present up to an Nth subgroup gene expression profile 1226 for an Nth group of individuals 1228 in which the neurodevelopmental condition is present. The computational biology system 102 can determine one or more candidate therapeutic combinations for the individuals included in the respective subgroups by analyzing the ranked list of genes included in the subgroup gene expression profiles with respect to the inverse of the ranked list of genes included in the merged gene expression profiles corresponding to a number of therapeutic combinations. The computational biology system 102 can determine measures of similarity between the subgroup gene expression profiles and the merged gene expression profiles of the therapeutic combinations to determine a probability of respective therapeutic combinations being candidate treatments for individuals of one or more respective subgroups in which the neurodevelopmental condition is present. In one or more illustrative examples, the computational biology system 102 can analyze the first subgroup gene expression profile 1222 with respect to the first merged gene expression profile 1210 to determine a probability of the combination of the Nth therapeutic 1208 and the second therapeutic 1214 being a candidate treatment for individuals included in the first subgroup 1224. Additionally, the computational biology system 102 can analyze the Nth subgroup gene expression profile 1226 with respect to the first merged gene expression profile 1210 to determine a probability of the combination of the Nth therapeutic 1208 and the second therapeutic 1214 being a candidate treatment for the Nth subgroup 1228.

In various examples, the individuals included in the first subgroup 1224 and the individuals included in the Nth subgroup 1228 can be clinically diagnosed with a same neurodevelopmental condition, but the individuals included in the first subgroup 1224 and the individuals included in the Nth subgroup 1228 can have different characteristics. For example, the individuals included in the first subgroup 1224 can be associated with one or more first phenotypes and the individuals included in the Nth subgroup 1228 can be associated with one or more second phenotypes that are different from at least one phenotype of the one or more first phenotypes. Additionally, the first subgroup gene expression profile 1222 for the first group of individuals 1224 can be different from the Nth subgroup gene expression profile 1226 for the Nth group of individuals 1228. In various examples, at least a portion of the first subgroup gene expression profile 1222 and the Nth subgroup gene expression profile 1226 can be different based on one or more first biological pathways being disrupted in the first subgroup 1224 of individuals and one or more second biological pathways being disrupted in the second subgroup 1228, where at least one of the one or more second biological pathways is different from at least one of the one or more first biological pathways.

In one or more implementations, the computational biology system 102 can generate a gene expression profile distance network that includes a plurality of nodes and a plurality of edges connecting the nodes. Each node of the gene expression profile distance network can correspond to a combination of at least two therapeutics and the edges can indicate a distance between the therapeutic combinations related to the respective nodes. In various examples, the computational biology system 102 can determine a measure of similarity between a merged gene expression profile for a therapeutic combination and a subgroup gene expression profile by adding the inverse of the subgroup gene expression profile into the gene expression profile distance network and determining a distance between the node of the therapeutic combination and the node of the inverse of the subgroup gene expression profile in the gene expression profile distance network. The computational biology system 102 can determine one or more combinations of therapeutics that can be candidate treatments for a subgroup of individuals by determining the one or more combinations of therapeutics in which the distance is minimized with respect to the node of the subgroup of individuals included in the gene expression profile distance network.

The computational biology system 102 can determine a distance between a therapeutic combination node and a location of a subgroup node in a gene expression profile distance network by determining one or more similarity metrics between a therapeutic combination node and a location of a subgroup node in a gene expression profile distance network. In one or more illustrative examples, the computational biology system 102 can analyze a number of up-regulated genes and a number of down-regulated genes of a merged gene expression profile for a combination of therapeutics in relation to a number of up-regulated genes and a number of down-regulated genes associated with a gene expression profile for a subgroup of individuals. For example, the computational biology system 102 can determine a subset of the up-regulated genes and a subset of down-regulated genes included in the first merged gene expression profile 1210. The computational biology system 102 can also determine a subset of up-regulated genes and a subset of down regulated genes included in the first subgroup gene expression profile 1222. In one or more implementations, the computational biology system 102 can determine a first profile similarity metric by analyzing each gene included in the subset of up-regulated genes of the first merged gene expression profile 1210 with respect to the subset of down-regulated genes of the first subgroup gene expression profile 1222 and by analyzing each gene included in the subset of down-regulated genes included in the first merged gene expression profile 1210 with respect to the subset of up-regulated genes of the first subgroup gene expression profile 1222.

In one or more illustrative examples, the computational biology system 102 can determine the 250 most up-regulated genes and the 250 most down-regulated genes included in the merged gene expression profile 1210 of the first combination of therapeutics 1212 that includes the Nth therapeutic 1208 and the second therapeutic 1214. The computational biology system 102 can also determine the 250 most up-regulated genes and the 250 most down-regulated genes included in first subgroup gene expression profile 1222. The computational biology system 102 can then determine a first similarity metric by identifying individual genes included in the 250 most up-regulated genes of the merged gene expression profile 1210 that are also included in the 250 most down-regulated genes of the first subgroup gene expression profile 1222. In addition, the computational biology system 102 can determine the first similarity metric by identifying individual genes included in the 250 most down-regulated genes of the first merged gene expression profile 1210 that are also included in the 250 most up-regulated genes of first subgroup gene expression profile 1222.

The computational biology system 102 can also determine a second profile similarity metric indicating a similarity between the first subgroup gene expression profile 1222 and the first merged gene expression profile 1210. In one or more implementations, the computational biology system 102 can analyze a number of up-regulated genes and a number of down-regulated genes of the first subgroup gene expression profile 1222 in relation to a number of up-regulated genes and a number of down-regulated genes included in the first merged gene expression profile 1210. For example, the computational biology system 102 can determine a second profile similarity metric by analyzing each gene included in a subset of up-regulated genes of the first subgroup gene expression profile 1222 with respect to a subset of down-regulated genes of the first merged gene expression profile 1210 and by analyzing each gene included in a subset of down-regulated genes in the first subgroup gene expression profile 1222 with respect to a subset of up-regulated genes of the first merged gene expression profile 1210.

The computational biology system 102 can determine a profile similarity score by combining the first profile similarity metric and the second profile similarity metric. The profile similarity score can then be used to determine a distance between the first merged gene expression profile 1210 and the first subgroup gene expression profile 1222 within a gene expression profile distance network. In turn, the distance between the merged gene expression profile 1210 and the first subgroup gene expression profile 1222 within a gene expression profile distance network can be used to determine a probability that the combination of the Nth therapeutic 1208 and the second therapeutic 1214 can be a candidate treatment for the first subgroup 1224 of individuals in which a neurodevelopmental condition is present. For example, the greater the magnitude of the profile similarity score between the first merged gene expression profile 1210 and the first subgroup gene expression profile 1222 of a gene expression profile distance network, the lower the probability of the combination of the Nth therapeutic 1208 and the second therapeutic 1214 being a candidate therapeutic for the first subgroup 1224.

In the illustrative example of Figure 12, the computational biology system 102 can determine that the probability of the first combination of therapeutics 1212, which includes the Nth therapeutic 1208 and the second therapeutic 1214, being a candidate treatment for the first subgroup 1224 is greater than a threshold probability. Thus, the computational biology system 102 can determine a probability that the first combination of therapeutics 1212 can be a candidate treatment that can alleviate one or more symptoms of the first subgroup 1224 in relation to the neurodevelopmental condition. Additionally, the computational biology system 102 can determine a probability of a second combination of therapeutics 1230, which includes a fifth therapeutic 1232 and a sixth therapeutic 1234, being a candidate treatment for the Nth subgroup 1228 is greater than a threshold probability. Accordingly, the computational biology system 102 can determine a probability that the second combination of therapeutics 1230 can be a candidate treatment that can alleviate one or more symptoms of the second subgroup 1228 in relation to the neurodevelopmental condition.

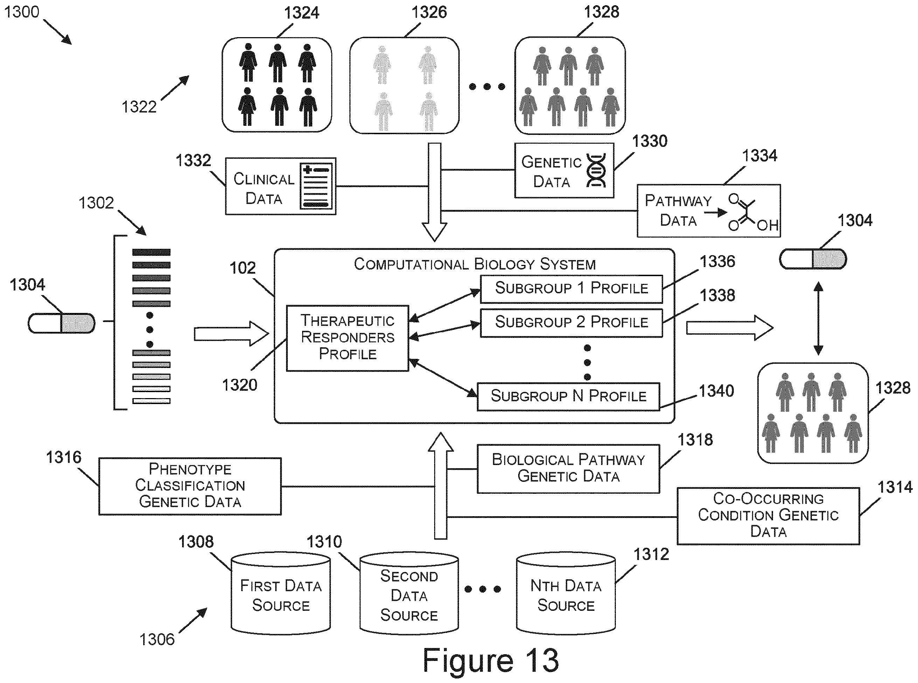

Figure 13 is a diagram of an example framework 1300 to determine genetic profiles of individuals that have at least a threshold probability of being responsive to a therapeutic for the treatment of a neurodevelopmental condition, in accordance with one or more example implementations. The framework 1300 can include a computational biology system 102 that can obtain gene expression profiles of a number of therapeutics. For example, the computational biology system 102 can obtain the gene expression profile 1302 that corresponds to the therapeutic 1304. The gene expression profile 1302 can indicate a first number of genes that can be up-regulated and a second number of genes that can be down-regulated in response to interactions between the therapeutic and cells that include a plurality of genes comprising at least the first number of genes and the second number of genes. In various examples, the computational biology system 102 can obtain the gene expression profile 1302, in addition to a number of other gene expression profiles, from one or more data stores. The one or more data stores can include one or more databases that are electronically accessible via a network. In one or more illustrative examples, the computational biology system 102 can obtain the gene expression profile 1302 from a database that is accessible via a website.

The framework 1300 can also include a number of data sources 1306 that store information about a biological condition, such as a neurodevelopmental condition. The number of data sources 1306 can include a first data source 1308, a second data source 1310, up to an Nth data source 1312. In one or more implementations, at least a portion of the number of data sources 1306 can include databases generated by the computational biology system 102. To illustrate, at least a portion of the number of data sources 1306 can include information stored by at least one of the curated databases 202 described with respect to Figure 2.

The computational biology system 102 can obtain co-occurring condition genetic data 1314 from at least one of the number of data sources 1306. The co-occurring condition genetic data 1314 can indicate a number of genes that are over-expressed and/or a number of genes that are under-expressed in individuals in which one or more biological conditions are present. The one or more biological conditions can also be present in individuals in which a neurodevelopmental condition is present. In one or more illustrative examples, at least a portion of the number of data sources 1306 can store information indicating genes that are up-regulated and/or down-regulated for individuals in which one or more biological conditions that are co-occurring with autism spectrum disorder can also be present.

The computational biology system 102 can also obtain phenotype classification genetic data 1316 from at least a portion of the number of data sources 1306. The phenotype classification genetic data 1316 can indicate one or more genes that are related to one or more phenotypes. In various examples, the one or more phenotypes can be associated with at least one biological condition. For example, the phenotype classification genetic data 1316 can indicate one or more genes that correspond to at least one phenotype that is associated with a neurodevelopmental condition, such as autism spectrum disorder. In one or more implementations, the phenotype classification genetic data 1316 can indicate one or more genes that are up-regulated and/or one or more genes that are down-regulated in individuals in which a respective phenotype is present.

Additionally, the computational biology system 102 can obtain biological pathway genetic data 1318 from at least a portion of the number of data sources 1306. The biological pathway genetic data 1318 can indicate one or more genes that are expressed in relation to at least one biological pathway. In one or more examples, the biological pathways for which at least a portion of the number of data sources 1306 store genetic data can be related to one or more biological conditions. To illustrate, the biological pathway genetic data 1318 obtained by computational biology system 102 can correspond to one or more biological pathways that can be disrupted for individuals in which a neurodevelopmental condition is present.

The computational biology system 102 can analyze the gene expression profile 1302 in relation to at least a portion of the information obtained from at least one of the number of data sources 1306 in relation to gene expression profiles of one or more therapeutics. For example, the computational biology system 102 can analyze at least one of the co-occurring condition genetic data 1314, the phenotype classification genetic data 1316, or the biological pathway genetic data 1318 in relation to the gene expression profile 1302. For example, the computational biology system 102 can determine one or more genes included in the gene expression profile 1302 that have at least a threshold amount of representation in at least one of the co-occurring condition genetic data 1314, the phenotype classification genetic data 1316, or the biological pathway genetic data 1318. The computational biology system 102 can determine a therapeutic responders profile 1320 with respect to the therapeutic 1304 based on an analysis of the gene expression profile 1302 and at least one of the co-occurring condition genetic data 1314, the phenotype classification genetic data 1316, or the biological pathway genetic data 1318. The therapeutic responders profile 1320 can indicate one or more genes of individuals that have at least a threshold probability of being responsive to the therapeutic 1304. To illustrate, the therapeutic responders profile 1320 can indicate one or more genes that can be present in individuals that have at least a threshold probability of one or more symptoms of the individuals that are related to the neurodevelopmental condition being alleviated in response to the therapeutic 1304.

In one or more implementations, the computational biology system 102 can perform one or more tests to determine a number of genes to include in the therapeutic responders profile 1320. For example, the computational biology system 102 can determine whether each gene included in the gene expression profile 1302 is overrepresented in at least one of the co-occurring condition genetic data 1314, the phenotype classification genetic data 1316, or the biological pathway genetic data 1318 according to a number of different statistical tests. In various examples, the genes of the gene expression profile 1302 that are identified by at least one statistical test as having at least a threshold amount of overrepresentation in at least one of the co-occurring condition genetic data 1314, the phenotype classification genetic data 1316, or the biological pathway genetic data 1318 can be included in the therapeutic responders profile 1320. In additional implementations, the genes of the gene expression profile 1302 that are identified by one or more statistical tests as having at least a threshold amount of overrepresentation in at least one of the co-occurring condition genetic data 1314, the phenotype classification genetic data 1316, or the biological pathway genetic data 1318 can be included in the therapeutic responders profile 1320.

In addition to the therapeutic responders profile 1320, the computational biology system 102 can determine an adverse responders profile that indicates one or more genes that can be present in individuals that have at least a threshold probability having an adverse reaction, such as one or more symptoms related to the neurodevelopmental condition becoming worse, in response to the therapeutic 1304. The adverse responders profile can be determined through an analysis of the gene expression profile 1302 and at least one of the co-occurring condition genetic data 1314, the phenotype classification genetic data 1316, or the biological pathway genetic data 1318.

In one or more illustrative examples, the computational biology system 102 can determine one or more genes included in the gene expression profile 1302 that have at least a threshold amount of representation in genetic data obtained from the number of data sources 1306 by performing a hypergeometric test. For example, the computational biology system 102 can determine a probability of at least a portion of the genes included in the gene expression profile 1302 also being included in at least one of the co-occurring condition genetic data 1314, the phenotype classification genetic data 1316, or the biological pathway genetic data 1318 in relation to a number of randomly selected genes being included in at least one of the co-occurring condition genetic data 1314, the phenotype classification genetic data 1316 or the biological pathway genetic data 1318 according to a hypergeometric distribution. In situations where the actual occurrences of one or more genes included in the gene expression profile 1302 meet and/or exceed a threshold number of occurrences in at least one of the co-occurring condition genetic data 1314, the phenotype classification genetic data 1316, or the biological pathway genetic data 1318 according to the hypergeometric distribution, the computational biology system 102 can add the one or more genes to the therapeutic responders profile 1320.

The computational biology system 102 can also determine one or more genes included in at least one of the co-occurring condition genetic data 1314, the phenotype classification genetic data 1316, or the biological pathway genetic data 1318 that are also included in the gene expression profile 1302 and determine a strength of measures of up-regulation and the measures of down-regulation of genes included in at least one of the co-occurring condition genetic data 1314, the phenotype classification genetic data 1316, or the biological pathway genetic data 1318. In various examples, the strength of individual measures of up-regulation and/or measures of down-regulation of genes included in the gene expression profile 1302 can be based on a threshold false discovery rate. In one or more implementations, the threshold false discovery rate can correspond to an amount of up-regulation and/or down-regulation of respective genes included in both the gene expression profile 1302 and at least one of the co-occurring condition genetic data 1314, the phenotype classification genetic data 1316, or the biological pathway genetic data 1318 by chance. In one or more illustrative examples, a strength of up-regulation of one or more genes and a strength of down-regulation of one or more genes included in the gene expression profile 1302 can be determined based on differences between the measures of up-regulation and the measures of down-regulation of respective genes included in the gene expression profile 1302 in relation to the amounts of up-regulation and down-regulation expected by chance for the genes of the gene expression profile 1302 that are also included in at least one of the co-occurring condition genetic data 1314, the phenotype classification genetic data 1316, or the biological pathway genetic data 1318.

In various examples, the strength of up-regulation of one or more genes and a strength of down-regulation of one or more genes included in the gene expression profile 1302 with respect to genes included in at least one of the co-occurring condition genetic data 1314, the phenotype classification genetic data 1316, or the biological pathway genetic data 1318 can be determined by identifying a threshold number of up-regulated genes and a threshold number of down-regulated genes included in the gene expression profile 1302 that are also included in the genes included in the co-occurring condition genetic data 1314, the phenotype classification genetic data 1316, or the biological pathway genetic data 1318. In one or more illustrative examples, the threshold number of up-regulated genes and down-regulated genes can be 250. Further, the strength of up-regulation of one or more genes and a strength of down-regulation of one or more genes included in the gene expression profile 1302 with respect to genes included in at least one of the co-occurring condition genetic data 1314, the phenotype classification genetic data 1316, or the biological pathway genetic data 1318 can be determined by determining a profile similarity score between the gene expression profile 1302 and a number of genes included in at least one of the co-occurring condition genetic data 1314, the phenotype classification genetic data 1316, or the biological pathway genetic data 1318. The profile similarity score can indicate a probability that genes included in at least one of the co-occurring condition genetic data 1314, the phenotype classification genetic data 1316, or the biological pathway genetic data 1318 are also included in the gene expression profile 1302.

In additional implementations, the computational biology system 102 can determine an amount of overrepresentation of at least a portion of the genes included in the gene expression profile 1302 in the genes included in at least one of the co-occurring condition genetic data 1314, the phenotype classification genetic data 1316, or the biological pathway genetic data 1318 (i.e., genes of interest) by performing a number of simulations that use a random or pseudo-random number generator to select genes from a pool of genes and determine the significance of an amount of overlap between the selected genes and the genes included in the gene expression profile 1302. In one or more examples, 10,000 synthetic gene sets of different sizes from 5 genes to 500 genes are generated via random selection of genes from the human genome. The computational biology system 102 can determine the amount of overrepresentation by determining the genes ranked as high as a certain rank threshold among the synthetic gene sets. In various implementations, the computational biology system 102 can determine the significance of an amount of overlap between the selected genes and the genes included in the gene expression profile 1302 at different rank threshold ranges, such as at rank thresholds 1-500, or rank thresholds 1-250, 1-100, 1-50, 1-10, or 1-50. The likelihood of observing the same number of genes of interest (included in at least one of the co-occurring condition genetic data 1314, the phenotype classification genetic data 1316, or the biological pathway genetic data 1318) ranked as high as each rank threshold is determined by comparing the observed area under the curve (AUC) to the one expected by chance, which is given by the distribution of 10,000 randomly simulated AUCs at each particular rank threshold. The computational biology system 102 can then determine the rank threshold within the range of evaluated rank thresholds that results in the highest significance, as well as the lowest and the highest ranks within the range of evaluated rank thresholds that reach significance.

The therapeutic responders profile 1320 can include features, in addition to genes, that can correspond to individuals that have at least a threshold probability of being responsive to the therapeutic 1304. For example, the therapeutic responders profile 1320 can indicate morphological features of individuals having at least a threshold probability of being responsive to the therapeutic 1304. Additionally, the therapeutic responders profile 1320 can indicate clinical observations and/or behavioral characteristics of individuals having at least a threshold probability of being responsive to the therapeutic 1304. Further, the therapeutic responders profile 1320 can indicate levels of analytes that can be present in individuals having at least a threshold probability of being responsive to the therapeutic 1304.

In various examples, the computational biology system 102 can determine additional features of individuals that have at least a threshold probability of being responsive to the therapeutic 1304 by analyzing the genes included in the therapeutic responders profile 1320 in relation to the features of individuals included in at least a portion of the number of data sources 1306, where the features correspond to at least a portion of the genes included in the therapeutic responders profile 1320. For example, the computational biology system 1302 can determine morphological features included in at least a portion of the number of data sources 1306 that correspond to one or more genes included in the therapeutic responders profile 1320. Additionally, the computational biology system 102 can determine clinical features and/or behavioral features included in at least a portion of the number of data sources 1306 that correspond to one or more genes included in the therapeutic responders profile 1320. Further, the computational biology system 102 can determine levels of analytes included in at least a portion of the number of data sources 1306 that correspond to one or more genes included in the therapeutic responders profile 1320.

The computational biology system 1302 can also analyze information about a number of subgroups 1322 of individuals in which a biological condition is present and identify one or more of the number of subgroups 1322 that correspond to the therapeutic responders profile 1320. In various examples, a neurodevelopmental condition can be present in the individuals included in the number of subgroups 1322. In one or more implementations, the number of subgroups 1322 can include a first subgroup 1324, a second subgroup 1326, up to an Nth subgroup 1328. The computational biology system 102 can obtain at least one of genetic data 1330, clinical data 1332, or pathway data 1334 corresponding to individuals included in the number of subgroups 1322. The computational biology system 102 can obtain the genetic data 1330, the clinical data 1332, and/or the pathway data 1334 from one or more data stores that are accessible to the computational biology system 102.

The genetic data 1330 can include at least one of DNA information or RNA information for at least a portion of the individuals included in the number of subgroups 1322. In various examples, the genetic data 1330 can include transcriptomic information. In one or more illustrative examples, the genetic data 1330 can include gene expression profiles for respective subgroups included in the number of subgroups 1322. For example, the genetic data 1330 can include at least a first gene expression profile that corresponds to the first subgroup 1324 and a second gene expression profile that corresponds to the second subgroup 1326. The computational biology system 102 can generate gene expression profiles for at least a portion of the number of subgroups 1322 using one or more implementations described with respect to generating the consensus gene expression profile 1146 described in relation to Figure 11.

The clinical data 1332 can include observations made by a healthcare practitioner in a clinical setting. The clinical data 1332 can also include physical observations corresponding to at least a portion of the individuals included in the number of subgroups 1322. For example, the clinical data 1332 can indicate morphological features of at least a portion of the individuals included in the number of subgroups 1322. Additionally, the clinical data 1332 can indicate results of one or more diagnostic tests, one or more analytical techniques, and/or one or more imaging procedures. In one or more illustrative examples, the clinical data 1332 can include measures of analytes within at least a portion of the individuals included in the number of subgroups 1322. In various examples, the clinical data 1332 can also indicate electrical activity, such as EEG or ECG measurements. The pathway data 1334 can indicate one or more biological pathways that are disrupted in at least a portion of the individuals included in the number of subgroups 1322. In one or more implementations, the pathway data 1334 can indicate genes that are up-regulated and/or genes that are down-regulated that are related to one or more biological pathways in at least a portion of the individuals included in the number of subgroups 1322.

The computational biology system 102 can generate subgroup profiles that indicate characteristics of the individuals included in respective subgroups of the number of subgroups 1322. For example, the computational biology system 102 can generate a first subgroup profile 1336 that indicates characteristics of individuals included in the first subgroup 1324 and a second subgroup profile 1338 that indicates characteristics of individuals included in the second subgroup 1326. In addition, the computational biology system 102 can generate an Nth subgroup profile 1340 that indicates characteristics of individuals included in the Nth subgroup 1328. In various examples, the subgroup profiles 1336, 1338, 1340 can indicate genetic data that corresponds to the respective subgroups 1324, 1326, 1328. In one or more illustrative examples, the computational biology system 102 can generate a first gene expression profile for the first subgroup 1324 to include in the first subgroup profile 1336, a second gene expression profile for the second subgroup 1326 to include in the second subgroup profile 1338, and an Nth gene expression profile for the Nth subgroup 1328 to include in the Nth subgroup profile 1340.

At least a portion of the subgroup profiles 1336, 1338, 1340 can also indicate clinical features and/or pathway features of individuals included in the respective subgroups 1324, 1326, 1328. To illustrate, at least a portion of the subgroup profiles 1336, 1338, 1340 can indicate at least one of one or more morphological features, one or more levels of analytes, one or more clinical observations, or one or more phenotypes that correspond to the respective subgroups 1324, 1326, 1328. In one or more implementations, the computational biology system 102 can determine a first set of clinical features that correspond to the first subgroup 1324 and include the first set of clinical features in the first subgroup profile 1336. Additionally, the computational biology system 102 can determine a second set of clinical features that correspond to the second subgroup 1326 and include the second set of clinical features in the second subgroup profile 1338. Further, the computational biology system 102 can determine a third set of clinical features that correspond to the Nth subgroup 1328 and include the third set of clinical features in the Nth subgroup profile 1340. The computational biology system 102 can also determine one or more disrupted biological pathways for at least a portion of the number of subgroups 1322 and include the one or more disrupted biological pathways in the respective subgroup profiles 1336, 1338, 1340.

The computational biology system 102 can analyze the therapeutic responders profile 1320 in relation to at least a portion of the subgroup profiles to determine a subgroup that can include individuals with at least a threshold probability of being responsive to the therapeutic 1304. For example, the computational biology system 102 can analyze the therapeutic responders profile 1320 with respect to at least one of the first subgroup profile 1336, the second subgroup profile 1338, or the Nth subgroup profile 1340 to determine whether individuals included in at least one of the first subgroup 1324, the second subgroup 1326, or the Nth subgroup 1328 have at least a threshold probability of being responsive to the therapeutic 1304.

In one or more implementations, the computational biology system 102 can identify subgroups that have at least a threshold probability of being responsive to a therapeutic by determining an amount of overlap between features included in the therapeutic responders profile 1320 and the features included in the respective subgroup profiles. Additionally, the computational biology system 102 can determine a threshold amount of overlap by analyzing the features included in the therapeutic responders profile 1320 with respect to features included in a number of groups of randomly selected or pseudo-randomly selected individuals. To illustrate, the computational biology system 102 can perform a number of simulations such that in each simulation, the computational biology system 102 can obtain features of a number of randomly selected or pseudo-randomly selected individuals from one or more data stores that are accessible to the computational biology system 102. Based on the results of the simulations, the computational biology system 102 can then determine a threshold amount of overlap related to the features included in the therapeutic responders profile 1320 and the features included in the respective groups of individuals that are randomly selected or pseudo-randomly selected by the computational biology system 102. The computational biology system 102 can then analyze the amount of overlap between the features included in the therapeutic responders profile 1320 and the respective subgroup profiles (e.g., subgroup profiles 1336, 1338, 1340) in relation to the threshold amount of overlap. In situations where the computational biology system 102 determines that the amount of overlap between the therapeutic responders profile 1320 and a respective subgroup profile corresponds to the threshold amount of overlap, the computational biology system 102 can determine that the individuals included in the respective subgroup have at least a threshold probability of being responsive to the therapeutic.

The computational biology system 102 can also determine individuals that can be responsive to the therapeutic 1304 by analyzing gene expression profiles of the individuals with respect to the gene expression profile 1302 of the therapeutic 1304. In one or more implementations, the computational biology system 102 can analyze an inverse composite gene expression profile related to one or more of the number of subgroups 1322 with respect to the gene expression profile 1302 to determine a measure of similarity between a respective gene expression profile of at least one of the number of subgroups 1322 and the gene expression profile 1302. For example, the computational biology system 102 can determine a distance between the gene expression profile 1302 and an inverse of a gene expression profile for a respective one of the number of subgroups 1322 by determining one or more similarity metrics between the respective gene expression profiles. In one or more illustrative examples, the computational biology system 102 can analyze a number of up-regulated genes and a number of down-regulated genes of the gene expression profile 1302 in relation to a number of up-regulated genes and a number of down-regulated genes associated with a gene expression profile for a subgroup of the number of subgroups 1322. To illustrate, the computational biology system 102 can determine a subset of the up-regulated genes and a subset of down-regulated genes included in the gene expression profile 1302. The computational biology system 102 can also determine a subset of up-regulated genes and a subset of down regulated genes included in an inverse of a gene expression profile for a respective subgroup of the number of subgroups 1322. In one or more implementations, the computational biology system 102 can determine a first profile similarity metric by analyzing each gene included in the subset of up-regulated genes of the gene expression profile 1302 with respect to the subset of down-regulated genes of a gene expression profile for a subgroup of the number of subgroups 1322 and by analyzing each gene included in the subset of down-regulated genes included in the gene expression profile 1302 with respect to the subset of up-regulated genes of the gene expression profile of the subgroup of the number of subgroups 1322.

In one or more illustrative examples, the computational biology system 102 can determine the 250 most up-regulated genes and the 250 most down-regulated genes included in the gene expression profile 1302 related to the therapeutic 1304. The computational biology system 102 can also determine the 250 most up-regulated genes and the 250 most down-regulated genes included in a gene expression profile of the first subgroup 1324. The computational biology system 102 can then determine a first similarity metric by identifying individual genes included in the 250 most up-regulated genes of the gene expression profile 1302 that are also included in the 250 most down-regulated genes of the gene expression profile of the first subgroup 1324. In addition, the computational biology system 102 can determine the first similarity metric by identifying individual genes included in the 250 most down-regulated genes of the gene expression profile 1302 that are also included in the 250 most up-regulated genes of gene expression profile of the first subgroup 1324.

The computational biology system 102 can also determine a second profile similarity metric indicating a similarity between the gene expression profile 1302 and the gene expression profile of a respective subgroup of the number of subgroups 1322. In one or more implementations, the computational biology system 102 can analyze a number of up-regulated genes and a number of down-regulated genes of the gene expression profile 1302 in relation to a number of up-regulated genes and a number of down-regulated genes included in the gene expression profile of the respective subgroup of the number of subgroups. For example, the computational biology system 102 can determine a second profile similarity metric by analyzing each gene included in s subset of up-regulated genes included in the gene expression profile 1302 with respect to a subset of down-regulated genes of the gene expression profile of the respective subgroup of the number of subgroups 1322 and by analyzing each gene included in a subset of down-regulated genes included in gene expression profile 1302 with respect to a subset of up-regulated genes of the gene expression profile of the respective subgroup of the number of subgroups 1322.

In one or more illustrative examples, the computational biology system 102 can analyze the therapeutic responders profile 1320 related to the therapeutic 1304 in relation to the Nth subgroup profile 1340. For example, the computational biology system 102 can determine an amount of overrepresentation of features included in the Nth subgroup profile 1340 with respect to the features included in the therapeutic responders profile 1320. To illustrate, the computational biology system 102 can determine an amount of overlap between features included in the Nth subgroup profile 1340 and the therapeutic responders profile 1320 in relation to a threshold amount of overlap. The computational biology system 102 can determine the threshold amount of overlap by determining amounts of overlap between features of groups of randomly or pseudo-randomly selected individuals and features included in the therapeutic responders profile 1320. In one or more implementations, the computational biology system 102 can determine that the amount of overlap between features included in the Nth subgroup profile 1340 meets and/or exceeds the threshold amount of overlap. In these scenarios, the computational biology system 102 can determine that the individuals included in the Nth subgroup 1328 can have at least a threshold probability of being responsive to the therapeutic 1304.

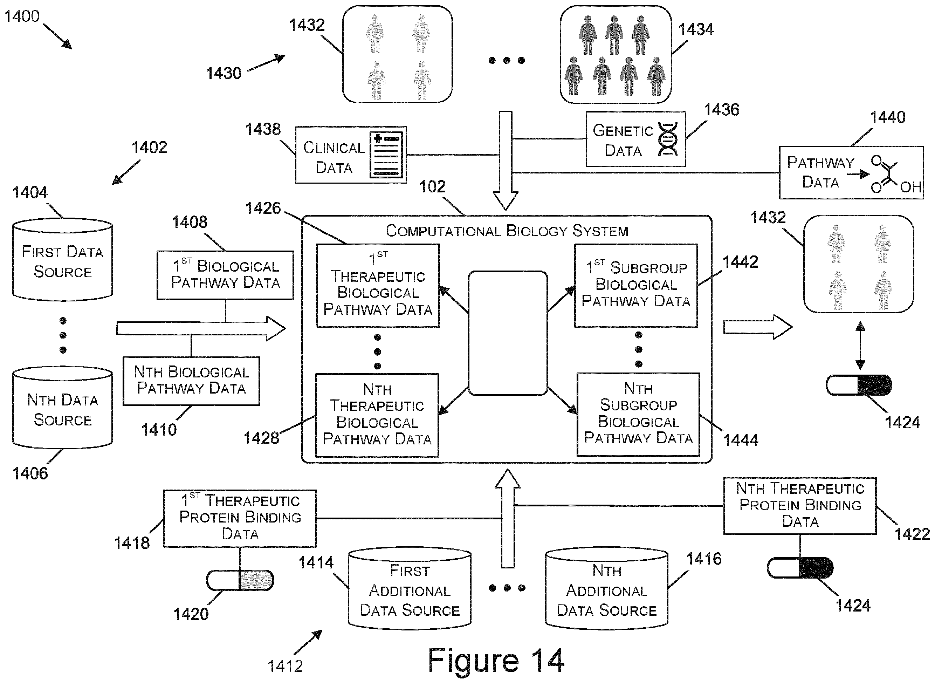

Figure 14 is a diagram of an example framework 1400 to determine therapeutics that can be candidate treatments for subgroups of individuals based on biological pathways affected by the therapeutics and biological pathways disrupted in individuals in which a neurodevelopmental condition is present, in accordance with one or more example implementations. The framework 1400 can include a computational biology system 102 that can obtain biological pathway data from a number of data sources 102. The number of data sources 1402 can include a first data source 1404 up to an Nth data source 1406. The number of data sources 1402 can include one or more databases that are electronically accessible to the computational biology system 102. The number of data sources 1402 can also include one or more websites that can be used by the computational biology system 102 to access information related to biological pathways. In one or more implementations, the computational biology system 102 can obtain first biological pathway data 1408 up to Nth biological pathway data 1410 from the one or more data sources 1402. The first biological pathway data 1408 and the Nth biological pathway data 1410 can indicate proteins that are related to one or more biological pathways. For example, the first biological pathway data 1408 can indicate one or more first proteins that are produced, modified, and/or participate in one or more chemical reactions in relation to a first biological pathway. In an additional example, the Nth biological pathway data 1410 can indicate one or more second proteins that are produced, modified, and/or participate in one or more chemical reactions in relation to a second biological pathway.

The computational biology system 102 can also obtain information from a number of additional data sources 1412, where the information is related to binding of proteins by therapeutics. The number of additional data sources 1412 can include a first additional data source 1414 up to an Nth additional data source 1416. The number of additional data sources 1412 can include one or more databases that are electronically accessible by the computational biology system 102. In addition, the number of additional data sources 1412 can include one or more websites that can be used by the computational biology system 102 to obtain information that corresponds to the binding of proteins by one or more therapeutics. In one or more implementations, the computational biology system 102 can obtain first therapeutic protein binding data 1418 that corresponds to a first therapeutic and Nth therapeutic protein binding data 1422 that corresponds to an Nth therapeutic 1424. In one or more illustrative examples, the first therapeutic protein binding data 1418 can indicate one or more measures of binding affinity between the first therapeutic 1420 and one or more proteins. The one or more measures of binding affinity can include one or more dissociation constants. Further, the Nth therapeutic protein binding data 1422 can indicate one or more measures of binding affinity between the Nth therapeutic 1424 and one or more proteins.

The computational biology system 102 can analyze information obtained from the one or more additional data sources 1412 to determine one or more proteins that have at least a threshold amount of binding affinity to one or more therapeutics. For example, the computational biology system 102 can analyze measures of binding affinity included in the first therapeutic protein binding data 1418 to determine one or more proteins that have at least a threshold amount of binding affinity with respect to the first therapeutic 1420. In addition, the computational biology system 102 can analyze measures of binding affinity included in the Nth therapeutic protein binding data 1422 to determine one or more proteins that have at least a threshold amount of binding affinity with respect to the Nth therapeutic 1424.

In one or more implementations, the computational biology system 102 can analyze the biological pathway data obtained from the number of data sources 1402 in relation to therapeutic protein binding data obtained from the number of additional data sources 1412 to determine one or more biological pathways that can be related to one or more therapeutics. In various examples, the computational biology system 102 can analyze biological pathway data and therapeutic protein binding data to determine one or more biological pathways that can be disrupted by one or more therapeutics. In one or more illustrative examples, the computational biology system 102 can generate first therapeutic biological pathway data 1426 that indicate one or more biological pathways related to the first therapeutic 1420. Additionally, the computational biology system 102 can generate Nth therapeutic biological pathway data 1428 that indicates one or more biological pathways related to the Nth therapeutic 1424.

The computational biology system 102 can determine one or more biological pathways related to one or more therapeutics by determining one or more shortest paths between respective biological pathways and respective therapeutics. For example, the computational biology system 102 can determine one or more shortest paths between one or more biological pathways and a therapeutic by analyzing the proteins related to the one or more biological pathways in relation to one or more proteins that have at least a threshold amount of binding affinity with respect to the therapeutic. The computational biology system 102 can implement one or more shortest path algorithms to determine one or more shortest paths between a biological pathway and a therapeutic based on one or more proteins related to the biological pathway and one or more proteins related to the therapeutic. For example, the computational biology system 102 can implement at least one of a Dijkstra algorithm, a Bellman-Ford algorithm, or a k-shortest path algorithm to determine one or more biological paths between a respective biological pathway and a respective therapeutic. In various examples, the computational biology system 102 can analyze one or more genes of one or more biological pathways to determine one or more shortest paths in relation to proteins that correspond to a respective therapeutic. The one or more shortest paths can be determined using a number of techniques, such as a priority queue coupled with a greedy/dynamic programming approach. In various implementations, the computational biology system 102 can use the first therapeutic biological pathway data 1426 up to the Nth therapeutic biological pathway data 1428 to determine the shortest path distances in a human protein interaction network between the genes included in the biological pathway and the proteins related to the therapeutic. The computational biology system 102 can determine a normal distribution for the distances in the human protein interaction network between proteins related to therapeutics and proteins related to genes of biological pathways that includes a mean and a number of standard deviations below the mean can indicate that proteins related to a therapeutic are closer to the genes of a biological pathway than the majority of other proteins related to other therapeutics. To illustrate, the first therapeutic biological pathway data can be determined as the biological pathways that are closer to the first therapeutic more than 2 standard deviation or more than 1.5 standard deviation or 1 standard deviation given the normal distribution for the distances.

The computational biology system 102 can also determine one or more subgroups of individuals in which a neurodevelopmental condition is present for which one or more therapeutics have at least a threshold probability of being candidate treatments for the neurodevelopmental condition. In one or more implementations, the computational biology system 102 can obtain information about a number of subgroups 1430 that include individuals in which a neurodevelopmental condition is present. For example, the number of subgroups 1430 can include at least a first subgroup 1432 up to an Nth subgroup 1434. The respective subgroups included in the number of subgroups 1430 can include individuals that have a profile of characteristics. To illustrate, the first subgroup 1432 can include individuals in which a neurodevelopmental condition is present and that have a first set of characteristics. In addition, the Nth subgroup 1434 can include individuals in which the neurodevelopmental condition is present and that have a second set of characteristics. In one or more illustrative examples, the first set of characteristics corresponding to the first subgroup 1432 can include one or more first morphological features, one or more first clinical features, levels of one or more first analytes, one or more first co-occurring conditions, one or more first disrupted biological pathways, one or more first genes, or one or more combinations thereof. Additionally, the second set of characteristics corresponding to the Nth subgroup 1434 can include one or more second morphological features, one or more second clinical features, levels of one or more second analytes, one or more second co-occurring conditions, one or more second disrupted biological pathways, one or more second genes, or one or more combinations thereof.

In one or more implementations, the computational biology system 102 can obtain at least one of genetic data 1436, clinical data 1438, or pathway data 1440 corresponding to individuals included in the number of subgroups 1430. The computational biology system 102 can obtain the genetic data 1436, the clinical data 1438, and/or the pathway data 1440 from one or more data stores that are accessible to the computational biology system 102. The genetic data 1436 can include at least one of DNA information or RNA information for at least a portion of the individuals included in the number of subgroups 1430. In various examples, the genetic data 1436 can include transcriptomic information. In one or more illustrative examples, the genetic data 1436 can include gene expression profiles for respective subgroups included in the number of subgroups 1430. For example, the genetic data 1436 can include at least a first gene expression profile that corresponds to the first subgroup 1432 and a second gene expression profile that corresponds to the Nth subgroup 1434. The computational biology system 102 can generate gene expression profiles for at least a portion of the number of subgroups 1430 using one or more implementations described with respect to generating the composite gene expression profiles 1146 described in relation to Figure 11.

The clinical data 1438 can include observations made by a healthcare practitioner in a clinical setting. The clinical data 1438 can also include physical observations corresponding to at least a portion of the individuals included in the number of subgroups 1430. For example, the clinical data 1438 can indicate morphological features of at least a portion of the individuals included in the number of subgroups 1430. Additionally, the clinical data 1438 can indicate results of one or more diagnostic tests, one or more analytical techniques, and/or one or more imaging procedures. In one or more illustrative examples, the clinical data 1438 can include measures of analytes within at least a portion of the individuals included in the number of subgroups 1430. In various examples, the clinical data 1438 can also indicate electrical activity, such as EEG measurements. The pathway data 1440 can indicate one or more biological pathways that are disrupted in at least a portion of the individuals included in the number of subgroups 1430. In one or more implementations, the pathway data 1440 can indicate genes that are up-regulated and/or genes that are down-regulated in at least a portion of the individuals included in the number of subgroups 1430.

The computational biology system 102 can analyze the information obtained about the number of subgroups 1430 and determine one or more biological pathways that correspond to respective subgroups of the number of subgroups 1430. To illustrate, the computational biology system 102 can analyze at least one of the genetic data 1436, the clinical data 1438, or the pathway data 1440 to determine one or more biological pathways that correspond to at least one of the number of subgroups 1430. In one or more illustrative examples, the computational biology system 102 can analyze at least one of the genetic data 1436, the clinical data 1438, or the pathway data 1440 related to the first subgroup 1432 to generate first subgroup biological pathway data 1442. The first subgroup biological pathway data 1442 can indicate one or more biological pathways that are disrupted in the first subgroup 1432 in relation to the neurodevelopmental condition present in the individuals included in the first subgroup 1432. Additionally, the computational biology system 102 can analyze at least one of the genetic data 1436, the clinical data 1438, or the pathway data 1440 related to the Nth subgroup 1434 to generate Nth subgroup biological pathway data 1444.

In various examples, the computational biology system 102 can determine one or more biological pathways disrupted in individuals included in the first subgroup 1432 by analyzing the amounts of expression of one or more genes of the individuals included in the first subgroup 1432. The computational biology system 102 can also determine one or more biological pathways disrupted in individuals included in the first subgroup 1432 by analyzing genetic variants of the individuals included in the first subgroup 1432. Additionally, the computational biology system 102 can determine one or more biological pathways disrupted in individuals included in the first subgroup 1432 by analyzing clinical features of the individuals included in the first subgroup 1432. The clinical features can include one or more morphological features, levels of one or more analytes, one or more observations made by a healthcare practitioner, results of one or more diagnostic procedures, results of one or more analytical tests, one or more phenotypes, one or more physical observable features, or one or more combinations thereof.

In one or more implementations, the computational biology system 102 can analyze the therapeutic biological pathway data with respect to the subgroup biological pathway data to determine one or more therapeutics that have at least a threshold probability of being candidate treatments for one or more subgroups of individuals in which a neurodevelopmental condition is present. For example, the computational biology system 102 can analyze at least one of the first therapeutic biological pathway data 1426 to the Nth therapeutic biological pathway data 1428 in relation to at least one of the first subgroup biological pathway data 1442 up to the Nth subgroup biological pathway data 1444 to determine an amount of similarity, such as a profile similarity score, between biological pathways related to one or more therapeutics and biological pathways related to individuals in which a neurodevelopmental condition is present. In various examples, the computational biology system 102 can determine an amount of overlap between one or more first genes corresponding to one or more first biological pathways related to a therapeutic and one or more second genes corresponding to one or more second biological pathways related to a subgroup of the number of subgroups 1430. In one or more illustrative examples, the computational biology system 102 can perform a Fisher's exact test to determine an amount of overlap between one or more first genes included in the first therapeutic biological pathway data 1426 and one or more additional genes included in the first subgroup biological pathway data 1442. In one or more additional illustrative examples, the computational biology system 102 can perform a Fisher's exact test to determine an amount of overlap between one or more first genes included in the first therapeutic biological pathway data 1426 and one or more additional genes included in the Nth subgroup biological pathway data 1444. In various examples, a threshold amount of overlap between one or more genes of biological pathways related to a therapeutic and one or more genes of biological pathways related to a subgroup of the number of subgroups 1430 can indicate a probability that the therapeutic can be a candidate treatment for the neurodevelopmental condition with respect to individuals included in a respective subgroup.

Further, the computational biology system 102 can determine a z-statistic in relation to biological pathway data for one or more therapeutics in relation to biological pathway data for one or more subgroups included in the number of subgroups 1430. For example, the computational biology system 102 can determine a z-statistic with respect to the first therapeutic biological pathway data 1426 and the first subgroup biological pathway data 1442 by determining an amount of overlap between one or more genes included in the first therapeutic biological pathway data 1426 and one or more genes included in the first subgroup biological pathway data 1442. The computational biology system 102 can also determine a threshold amount of overlap between one or more genes included in the first therapeutic biological pathway data 1426 and one or more genes that are randomly or pseudo-randomly selected from a set of genes. The computational biology system 102 can then analyze the amount of overlap between one or more genes included in the first therapeutic biological pathway data 1426 and one or more genes included in the first subgroup biological pathway data 1442 in relation to the threshold amount of overlap to determine the z-statistic. In additional implementations, the computational biology system 102 can perform one or more multi-test correction procedures after performing the computations of the Fisher's exact test and/or after determining the z-statistic. The computational biology system 102 can perform the one or more multiple hypothesis testing correction procedures in relation to one or more false discovery rate threshold values.

The computational biology system 102 can use one or more multiple-testing correction techniques to determine the strengths of association one or more genes included in the first therapeutic biological pathway data 1426 and one or more genes that are randomly or pseudo-randomly selected from a set of genes, by modifying one or more p-values generated using Fisher's exact test to produce one or more adjusted p-values. In one or more illustrative examples, the computational biology system 102 can perform a Bonferroni one-step correction to determine p-values (associations) that pass this multiple-test correction. Additionally, the computational biology system 102 can perform a Sidak one-step correction to generate one or more adjusted p-vales from one or more p-values produced using the Fisher's exact test. Further, the computational biology system 102 can produce one or more adjusted p-values using a Holm- Sidak step-down method with Sidak adjustments based on one or more p-values generated using the Fisher's exact test. The computational biology system 102 can also perform a Holm step-down method using Bonferroni adjustments with respect to one or more *p*-values to generate one or more adjusted p-values. In various implementations, the computational biology system 102 can perform a Simes-Hochberg step-up method to produce one or more adjusted *p*-values based on one or more adjusted *p*-values generated by the computational biology system 102 using Fisher's exact test. In one or more additional implementations, the computational biology system 102 can generate one or more adjusted *p*-values using a Hommel closed method based on Simes tests with respect to one or more *p*-values generated by the computational biology system 102 using Fisher's exact test. In one or more further implementations, the computational biology system 102 can use a one stage Benjamini-Hochberg procedure for false discovery rate correction or a two stage Benjamini-Hochberg false discovery rate correction to produce one or more adjusted *p-*values with respect to one or more *p*-values determined by the computational biology system 102 using Fisher's exact test. In one or more illustrative examples, the computational biology system 102 can implement a one stage Benjamini-Yekutieli procedure for false discovery rate correction or a two stage Benjamini-Yekutieli false discovery rate correction to generate one or more adjusted *p*-values from one or more *p*-values produced by the computational biology system 102 using Fisher's exact test.

In the illustrative example of Figure 14, the computational biology system 102 can analyze the first therapeutic biological pathway data 1426 in relation to the Nth subgroup biological pathway data 1444. The computational biology system 102 can determine at least a threshold amount of overlap between biological pathways included in the first therapeutic biological pathway data 1426 and biological pathways included in the Nth subgroup biological pathway data 1444. For example, the computational biology system 102 can determine at least a threshold amount of overlap between first genes related to a first number of pathways included in the first therapeutic biological pathway data 1426 and second genes related to a second number of pathways included in the Nth subgroup biological pathway data 1444. In one or more illustrative examples, the computational biology system 102 can perform at least one of a Fisher's exact test or determine a z-statistic to determine that the amount of overlap between first genes related to a first number of pathways included in the first therapeutic biological pathway data 1426 and second genes related to a second number of biological pathways included in the Nth subgroup biological pathway data 1444 is at least a threshold amount of overlap. In various examples, based on the amount of overlap between first genes related to a first number of pathways included in the first therapeutic biological pathway data 1426 and second genes related to a second number of pathways included in the Nth subgroup biological pathway data 1444 being at least a threshold amount of overlap, the computational biology system 102 can determine that the Nth therapeutic 1424 has at least a threshold probability of being a candidate therapeutic to treat the neurodevelopmental condition in the first subgroup 1432.

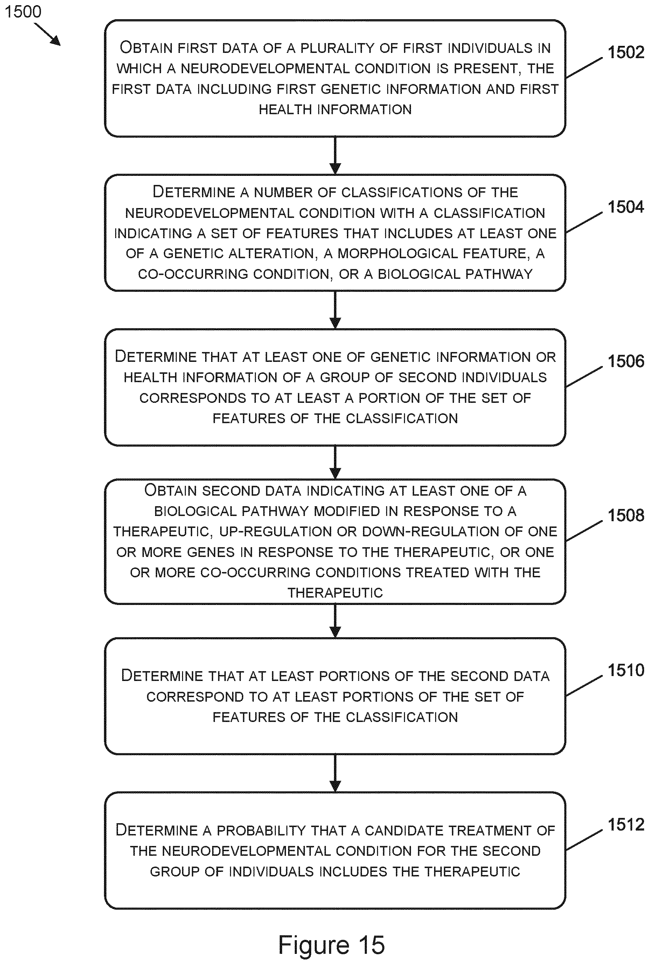

Figures 15-21 illustrate example methods for classifying individuals diagnosed with a neurodevelopmental condition and determining one or more treatments for the individuals. The example processes are illustrated as collections of blocks in logical flow graphs, which represent sequences of operations that can be implemented in hardware, software, or a combination thereof. The blocks are referenced by numbers. In the context of software, the blocks represent computer-executable instructions stored on one or more computer-readable media that, when executed by one or more processing units (such as hardware microprocessors), perform the recited operations. Generally, computer-executable instructions include routines, programs, objects, components, data structures, and the like that perform particular functions or implement particular data types. The order in which the operations are described is not intended to be construed as a limitation, and any number of the described blocks can be combined in any order and/or in parallel to implement the process.

Figure 15 is a flow diagram of an example process 1500 to determine a candidate treatment for individuals in which a neurodevelopmental condition is present, in accordance with one or more example implementations. The process 1500 can include, at operation 1502, obtaining first data of a plurality of first individuals in which a neurodevelopmental condition is present. The first data can include first genetic data and first health data of the plurality of individuals. In one or more illustrative examples, the neurodevelopmental condition can include autism spectrum disorder.

The genetic data can include full genome sequencing with respect to the number of individuals. In additional implementations, the genetic data can indicate the presence or absence of one or more gene alterations within the number of individuals. The genetic data can include deoxyribonucleic acid (DNA) information of individuals, ribonucleic acid (RNA) information of individuals, or combinations thereof. The genetic data can also include the expression levels of one or more genes of the individuals. In various implementations, the expression level of a gene can correspond to a level of one or more proteins or RNA transcripts that are produced in relation to the gene.

Additionally, the health data can include observations made by healthcare practitioners. In one or more implementations, the health data can include diagnoses of individuals with one or more biological conditions. The health data can also include laboratory test results for individuals. The laboratory test results can include levels of one or more analytes present in individuals. The analytes can include substances that can be detected through the blood, one or more additional fluids of individuals, and/or tissue. In addition, the health data can include results of diagnostic tests of individuals. The diagnostic test results can include imaging information obtained via one or more medical imaging technologies, such as electroencephalogram (EEG) data and electrocardiogram (ECG) data. In one or more implementations, the EEG data and/or the ECG data can also be included in the health data as electrical activity data. The diagnostic test results can also include results from one or more assays that have been performed with respect to fluids and/or tissue of individuals.

At operation 1504, the process 1500 can include determining a number of classifications of the neurodevelopmental condition with a classification of the number of classifications indicating a set of features that includes at least one of a genomic region, a morphological region, a co-occurring condition, or a biological pathway. In various examples, each classification can indicate a set of features that includes at least one of one or more genomic regions present in individuals in which the neurodevelopmental condition is present, one or more morphological features regions present in individuals in which the neurodevelopmental condition is present, one or more co-occurring conditions that are also present in individuals in which the neurodevelopmental condition is present, or one or more biological pathways that are disrupted in individuals in which the neurodevelopmental condition is present.

The process 1500 can include, at operation 1506, determining that at least one of genetic information or health information of a group of second individuals corresponds to at least a portion of the set of features of the classification. In this way, the group of second individuals can be associated with the classification. Additionally, at operation 1508 the process 1500 can include obtaining second data indicating at least one of a biological pathway modified in response to a therapeutic, up-regulation of one or more first genes in response to the therapeutic, down-regulation of one or more second genes in response to the therapeutic, or one or more additional conditions treated with the therapeutic.

Further, at operation 1510, the process 1500 can include determining that at least portions of the second data correspond to at least portions of the set of features of the classification. The process 1500 can also include, at operation 1512, determining a probability that a candidate treatment of the neurodevelopmental condition for the group of second individuals includes the therapeutic. In one or more illustrative examples, user interface data can be generated that indicates the probability that the therapeutic is a candidate treatment of the neurodevelopmental condition for the individual. In one or more implementations, the probability that a candidate treatment for the group of second individuals with respect to the neurodevelopmental condition can be based on information corresponding to a group of third individuals in which a co-occurring condition related to the neurodevelopmental condition is present. The third group of individuals can have at least a threshold decrease in one or more additional symptoms of an additional condition that is co-occurring with respect to the neurodevelopmental condition in response to the therapeutic. In various examples, the probability that the candidate treatment of the neurodevelopmental condition for the second group of individuals includes the therapeutic can be based at least partly on a measure of similarity between the group of second individuals and the group of third individuals.

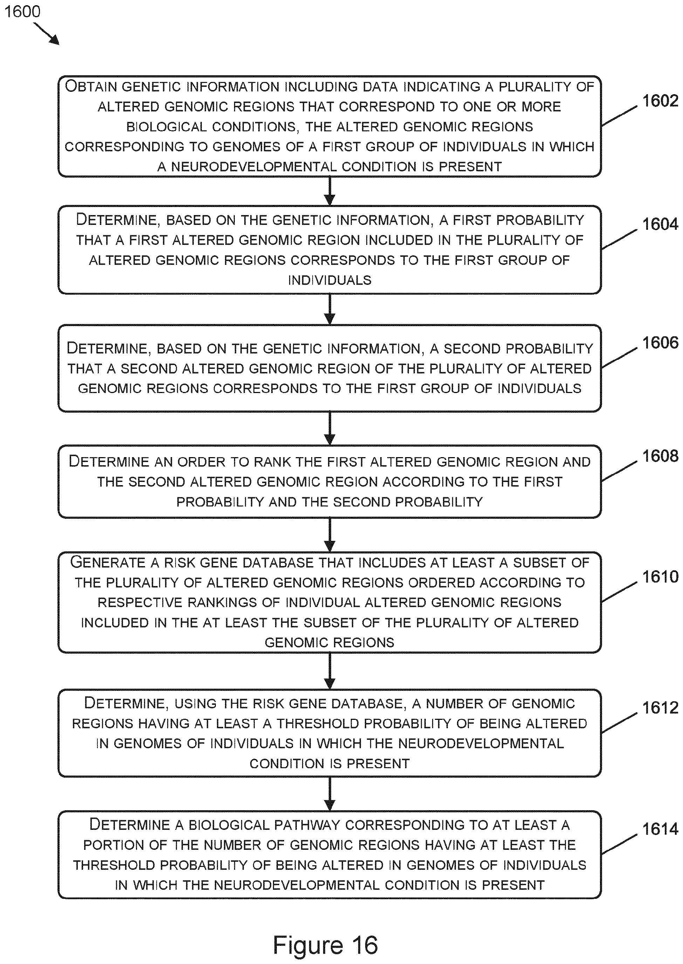

Figure 16 is a flow diagram of an example process 1600 to determine one or more biological pathways that can be used to determine subgroups of individuals in which a neurodevelopmental condition is present, in accordance with one or more example implementations.

At operation 1602, the process 1600 can include obtaining genetic information including data indicating a plurality of altered genomic regions that correspond to one or more biological conditions. The altered genomic regions can correspond to genomes of individuals in which at least one biological condition of the one or more biological conditions is present in relation to genomes of individuals in which the at least one biological condition is not present.

The process 1600 can include, at operation 1604, determining a first probability that a first altered genomic region included in the plurality of altered genomic regions corresponds to a group of individuals in which a neurodevelopmental condition is present. In addition, at operation 1606, the process 1600 can include determining a second probability that a second altered genomic region of the plurality of altered genomic regions corresponds to the group of individuals in which the neurodevelopmental condition is present. In various implementations, a probability that an altered genomic region corresponds to the group of individuals in which the neurodevelopmental condition is present can be determined by analyzing genetic information related to one or more biological pathways that may be disrupted in individuals in which the neurodevelopmental condition is present. Additionally, a probability that an altered genomic region corresponds to the group of individuals in which the neurodevelopmental condition is present can be determined based on altered genomic regions that are associated with biological conditions that correspond to one or more phenotypes that are related to individuals in which the neurodevelopmental condition is present.

Further, at operation 1608, the process 1600 can include determining an order to rank the first altered genomic region and the second altered genomic region according to the first probability and the second probability. In one or more illustrative examples, the first altered genomic region and the second altered genomic region can have respective allele frequencies that are individually no greater than about 0.001. In these scenarios, a first ranking of the first altered genomic region can be determined based on a first measure of conservation between a first gene related to the first altered genomic region and the first altered genomic region and a second ranking of the second altered genomic region can be determined based on a second measure of conservation between a second gene related to the second altered genomic region and the second altered genomic region. In one or more implementations, the first ranking can be greater than the second ranking based on the first measure of conservation being greater than the second measure of conservation.

The process 1600 can also include, at operation 1610, generating a risk gene database that includes at least a subset of the plurality of altered genomic regions ordered according to respective rankings of individual altered genomic regions included in the at least the subset of the plurality of altered genomic regions. Additionally, at operation 1612, the process 1600 can include determining, using the risk genes database, a number of altered genomic regions having at least a threshold probability of being present in genomes of individuals in which the neurodevelopmental condition is present.

At operation 1614, the process 1600 can include determining a biological pathway corresponding to at least a portion of the number of altered genomic regions having at least the threshold probability of being present in the genomes of individuals in which the neurodevelopmental condition is present. The biological pathway can be one biological pathway of a number of biological pathways that are associated with at least one altered genomic region included in the risk gene database, where each of the altered genomic regions have at least a threshold probability of being present in genomes of individuals in which the neurodevelopmental condition is present.

In one or more implementations, the number of biological pathways to use in the grouping of individuals can be minimized. That is, some biological pathways that are related to genes corresponding to altered genes in individuals in which the neurodevelopmental condition is present can be redundant with respect to other biological pathways that are related to genes that correspond to altered genes in individuals in which the neurodevelopmental condition is present. In various examples, the process used to eliminate redundant biological pathways can be based on whether or not the biological pathways have a parent-child relationship. Additionally, the elimination of redundant biological pathways can also depend of a number of genes associated with each biological pathway that also have at least a threshold probability of being altered in genomes of individuals in which the neurodevelopmental condition is present.

In one or more illustrative examples, for each biological pathway of the plurality of biological pathways that is associated with at least one gene included in the risk genes database having at least a threshold probability of being altered in genomes of individuals in which the neurodevelopmental condition is present, the number of the risk genes can be counted. The biological pathways that has at least a threshold number of risk genes can then be filtered based on the number of genes they share such that a subset of the plurality of biological pathways is produced and biological pathways corresponding to broader biological processes are excluded. Subsequently, pairs of biological pathways can be determined that have at least a threshold number of genes in common relative to the number of all genes in the biological pathways using overlap coefficient, Jaccard Index, Sørensen-Dice coefficient, Tversky index and/or mutual information. For example, one or more pairs of biological pathways having an overlap coefficient of at least 0.8 can be identified for genes in a first pathway in common with the genes in the second pathway relative to the number of genes in the smaller of the first and second pathways. In situations where a pair of biological pathways has a parent-child relationship, the biological pathway with the greater number of genes, including but not limited to risk genes can be determined to be redundant with respect to the biological pathway associated with fewer genes. In scenarios where there is not a parent-child relationship between a pair of biological pathways, each biological pathway included in the pair may not be considered redundant with respect to one another. In one or more implementations, the non-redundant pathways can be used to determine a probability that a therapeutic is a candidate treatment for the neurodevelopmental condition.

Although the illustrative example of FIG. 16 is directed to determining a biological pathway that corresponds to genomic regions that have at least a threshold probability of being altered in genomes of individuals in which the neurodevelopmental condition is present, at least a portion of the operations described with respect to FIG. 16 can also be performed in relation to determining one or more co-occurring conditions and/or one or more phenotypes that correspond to genomic regions that have at least the threshold probability of being altered in genomes of individuals in which the neurodevelopmental condition is present. For example, based on the number of genomic regions having at least the threshold probability of being altered in genomes of individuals in which the neurodevelopmental condition is present, as performed with respect to operation 1612, a co-occurring condition can be identified that corresponds to these genomic regions. In various examples, redundant co-occurring conditions that include one or more of the genomic regions having at least the threshold probability of being altered in genomes of individuals in which the neurodevelopmental condition is present can be eliminated in a manner similar to that described above with respect to biological pathways. To illustrate, for each co-occurring condition, a number of genomic regions included in the set of genomic regions having at least the threshold probability of being altered in genomes of individuals in which the neurodevelopmental condition is present can be determined. Additionally, co-occurring conditions having at least the threshold probability of being present in individuals with the neurodevelopmental condition that have larger numbers of the genomic regions can be eliminated from a group of co-occurring conditions and the non-redundant co-occurring conditions can be used to determine a group of altered genomic regions that can characterize individuals in which the neurodevelopmental condition is present.

In a further illustrative example, based on the number of genomic regions having at least the threshold probability of being altered in genomes of individuals in which the neurodevelopmental condition is present, as performed with respect to operation 1612, a phenotype can be identified that corresponds to these genomic regions. In various examples, redundant phenotypes that include one or more of the genomic regions having at least the threshold probability of being altered in genomes of individuals in which the neurodevelopmental condition is present can be eliminated in a manner similar to that described above with respect to biological pathways and co-occurring conditions. To illustrate, for each phenotype, a number of genomic regions included in the set of genomic regions having at least the threshold probability of being altered in genomes of individuals in which the neurodevelopmental condition is present can be determined. Additionally, phenotypes having at least the threshold probability of being included in individuals with the neurodevelopmental condition that have larger numbers can be eliminated from a group of phenotypes and the non-redundant phenotypes can be used to determine a group of altered genomic regions that can characterize individuals in which the neurodevelopmental condition is present.

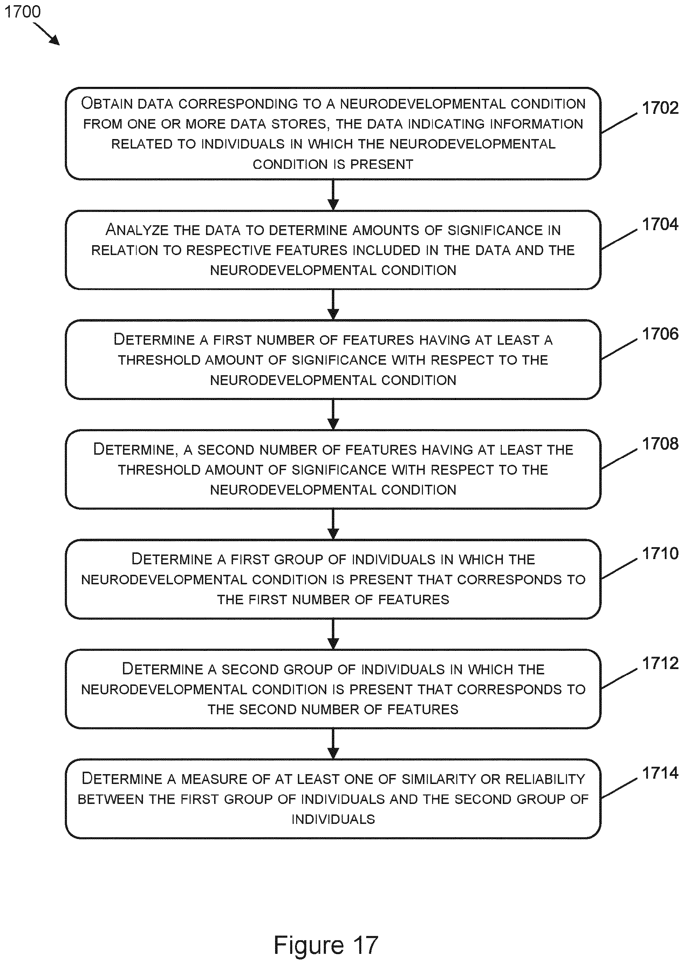

Figure 17 is a flow diagram of a first example process 1700 to determine features that can be used to identify subgroups of individuals in which a neurodevelopmental condition is present, in accordance with one or more example implementations.

The process 1700 can include, at operation 1702, obtaining data corresponding to a neurodevelopmental condition from one or more data stores, the data indicating information related to individuals in which the neurodevelopmental condition is present. At operation 1704, the process 1700 can include analyzing the data to determine amounts of significance in relation to respective features included in the data and the neurodevelopmental condition.

In addition, the process 1700 can include, at operation 1706, determining a first number of features having at least a threshold amount of significance with respect to the neurodevelopmental condition. The first number of features including at least one of one or more first genetic features, one or more first observable features, one or more first co-occurring conditions, or one or more first analytes.

Further, at operation 1708, the process 1700 can include determining a second number of features having at least the threshold amount of significance with respect to the neurodevelopmental condition. The second group of features including at least one of one or more second genetic features, one or more second observable features, one or more second co-occurring conditions, or one or more second analytes, wherein at least one feature included in the second number of features is different from at least one feature included in the first number of features. In one or more illustrative examples, the first group of features and the second group of features can be determined using at least one of one or more hierarchical-based clustering techniques, one or more partitioning-based clustering techniques, one or more density-based clustering techniques, one or more model-based clustering techniques such as a latent class analysis techniques, or one or more graph-based clustering techniques.

The process 1700 can also include, at operation 1710, determining a first group of individuals in which the neurodevelopmental condition is present that correspond to the first number of features. Additionally, at operation 1712, the process 1700 can include determining a second group of individuals in which the neurodevelopmental condition is present that corresponds to the second number of features. The second group of individuals can include at least one individual that is different from at least one individual included in the first group of individuals.

At operation 1714, the process 1700 can include determining a measure of reliability and/or similarity between the first group of individuals and the second group of individuals. In various implementations where the measure of reliability is less than a threshold measure of reliability, different sets of features can be determined for use in grouping individuals. For example, a third group of features can be determined that has at least the threshold amount of significance with respect to the neurodevelopmental condition. The third group of features can include at least one of one or more third genetic features, one or more third observable features, one or more third co-occurring conditions, or one or more third analytes and at least one feature included in the third number of features can be different from at least one feature included in the first number of features and at least one feature included in the second number of features. In addition, a fourth group of features can be determined that has at least the threshold amount of significance with respect to the neurodevelopmental condition. The fourth group of features can include at least one of one or more fourth genetic features, one or more fourth observable features, one or more fourth co-occurring conditions, or one or more fourth analytes. Further, at least one feature included in the fourth number of features can be different from at least one feature included in the first number of features, at least one feature included in the second number of features, and at least one feature included in the third number of features. In these situations, a third group of individuals in which the neurodevelopmental condition is present and that correspond to the third number of features can be determined where the third group of individuals includes at least one individual that is different from at least one individual included in the first group of individuals and at least one individual included in the second group of individuals. In addition, a fourth group of individuals in which the neurodevelopmental condition is present and that corresponds to the fourth number of features can be determined, where the fourth group of individuals includes at least one individual that is different from at least one individual included in the first group of individuals, at least one individual included in the second group of individuals, and at least one individual included in the third group of individuals. An additional measure of reliability can then be determined with respect to the third group of individuals and the fourth group of individuals.

In one or more implementations, the measures of reliability for groups of individuals that are determined using different sets of features can correspond to the use of different clustering techniques to determine the sets of features. For example, a first set of features can be determined using a k-means clustering technique, a second set of features can be determined using a latent class analysis technique, and a third set of features can be determined using a graph-based clustering technique. In various examples, the respective measures of reliability of the groupings of individuals determined using the sets of features generated by the different clustering techniques can be used to identify the clustering technique that has a best fit to the data obtained that corresponds to individuals in which the neurodevelopmental condition is present. Further, the sets of features and groupings of individuals determined using the clustering technique that has a best fit to the data can be prioritized in relation to sets of features and groupings of individuals determined using other clustering techniques.

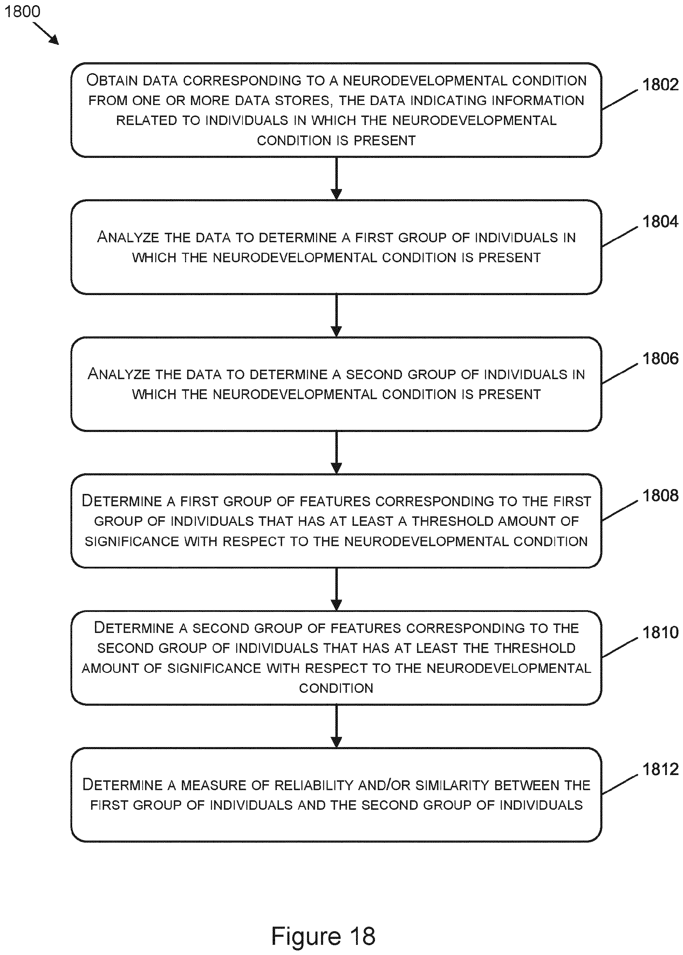

Figure 18 is a flow diagram of a second example process 1800 to determine features that can be used to identify subgroups of individuals in which a neurodevelopmental condition is present, in accordance with one or more example implementations.
The process 1800 can include, at operation 1802, obtaining data corresponding to a neurodevelopmental condition from one or more data stores, the data indicating information related to individuals in which the neurodevelopmental condition is present. At operation 1804, the process 1800 can include analyzing the data to determine a first group of individuals in which the neurodevelopmental condition is present. Additionally, the process 1800 can include, at operation 1806, analyzing the data to determine a second group of individuals in which the neurodevelopmental condition is present. The second group of individuals can include at least one individual that is different from at least one individual of the first group of individuals.

In one or more illustrative examples, the first group of individuals and the second group of individuals can be determined using at least one of one or more hierarchical-based clustering techniques, one or more partitioning-based clustering techniques, one or more density-based clustering techniques, one or more model-based clustering techniques such as a latent class analysis techniques, or one or more graph-based clustering techniques

Further, at operation 1808, the process 1800 can include determining a first group of features corresponding to the first group of individuals that has at least a threshold amount of significance with respect to the neurodevelopmental condition. The first group of features can include at least one of one or more first genetic features, one or more first observable features, one or more first co-occurring conditions, or one or more first analytes.

The process 1800 can also include, at operation 1810, determining a second group of features corresponding to the second group of individuals that has at least the threshold amount of significance with respect to the neurodevelopmental condition. The second group of features can include at least one of one or more second genetic features, one or more second observable features, one or more second co-occurring conditions, or one or more second analytes. At least one feature included in the second group of features can be different from at least one feature included in the first group of features.

In addition, at operation 1812, the process 1800 can include determining a measure of reliability and/or similarity between the first group of individuals and the second group of individuals. The measure of reliability can be determined by determining a first amount of similarity between first individuals included in the first group of individuals and determining a second similarity between the first individuals and second individuals included in the second group of individuals. In these situations, the measure of reliability can be determined based on the first amount of similarity and the second amount of similarity. In various examples, the measure of reliability between the first group of individuals and the second group of individuals can be determined based on determining levels of preservation between groups of individuals that have been determined using different sources of data.

In one or more implementations, the respective levels of preservation can be based on the number of features that are shared by individuals included in the respective pairs of groupings. To illustrate, the first level of preservation can be based on the number of features shared between the first individuals included in the first grouping and the number of the second individuals included in the second grouping. Additionally, a level of preservation for a pair of groupings can correspond to a threshold level of preservation in situations where at least a threshold number of individuals included in one grouping of the pair are also included in the other grouping of the pair. Further, in situations where the first level of preservation corresponds to a threshold level of preservation, the first group of individuals can include individuals from both the first grouping and the second grouping. That is, individuals from groupings that are generated using data from different data sources can be included in a single group of individuals that are associated with a particular set of features.

Further, the measures of reliability for groups of individuals can correspond to the use of different clustering techniques to determine groups of individuals. For example, a first group of individuals can be determined using a k-means clustering technique, a second group of individuals can be determined using a latent class analysis technique, and a third group of individuals can be determined using a graph-based clustering technique. In various examples, the respective measures of reliability of the groupings of individuals determined generated by the different clustering techniques can be used to identify the clustering technique that has a best fit to the data obtained that corresponds to individuals in which the neurodevelopmental condition is present. Further, the groupings of individuals determined using the clustering technique that has a best fit to the data can be prioritized in relation to groupings of individuals determined using other clustering techniques.

In various examples, a number of groups of individuals can be merged to produce a modified group of individuals in order to produce an increased measure of reliability and/or an increased level of preservation for the modified group in relation to the respective groups of individuals that comprise the modified group. For example, the first group of individuals can be combined with the second group of individuals to produce a modified group of individuals that has an increased measure of reliability and/or an increased level of preservation with respect to additional groups of individuals. The modified group of individuals can then be used to identify features that can be used to group additional individuals in which the neurodevelopmental condition is present.

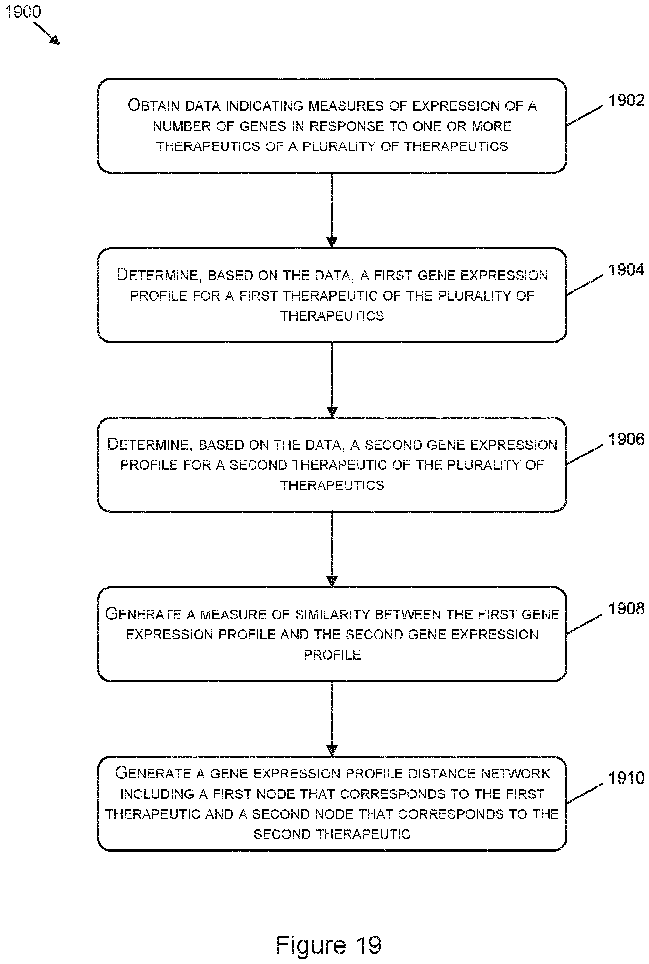

Figure 19 is a flow diagram of an example process 1900 to generate a gene expression profile distance network, in accordance with one or more example implementations.

At operation 1902, the process 1900 can include obtaining data indicating measures of expression of a number of genes in response to one or more therapeutics of a plurality of therapeutics. The process 1900 can include, at operation 1904, determining, based on the data a first gene expression profile for a first therapeutic of the plurality of therapeutics. The first gene expression profile can include a first group of genes that includes from about 50 to 500 first genes having at least a threshold amount of up-regulation in response to the therapeutic. The first gene expression profile can also include a second group of genes that includes from about 50 to 500 second genes having at least a threshold amount of down-regulation in response to the therapeutic. In one or more implementations, the first gene expression profile can be produced by generating a ranked list of genes based on respective amounts of up-regulation in response to the first therapeutic and respective amounts of down-regulation of the genes in response to the first therapeutic. In one or more illustrative examples, genes that are up-regulated in response to the first therapeutic can have greater rankings that genes that are down-regulated in response to the therapeutic with the ranking of each gene being based a magnitude of the amount of up-regulation for the gene or the amount of down-regulation for the gene. For example, the ranked list can indicate a most up-regulated gene having a highest ranking and move in descending order of ranking until the gene being most down-regulated in response to the therapeutic has the lowest ranking. In addition, a first number of genes of the ranked list can be identified that have at least a threshold amount of up-regulation and a second number of genes of the ranked list can be identified that have at least a threshold amount of down-regulation. In various examples, the first number of genes of the ranked list that have at least the threshold amount of up-regulation can correspond to the first group of genes included in the first gene expression profile. Further, the second number of genes of the ranked list that have at least the threshold amount of down-regulation can correspond to the second group of genes included in the first gene expression profile.

Also, at operation 1906, the process 1900 can include determining, based on the data, a second gene expression profile for a second therapeutic of the plurality of therapeutics. Similar to the first gene expression profile, the second gene expression profile can include a third group of genes that includes from about 50 to 500 third genes having at least the threshold amount of up-regulation in response to the therapeutic. In addition, the second gene expression profile can include a fourth group of genes that includes from about 50 to 500 fourth genes having at least the threshold amount of down-regulation in response to the therapeutic. The up-regulated third genes and the down-regulated fourth genes can be based on additional ranked list of genes that indicates respective amounts of up-regulation in response to the second therapeutic and respective amounts of down-regulation of the genes in response to the second therapeutic.

In addition, at operation 1908, the process 1900 can include generating a measure of similarity between the first gene expression profile distance network and the second gene expression profile distance network. The measure of similarity can be determined by analyzing the first group of genes with respect to a third group of genes to determine one or more first shared genes that are included in the first group of genes and included in the third group of genes. In addition, the second group of genes can be analyzed with respect to the fourth group of genes to determine one or more second shared genes that are included in the second group of genes and included in the fourth group of genes. In this way, genes of the first gene expression profile can be analyzed with respect to the second gene expression profile to identify shared genes between the first gene expression profile and the second gene expression profile. Additionally, the second gene expression profile can be analyzed with respect to the first gene expression profile to determine shared genes between the second gene expression profile and the first gene expression profile. For example, the third group of genes can be analyzed with respect to the first group of genes to determine one or more third shared genes that are included in the third group of genes and included in the first group of genes and the fourth group of genes can be analyzed with respect to the second group of genes to determine one or more fourth shared genes that are included in the fourth group of genes and included in the second group of genes. In various examples, the one or more first shared genes can be determined by performing a comparison in relation to a plurality of first pairs of genes with each first pair of genes including one gene selected from the first group of genes and an additional gene selected from the third group of genes. In addition, the one or more second shared genes can be determined by performing a comparison in relation to a plurality of second pairs of genes with each second pair of genes including one gene selected from the second group of genes and an additional gene selected from the fourth group of genes.

In one or more implementations, the measure of similarity can also be determined by determining a first component of the measure of similarity based on genes that are shared and genes that are not shared between the first gene expression profile and the second gene expression profile and determining a second component of the measure of similarity based on genes that are shared and genes that are not shared between the second gene expression profile and the first gene expression profile. In various examples, the first component of the measure of similarity can be combined with the second component of the measure of similarity. To illustrate, an average of the first component of the measure of similarity and the second component of the measure of similarity can be determined. In one or more illustrative examples, the measure of similarity can be determined by comparing a first gene selected from the first group of genes with a second gene selected from the third group of genes and determining that the first gene corresponds to the second gene. Consequently, a value of a first measure of similarity can be increased based on the first gene corresponding to the second gene. Additionally, a third gene selected from the first group of genes can be compared with a fourth gene selected from the third group of genes and, in one or more examples, the third gene does not correspond to the fourth gene. In these scenarios, the value of the first measure of similarity can be decreased based on a lack of correspondence between the third gene and the fourth gene. Thus, for each gene that is shared between pairs of groups of up-regulated genes and pairs of groups of down-regulated genes, a component of the measure of similarity can be increased. Further, for each gene that is not shared between pairs of groups of up-regulated genes and pairs of groups of down-regulated genes, a component of the measure of similarity can be decreased.

Further, the process 1900 can include, at operation 1910, generating a gene expression profile distance network include a first node that corresponds to the first therapeutic and a second node that corresponds to the second therapeutic. In one or more implementations, the gene expression profile distance network can include hundreds, up to thousands of nodes that each correspond to at least one therapeutic with the nodes being connected by edges. The length of the edges between respective nodes can correspond to a similarity between the gene expression profiles of the one or more therapeutics that correspond to the respective nodes. Additionally, the gene expression profile distance network can be used to determine probabilities that one or more therapeutics represented in the gene expression profile distance network can be candidate treatments for one or more groups of individuals in which a neurodevelopmental condition is present. For example, the genes of the group of individuals that are up-regulated can be compared with genes of one or more therapeutics that are down-regulated and the genes of the group of individuals that are down-regulated can be compared with genes of one or more therapeutics that are up-regulated.

Further, gene expression profile distance networks can be determined using information other than up-regulation and down-regulation of genes by therapeutics. For example, additional data can be obtained that indicates additional measures of expression of an additional number of genes in response to one or more additional sources of genetic expression modification that are different from therapeutics. In one or more illustrative examples, the additional source of genetic expression can include a biological condition, such as a disease or disorder, or short hairpin ribonucleic acid (shRNA) molecules. Based on the additional data, a first additional gene expression profile can be determined for a first additional source of genetic expression modification with the first additional gene expression profile indicating up-regulation of a first additional group of genes in response to the first additional source of genetic expression modification and down-regulation of a second additional group of genes in response to the first additional source of genetic expression modification. In addition, based on the additional data, a second additional gene expression profile can be determined for a second additional source of genetic expression modification with the second additional gene expression profile indicating up-regulation of a third additional group of genes in response to the second additional source of genetic expression modification and down-regulation of a fourth additional group of genes in response to the second additional source of genetic expression modification. In various examples, an additional measure of similarity between the first additional gene expression profile and the second additional gene expression profile can be generated. The additional measure of similarity can indicate a number of the first additional group of genes that correspond to a number of the third additional group of genes and a number of the second additional group of genes that correspond to a number of the fourth additional group of genes. In one or more implementations, an additional gene expression profile distance network can be generated with the additional gene expression profile distance network including a first additional node that corresponds to the first additional source of genetic expression modification and a second additional node that corresponds to the second additional source of genetic expression modification. The additional gene expression profile distance network can also include an additional edge between the first additional node and the second additional node with a length of the additional edge corresponding to the additional measure of similarity.

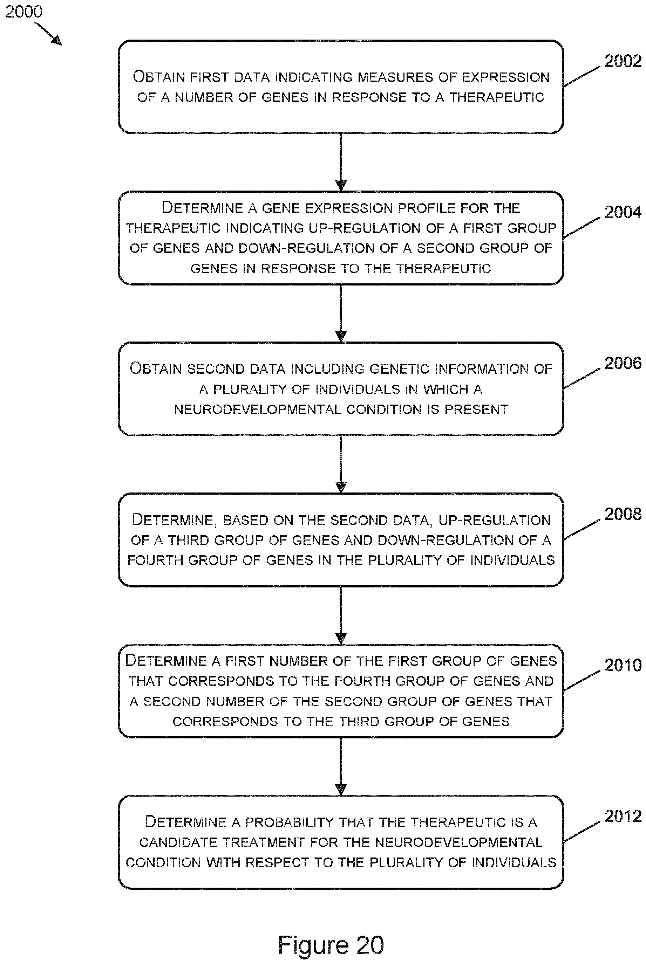

Figure 20 is a flow diagram of an example process 2000 to determine one or more therapeutics that can be candidate treatments for one or more subgroups of individuals in which a neurodevelopmental condition is present, in accordance with one or more example implementations.

At operation 2002, the process 2000 can include obtaining first data indicating measures of expression of a number of genes in response to a therapeutic. In addition, the process 2000 can include, at operation 2004, determining, based on the first data, a gene expression profile for the therapeutic, the gene expression profile indicating up-regulation of a first group of genes in response and down-regulation of a second group of genes in response to the therapeutic.

Further, at operation 2006, the process 2000 can include obtaining second data including genetic information of a plurality of individuals in which a neurodevelopmental condition is present. The process 2000 can also include, at operation 2008, determining, based on the second data, up-regulation of a third group of genes in the plurality of individuals and down-regulation of a fourth group of genes in the plurality of individuals. In various examples, a gene expression profile can be generated for the plurality of individuals that includes the third group of genes and the respective measures of up-regulation of the third group of genes in addition to the fourth group of genes and the respective measures of down-regulation of the fourth group of genes. To illustrate, an additional gene expression profile can be determined for the plurality of individuals. The additional gene expression profile can include the third group of genes and the fourth group of genes.

In one or more examples, the plurality of individuals can comprise a subgroup of a population of individuals in which the neurodevelopmental condition is present. In one or more implementations, different gene expression profiles can be generated for different subgroups within the population. For example, genetic information of an additional subgroup of the population of individuals in which the neurodevelopmental condition is present can be obtained and the genetic information can be used to generate an additional gene expression profile that indicates up-regulation of a fifth group of genes in the additional sub-group and down-regulation of a sixth group of genes in the additional subgroup. In one or more illustrative examples, the genes that are up-regulated and down-regulated in one subgroup can be different from the genes that are up-regulated and down-regulated in another subgroup. To illustrate, at least one gene of the fifth group of genes can be different from at least one gene included in the third group of genes and at least one gene of the sixth group of genes can be different from at least one gene included in the fourth group of genes.

In one or more examples, the gene expression profile for a subgroup of individuals can be comprised of a combination of gene expression profiles for at least a portion of the individuals included in the subgroup. In one or more implementations, a first additional gene expression profile can be generated for a first individual included in the plurality of individuals. The first additional gene expression profile can indicate up-regulation of a first additional group of genes in the first individual and down-regulation of a second additional group of genes in the first individual. In addition, a second additional gene expression profile can be generated for a second individual included in the plurality of individuals. The second additional gene expression profile can indicate up-regulation of a third additional group of genes in the second individual and down-regulation of a fourth additional group of genes in the second individual. Further, a consensus gene expression profile can be generated based on at least the first additional gene expression profile and the second additional gene expression profile. The consensus gene expression profile can indicate up-regulation of a fifth additional group of genes and down-regulation of a sixth additional group of genes. The fifth additional group of genes can include at least a portion of the first additional group of genes and at least a portion of the third additional group of genes. The sixth additional group of genes can include at least a portion of the second additional group of genes and at least a portion of the fourth additional group of genes.

In one or more illustrative implementations, the consensus gene expression profile can be produced by generating a plurality of additional gene expression profiles for a plurality of individuals with each additional gene expression profile indicating up-regulation of one or more genes and down-regulation of one or more genes of a respective individual included in the plurality of individuals. Subsequently, a plurality of merged gene expression profiles can be generated based on the plurality of additional gene expression profiles. Each merged gene expression profile can be generated based on a first plurality of up-regulated genes and a first plurality of down-regulated genes of a first additional gene expression profile of the plurality of additional gene expression profiles that corresponds to a first individual and on a second plurality of up-regulated genes and a second plurality of down-regulated genes of a second additional gene expression profile of the plurality of additional gene expression profiles that corresponds to a second individual. For example, a merged gene expression profile can include average rankings of at least a portion of the up-regulated and down-regulated genes from the first additional gene expression profile and at least a portion of the up-regulated and down-regulated genes from the second additional gene expression profile. The merged gene expression profiles can also include a number of up-regulated genes from each individual gene expression profile, such as the top 125 up-regulated genes, and a number of down-regulated genes from each individual gene expression profile, such as the bottom 125 down-regulated genes. Further, an overall ranking of genes included in the plurality of additional gene expression profiles can be determined based on rankings of the genes in at least a portion of the plurality of merged gene expression profiles and the overall ranking of genes can correspond to the consensus gene expression profile. In some cases, a first number of the overall up-regulated genes (e.g., top 250) from the merged profiles can be included in the consensus gene expression profile and a second number of overall down-regulated genes (e.g., bottom 250) from the merged profiles can be included in the consensus gene expression profile.

At operation 2010, the process 2000 can include determining a first number of the first group of genes that corresponds to the fourth group of genes and a second number of the second group of genes that corresponds to the third group of genes. In this way, the inverse of the gene expression profile of the plurality of individuals is analyzed with respect to the gene expression profile of the therapeutic.

Additionally, at operation 2012, the process 2000 can include determining, based on the first number of the first group of genes that corresponds to the fourth group of genes and the second number of the second group of genes that corresponds to the third group of genes, a probability that the therapeutic is a candidate treatment for the neurodevelopmental condition with respect to the plurality of individuals. In various examples, the probability that the therapeutic is a candidate treatment for the neurodevelopmental condition with respect to the plurality of individuals can be less than a threshold probability. In one or more implementations, a gene expression profile of an additional therapeutic can be analyzed with respect to at least a portion of the up-regulated and down-regulated genes of the plurality of individuals to determine an additional probability of the additional therapeutic being a candidate treatment for the neurodevelopmental condition with respect to the plurality of individuals. In additional examples, a gene expression profile indicated a number of up-regulated genes and a number of down-regulated genes for a combination of therapeutics can be analyzed with respect to at least a portion of the up-regulated genes and down-regulated genes of the plurality of individuals to determine a probability that the combination of therapeutics can be a candidate treatment for the neurodevelopmental condition with respect to the plurality of individuals. In one or more illustrative examples, a combination of therapeutics can have greater than a threshold probability of being a candidate treatment for the neurodevelopmental condition with respect to the plurality of individuals in situations where at least one of the therapeutics included in the combination of therapeutics does not individually have at least the threshold probability of being a candidate treatment for the neurodevelopmental condition with respect to the plurality of individuals.

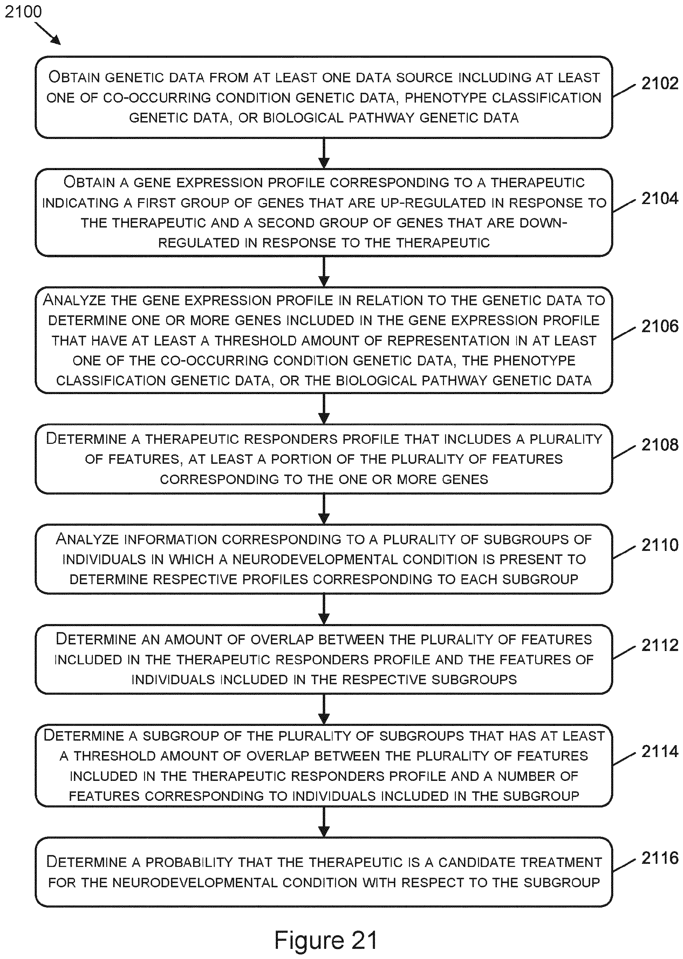

Figure 21 is a flow diagram of an example process 2100 to determine features of a group of individuals that have at least a threshold probability of being responsive to a therapeutic in relation to a neurodevelopmental condition, in accordance with one or more example implementations.

At operation 2102, the process 2100 can include obtaining genetic data from at least one data source including at least one of co-occurring condition genetic data, phenotype classification genetic data, or biological pathway genetic data. The co-occurring condition genetic data can be related to a first number of genes that are over-expressed in individuals in which a neurodevelopmental condition and an additional biological condition are present and a second number of genes that are under-expressed in individuals in which the neurodevelopmental condition and the additional biological condition are present. The phenotype classification genetic data can indicate one or more genes that correspond to at least one phenotype. The biological pathway genetic data can indicate one or more genes that are expressed in relation to at least one biological pathway. In one or more examples, the group of individuals from which the co-occurring condition genetic data is generated can include one or more individuals that are different from the group of individuals from which the phenotype classification data and the biological pathway genetic data are generated. Additionally, the group of individuals from which the phenotype classification data are generated can be different from the group of individuals from which the biological pathway genetic data is generated.

The process 2100 can also include, at operation 2104, obtaining a gene expression profile corresponding to a therapeutic. The gene expression profile can indicate a first group of genes that are up-regulated in response to the therapeutic and a second group of genes that are down-regulated in response to the therapeutic. In one or more implementations, the gene expression profile can be produced by generating a ranked list of genes based on respective amounts of up-regulation and respective amounts of down-regulation of a plurality of genes in response to the therapeutic. Additionally, a first number of genes of the ranked list that have at least a threshold amount of up-regulation can be determined and a second number of genes of the ranked list that have at least a threshold amount of down-regulation can also be determined. In these scenarios, the first group of genes included in the gene expression profile can include the first number of genes having at least the threshold amount of up-regulation and the second group of genes included in the gene expression profile can include the second number of genes having at least the threshold amount of down-regulation.

In addition, at operation 2106, the process 2100 can include analyzing the gene expression profile in relation to the genetic data obtained from the at least one data source to determine one or more genes included in the gene expression profile that have at least a threshold amount of representation in at least one of the co-occurring condition genetic data, the phenotype classification genetic data, or the biological pathway genetic data.

Further, the process 2100 can include, at operation 2108, determining a therapeutic responders profile that includes a plurality of features, at least a portion of the plurality of features corresponding to the one or more genes. The therapeutic responders profile can include at least one of a biological pathway that is disrupted in individuals in which the neurodevelopmental condition is present, a co-occurring biological condition that is present in individuals in which the neurodevelopmental condition is present, a phenotype that is related to individuals in which the neurodevelopmental condition is present, levels of analytes present in individuals in which the neurodevelopmental condition is present, or a morphological condition that is present in individuals in which the neurodevelopmental condition is present.

In various examples, the therapeutic responders profile can include an additional gene expression profile that is generated by determining a first ranking of a gene included in the gene expression profile based on an amount of up-regulation of the gene in response to the therapeutic or an amount of down-regulation of the gene in response to the therapeutic. Additionally, a determination can be made that the gene is present in at least one of the co-occurring condition genetic data, phenotype classification genetic data, or biological pathway genetic data. The additional gene expression profile can be generated that includes the gene, with the gene having a second ranking in the additional gene expression profile where the second ranking is based on the first ranking. In one or more illustrative examples, the first ranking can be different from the second ranking. In one or more implementations, a determination can be made that an additional gene included in the gene expression profile is not present in at least one of the co-occurring condition genetic data, phenotype classification genetic data, or biological pathway genetic data. In these scenarios, the additional gene may be excluded from the additional gene expression profile.

The process 2100 can also include, at operation 2110, analyzing information corresponding to a plurality of subgroups of individuals in which a neurodevelopmental condition is present to determine respective profiles corresponding to each of the subgroups. The respective profiles each can indicate features of individuals included in the respective subgroups.

Additionally, the process 2100 can include, at operation 2112, determining an amount of overlap between the plurality of features included in the therapeutic responders profile and the features of individuals included in the respective subgroups.

At operation 2114, the process 2100 can include determining a subgroup of the plurality of subgroups that has at least a threshold amount of overlap between the plurality of features included in the therapeutic responders profile and a number of features corresponding to individuals included in the subgroup.

The process 2100 can include, at operation 2116, determining a probability that the therapeutic is a candidate treatment for the neurodevelopmental condition with respect to the subgroup. In one or more illustrative examples, the probability that the therapeutic is a candidate treatment for the neurodevelopmental condition with respect to the subgroup can correspond to a probability that the therapeutic alleviates at least a portion of the symptoms of the subgroup with respect to the neurodevelopmental condition. In one or more implementations, the probability that the therapeutic is a candidate treatment for the neurodevelopmental condition with respect to the subgroup can be based on a measure of similarity related to an amount of overlap between the therapeutic responders profile and the number of features corresponding to the individuals included in the subgroup.

In various examples, the measure of similarity can be based on an amount of overlap between the additional gene expression profile included in the therapeutic responders profile and an inverse of a further gene expression profile for the subgroup. The further gene expression profile can indicate a third group of genes that are up-regulated in individuals included in the subgroup and a fourth group of genes that are down-regulated in individuals included in the subgroup. In one or more implementations, the subgroup gene expression profile can be produced by determining up-regulation of a fifth group of genes in the plurality of individuals and down-regulation of a sixth group of genes in the plurality of individuals. The information used to generate the subgroup gene expression profile can include genetic information of a plurality of individuals included in a plurality of subgroups of individuals in which the neurodevelopmental condition is present.

A first measure of similarity used to determine an overall measure of similarity that corresponds to the probability that the therapeutic is a candidate treatment for the neurodevelopmental condition with respect to the subgroup can be produced by comparing individual genes selected from the up-regulated group of genes included in the gene expression profile of the therapeutic responders profile with individual genes included in the down-regulated group of genes included in the subgroup gene expression profile. In these situations, a number of first pairs of shared genes can be determined between the individual genes included in the up-regulated group of genes of the gene expression profile of the therapeutic responders profile and the individual genes included in the down-regulated group of genes of the subgroup gene expression profile. Each first pair of shared genes can include one gene included in the up-regulated group of genes of the gene expression profile of the therapeutic responders profile and an additional gene included in the down-regulated group of genes included in the subgroup gene expression profile. Further, a number of first pairs of differing genes between the individual genes included in the up-regulated group of genes of the gene expression profile of the therapeutic responders profile and the individual genes included in the down-regulated group of genes included in the subgroup gene expression profile. Each first pair of differing genes can include a gene included in the up-regulated group of genes of the gene expression profile of the therapeutic responders profile and another gene included in the down-regulated group of genes included in the subgroup gene expression profile. In one or more illustrative examples, a value of a first measure of similarity can be increased based on the number of first pairs of shared genes and the value of the first measure of similarity can be decreased based on the number of first pairs of differing genes.

In addition, a second measure of similarity used to determine the overall measure of similarity that corresponds to the probability that the therapeutic is a candidate treatment for the neurodevelopmental condition with respect to the subgroup can be produced by comparing individual genes selected from the down-regulated group of genes of the gene expression profile of the therapeutic responders profile with individual genes included in the up-regulated group of genes of the subgroup gene expression profile. In one or more implementations, a number of second pairs of shared genes can be determined between the individual genes included in the down-regulated group of genes of the gene expression profile of the therapeutic responders profile and the individual genes included in the up-regulated group of genes of the subgroup gene expression profile. Each second pair of shared genes can include one gene included in the down-regulated group of genes of the gene expression profile of the therapeutic responders profile and an additional gene included in the up-regulated group of genes of the subgroup gene expression profile. Also, a number of second pairs of differing genes between the individual genes included in the down-regulated group of genes of the gene expression profile of the therapeutic responders profile and the individual genes included in the up-regulated group of genes of the subgroup gene expression profile. Each second pair of differing genes can include a gene included in the down-regulated group of genes of the gene expression profile of the therapeutic responders profile and another gene included in the up-regulated group of genes of the subgroup gene expression profile. In these scenarios, the value of the second measure of similarity can be increased based on the number of second pairs of shared genes and the value of the second measure of similarity can be decreased based on the number of second pairs of shared genes. The second measure of similarity can then be combined with the first measure of similarity to generate the overall measure of similarity.

In further examples, features included in the therapeutic responders profile can be determined using human protein interaction data indicating a group of genes within a network of genes that are regulated, such as up-regulated and/or down-regulated, in response to the therapeutic. The human protein interaction data can also indicate interactions between the group of genes and proteins found in one or more populations of humans. The human protein interaction data can be obtained from one or more data sources. In various examples, an amount of representation of the genes included in the network of genes can be determined in relation to the co-occurring condition genetic data, the phenotype classification data, and/or the biological pathway genetic data. One or more genes having at least a threshold amount of representation can be included in the therapeutic responders profile. In this way, at least a portion of the genes included in the therapeutic responders profile can be identified based on interactions between proteins and a group of genes that are regulated by the therapeutic.

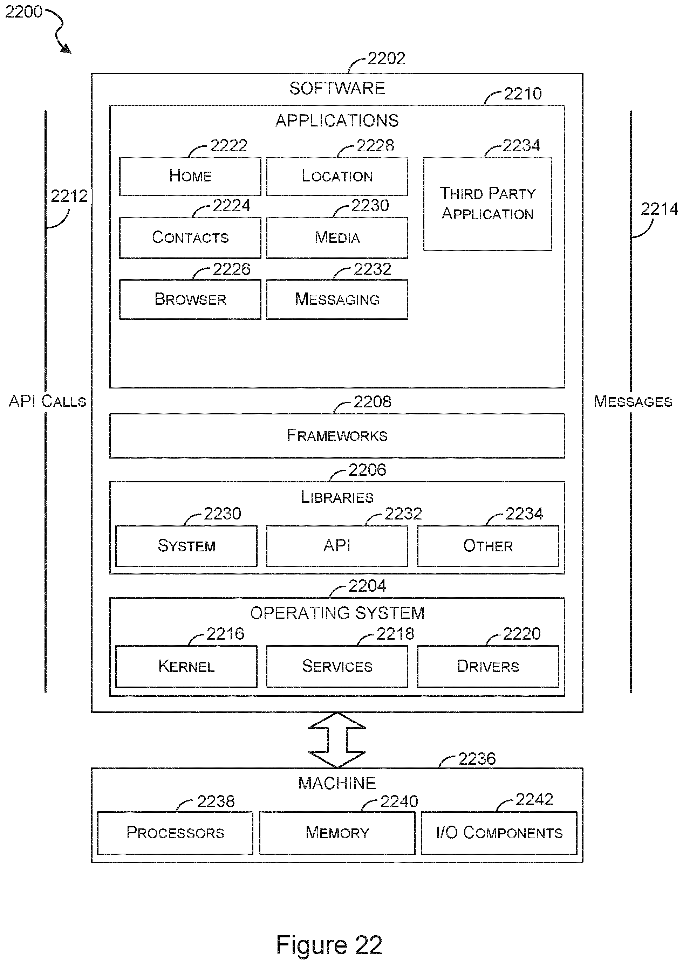

Figure 22 is a block diagram illustrating an example architecture 2200 of software 2202, which can be installed on any one or more of the devices described herein, in accordance with one or more example implementations. Figure 22 is merely a non-limiting example of a software architecture, and it will be appreciated that many other architectures can be implemented to facilitate the functionality described herein. In various embodiments, the software 2202 is implemented by hardware such as a machine 2236 that includes processors 2238, memory 2240, and input/output (I/O) components 2242. In this example architecture 2200, the software 2202 can be conceptualized as a stack of layers where each layer may provide a particular functionality. For example, the software 2202 includes layers such as an operating system 2004, libraries 2206, frameworks 2208, and applications 2210. Operationally, the applications 2210 invoke API calls 2212 through the software stack and receive messages 2214 in response to the API calls 2212, consistent with one or more implementations.

In various implementations, the operating system 2204 manages hardware resources and provides common services. The operating system 2204 includes, for example, a kernel 2216, services 2218, and drivers 2220. The kernel 2216 acts as an abstraction layer between the hardware and the other software layers, consistent with one or more implementations. For example, the kernel 2216 provides memory management, processor management (e.g., scheduling), component management, networking, and security settings, among other functionality. The services 2218 can provide other common services for the other software layers. The drivers 2220 are responsible for controlling or interfacing with the underlying hardware, according to some embodiments. For instance, the drivers 2220 can include display drivers, camera drivers, BLUETOOTH® or BLUETOOTH® Low-Energy drivers, flash memory drivers, serial communication drivers (e.g., Universal Serial Bus (USB) drivers), Wi-Fi® drivers, audio drivers, power management drivers, and so forth.

In one or more implementations, the libraries 2206 provide a low-level common infrastructure utilized by the applications 2210. The libraries 2206 can include system libraries 2230 (e.g., C standard library) that can provide functions such as memory allocation functions, string manipulation functions, mathematic functions, and the like. In addition, the libraries 2206 can include API libraries 2232 such as media libraries (e.g., libraries to support presentation and manipulation of various media formats such as Moving Picture Experts Group-4 (MPEG4), Advanced Video Coding (H.264 or AVC), Moving Picture Experts Group Layer-3 (MP3), Advanced Audio Coding (AAC), Adaptive Multi-Rate (AMR) audio codec, Joint Photographic Experts Group (JPEG or JPG), or Portable Network Graphics (PNG)), graphics libraries (e.g., an OpenGL framework used to render in 2D and 3D in a graphic context on a display), database libraries (e.g., SQLite to provide various relational database functions), web libraries (e.g., WebKit to provide web browsing functionality), and the like. The libraries 2206 can also include a wide variety of other libraries 2234 to provide many other APIs to the applications 2210.

The frameworks 2208 provide a high-level common infrastructure that can be utilized by the applications 2210, according to some embodiments. For example, the frameworks 2208 provide various graphical user interface (GUI) functions, high-level resource management, high-level location services, and so forth. The frameworks 2208 can provide a broad spectrum of other APIs that can be utilized by the applications 2210, some of which may be specific to a particular operating system 2204 or platform.

In one or more example implementations, the applications 2210 can include at least one of a home application 2222, a contacts application 2224, a browser application 2226, a location application 2228, a media application 2230, a messaging application 2232, or a third-party application 2234. According to one or more implementations, the applications 2210 are programs that execute functions defined in the programs. Various programming languages can be employed to create one or more of the applications 2210, structured in a variety of manners, such as object-oriented programming languages (e.g., Objective-C, Java, or C++) or procedural programming languages (e.g., C or assembly language). In an example, the third-party application 2234 (e.g., an application developed using the ANDROID™ or IOS™ software development kit (SDK) by an entity other than the vendor of the particular platform) may be mobile software running on a mobile operating system such as IOS™, ANDROID™, WINDOWS® Phone, or another mobile operating system. In this example, the third-party application 2234 can invoke the API calls 2212 provided by the operating system 2204 to facilitate functionality described herein.

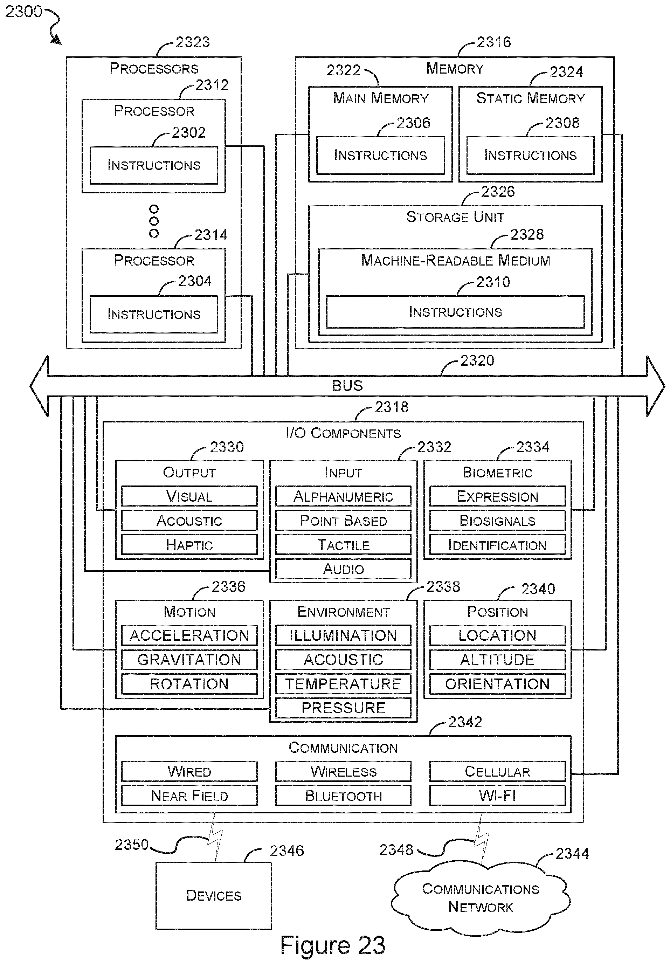

Figure 23 illustrates a diagrammatic representation of an example machine 2300 in the form of a computer system within which a set of instructions may be executed for causing the machine 2300 to perform any one or more of the methodologies discussed herein, in accordance with one or more example implementations. Specifically, Figure 23 shows a diagrammatic representation of the machine 2300 in the example form of a computer system, within which instructions (e.g., software, a program, an application, an applet, an app, or other executable code) for causing the machine 2300 to perform any one or more of the methodologies discussed herein may be executed. For example, the instructions 2316 may cause the machine 2300 to execute one or more of the operations described with respect to Figures 1-21. Instructions can transform the general, non-programmed machine 2300 into a particular machine 2300 programmed to carry out the described and illustrated functions in the manner described. In one or more implementations, the machine 2300 operates as a standalone device or may be coupled (e.g., networked) to other machines. In a networked deployment, the machine 2300 can operate in the capacity of a server machine or a client machine in a server-client network environment, or as a peer machine in a peer-to-peer (or distributed) network environment. The machine 2300 can comprise, but not be limited to, a server computer, a client computer, a personal computer (PC), a tablet computer, a laptop computer, a netbook, a set-top box (STB), a personal digital assistant (PDA), an entertainment media system, a cellular telephone, a smart phone, a mobile device, a wearable device (e.g., a smart watch), a smart home device (e.g., a smart appliance), other smart devices, a web appliance, a network router, a network switch, a network bridge, or any machine capable of executing computer-readable instructions, sequentially or otherwise, that specify actions to be taken by the machine 2300. Further, while only a single machine 2300 is illustrated, the term "machine" shall also be taken to include a collection of machines 2300 that individually or jointly execute the instructions 2316 to perform any one or more of the methodologies discussed herein.

The machine 2300 may include processors 2321, memory 2316, and I/O components 2318, that can be configured to communicate with each other such as via a bus 2320. In one or more example implementations, the processors 2321 (e.g., a central processing unit (CPU), a reduced instruction set computing (RISC) processor, a complex instruction set computing (CISC) processor, a graphics processing unit (GPU), a digital signal processor (DSP), an application-specific integrated circuit (ASIC), a radio-frequency integrated circuit (RFIC), another processor, or any suitable combination thereof) may include, for example, a processor 2312 and a processor 2314 that may execute the instructions 2302 and 2304. The term "processor" is intended to include multi-core processors that may comprise two or more independent processors (sometimes referred to as "cores") that may execute instructions 2302, 2304 contemporaneously. Although Figure 23 shows multiple processors 2321, the machine 2300 can include a single processor 2312 with a single core, a single processor 2312 with multiple cores (e.g., a multi-core processor 2312), multiple processors 2312, 2314 with a single core, multiple processors 2312, 2314 with multiple cores, or any combination thereof.

The memory 2316 may include a main memory 2322, a static memory 2324, and a storage unit 2326, each accessible to the processors 2321 such as via the bus 2320. The main memory 2322, the static memory 2324, and the storage unit 2326 store the instructions 2306, 2308, 2310 embodying any one or more of the methodologies or functions described herein. The instructions 2306, 2308, 2310 can also reside, completely or partially, within the main memory 2322, within the static memory 2324, within the storage unit 2326, within at least one of the processors 2321 (e.g., within the processor's cache memory), or any suitable combination thereof, during execution thereof by the machine 2300. The instructions 2310 of the storage unit 2326 can be stored by a machine-readable medium 2328.

The I/O components 2318 can include a wide variety of components to receive input, provide output, produce output, transmit information, exchange information, capture measurements, and so on. The specific I/O components 2318 that are included in a particular machine 2300 will depend on the type of machine. For example, portable machines such as mobile phones will likely include a touch input device or other such input mechanisms, while a headless server machine will likely not include such a touch input device. It will be appreciated that the I/O components 2318 can include many other components that are not shown in Figure 23. The I/O components 2318 are grouped according to functionality merely for simplifying the following discussion, and the grouping is in no way limiting. In various example implementations, the I/O components 2318 can include output components 2330 and input components 2332. The output components 2330 can include visual components (e.g., a display such as a plasma display panel (PDP), a light-emitting diode (LED) display, a liquid crystal display (LCD), a projector, or a cathode ray tube (CRT)), acoustic components (e.g., speakers), haptic components (e.g., a vibratory motor, resistance mechanisms), other signal generators, and so forth. The input components 2332 can include alphanumeric input components (e.g., a keyboard, a touch screen configured to receive alphanumeric input, a photo-optical keyboard, or other alphanumeric input components), point-based input components (e.g., a mouse, a touchpad, a trackball, a joystick, a motion sensor, or another pointing instrument), tactile input components (e.g., a physical button, a touch screen that provides location and/or force of touches or touch gestures, or other tactile input components), audio input components (e.g., a microphone), and the like.

In further example embodiments, the I/O components 2318 can include biometric components 2334, motion components 2336, environmental components 2338, and/or position components 2340, among a wide array of other components. For example, the biometric components 2334 can include components to detect expressions (e.g., hand expressions, facial expressions, vocal expressions, body gestures, or eye tracking), measure biosignals (e.g., blood pressure, heart rate, body temperature, perspiration, or brain waves), identify a person (e.g., voice identification, retinal identification, facial identification, fingerprint identification, or electroencephalogram-based identification), and the like. The motion components 2336 can include acceleration sensor components (e.g., accelerometer), gravitation sensor components, rotation sensor components (e.g., gyroscope), and so forth. The environmental components 2338 can include, for example, illumination sensor components (e.g., photometer), temperature sensor components (e.g., one or more thermometers that detect ambient temperature), humidity sensor components, pressure sensor components (e.g., barometer), acoustic sensor components (e.g., one or more microphones that detect background noise), proximity sensor components (e.g., infrared sensors that detect nearby objects), gas sensors (e.g., gas detection sensors to detect concentrations of hazardous gases for safety or to measure pollutants in the atmosphere), or other components that may provide indications, measurements, or signals corresponding to a surrounding physical environment. The position components 2340 can include location sensor components (e.g., a Global Positioning System (GPS) receiver component), altitude sensor components (e.g., altimeters or barometers that detect air pressure from which altitude may be derived), orientation sensor components (e.g., magnetometers), and the like.

Communication may be implemented using a wide variety of technologies. The I/O components 2318 can include communication components 2342 operable to couple the machine 2300 to a network 2344 or devices 2346 via a coupling 2348 and a coupling 2350, respectively. For example, the communication components 2342 can include a network interface component or another suitable device to interface with the network 2344. In further examples, the communication components 2342 can include wired communication components, wireless communication components, cellular communication components, near field communication (NFC) components, Bluetooth® components (e.g., Bluetooth® Low Energy), Wi-Fi® components, and other communication components to provide communication via other modalities. The devices 2346 can be another machine or any of a wide variety of peripheral devices (e.g., coupled via a USB).

Moreover, the communication components 2342 can detect identifiers or include components operable to detect identifiers. For example, the communication components 2342 can include radio-frequency identification (RFID) tag reader components, NFC smart tag detection components, optical reader components (e.g., an optical sensor to detect one-dimensional bar codes such as Universal Product Code (UPC) bar code, multi-dimensional bar codes such as QR code, Aztec code, Data Matrix, Dataglyph, MaxiCode, PDF417, Ultra Code, UCC RSS-2D bar code, and other optical codes), or acoustic detection components (e.g., microphones to identify tagged audio signals). In addition, a variety of information may be derived via the communication components 2342, such as location via Internet Protocol (IP) geolocation, location via Wi-Fi® signal triangulation, location via detecting an NFC beacon signal that may indicate a particular location, and so forth.

The various memories (i.e., 2322, 2324, 2326, and/or memory of the processor(s) 2321) and/or the machine-readable medium 2310 can store one or more sets of instructions 2302, 2304, 2306, 2308, 2310 and data structures (e.g., software) embodying or utilized by any one or more of the methodologies or functions described herein. These instructions (e.g., the instructions 2302 2304, 2306, 2308, 2310), when executed by the processor(s) 2321, cause various operations to implement the disclosed embodiments.

As used herein, the terms "machine-storage medium," "device-storage medium," and "computer-storage medium" mean the same thing and may be used interchangeably. The terms refer to a single or multiple storage devices and/or media (e.g., a centralized or distributed database, and/or associated caches and servers) that store executable instructions 2302, 2304, 2306, 2308, 2310, and/or data. The terms shall accordingly be taken to include, but not be limited to, solid-state memories, and optical and magnetic media, including memory internal or external to processors 2321. Specific examples of machine-storage media, computer-storage media, and/or device-storage media include non-volatile memory, including by way of example semiconductor memory devices, e.g., erasable programmable read-only memory (EPROM), electrically erasable programmable read-only memory (EEPROM), field-programmable gate array (FPGA), and flash memory devices; magnetic disks such as internal hard disks and removable disks; magneto-optical disks; and CD-ROM and DVD-ROM disks. The terms "machine-storage media," "computer-storage media," and "device-storage media" specifically exclude carrier waves, modulated data signals, and other such media, at least some of which are covered under the term "signal medium" discussed below.

In various example embodiments, one or more portions of the network 2344 may be an ad hoc network, an intranet, an extranet, a virtual private network (VPN), a local-area network (LAN), a wireless LAN (WLAN), a wide-area network (WAN), a wireless WAN (WWAN), a metropolitan-area network (MAN), the Internet, a portion of the Internet, a portion of the public switched telephone network (PSTN), a plain old telephone service (POTS) network, a cellular telephone network, a wireless network, a Wi-Fi® network, another type of network, or a combination of two or more such networks. For example, the network 2344 or a portion of the network 2344 can include a wireless or cellular network, and the coupling 2348 may be a Code Division Multiple Access (CDMA) connection, a Global System for Mobile communications (GSM) connection, or another type of cellular or wireless coupling. In this example, the coupling 2348 can implement any of a variety of types of data transfer technology, such as Single Carrier Radio Transmission Technology (1xRTT), Evolution-Data Optimized (EVDO) technology, General Packet Radio Service (GPRS) technology, Enhanced Data rates for GSM Evolution (EDGE) technology, third Generation Partnership Project (3GPP) including 3G, fourth generation wireless (4G) networks, Universal Mobile Telecommunications System (UMTS), High-Speed Packet Access (HSPA), Worldwide Interoperability for Microwave Access (WiMAX), Long-Term Evolution (LTE) standard, others defined by various standard-setting organizations, other long-range protocols, or other data transfer technology.

The instructions 2302, 2304, 2306, 2308, 2310 can be transmitted or received over the network 2344 using a transmission medium via a network interface device (e.g., a network interface component included in the communication components 2342) and utilizing any one of a number of well-known transfer protocols (e.g., Hypertext Transfer Protocol (HTTP)). Similarly, the instructions 2302, 2304, 2306, 2308, 2310 can be transmitted or received using a transmission medium via the coupling 2350 (e.g., a peer-to-peer coupling) to the devices 2346. The terms "transmission medium" and "signal medium" mean the same thing and may be used interchangeably in this disclosure. The terms "transmission medium" and "signal medium" shall be taken to include any intangible medium that is capable of storing, encoding, or carrying the instructions 2302, 2304, 2306, 2308, 2310 for execution by the machine 2300, and include digital or analog communications signals or other intangible media to facilitate communication of such software. Hence, the terms "transmission medium" and "signal medium" shall be taken to include any form of modulated data signal, carrier wave, and so forth. The term "modulated data signal" means a signal that has one or more of its characteristics set or changed in such a manner as to encode information in the signal.

The terms "machine-readable medium," "computer-readable medium," and "device-readable medium" mean the same thing and may be used interchangeably in this disclosure. The terms are defined to include both machine-storage media and transmission media. Thus, the terms include both storage devices/media and carrier waves/modulated data signals.

### Example Implementations

Implementation 1. A method comprising: obtaining, by a computing system including one or more hardware processors and memory, first data of a plurality of first individuals in which a neurodevelopmental condition is present, the first data including first genetic data and first health data of the plurality of first individuals; determining, by the computing system and using the first data, a number of classifications of the neurodevelopmental condition, a classification of the number of classifications indicating a set of features of a group of first individuals of the plurality of individuals, the set of features including at least one of one or more genetic alterations present in conjunction with the neurodevelopmental condition, one or more morphological features present in conjunction with the neurodevelopmental condition, one or more additional conditions present in conjunction with the neurodevelopmental condition, or one or more biological pathways modified in response to the neurodevelopmental condition; determining, by the computing system, that at least one of a portion of second genetic information of a group of second individuals or a portion of second health information of the group of second individuals corresponds to the set of features of the classification; obtaining, by the computing system, second data indicating at least one of a biological pathway modified in response to a therapeutic, up-regulation of one or more first genes in response to the therapeutic, down-regulation of one or more second genes in response to the therapeutic, or one or more additional conditions treated with the therapeutic; determining, by the computing system, that at least portions of the second data correspond to at least portions of the set of features of the classification; and determining, by the computing system, a probability that a candidate treatment of the neurodevelopmental condition for the group of second individuals includes the therapeutic.
Implementation 2. The method of implementation 1, wherein the neurodevelopmental condition is autism spectrum disorder.
Implementation 3. The method of implementations 1 or 2, comprising: analyzing, by the computing system, at least one of deoxyribonucleic acid (DNA) sequencing information or ribonucleic acid (RNA) sequencing information of the group of first individuals to determine the one or more genes included in the set of features.
Implementation 4. The method of any one of implementations 1-3, comprising: analyzing, by the computing system, at least one of DNA sequencing information, RNA sequencing information, or metabolomic information of the group of first individuals to determine the one or more biological pathways modified in response to the neurodevelopmental condition.
Implementation 5. The method of any one of implementations 1-4, wherein the first health information includes laboratory test results that indicate levels of analytes in at least a portion of the plurality of first individuals; and the method comprising: analyzing, by the computing system, the levels of analytes in at least the portion of the plurality of first individuals to determine at least one of the one or more biological pathways modified in response to the neurodevelopmental condition or the one or more additional conditions present in conjunction with the neurodevelopmental condition.
Implementation 6. The method of any one of implementations 1-5, wherein the first health information includes electronic medical records of at least a portion of the plurality of first individuals, and the method comprising: analyzing, by the computing system, the electronic medical records to determine at least one of the one or more morphological features or the one or more additional conditions present in conjunction with the neurodevelopmental condition.
Implementation 7. The method of any one of implementations 1-6, comprising: obtaining, by the computing system, third data of third individuals having at least a threshold decrease in one or more symptoms of an additional condition of the one or more additional conditions in response to treatment of the third individuals with the therapeutic or third data of third individuals where a subset of those have at least a threshold decrease in one or more symptoms of a neurodevelopmental condition in response to treatment of the third individuals with the therapeutic, the third data including third genetic information of the third individuals and third health information of the third individuals; and determining, by the computing system, a measure of similarity between at least one of a portion of the third genetic information or a portion of the third health information with respect to the set of features of the classification; and wherein determining the probability that the candidate treatment of the neurodevelopmental condition for the second group of individuals includes the therapeutic is based at least partly on the measure of similarity.
Implementation 8. A system comprising: one or more processors; and one or more non-transitory computer-readable storage media that include computer-readable instructions that, when executed by the one or more processors, cause the one or more processors to: obtain first data of a plurality of individuals in which a neurodevelopmental condition is present, the first data including first genetic data and first health data of the plurality of first individuals; determine, using the first data, a number of classifications of the neurodevelopmental condition, a classification of the number of classifications indicating a set of features of a group of first individuals of the plurality of first individuals, the set of features including at least one of one or more genetic alterations present in conjunction with the neurodevelopmental condition, one or more morphological features present in conjunction with the neurodevelopmental condition, one or more additional conditions present in conjunction with the neurodevelopmental condition, or one or more biological pathways modified in response to the neurodevelopmental condition; determine that at least one of a portion of second genetic information of a group of second individuals or a portion of second health information of a group of second individuals corresponds to the set of features of the classification; obtain second data indicating at least one of a biological pathway modified in response to a therapeutic, up-regulation of one or more first genes in response to the therapeutic, down-regulation of one or more second genes in in response to the therapeutic, or one or more additional conditions treated with the therapeutic; determine that at least portions of the second data correspond to at least portions of the set of features of the classification; and determine a probability that a candidate treatment of the neurodevelopmental condition for the group of second individuals includes the therapeutic.
Implementation 9. The system of implementation 8, wherein the one or more non-transitory computer-readable storage media include additional computer-readable instructions that, when executed by the one or more processors, cause the one or more processors to: obtain third data indicating measures of expression of a number of genes in response to a plurality of additional therapeutics; determine, based on the third data, a first gene expression profile for a first additional therapeutic of the plurality of additional therapeutics, the first gene expression profile indicating up-regulation of a first group of genes in response the first additional therapeutic and down-regulation of a second group of genes in response the first additional therapeutic; determine, based on the third data, a second gene expression profile for a second additional therapeutic of the plurality of additional therapeutics, the second gene expression profile indicating up-regulation of a third group of genes in response to the second therapeutic and down-regulation of a fourth group of genes in response to the second therapeutic.
Implementation 10. The system of implementation 9, wherein the one or more non-transitory computer-readable storage media include additional computer-readable instructions that, when executed by the one or more processors, cause the one or more processors to: generate a measure of similarity between the first expression profile and the second expression profile, the measure of similarity indicating a number of the first genes that correspond to a number of the third genes and a number of the second genes that correspond to a number of the fourth genes; and generate a gene expression profile distance network, the gene expression profile distance network including a first node that corresponds to the first additional therapeutic and a second node that corresponds to the second additional therapeutic with a distance between the first node and the second node in the gene expression profile distance network corresponding to the measure of similarity.
Implementation 11. The system of any one of implementations 8-10, wherein the neurodevelopmental condition is autism spectrum disorder and the one or more additional conditions present in relation to the neurodevelopmental condition can include at least one of seizures, allodynia, or hyperalgesia.
Implementation 12. The system of anyone of implementations 8-11, wherein: a first group of first additional genes are expressed in relation to a first biological pathway of a group of biological pathways, the first group of first genes having at least a threshold probability that a presence of the first group of first genes in an individual corresponds to the neurodevelopmental condition being present in the individual; a second group of second genes are expressed in relation to a second biological pathway of the group of biological pathways, the second group of second genes having at least the threshold probability that a presence of the second group of second genes in the individual corresponds to the neurodevelopmental condition being present in the individual; and at least a portion of the first group of the first genes include at least a portion of the second group of the second genes.
Implementation 13. The system of implementation 12, wherein the one or more non-transitory computer-readable storage media include additional computer-readable instructions that, when executed by the one or more processors, cause the one or more processors to: determine an amount of overlap between the first group of first genes and the second group of second genes; and remove, based on the amount of overlap, the second biological pathway from the group of biological pathways to generate a modified group of biological pathways.
Implementation 14. The system of any one of implementations 8-13, wherein the one or more non-transitory computer-readable storage media include additional computer-readable instructions that, when executed by the one or more processors, cause the one or more processors to: generate user interface data indicating the probability that the therapeutic is a candidate treatment of the neurodevelopmental condition for the individual.
Implementation 15. The system of any one of implementations 8-14, wherein the set of features includes a gene of the one or more genes present in conjunction with the neurodevelopmental condition, a morphological feature of the one or more morphological features present in conjunction with the neurodevelopmental condition, an additional condition of the one or more additional conditions present in conjunction with the neurodevelopmental condition, and a biological pathway of the one or more biological pathways modified in response to the neurodevelopmental condition.
Implementation 16. A method comprising: obtaining, by a computing system including one or more hardware processors and memory, genetic information including data indicating a plurality of altered genomic regions that correspond to one or more biological conditions, the plurality of altered genomic regions corresponding to genomes of a first group of individuals in which a neurodevelopmental condition is present in relation to genomes of a second group of individuals in which the neurodevelopmental condition is not present; determining, by the computing system and based on the genetic information, a first probability that a first altered genomic region included in the plurality of altered genomic regions corresponds to the first group of individuals; determining, by the computing system and based on the genetic information, a second probability that a second altered genomic region of the plurality of altered genomic regions corresponds to the first group of individuals; determining, by the computing system, an order to rank the first altered genomic region and the second altered genomic region according to the first probability and the second probability; generating, by the computing system, a risk gene database that includes at least a subset of the plurality of altered genomic regions ordered according to respective rankings of individual altered genomic regions included in the at least the subset of the plurality of altered genomic regions; determining, by the computing system and using the risk gene database, a number of genomic regions having at least a threshold probability of being altered in genomes of the first group of individuals; and determining, by the computing system, one or more biological pathways corresponding to at least a portion of the number of genomic regions having at least the threshold probability of being altered in the genomes of the first group of individuals.
Implementation 17. The method of implementation 16, wherein the genetic information includes copy number variant information comprising at least one of: duplications indicating that a number of copies of one or more genomic regions present in genomes of the first group individuals is greater than a number of copies of the one or more genomic regions present in genomes of the second group of individuals, inversions indicating locations of one or more first additional genomic regions included in genomes of the first group of individuals is switched with respect to locations of the one or more first additional genomic regions in the genomes of the second group of individuals, or deletions indicating that a number of copies of one or more second additional genomic regions present in the genomes of the first group of individuals is less than a number of copies of the one or more second additional genomic regions present in the genomes of the second group of individuals.
Implementation 18. The method of implementations 16 or 17, wherein the genetic information includes at least one of: associated phenotype genes indicating genes that are present in individuals having one or more phenotypes that are co-occurring with respect to the neurodevelopmental condition; risk gene protein-protein interactions indicate interactions between at least one first protein and a second protein that is produced in association with the expression of a genomic region that has at least the threshold probability of being altered in genomes of individuals in which a neurodevelopmental condition is present; RNA sequence derived gene-phenotype associations indicating altered genomic regions identified from RNA sequence information of individuals in which the neurodevelopmental condition is present in relation to RNA sequence information of individuals in which the neurodevelopmental condition is not present; DNA sequence derived gene-phenotype associations indicating one or more altered genomic regions identified from DNA sequence information of individuals in which the neurodevelopmental condition is present in relation to DNA sequence information of individuals in which the neurodevelopmental condition is not present; genome-wide association studies (GWAS) derived gene-phenotype associations; or gene mutation rates corresponding to expected mutation rates based on gene length and sequence context and observed mutation rates in a control population in which the neurodevelopmental condition is not present.
Implementation 19. The method of any one of implementations 16-18, wherein: the alteration in the first genomic region has a first allele frequency that is no greater than about 0.001 and the alteration in the second genomic region has a second allele frequency that is no greater than about 0.001; a first ranking of the first genomic region is determined based on a first measure of conservation between a first gene related to the first genomic region and the first genomic region; a second ranking of the second genomic region is determined based on a second measure of conservation between a second gene related to the second genomic region and the second genomic region.
Implementation 20. The method of implementation 19, wherein the first ranking is greater than the second ranking based on the first measure of conservation being greater than the second measure of conservation.
Implementation 21. The method of any one of implementations 16-20, comprising: determining a first plurality of altered genomic regions corresponding to a first biological condition that is co-occurring with respect to the neurodevelopmental condition; determining a second plurality of altered genomic regions corresponding to a second biological condition that is co-occurring with respect to the neurodevelopmental condition; and determining a set of genomic regions that is common between the first plurality of altered genomic regions and the second plurality of altered genomic regions.
Implementation 22. The method of implementation 21, wherein the set of genomic regions contains genes involved in one or more biological pathways.
Implementation 23. The method of any one of implementations 16-22, comprising: determining a strength of association between a first phenotype and a second phenotype; determining that the strength of association is greater than a threshold strength of association; determining one or more first biological conditions corresponding to the first phenotype and one or more second biological conditions corresponding to the second phenotype; determining a number of altered genomic regions that are common between at least a portion of the one or more first biological conditions and at least a portion of the one or more second biological conditions; and determining that the first phenotype is related to the number of altered genomic regions that are common between at least a portion of the one or more first biological conditions and at least a portion of the one or more second biological conditions.
Implementation 24. The method of implementation 23, wherein: the first phenotype is associated with a first number of items including at least one of one or more first genomic regions, one or more first biological pathways, one or more first symptoms, one or more first anatomical features, one or more first morphological features, one or more first clinical observations, one or more first clinical diagnoses, or one or more first publications related to the first phenotype; the second phenotype is associated with a second number of items including at least one of one or more second genomic regions, one or more second biological pathways, one or more second symptoms, one or more second anatomical features, one or more second morphological features, one or more second clinical observations, one or more second clinical diagnoses, or one or more second publications related to the second phenotype; and the strength of association between the first phenotype and the second phenotype is based on analyzing the first number of items in relation to the second number of items.
Implementation 25. A system comprising: one or more hardware processors; one or more non-transitory computer-readable storage media storing computer readable instructions that, when executed by the one or more hardware processors, cause the one or more hardware processors to perform operations comprising: obtaining genetic information including data indicating a plurality of altered genomic regions that correspond to one or more biological conditions, the altered genomic regions corresponding to genomes of individuals in which at least one biological condition of the one or more biological conditions is present in relation to genomes of individuals in which the at least one biological condition is not present; determining a first probability that a first altered genomic region included in the plurality of altered genomic regions corresponds to a group of individuals in which a neurodevelopmental condition is present; determining a second probability that a second altered genomic region of the plurality of altered genomic regions corresponds to the group of individuals in which the neurodevelopmental condition is present; determining an order to rank the first altered genomic region and the second altered genomic region according to the first probability and the second probability; generating a risk gene database that includes at least a subset of the plurality of altered genomic regions ordered according to respective rankings of individual altered genomic regions included in the at least the subset of the plurality of altered genomic regions; determining, using the risk gene database, a number of genomic regions having at least a threshold probability of being altered in genomes of individuals in which the neurodevelopmental condition is present; and determining a biological pathway corresponding to at least a portion of the number of genomic regions having at least the threshold probability of being altered in the genomes of individuals in which the neurodevelopmental condition is present.
Implementation 26. The system of implementation 25, wherein the biological pathway is one of a plurality of biological pathways that are associated with at least one genomic region included in the gene risk database, the at least one genomic region having at least the threshold probability of being altered in genomes of individuals in which the neurodevelopmental condition is present.
Implementation 27. The system of implementation 26, wherein the one or more computer-readable storage media store additional computer readable instructions that, when executed by the one or more hardware processors, cause the one or more hardware processors to perform operations comprising: determining a number of genomic regions corresponding to each biological pathway of the plurality of biological pathways that have at least the threshold probability of being altered in genomes of individuals in which the neurodevelopmental condition is present; and determining a subset of the plurality of biological pathways having at least a threshold number of genomic regions that have at least the threshold probability of being altered in genomes of individuals in which the neurodevelopmental condition is present.
Implementation 28. The system of implementation 27, wherein the one or more computer-readable storage media store additional computer readable instructions that, when executed by the one or more hardware processors, cause the one or more hardware processors to perform operations comprising: determining an overlap coefficient of at least 0.8 for first genomic regions of a first biological pathway included in the subset of the plurality of biological pathways that are common between second genomic regions of a second biological pathway included in the subset of the plurality of biological pathways; and determining that the second biological pathway is redundant with respect to the first biological pathway.
Implementation 29. The system of implementation 28, wherein: the one or more computer-readable storage media store additional computer readable instructions that, when executed by the one or more hardware processors, cause the one or more hardware processors to perform operations comprising determining that a number of the first genomic regions is less than a number of the second genomic regions; the first biological pathway and the second biological pathway have a parent-child relationship; the second biological pathway is redundant with respect to the first biological pathway based on the number of the first genomic regions being less than the number of the second genomic regions; and the one or more computer-readable storage media store further computer readable instructions that, when executed by the one or more hardware processors, cause the one or more hardware processors to perform operations comprising determining that one or more therapeutics are candidate treatments for the neurodevelopmental condition based on the first biological pathway.
Implementation 30. The system of implementation 27, wherein the one or more computer-readable storage media store additional computer readable instructions that, when executed by the one or more hardware processors, cause the one or more hardware processors to perform operations comprising: determining an overlap coefficient of at least 0.8 for first genomic regions of a first biological pathway included in the subset of the plurality of biological pathways that are common between second genomic regions of a second biological pathway included in the subset of the plurality of biological pathways, wherein the first biological pathway and the second biological pathway do not have a parent-child relationship; determining that a number of the first genomic regions is less than a number of the second genomic regions; determining that the second biological pathway is not redundant with respect to the first biological pathway; and determining one or more probabilities that one or more therapeutics are candidate treatments for the neurodevelopmental condition based on the first biological pathway and the second biological pathway.
Implementation 31. A method comprising: obtaining, by a computing system including one or more hardware processors and memory, data corresponding to a neurodevelopmental condition from one or more data stores, the data indicating information related to individuals in which the neurodevelopmental condition is present; analyzing, by the computing system, the data to determine amounts of significance in relation to respective features included in the data and the neurodevelopmental condition; determining, by the computing system, a first number of features having at least a threshold amount of significance with respect to the neurodevelopmental condition, the first number of features including at least one of one or more first genetic features, one or more first observable features, one or more first co-occurring conditions, or one or more first analytes; determining, by the computing system, a second number of features having at least the threshold amount of significance with respect to the neurodevelopmental condition, the second number of features including at least one of one or more second genetic features, one or more second observable features, one or more second co-occurring conditions, or one or more second analytes, wherein at least one feature included in the second number of features is different from at least one feature included in the first number of features; determining, by the computing system, a first group of individuals in which the neurodevelopmental condition is present that correspond to the first number of features; determining, by the computing system, a second group of individuals in which the neurodevelopmental condition is present that corresponds to the second number of features, the second group of individuals including at least one individual that is different from at least one individual included in the first group of individuals; and determining, by the computing system, a measure of at least one of similarity or reliability between the first group of individuals and the second group of individuals.
Implementation 32. The method of implementation 31 comprising: determining that the measure of reliability between the first group of individuals and the second group of individuals is less than a threshold measure of reliability; determining, based on the measure of reliability being less than the threshold measure of reliability, a third group of features having at least the threshold amount of significance with respect to the neurodevelopmental condition, the third group of features including at least one of one or more third genetic features, one or more third observable features, one or more third co-occurring conditions, or one or more third analytes, wherein at least one feature included in the third number of features is different from at least one feature included in the first number of features and at least one feature included in the second number of features; and determining, based on the measure of reliability being less than the threshold measure of reliability, a fourth group of features having at least the threshold amount of significance with respect to the neurodevelopmental condition, the fourth group of features including at least one of one or more fourth genetic features, one or more fourth observable features, one or more fourth co-occurring conditions, or one or more fourth analytes, wherein at least one feature included in the fourth number of features is different from at least one feature included in the first number of features, at least one feature included in the second number of features, and at least one feature included in the third number of features.
Implementation 33. The method of implementation 32, comprising: determining a third group of individuals in which the neurodevelopmental condition is present that correspond to the third number of features, the third group of individuals including at least one individual that is different from at least one individual included in the first group of individuals and at least one individual included in the second group of individuals; determining a fourth group of individuals in which the neurodevelopmental condition is present that corresponds to the fourth number of features, the fourth group of individuals including at least one individual that is different from at least one individual included in the first group of individuals, at least one individual included in the second group of individuals, and at least one individual included in the third group of individuals; determining an additional measure of reliability between the third group of individuals and the fourth group of individuals; and determining that the additional measure of reliability satisfies the threshold measure of reliability.
Implementation 34. The method of implementation 33, comprising: obtaining additional data corresponding to the neurodevelopmental condition from the one or more data stores, the additional data indicating additional information related to additional individuals in which the neurodevelopmental condition is present; analyzing the additional information related to the additional individuals in relation to the third group of features to determine that a first measure of similarity between features of a first portion of the additional individuals and the third group of features is at least a threshold measure of similarity; analyzing the additional information related to the additional individuals in relation to the fourth group of features to determine that a second measure of similarity between features of a second portion of the additional individuals and the fourth group of features is at least the threshold measure of similarity; determining, based on the first similarity metric being at least the threshold similarity metric, that the first portion of the additional individuals are included in the third group of individuals; and determining, based on the second similarity metric being at least the threshold similarity metric, that the second portion of the additional individuals are included in the fourth group of individuals.
Implementation 35. The method of any one of implementations 31-34, wherein the first genetic features are obtained from a first version of a risk genes data store that includes a first plurality of genes having a first false discovery rate for the neurodevelopmental condition that is no greater than about 0.30, and the method comprising: generating a second version of the risk genes data store that includes a second plurality of genes having a second false discovery rate for the neurodevelopmental condition that is no greater than about 0.20.
Implementation 36. The method of any one of implementations 31-35, wherein the first genetic features are obtained from a risk variants data store that includes a number of variants of genes that have at least a threshold strength of association with the neurodevelopmental condition, and the method comprising: determining a *p*-value for a variant of a gene and the neurodevelopmental condition using a Fisher's exact test; and modifying the p-value to produce an adjusted *p*-value based on one or more multiple-testing correction techniques; and determining a strength of association between the variant of the gene and the neurodevelopmental condition based on the adjusted *p*-value.
Implementation 37. The method of any one of implementations 31-36, wherein the first group of features and the second group of features are determined using at least one of one or more hierarchical-based clustering techniques, one or more partitioning-based clustering techniques such as k-means clustering techniques, one or more model-based clustering techniques such as latent class analysis techniques, one or more density-based clustering techniques or one or more graph-based clustering techniques.
Implementation 38 A system comprising: one or more hardware processors; one or more non-transitory computer-readable storage media storing computer readable instructions that, when executed by the one or more hardware processors, cause the one or more hardware processors to perform operations comprising: obtaining data corresponding to a neurodevelopmental condition from one or more data stores, the data indicating information related to individuals in which the neurodevelopmental condition is present; analyzing the data to determine a first group of individuals in which the neurodevelopmental condition is present; analyzing the data to determine a second group of individuals in which the neurodevelopmental condition is present, the second group of individuals including at least one individual that is different from at least one individual of the first group of individuals; determining a first group of features corresponding to the first group of individuals that has at least a threshold amount of significance with respect to the neurodevelopmental condition, the first group of features including at least one of one or more first genetic features, one or more first observable features, one or more first co-occurring conditions, or one or more first analytes; determining a second group of features corresponding to the second group of individuals that has at least the threshold amount of significance with respect to the neurodevelopmental condition, the second group of features including at least one of one or more second genetic features, one or more second observable features, one or more second co-occurring conditions, or one or more second analytes, wherein at least one feature included in the second group of features is different from at least one feature included in the first group of features; and determining a measure of at least one of similarity or reliability between the first group of individuals and the second group of individuals.
Implementation 39. The system of implementation 38, wherein: the one or more non-transitory computer-readable storage media store additional computer-readable instructions that, when executed by the one or more hardware processors, cause the one or more hardware processors to perform operations comprising: determining a first amount of similarity between first individuals included in the first group of individuals; and determining a second similarity between the first individuals and second individuals included in the second group of individuals; and the measure of reliability is determined based on the first amount of similarity and the second amount of similarity.
Implementation 40. The system of implementations 38 or 39, wherein: the one or more non-transitory computer-readable storage media store additional computer-readable instructions that, when executed by the one or more hardware processors, cause the one or more hardware processors to perform operations comprising: determining a first number of groupings of the individuals in which the neurodevelopmental condition is present using first information obtained from one or more first sources of information storing genetic information of a first plurality of individuals in which the neurodevelopmental condition is present; determining a second number of groupings of the individuals in which the neurodevelopmental condition is present using second information obtained from one or more second sources of information storing biological pathway information of a second plurality of individuals in which the neurodevelopmental condition is present; determining a third number of groupings of the individuals in which the neurodevelopmental condition is present using third information obtained from one or more third sources of information storing phenotypic information of a third plurality of individuals in which the neurodevelopmental condition is present; and determining a fourth number of groupings of individuals in which the neurodevelopmental condition is present using fourth information obtained from one or more fourth sources of information storing clinical data of a fourth plurality of individuals in which the neurodevelopmental condition is present.
Implementation 41. The system of implementation 40, wherein at least a portion of the individuals included in a first grouping of the first number of groupings is included in at least one of a second grouping of the second number of groupings, a third grouping of the third number of groupings, or a fourth grouping of the fourth number of groupings.
Implementation 42. The system of implementation 40, wherein the one or more non-transitory computer-readable storage media store additional computer-readable instructions that, when executed by the one or more hardware processors, cause the one or more hardware processors to perform operations comprising: determining a first level of preservation between the first individuals included in the first grouping and a portion of the second individuals included in a second grouping of the second number of groupings; determining a second level of preservation between the first individuals included in the first grouping and a portion of the third individuals included in a third grouping of the third number of groupings; determining a third level of preservation between the first individuals included in the first grouping and a portion of the third individuals included in a third grouping of the third number of groupings; and determining that at least one of the first level of preservation, the second level of preservation, or the third level of preservation corresponds to a threshold level of preservation.
Implementation 43. The system of implementation 42, wherein: the one or more non-transitory computer-readable storage media store additional computer-readable instructions that, when executed by the one or more hardware processors, cause the one or more hardware processors to perform operations comprising determining an amount of overlap between the first individuals included in the first grouping and a number of the second individuals included in the second grouping; and the first level of preservation is based on the amount of overlap in relation to an expected amount of overlap, the expected amount of overlap corresponding to respective amounts of overlap between individuals included in a number of pairs of groupings that are selected in a random or pseudo-random manner.
Implementation 44. The system of implementation 42, wherein: the one or more non-transitory computer-readable storage media store additional computer-readable instructions that, when executed by the one or more hardware processors, cause the one or more hardware processors to perform operations comprising determining a number of features of the first individuals included in the first grouping that are shared with a number of the second individuals included in the second grouping; and the first level of preservation is based on the number of features shared between the first individuals included in the first grouping and the number of the second individuals included in the second grouping.
Implementation 45. The system of any one of implementations 42-44, wherein: the one or more non-transitory computer-readable storage media store additional computer-readable instructions that, when executed by the one or more hardware processors, cause the one or more hardware processors to perform operations comprising determining that the first level of preservation corresponds to the threshold level of preservation; and at least a portion of the first individuals included in the first grouping and at least a portion of the individuals included in the second grouping are included in the first group of individuals.
Implementation 46 A method comprising: obtaining, by a computing system including one or more hardware processors and memory, data indicating measures of expression of a number of genes in response to one or more therapeutics of a plurality of therapeutics; determining, by the computing system and based on the data, a first gene expression profile for a first therapeutic of the plurality of therapeutics, the first gene expression profile indicating up-regulation of a first group of genes in response to the first therapeutic and down-regulation of a second group of genes in response to the first therapeutic; determining, by the computing system and based on the data, a second gene expression profile for a second therapeutic of the plurality of therapeutics, the second expression profile indicating up-regulation of a third group of genes in response to the second therapeutic and down-regulation of a fourth group of genes in response to the second therapeutic; generating, by the computing system, a measure of similarity between the first gene expression profile and the second gene expression profile, the measure of similarity indicating a number of the first group of genes that correspond to a number of the third group of genes and a number of the second group of genes that correspond to a number of the fourth group of genes; and generating, by the computing system, a gene expression profile distance network, the gene expression profile distance network including a first node that corresponds to the first therapeutic and a second node that corresponds to the second therapeutic with a distance between the first node and the second node in the therapeutics network corresponding to the measure of similarity.
Implementation 47. The method of implementation 46, comprising: determining, by the computing system, an amount of expression of a gene with respect to a therapeutic based on RNA sequencing information obtained after profiling one or more cell lines with respect to the therapeutic.
Implementation 48. The method of implementations 46 or 47, wherein: the first group of genes includes from about 50 to 500 first genes having at least a threshold amount of up-regulation in response to the first therapeutic and includes the second group of genes includes from about 50 to 500 second genes having at least a threshold amount of down-regulation in response to the first therapeutic; and the third group of genes includes from about 50 to 500 third genes having at least the threshold amount of up-regulation in response to the second therapeutic and includes the fourth group of genes includes from about 50 to 500 fourth genes having at least the threshold amount of down-regulation in response to the second therapeutic.
Implementation 49. The method of any one of implementations 46-48, wherein the measure of similarity is determined by: analyzing the first group of genes with respect to the third group of genes to determine one or more first shared genes that are included in the first group of genes and included in the third group of genes; analyzing the second group of genes with respect to the fourth group of genes to determine one or more second shared genes that are included in the second group of genes and included in the fourth group of genes; analyzing the third group of genes with respect to the first group of genes to determine one or more third shared genes that are included in the third group of genes and included in the first group of genes; and analyzing the fourth group of genes with respect to the second group of genes to determine one or more fourth shared genes that are included in the fourth group of genes and included in the second group of genes.
Implementation 50. The method of implementation 49, comprising: determining the one or more first shared genes by performing a comparison in relation to a plurality of first pairs of genes with each first pair of genes including one gene selected from the first group of genes and an additional gene selected from the third group of genes; and determining the one or more second shared genes by performing a comparison in relation to a plurality of second pairs of genes with each second pair of genes including one gene selected from the second group of genes and an additional gene selected from the fourth group of genes.
Implementation 51. The method of implementation 50, comprising: comparing a first gene selected from the first group of genes with a second gene selected from the third group of genes; determining that the first gene corresponds to the second gene; increasing a value of a first measure of similarity based on the first gene corresponding to the second gene; comparing a third gene selected from the first group of genes with a fourth gene selected from the third group of genes; determining that the third gene does not correspond to the fourth gene; and decreasing the value of the first measure of similarity based on a lack of correspondence between the third gene and the fourth gene.
Implementation 52. The method of implementation 51, comprising: comparing a fifth gene selected from the third group of genes with a sixth gene selected from the first group of genes; determining that the fifth gene corresponds to the sixth gene; increasing a value of a second measure of similarity based on the fifth gene corresponding to the sixth gene; comparing a seventh gene selected from the third group of genes with an eighth gene selected from the first group of genes; determining that the seventh gene does not correspond to the eighth gene; and decreasing the value of the second measure of similarity based on a lack of correspondence between the seventh gene and the eighth gene.
Implementation 53. The method of implementation 52, comprising combining the value of the first measure of similarity with the value of the second measure of similarity to determine a value of the measure of similarity between the first gene expression profile and the second gene expression profile.
Implementation 54. A system comprising: one or more hardware processors; one or more non-transitory computer-readable storage media storing computer readable instructions that, when executed by the one or more hardware processors, cause the one or more hardware processors to perform operations comprising: obtaining data indicating measures of expression of a number of genes in response to one or more therapeutics of a plurality of therapeutics; determining, based on the data, a first gene expression profile for a first therapeutic of the plurality of therapeutics, the first gene expression profile indicating up-regulation of a first group of genes in response to the first therapeutic and down-regulation of a second group of genes in response to the first therapeutic; determining, based on the data, a second gene expression profile for a second therapeutic of the plurality of therapeutics, the second gene expression profile indicating up-regulation of a third group of genes in response to the second therapeutic and down-regulation of a fourth group of genes in response to the second therapeutic; generating a measure of similarity between the first gene expression profile and the second gene expression profile, the measure of similarity indicating a number of the first group of genes that correspond to a number of the third group of genes and a number of the second group of genes that correspond to a number of the fourth group of genes; and generating a gene expression profile distance network, the gene expression profile distance network including a first node that corresponds to the first therapeutic and a second node that corresponds to the second therapeutic with a distance between the first node and the second node in the therapeutics network corresponding to the measure of similarity.
Implementation 55. The system of implementation 54, wherein: the one or more non-transitory computer-readable storage media store additional computer readable instructions that, when executed by the one or more hardware processors, cause the one or more hardware processors to perform operations comprising: generating a ranked list of genes based on respective amounts of up-regulation and respective amounts of down-regulation of the genes in response to the first therapeutic; determining a first number of genes of the ranked list that have at least a threshold amount of up-regulation; and determining a second number of genes of the ranked list that have at least a threshold amount of down-regulation; and the first group of genes included in the first gene expression profile includes the first number of genes and the second group of genes included in the first gene expression profile includes the second number of genes.
Implementation 56. The system of implementation 54, wherein one or more non-transitory computer-readable storage media store additional computer readable instructions that, when executed by the one or more hardware processors, cause the one or more hardware processors to perform operations comprising: comparing individual genes selected from the first group of genes with individual genes included in the third group of genes; determining a number of first pairs of shared genes between the individual genes included in the first group of genes and the individual genes included in the third group of genes with each first pair of shared genes including one gene included in the first group of genes and an additional gene included in the third group of genes; determining a number of first pairs of differing genes between the individual genes included in the first group of genes and the individual genes included in the third group of genes with each first pair of differing genes including a gene included in the first group of genes and another gene included in the third group of genes; increasing a value of at least a portion of the measure of similarity based on the number of first pairs of shared genes; and decreasing the value of the at least a portion of the measure of similarity based on the number of first pairs of differing genes.
Implementation 57. The system of implementation 56, wherein one or more non-transitory computer-readable storage media store additional computer readable instructions that, when executed by the one or more hardware processors, cause the one or more hardware processors to perform operations comprising: comparing individual genes selected from the second group of genes with individual genes included in the fourth group of genes; determining a number of second pairs of shared genes between the individual genes included in the second group of genes and the individual genes included in the fourth group of genes with each second pair of shared genes including one gene included in the second group of genes and an additional gene included in the fourth group of genes; determining a number of second pairs of differing genes between the individual genes included in the second group of genes and the individual genes included in the fourth group of genes with each pair of second pair of differing genes including a gene included in the second group of genes and another gene included in the fourth group of genes; increasing the value of the at least a portion of the measure of similarity based on the number of second pairs of shared genes; and decreasing the value of the at least a portion of the measure of similarity based on the number of second pairs of shared genes.
Implementation 58. The system of any one of implementations 54-57, wherein: the data indicating the measures of expression of the number of genes in response to the one or more therapeutics is obtained from a first data source; and the one or more non-transitory computer-readable storage media store additional computer readable instructions that, when executed by the one or more hardware processors, cause the one or more hardware processors to perform operations comprising: obtaining additional data indicating additional measures of expression of an additional number of genes in response to one or more additional sources of genetic expression modification that are different from therapeutics; determining, based on the additional data, a first additional gene expression profile for a first additional source of genetic expression modification, the first additional gene expression profile indicating up-regulation of a first additional group of genes in response to the first additional source of genetic expression modification and down-regulation of a second additional group of genes in response to the first additional source of genetic expression modification; determining, based on the additional data, a second additional gene expression profile for a second additional source of genetic expression modification, the second additional gene expression profile indicating up-regulation of a third additional group of genes in response to the second additional source of genetic expression modification and down-regulation of a fourth additional group of genes in response to the second additional source of genetic expression modification; generating an additional measure of similarity between the first additional gene expression profile and the second additional gene expression profile, the additional measure of similarity indicating a number of the first additional group of genes that correspond to a number of the third additional group of genes and a number of the second additional group of genes that correspond to a number of the fourth additional group of genes; and generating an additional gene expression profile distance network, the additional gene expression profile distance network including a first additional node that corresponds to the first additional source of genetic expression modification and a second additional node that corresponds to the second additional source of genetic expression modification with an additional edge between the first additional node and the second additional node, a length of the additional edge corresponding to the additional measure of similarity.
Implementation 59. The system of implementation 58, wherein the additional source of genetic expression modification includes a biological condition or short hairpin ribonucleic acid (shRNA) molecules.
Implementation 60. The system of any one of implementations 55-59, wherein: the one or more non-transitory computer-readable storage media store additional computer readable instructions that, when executed by the one or more hardware processors, cause the one or more hardware processors to perform operations comprising: generating a third gene expression profile indicating up-regulation of a fifth group of genes in a group of individuals in which a neurodevelopmental condition is present and a down-regulation of a sixth group of genes in the group of individuals; generating a further measure of similarity between the first gene expression profile and the third gene expression profile, the further measure of similarity indicating a number of the first group of genes that correspond to the sixth group of genes and a number of the second group of genes that correspond to the fifth group of genes; determining, based on the further measure of similarity, a probability that the first therapeutic is a candidate treatment for the group of individuals in relation to the neurodevelopmental condition.
Implementation 61. A method comprising: obtaining, by a computing system including one or more hardware processors and memory, first data indicating measures of expression of a number of genes in response to a therapeutic; determining, by the computing system and based on the first data, a gene expression profile for the therapeutic, the gene expression profile indicating up-regulation of a first group of genes in response to the therapeutic and down-regulation of a second group of genes in response to the therapeutic; obtaining, by the computing system, second data including genetic information of a plurality of individuals in which a neurodevelopmental condition is present; determining, by the computing system and based on the second data, up-regulation of a third group of genes in the plurality of individuals and down-regulation of a fourth group of genes in the plurality of individuals; determining, by the computing system, a first number of the first group of genes that corresponds to the fourth group of genes and a second number of the second group of genes that corresponds to the third group of genes; and determining, by the computing system and based on the first number of the first group of genes and the second number of the second group of genes, a probability that the therapeutic is a candidate treatment for the neurodevelopmental condition with respect to the plurality of individuals.
Implementation 62. The method of implementation 61, wherein the plurality of individuals comprises a subgroup of a population of individuals in which the neurodevelopmental condition is present; and the method comprising: obtaining, by the computing system, third data including genetic information of an additional subgroup of the population of individuals in which the neurodevelopmental condition is present; determining, by the computing system and based on the third data, up-regulation of a fifth group of genes in the additional subgroup and down-regulation of a sixth group of genes in the additional subgroup; determining, by the computing system, a third number of the first group of genes that corresponds to the sixth group of genes and a fourth number of the second group of genes that corresponds to the fifth group of genes; and determining, by the computing system and based on the third number of the first group of genes and the fourth number of the second group of genes, an additional probability that the therapeutic is an additional candidate treatment for the neurodevelopmental condition with respect to the additional subgroup of the population.
Implementation 63. The method of implementation 62, wherein the additional probability that the therapeutic is an additional candidate treatment for the neurodevelopmental condition with respect to the additional sub-group of the population is less than the probability that the therapeutic is a candidate treatment for the neurodevelopmental condition with respect to the plurality of individuals.
Implementation 64. The method of implementation 61, wherein at least one gene of the fifth group of genes is different from at least one gene included in the third group of genes and at least one gene of the sixth group of genes is different from at least one gene included in the fourth group of genes.
Implementation 65. The method of implementation 61, comprising: obtaining third data indicating measures of expression of an additional number of genes in response to an additional therapeutic; determining, based on the third data, an additional gene expression profile for the additional therapeutic, the additional gene expression profile indicating up-regulation of a fifth group of genes in response to the additional therapeutic and down-regulation of a sixth group of genes in response to the additional therapeutic; determining, by the computing system, a third number of the fifth group of genes that corresponds to the fourth group of genes and a fourth number of the sixth group of genes that corresponds to the third group of genes; and determining, by the computing system and based on the third number of the fifth group of genes and the fourth number of the sixth group of genes, an additional probability that the additional therapeutic is an additional candidate treatment for the neurodevelopmental condition with respect to the plurality of individuals.
Implementation 66. The method of implementation 65, comprising: determining that the probability that the therapeutic is a candidate treatment for the neurodevelopmental condition with respect to the plurality of individuals is at least a threshold probability; determining that the additional probability that the additional therapeutic is an additional candidate treatment for the neurodevelopmental condition with respect to the plurality of individuals is at least the threshold probability; and determining that a further candidate treatment for the neurodevelopmental condition with respect to the plurality of individuals includes a combination of the first therapeutic and the second therapeutic.
Implementation 67. A system comprising one or more hardware processors; one or more non-transitory computer-readable storage media storing computer readable instructions that, when executed by the one or more hardware processors, cause the one or more hardware processors to perform operations comprising: obtaining first data indicating measures of expression of a number of genes in response to a therapeutic; determining, based on the first data, a gene expression profile for the therapeutic, the gene expression profile indicating up-regulation of a first group of genes in response to the therapeutic and down-regulation of a second group of genes in response to the therapeutic; obtaining second data including genetic information of a plurality of individuals in which a neurodevelopmental condition is present; determining, based on the second data, up-regulation of a third group of genes in the plurality of individuals and down-regulation of a fourth group of genes in the plurality of individuals; determining a first number of the first group of genes that corresponds to the fourth group of genes and a second number of the second group of genes that corresponds to the third group of genes; and determining, based on the first number of the first group of genes and the second number of the second group of genes, a probability that the therapeutic is a candidate treatment for the neurodevelopmental condition with respect to the plurality of individuals.
Implementation 68. The system of implementation 67, wherein the first group of genes includes from about 50 to 500 genes having at least a threshold amount of up-regulation in response to the therapeutic and the second group of genes includes from about 50 to 500 genes having at least a threshold amount of down-regulation in response to the therapeutic.
Implementation 69. The system of implementations 67 or 68, wherein the one or more non-transitory computer-readable storage media store additional computer readable instructions that, when executed by the one or more hardware processors, cause the one or more hardware processors to perform operations comprising: determining, based on the second data, a first additional gene expression profile for a first individual included in the plurality of individuals, the first additional gene expression profile indicating up-regulation of a first additional group of genes in the first individual and down-regulation of a second additional group of genes in the first individual; determining, based on the second data, a second additional gene expression profile for a second individual included in the plurality of individuals, the second additional gene expression profile indicating up-regulation of a third additional group of genes in the second individual and down-regulation of a fourth additional group of genes in the second individual; and generating a consensus gene expression profile based on at least the first additional gene expression profile and the second additional gene expression profile, the consensus gene expression profile indicating up-regulation of a fifth additional group of genes and down-regulation of a sixth additional group of genes, the fifth additional group of genes including at least a portion of the first additional group of genes and at least a portion of the third additional group of genes and the sixth additional group of genes including at least a portion of the second additional group of genes and at least a portion of the fourth additional group of genes.
Implementation 70. The system of implementation 69, wherein the one or more non-transitory computer-readable storage media store additional computer readable instructions that, when executed by the one or more hardware processors, cause the one or more hardware processors to perform operations comprising: generating a plurality of additional gene expression profiles for a plurality of individuals, each additional gene expression profile of the plurality of gene expression profiles indicating up-regulation of one or more genes and down-regulation of one or more genes of a respective individual included in the plurality of individuals; generating a plurality of merged gene expression profiles based on the plurality of additional gene expression profiles, each merged gene expression profile of the plurality of merged gene expression profiles being generated based on a first plurality of up-regulated genes and a first plurality of down-regulated genes of a first additional gene expression profile of the plurality of additional gene expression profiles and on a second plurality of up-regulated genes and a second plurality of down-regulated genes of a second additional gene expression profile of the plurality of additional gene expression profiles; and determining an overall ranking of genes included in the plurality of additional gene expression profiles based on rankings of the genes in at least a portion of the plurality of merged gene expression profiles, the overall ranking of genes corresponding to the consensus gene expression profile.
Implementation 71. The system of implementations 67 or 68, wherein the one or more non-transitory computer-readable storage media store additional computer readable instructions that, when executed by the one or more hardware processors, cause the one or more hardware processors to perform operations comprising: determining an additional gene expression profile for the plurality of individuals, the additional gene expression profile including the third group of genes and the fourth group of genes; analyzing the first group of genes with respect to the fourth group of genes to determine one or more first shared genes that are included in the first group of genes and included in the fourth group of genes; analyzing the second group of genes with respect to the third group of genes to determine one or more second shared genes that are included in the second group of genes and included in the third group of genes; analyzing the fourth group of genes with respect to the first group of genes to determine one or more third shared genes that are included in the fourth group of genes and included in the first group of genes; analyzing the third group of genes with respect to the second group of genes to determine one or more fourth shared genes that are included in the third group of genes and included in the second group of genes; and determining a measure of similarity between the gene expression profile and the additional gene expression profile based on a number of the one or more first shared genes, a number of the one or more second shared genes, a number of the one or more third shared genes, and a number of the one or more fourth shared genes.
Implementation 72. The system of implementation 71, wherein the one or more non-transitory computer-readable storage media store additional computer readable instructions that, when executed by the one or more hardware processors, cause the one or more hardware processors to perform operations comprising: determining the one or more first shared genes by performing a comparison in relation to a plurality of first pairs of genes with each first pair of genes including one gene selected from the first group of genes and an additional gene selected from the fourth group of genes; and determining the one or more second shared genes by performing a comparison in relation to a plurality of second pairs of genes with each second pair of genes including one gene selected from the second group of genes and an additional gene selected from the third group of genes.
Implementation 73. The system of implementation 72, wherein the one or more non-transitory computer-readable storage media store additional computer readable instructions that, when executed by the one or more hardware processors, cause the one or more hardware processors to perform operations comprising: comparing a first gene selected from the first group of genes with a second gene selected from the fourth group of genes; determining that the first gene corresponds to the second gene; increasing a value of a first measure of similarity based on the first gene corresponding to the second gene; comparing a third gene selected from the first group of genes with a fourth gene selected from the fourth group of genes; determining that the third gene does not correspond to the fourth gene; and decreasing the value of the first measure of similarity based on a lack of correspondence between the third gene and the fourth gene.
Implementation 74. The system of implementation 73, wherein the one or more non-transitory computer-readable storage media store additional computer readable instructions that, when executed by the one or more hardware processors, cause the one or more hardware processors to perform operations comprising: comparing a fifth gene selected from the fourth group of genes with a sixth gene selected from the first group of genes; determining that the fifth gene corresponds to the sixth gene; increasing a value of a second measure of similarity based on the fifth gene corresponding to the sixth gene; comparing a seventh gene selected from the fourth group of genes with an eighth gene selected from the first group of genes; determining that the seventh gene does not correspond to the eighth gene; and decreasing the value of the second measure of similarity based on a lack of correspondence between the seventh gene and the eighth gene.
Implementation 75. The system of implementation 74, wherein the one or more non-transitory computer-readable storage media store additional computer readable instructions that, when executed by the one or more hardware processors, cause the one or more hardware processors to perform operations comprising: combining the value of the first measure of similarity with the value of the second measure of similarity to determine a value of the measure of similarity between the gene expression profile and the additional gene expression profile.
Implementation 76. A method comprising: obtaining, by a computing system including one or more hardware processors and memory, genetic data from at least one data source including at least one of co-occurring condition genetic data, phenotype classification genetic data, or biological pathway genetic data; obtaining, by the computing system, a gene expression profile corresponding to a therapeutic, the gene expression profile indicating a first group of genes that are up-regulated in response to the therapeutic and a second group of genes that are down-regulated in response to the therapeutic; analyzing, by the computing system, the gene expression profile in relation to the genetic data obtained from the at least one data source to determine one or more genes included in the gene expression profile that have at least a threshold amount of representation in at least one of the co-occurring condition genetic data, the phenotype classification genetic data, or the biological pathway genetic data; determining, by the computing system, a therapeutic responders profile that includes a plurality of features, at least a portion of the plurality of features corresponding to the one or more genes; analyzing, by the computing system, information corresponding to a plurality of subgroups of individuals in which a neurodevelopmental condition is present to determine respective profiles corresponding to each of the subgroups, the respective subgroup profiles each indicating features of individuals included in the respective subgroups; determining, by the computing system, an amount of overlap between the plurality of features included in the therapeutic responders profile and the features of individuals included in the respective subgroup profiles; determining, by the computing system, a subgroup of the plurality of subgroups that has at least a threshold amount of overlap between the plurality of features included in the therapeutic responders profile and a number of features corresponding to individuals included in the subgroup profile; and determining, by the computing system, a probability that the therapeutic is a candidate treatment for the neurodevelopmental condition with respect to the subgroup.
Implementation 77. The method of implementation 76, wherein: the co-occurring condition genetic data is related to a first number of genes that are over-expressed in a group of first individuals in which the neurodevelopmental condition and an additional biological condition are present and a second number of genes that are under-expressed in the group of first individuals in which the neurodevelopmental condition and the additional biological condition are present; the phenotype classification genetic data indicates one or more genes that correspond to at least one phenotype of a group of second individuals in which the neurodevelopmental condition is present; and the biological pathway genetic data indicates one or more genes that are expressed in relation to disruption of at least one biological pathway in a group of third individuals in which the neurodevelopmental condition is present.
Implementation 78. The method of implementation 77, wherein at least one first individual included in the group of first individuals is different from at least one second individual included in the group of second individuals and from at least one third individual included in the group of third individuals; and at least one additional second individual included in the group of second individuals is different from at least one additional third individual included in the group of third individuals.
Implementation 79. The method of any one of implementations 76-78, comprising: determining a first ranking of a gene included in the gene expression profile based on an amount of up-regulation of the gene in response to the therapeutic or an amount of down-regulation of the gene in response to the therapeutic; determining that the gene is present in at least one of the co-occurring condition genetic data, phenotype classification genetic data, or biological pathway genetic data; and generating an additional gene expression profile that includes the gene with the gene having a second ranking in the additional gene expression profile, the second ranking being based on the first ranking and the additional gene expression profile being included in the therapeutic responders profile.
Implementation 80. The method of implementation 79, comprising: determining that an additional gene included in the gene expression profile is not present in at least one of the co-occurring condition genetic data, phenotype classification genetic data, or biological pathway genetic data; and determining that the additional gene is to be excluded from the additional gene expression profile.
Implementation 81. The method of any one of 79 or 80, comprising: generating a further gene expression profile for the subgroup of the plurality of subgroups, the further gene expression profile indicating a third group of genes that are up-regulated in individuals included in the subgroup and a fourth group of genes that are down-regulated in individuals included in the subgroup; and determining a measure of similarity between the additional gene expression profile included in the therapeutic responders profile and the further gene expression profile for the subgroup, the measure of similarity corresponding to at least a portion of the amount of overlap between the plurality of features included in the therapeutic responders profile and the number of features corresponding to the individuals included in the subgroup.
Implementation 82. The method of any one of implementations 76-81, wherein the therapeutic responders profile includes at least one of a biological pathway that is disrupted in individuals in which the neurodevelopmental condition is present, a co-occurring biological condition that is present in individuals in which the neurodevelopmental condition is present, a phenotype that is related to individuals in which the neurodevelopmental condition is present, levels of analytes present in individuals in which the neurodevelopmental condition is present, or a morphological condition that is present in individuals in which the neurodevelopmental condition is present.
Implementation 83. The method of any one of implementations 76-82, wherein the probability that the therapeutic is a candidate treatment for the neurodevelopmental condition with respect to the subgroup corresponds to a probability that the therapeutic alleviates at least a portion of the symptoms of the subgroup with respect to the neurodevelopmental condition.
Implementation 84. A system comprising: one or more hardware processors; one or more non-transitory computer-readable storage media storing computer readable instructions that, when executed by the one or more hardware processors, cause the one or more hardware processors to perform operations comprising: obtaining genetic data from at least one data source including at least one of co-occurring condition genetic data, phenotype classification genetic data, or biological pathway genetic data; obtaining a gene expression profile corresponding to a therapeutic, the gene expression profile indicating a first group of genes that are up-regulated in response to the therapeutic and a second group of genes that are down-regulated in response to the therapeutic; analyzing the gene expression profile in relation to the genetic data obtained from the at least one data source to determine one or more genes included in the gene expression profile that have at least a threshold amount of representation in at least one of the co-occurring condition genetic data, the phenotype classification genetic data, or the biological pathway genetic data; determining a therapeutic responders profile that includes a plurality of features, at least a portion of the plurality of features corresponding to the one or more genes; analyzing information corresponding to a plurality of subgroups of individuals in which a neurodevelopmental condition is present to determine respective subgroup profiles corresponding to each of the subgroups, the respective subgroup profiles each indicating features of individuals included in the respective subgroups; determining an amount of overlap between the plurality of features included in the therapeutic responders profile and the features of individuals included in the respective subgroup profiles; determining a subgroup of the plurality of subgroups that has at least a threshold amount of overlap between the plurality of features included in the therapeutic responders profile and a number of features corresponding to individuals included in the subgroup profile; and determining a probability that the therapeutic is a candidate treatment for the neurodevelopmental condition with respect to the subgroup.
Implementation 85. The system of implementation 84, wherein: the one or more non-transitory computer-readable storage media store additional computer readable instructions that, when executed by the one or more hardware processors, cause the one or more hardware processors to perform operations comprising: generating a ranked list of genes based on respective amounts of up-regulation and respective amounts of down-regulation of a plurality of genes in response to the therapeutic; determining a first number of genes of the ranked list that have at least a threshold amount of up-regulation; and determining a second number of genes of the ranked list that have at least a threshold amount of down-regulation; and the first group of genes included in the gene expression profile includes the first number of genes and the second group of genes included in the gene expression profile includes the second number of genes.
Implementation 86. The system of implementations 84 or 85, wherein the one or more non-transitory computer-readable storage media store additional computer readable instructions that, when executed by the one or more hardware processors, cause the one or more hardware processors to perform operations comprising: determining a first ranking of a gene included in the gene expression profile based on an amount of up-regulation of the gene in response to the therapeutic or an amount of down-regulation of the gene in response to the therapeutic; determining that the gene is present in at least one of the co-occurring condition genetic data, phenotype classification genetic data, or biological pathway genetic data; and generating an additional gene expression profile that includes the gene with the gene having a second ranking in the additional gene expression profile, wherein the second ranking is based on the first ranking, the additional gene expression profile is included in the therapeutic responders profile, and the additional gene expression profile indicates up-regulation of a third group of genes that includes at least a portion of the first group of genes and down-regulation of a fourth group of genes that includes at least a portion of the second group of genes.
Implementation 87. The system of implementation 86, wherein: the information corresponding to the plurality of subgroups of individuals in which the neurodevelopmental condition is present includes genetic information of a plurality of individuals included in the plurality of subgroups; the one or more non-transitory computer-readable storage media store additional computer readable instructions that, when executed by the one or more hardware processors, cause the one or more hardware processors to perform operations comprising: determining, based on the genetic information, up-regulation of a fifth group of genes in the plurality of individuals and down-regulation of a sixth group of genes in the plurality of individuals; and generating a subgroup gene expression profile that includes the fifth group of genes and the sixth group of genes, the subgroup gene expression profile being included in the subgroup profile.
Implementation 88. The system of implementation 87, wherein the one or more non-transitory computer-readable storage media store additional computer readable instructions that, when executed by the one or more hardware processors, cause the one or more hardware processors to perform operations comprising: comparing individual genes selected from the third group of genes with individual genes included in the sixth group of genes; determining a number of first pairs of shared genes between the individual genes included in the third group of genes and the individual genes included in the sixth group of genes with each first pair of shared genes including one gene included in the third group of genes and an additional gene included in the sixth group of genes; determining a number of first pairs of differing genes between the individual genes included in the third group of genes and the individual genes included in the sixth group of genes with each first pair of differing genes including a gene included in the third group of genes and another gene included in the sixth group of genes; increasing a value of a first measure of similarity based on the number of first pairs of shared genes; and decreasing the value of the first measure of similarity based on the number of first pairs of differing genes.
Implementation 89. The system of implementation 88, wherein one or more non-transitory computer-readable storage media store additional computer readable instructions that, when executed by the one or more hardware processors, cause the one or more hardware processors to perform operations comprising: comparing individual genes selected from the fourth group of genes with individual genes included in the fifth group of genes; determining a number of second pairs of shared genes between the individual genes included in the fourth group of genes and the individual genes included in the fifth group of genes with each second pair of shared genes including one gene included in the fourth group of genes and an additional gene included in the fifth group of genes; determining a number of second pairs of differing genes between the individual genes included in the fourth group of genes and the individual genes included in the fifth group of genes with each second pair of differing genes including a gene included in the fourth group of genes and another gene included in the fifth group of genes; increasing the value of a second measure of similarity based on the number of second pairs of shared genes; and decreasing the value of the second measure of similarity based on the number of second pairs of shared genes.
Implementation 90. The system of implementation 89, wherein one or more non-transitory computer-readable storage media store additional computer readable instructions that, when executed by the one or more hardware processors, cause the one or more hardware processors to perform operations comprising: combining the first measure of similarity and the second measure of similarity to generate an overall measure of similarity between the additional gene expression profile and the subgroup gene expression profile, wherein the probability that the therapeutic is a candidate treatment for the neurodevelopmental condition with respect to the subgroup is based at least partly on the overall measure of similarity.
Implementation 91. A system comprising: one or more hardware processors; one or more non-transitory computer-readable storage media storing computer readable instructions that, when executed by the one or more hardware processors, cause the one or more hardware processors to perform operations comprising: obtaining genetic data from at least one data source including at least one of co-occurring condition genetic data, phenotype classification genetic data, or biological pathway genetic data; obtaining target profile data of a therapeutic and human protein interaction data indicating a group of genes in a network of genes that are regulated in response to the therapeutic and interactions between the group of genes with proteins in a population of humans; analyzing the target profile data and protein interaction data in relation to the genetic data obtained from the at least one data source to determine one or more genes included in the network of genes that have at least a threshold amount of representation in at least one of the co-occurring condition genetic data, the phenotype classification genetic data, or the biological pathway genetic data; determining a therapeutic responders profile that includes a plurality of features, at least a portion of the plurality of features corresponding to the one or more genes; analyzing information corresponding to a plurality of subgroups of individuals in which a neurodevelopmental condition is present to determine respective subgroup profiles corresponding to each of the subgroups, the respective subgroup profiles each indicating features of individuals included in the respective subgroups; determining an amount of overlap between the plurality of features included in the therapeutic responders profile and the features of individuals included in the respective subgroup profiles; determining a subgroup of the plurality of subgroups that has at least a threshold amount of overlap between the plurality of features included in the therapeutic responders profile and a number of features corresponding to individuals included in the subgroup profile; and determining a probability that the therapeutic is a candidate treatment for the neurodevelopmental condition with respect to the subgroup.

## Claims

1. A method comprising:
obtaining, by a computing system (102) including one or more hardware processors (2312) and memory (2316), first data of a plurality of first individuals in which a neurodevelopmental condition is present, the first data including first genetic data (110) and first health data (108) of the plurality of first individuals;
determining, by the computing system and using the first data, a number of classifications (120, 122) of the neurodevelopmental condition, a classification (120) of the number of classifications indicating a set of features (124) of a group of first individuals of the plurality of individuals, the set of features including at least one of one or more genetic alterations present in conjunction with the neurodevelopmental condition, one or more morphological features present in conjunction with the neurodevelopmental condition, one or more additional conditions present in conjunction with the neurodevelopmental condition, or one or more biological pathways modified in response to the neurodevelopmental condition;
determining, by the computing system, that at least one of a portion of second genetic information of a group of second individuals or a portion of second health information of the group of second individuals corresponds to the set of features of the classification;
obtaining, by the computing system, second data (116) indicating at least one of a biological pathway modified in response to a therapeutic (136), up-regulation of one or more first genes in response to the therapeutic, down-regulation of one or more second genes in response to the therapeutic, or one or more additional conditions treated with the therapeutic;
determining, by the computing system, that at least portions of the second data correspond to at least portions of the set of features of the classification; and
determining, by the computing system, a probability that a candidate treatment of the neurodevelopmental condition for the group of second individuals includes the therapeutic.

2. The method of claim 1, wherein the neurodevelopmental condition is autism spectrum disorder.

3. The method of claim 1 or 2, comprising:
analyzing, by the computing system, at least one of deoxyribonucleic acid (DNA) sequencing information or ribonucleic acid (RNA) sequencing information of the group of first individuals to determine the one or more genes included in the set of features.

4. The method of any one of claims 1-3, comprising:
analyzing, by the computing system, at least one of DNA sequencing information, RNA sequencing information, or metabolomic information of the group of first individuals to determine the one or more biological pathways modified in response to the neurodevelopmental condition.

5. The method of any one of claims 1-4, wherein the first health information includes laboratory test results that indicate levels of analytes in at least a portion of the plurality of first individuals; and the method comprising:
analyzing, by the computing system, the levels of analytes in at least the portion of the plurality of first individuals to determine at least one of the one or more biological pathways modified in response to the neurodevelopmental condition or the one or more additional conditions present in conjunction with the neurodevelopmental condition.

6. The method of any one of claims 1-5, wherein the first health information includes electronic medical records of at least a portion of the plurality of first individuals, and the method comprising:
analyzing, by the computing system, the electronic medical records to determine at least one of the one or more morphological features or the one or more additional conditions present in conjunction with the neurodevelopmental condition.

7. The method of any one of claims 1-6, comprising:
obtaining, by the computing system, third data of third individuals having at least a threshold decrease in one or more symptoms of an additional condition of the one or more additional conditions in response to treatment of the third individuals with the therapeutic, the third data including third genetic information of the third individuals and third health information of the third individuals; and
determining, by the computing system, a measure of similarity between at least one of a portion of the third genetic information or a portion of the third health information with respect to the set of features of the classification; and
wherein determining the probability that the candidate treatment of the neurodevelopmental condition for the second group of individuals includes the therapeutic is based at least partly on the measure of similarity.

8. A system (102) comprising:
one or more processors (2312); and
one or more non-transitory computer-readable storage media (2322, 2324 2326) that include computer-readable instructions (2306, 2308, 2310) that, when executed by the one or more processors, cause the one or more processors to:
obtain first data of a plurality of individuals in which a neurodevelopmental condition is present, the first data including first genetic data (110) and first health data (108) of the plurality of first individuals;
determine, using the first data, a number of classifications (120, 122) of the neurodevelopmental condition, a classification (120) of the number of classifications indicating a set of features (124) of a group of first individuals of the plurality of first individuals, the set of features including at least one of one or more genetic alterations present in conjunction with the neurodevelopmental condition, one or more morphological features present in conjunction with the neurodevelopmental condition, one or more additional conditions present in conjunction with the neurodevelopmental condition, or one or more biological pathways modified in response to the neurodevelopmental condition;
determine that at least one of a portion of second genetic information of a group of second individuals or a portion of second health information of a group of second individuals corresponds to the set of features of the classification;
obtain second data (116) indicating at least one of a biological pathway modified in response to a therapeutic (136), up-regulation of one or more first genes in response to the therapeutic, down-regulation of one or more second genes in in response to the therapeutic, or one or more additional conditions treated with the therapeutic;
determine that at least portions of the second data correspond to at least portions of the set of features of the classification; and
determine a probability that a candidate treatment of the neurodevelopmental condition for the group of second individuals includes the therapeutic.

9. The system of claim 8, wherein the one or more non-transitory computer-readable storage media include additional computer-readable instructions that, when executed by the one or more processors, cause the one or more processors to:
obtain third data indicating measures of expression of a number of genes in response to a plurality of additional therapeutics;
determine, based on the third data, a first gene expression profile for a first additional therapeutic of the plurality of additional therapeutics, the first gene expression profile indicating up-regulation of a first group of genes in response the first additional therapeutic and down-regulation of a second group of genes in response the first additional therapeutic;
determine, based on the third data, a second gene expression profile for a second additional therapeutic of the plurality of additional therapeutics, the second gene expression profile indicating up-regulation of a third group of genes in response to the second therapeutic and down-regulation of a fourth group of genes in response to the second therapeutic.

10. The system of claim 9, wherein the one or more non-transitory computer-readable storage media include additional computer-readable instructions that, when executed by the one or more processors, cause the one or more processors to:
generate a measure of similarity between the first expression profile and the second expression profile, the measure of similarity indicating a number of the first genes that correspond to a number of the third genes and a number of the second genes that correspond to a number of the fourth genes; and
generate a gene expression profile distance network, the gene expression profile distance network including a first node that corresponds to the first additional therapeutic and a second node that corresponds to the second additional therapeutic with a distance between the first node and the second node in the gene expression profile distance network corresponding to the measure of similarity.

11. The system of any one of claims 8-10, wherein the neurodevelopmental condition is autism spectrum disorder and the one or more additional conditions present in relation to the neurodevelopmental condition can include at least one of seizures, allodynia, or hyperalgesia.

12. The system of any one of claims 8-11, wherein:
a first group of first additional genes are expressed in relation to a first biological pathway of a group of biological pathways, the first group of first genes having at least a threshold probability that a presence of the first group of first genes in an individual corresponds to the neurodevelopmental condition being present in the individual;
a second group of second genes are expressed in relation to a second biological pathway of the group of biological pathways, the second group of second genes having at least the threshold probability that a presence of the second group of second genes in the individual corresponds to the neurodevelopmental condition being present in the individual; and
at least a portion of the first group of the first genes include at least a portion of the second group of the second genes.

13. The system of claim 12, wherein the one or more non-transitory computer-readable storage media include additional computer-readable instructions that, when executed by the one or more processors, cause the one or more processors to:
determine an amount of overlap between the first group of first genes and the second group of second genes; and
remove, based on the amount of overlap, the second biological pathway from the group of biological pathways to generate a modified group of biological pathways.

14. The system of any one of claims 8-13, wherein the one or more non-transitory computer-readable storage media include additional computer-readable instructions that, when executed by the one or more processors, cause the one or more processors to:
generate user interface data indicating the probability that the therapeutic is a candidate treatment of the neurodevelopmental condition for the individual.

15. The system of any one of claims 8-14, wherein the set of features includes a gene of the one or more genes present in conjunction with the neurodevelopmental condition, a morphological feature of the one or more morphological features present in conjunction with the neurodevelopmental condition, an additional condition of the one or more additional conditions present in conjunction with the neurodevelopmental condition, and a biological pathway of the one or more biological pathways modified in response to the neurodevelopmental condition.

16. A method comprising:
obtaining, by a computing system including one or more hardware processors and memory, genetic information including data indicating a plurality of altered genomic regions that correspond to one or more biological conditions, the plurality of altered genomic regions corresponding to genomes of a first group of individuals in which a neurodevelopmental condition is present in relation to genomes of a second group of individuals in which the neurodevelopmental condition is not present;
determining, by the computing system and based on the genetic information, a first probability that a first altered genomic region included in the plurality of altered genomic regions corresponds to the first group of individuals;
determining, by the computing system and based on the genetic information, a second probability that a second altered genomic region of the plurality of altered genomic regions corresponds to the first group of individuals;
determining, by the computing system, an order to rank the first altered genomic region and the second altered genomic region according to the first probability and the second probability;
generating, by the computing system, a risk gene database that includes at least a subset of the plurality of altered genomic regions ordered according to respective rankings of individual altered genomic regions included in the at least the subset of the plurality of altered genomic regions;
determining, by the computing system and using the risk gene database, a number of genomic regions having at least a threshold probability of being altered in genomes of the first group of individuals; and
determining, by the computing system, one or more biological pathways corresponding to at least a portion of the number of genomic regions having at least the threshold probability of being altered in the genomes of the first group of individuals.

17. The method of claim 16, wherein the genetic information includes copy number variant information comprising at least one of:
duplications indicating that a number of copies of one or more genomic regions present in genomes of the first group individuals is greater than a number of copies of the one or more genomic regions present in genomes of the second group of individuals,
inversions indicating locations of one or more first additional genomic regions included in genomes of the first group of individuals is switched with respect to locations of the one or more first additional genomic regions in the genomes of the second group of individuals, or
deletions indicating that a number of copies of one or more second additional genomic regions present in the genomes of the first group of individuals is less than a number of copies of the one or more second additional genomic regions present in the genomes of the second group of individuals.

18. The method of claim 16 or 17, wherein the genetic information includes at least one of:
associated phenotype genes indicating genes that are present in individuals having one or more phenotypes that are co-occurring with respect to the neurodevelopmental condition;
risk gene protein-protein interactions indicate interactions between at least one first protein and a second protein that is produced in association with the expression of a genomic region that has at least the threshold probability of being altered in genomes of individuals in which a neurodevelopmental condition is present;
RNA sequence derived gene-phenotype associations indicating altered genomic regions identified from RNA sequence information of individuals in which the neurodevelopmental condition is present in relation to RNA sequence information of individuals in which the neurodevelopmental condition is not present;
DNA sequence derived gene-phenotype associations indicating one or more altered genomic regions identified from DNA sequence information of individuals in which the neurodevelopmental condition is present in relation to DNA sequence information of individuals in which the neurodevelopmental condition is not present;
genome-wide association studies (GWAS) derived gene-phenotype associations; or
gene mutation rates corresponding to expected mutation rates based on gene length and sequence context and observed mutation rates in a control population in which the neurodevelopmental condition is not present.

19. The method of any one of claims 16-18, wherein:
the alteration in the first genomic region has a first allele frequency that is no greater than about 0.001 and the alteration in the second genomic region has a second allele frequency that is no greater than about 0.001;
a first ranking of the first genomic region is determined based on a first measure of conservation between a first gene related to the first genomic region and the first genomic region;
a second ranking of the second genomic region is determined based on a second measure of conservation between a second gene related to the second genomic region and the second genomic region.

20. The method of claim 19, wherein the first ranking is greater than the second ranking based on the first measure of conservation being greater than the second measure of conservation.

21. The method of any one of claims 16-20, comprising:
determining a first plurality of altered genomic regions corresponding to a first biological condition that is co-occurring with respect to the neurodevelopmental condition;
determining a second plurality of altered genomic regions corresponding to a second biological condition that is co-occurring with respect to the neurodevelopmental condition; and
determining a set of genomic regions that is common between the first plurality of altered genomic regions and the second plurality of altered genomic regions.

22. The method of claim 21, wherein the set of genomic regions contains genes involved in one or more biological pathways.

23. The method of any one of claims 16-22, comprising:
determining a strength of association between a first phenotype and a second phenotype;
determining that the strength of association is greater than a threshold strength of association;
determining one or more first biological conditions corresponding to the first phenotype and one or more second biological conditions corresponding to the second phenotype;
determining a number of altered genomic regions that are common between at least a portion of the one or more first biological conditions and at least a portion of the one or more second biological conditions; and
determining that the first phenotype is related to the number of altered genomic regions that are common between at least a portion of the one or more first biological conditions and at least a portion of the one or more second biological conditions.

24. The method of claim 23, wherein:
the first phenotype is associated with a first number of items including at least one of one or more first genomic regions, one or more first biological pathways, one or more first symptoms, one or more first anatomical features, one or more first morphological features, one or more first clinical observations, one or more first clinical diagnoses, or one or more first publications related to the first phenotype;
the second phenotype is associated with a second number of items including at least one of one or more second genomic regions, one or more second biological pathways, one or more second symptoms, one or more second anatomical features, one or more second morphological features, one or more second clinical observations, one or more second clinical diagnoses, or one or more second publications related to the second phenotype; and
the strength of association between the first phenotype and the second phenotype is based on analyzing the first number of items in relation to the second number of items.

25. A system comprising:
one or more hardware processors;
one or more non-transitory computer-readable storage media storing computer readable instructions that, when executed by the one or more hardware processors, cause the one or more hardware processors to perform operations comprising:
obtaining genetic information including data indicating a plurality of altered genomic regions that correspond to one or more biological conditions, the altered genomic regions corresponding to genomes of individuals in which at least one biological condition of the one or more biological conditions is present in relation to genomes of individuals in which the at least one biological condition is not present;
determining a first probability that a first altered genomic region included in the plurality of altered genomic regions corresponds to a group of individuals in which a neurodevelopmental condition is present;
determining a second probability that a second altered genomic region of the plurality of altered genomic regions corresponds to the group of individuals in which the neurodevelopmental condition is present;
determining an order to rank the first altered genomic region and the second altered genomic region according to the first probability and the second probability;
generating a risk gene database that includes at least a subset of the plurality of altered genomic regions ordered according to respective rankings of individual altered genomic regions included in the at least the subset of the plurality of altered genomic regions;
determining, using the risk gene database, a number of genomic regions having at least a threshold probability of being altered in genomes of individuals in which the neurodevelopmental condition is present; and
determining a biological pathway corresponding to at least a portion of the number of genomic regions having at least the threshold probability of being altered in the genomes of individuals in which the neurodevelopmental condition is present.

26. The system of claim 25, wherein the biological pathway is one of a plurality of biological pathways that are associated with at least one genomic region included in the gene risk database, the at least one genomic region having at least the threshold probability of being altered in genomes of individuals in which the neurodevelopmental condition is present.

27. The system of claim 26, wherein the one or more computer-readable storage media store additional computer readable instructions that, when executed by the one or more hardware processors, cause the one or more hardware processors to perform operations comprising:
determining a number of genomic regions corresponding to each biological pathway of the plurality of biological pathways that have at least the threshold probability of being altered in genomes of individuals in which the neurodevelopmental condition is present; and
determining a subset of the plurality of biological pathways having at least a threshold number of genomic regions that have at least the threshold probability of being altered in genomes of individuals in which the neurodevelopmental condition is present.

28. The system of claim 27, wherein the one or more computer-readable storage media store additional computer readable instructions that, when executed by the one or more hardware processors, cause the one or more hardware processors to perform operations comprising:
determining an overlap of at least a threshold amount of overlap for first genomic regions of a first biological pathway included in the subset of the plurality of biological pathways that are common between second genomic regions of a second biological pathway included in the subset of the plurality of biological pathways; and
determining that the second biological pathway is redundant with respect to the first biological pathway.

29. The system of claim 28, wherein:
the one or more computer-readable storage media store additional computer readable instructions that, when executed by the one or more hardware processors, cause the one or more hardware processors to perform operations comprising determining that a number of the first genomic regions is less than a number of the second genomic regions;
the first biological pathway and the second biological pathway have a parent-child relationship;
the second biological pathway is redundant with respect to the first biological pathway based on the number of the first genomic regions being less than the number of the second genomic regions; and
the one or more computer-readable storage media store further computer readable instructions that, when executed by the one or more hardware processors, cause the one or more hardware processors to perform operations comprising determining that one or more therapeutics are candidate treatments for the neurodevelopmental condition based on the first biological pathway.

30. The system of claim 27, wherein the one or more computer-readable storage media store additional computer readable instructions that, when executed by the one or more hardware processors, cause the one or more hardware processors to perform operations comprising:
determining an overlap of at most a threshold amount of overlap for first genomic regions of a first biological pathway included in the subset of the plurality of biological pathways that are common between second genomic regions of a second biological pathway included in the subset of the plurality of biological pathways;
determining that a number of the first genomic regions is less than a number of the second genomic regions;
determining that the second biological pathway is not redundant with respect to the first biological pathway; and
determining one or more probabilities that one or more therapeutics are candidate treatments for the neurodevelopmental condition based on the first biological pathway and the second biological pathway.

31. A method comprising:
obtaining, by a computing system including one or more hardware processors and memory, data corresponding to a neurodevelopmental condition from one or more data stores, the data indicating information related to individuals in which the neurodevelopmental condition is present;
analyzing, by the computing system, the data to determine amounts of significance in relation to respective features included in the data and the neurodevelopmental condition;
determining, by the computing system, a first number of features having at least a threshold amount of significance with respect to the neurodevelopmental condition, the first number of features including at least one of one or more first genetic features, one or more first observable features, one or more first co-occurring conditions, or one or more first analytes;
determining, by the computing system, a second number of features having at least the threshold amount of significance with respect to the neurodevelopmental condition, the second number of features including at least one of one or more second genetic features, one or more second observable features, one or more second co-occurring conditions, or one or more second analytes, wherein at least one feature included in the second number of features is different from at least one feature included in the first number of features;
determining, by the computing system, a first group of individuals in which the neurodevelopmental condition is present that correspond to the first number of features;
determining, by the computing system, a second group of individuals in which the neurodevelopmental condition is present that corresponds to the second number of features, the second group of individuals including at least one individual that is different from at least one individual included in the first group of individuals; and
determining, by the computing system, a measure of at least one of similarity or reliability between the first group of individuals and the second group of individuals.

32. The method of claim 31 comprising:
determining that the measure of reliability between the first group of individuals and the second group of individuals is less than a threshold measure of reliability;
determining, based on the measure of reliability being less than the threshold measure of reliability, a third group of features having at least the threshold amount of significance with respect to the neurodevelopmental condition, the third group of features including at least one of one or more third genetic features, one or more third observable features, one or more third co-occurring conditions, or one or more third analytes, wherein at least one feature included in the third number of features is different from at least one feature included in the first number of features and at least one feature included in the second number of features; and
determining, based on the measure of reliability being less than the threshold measure of reliability, a fourth group of features having at least the threshold amount of significance with respect to the neurodevelopmental condition, the fourth group of features including at least one of one or more fourth genetic features, one or more fourth observable features, one or more fourth co-occurring conditions, or one or more fourth analytes, wherein at least one feature included in the fourth number of features is different from at least one feature included in the first number of features, at least one feature included in the second number of features, and at least one feature included in the third number of features.

33. The method of claim 32, comprising:
determining a third group of individuals in which the neurodevelopmental condition is present that correspond to the third number of features, the third group of individuals including at least one individual that is different from at least one individual included in the first group of individuals and at least one individual included in the second group of individuals;
determining a fourth group of individuals in which the neurodevelopmental condition is present that corresponds to the fourth number of features, the fourth group of individuals including at least one individual that is different from at least one individual included in the first group of individuals, at least one individual included in the second group of individuals, and at least one individual included in the third group of individuals;
determining an additional measure of reliability between the third group of individuals and the fourth group of individuals; and
determining that the additional measure of reliability satisfies the threshold measure of reliability.

34. The method of claim 33, comprising:
obtaining additional data corresponding to the neurodevelopmental condition from the one or more data stores, the additional data indicating additional information related to additional individuals in which the neurodevelopmental condition is present;
analyzing the additional information related to the additional individuals in relation to the third group of features to determine that a first measure of similarity between features of a first portion of the additional individuals and the third group of features is at least a threshold measure of similarity;
analyzing the additional information related to the additional individuals in relation to the fourth group of features to determine that a second measure of similarity between features of a second portion of the additional individuals and the fourth group of features is at least the threshold measure of similarity;
determining, based on the first similarity metric being at least the threshold similarity metric, that the first portion of the additional individuals are included in the third group of individuals; and
determining, based on the second similarity metric being at least the threshold similarity metric, that the second portion of the additional individuals are included in the fourth group of individuals.

35. The method of any one of claims 31-34, wherein the first genetic features are obtained from a first version of a risk genes data store that includes a first plurality of genes having a first false discovery rate for the neurodevelopmental condition that is no greater than about 0.30, and the method comprising:
generating a second version of the risk genes data store that includes a second plurality of genes having a second false discovery rate for the neurodevelopmental condition that is no greater than about 0.20.

36. The method of any one of claims 31-35, wherein the first genetic features are obtained from a risk variants data store that includes a number of variants of genes that have at least a threshold strength of association with the neurodevelopmental condition, and the method comprising:
determining a p-value for a variant of a gene and the neurodevelopmental condition using a Fisher's exact test; and
modifying the p-value to produce an adjusted *p*-value based on one or more multiple-testing correction techniques; and
determining a strength of association between the variant of the gene and the neurodevelopmental condition based on the adjusted p-value.

37. The method of any one of claims 31-36, wherein the first group of features and the second group of features are determined using at least one of one or more hierarchical-based clustering techniques, one or more partitioning-based clustering techniques, one or more density-based clustering techniques, one or more model-based clustering techniques, or one or more graph-based clustering techniques.

38. A system comprising:
one or more hardware processors;
one or more non-transitory computer-readable storage media storing computer readable instructions that, when executed by the one or more hardware processors, cause the one or more hardware processors to perform operations comprising:
obtaining data corresponding to a neurodevelopmental condition from one or more data stores, the data indicating information related to individuals in which the neurodevelopmental condition is present;
analyzing the data to determine a first group of individuals in which the neurodevelopmental condition is present;
analyzing the data to determine a second group of individuals in which the neurodevelopmental condition is present, the second group of individuals including at least one individual that is different from at least one individual of the first group of individuals;
determining a first group of features corresponding to the first group of individuals that has at least a threshold amount of significance with respect to the neurodevelopmental condition, the first group of features including at least one of one or more first genetic features, one or more first observable features, one or more first co-occurring conditions, or one or more first analytes;
determining a second group of features corresponding to the second group of individuals that has at least the threshold amount of significance with respect to the neurodevelopmental condition, the second group of features including at least one of one or more second genetic features, one or more second observable features, one or more second co-occurring conditions, or one or more second analytes, wherein at least one feature included in the second group of features is different from at least one feature included in the first group of features; and
determining a measure of at least one of similarity and/or reliability between the first group of individuals and the second group of individuals.

39. The system of claim 38, wherein:
the one or more non-transitory computer-readable storage media store additional computer-readable instructions that, when executed by the one or more hardware processors, cause the one or more hardware processors to perform operations comprising:
determining a first amount of similarity between first individuals included in the first group of individuals; and
determining a second similarity between the first individuals and second individuals included in the second group of individuals; and
the measure of reliability is determined based on the first amount of similarity and the second amount of similarity.

40. The system of claim 38 or 39, wherein:
the one or more non-transitory computer-readable storage media store additional computer-readable instructions that, when executed by the one or more hardware processors, cause the one or more hardware processors to perform operations comprising:
determining a first number of groupings of the individuals in which the neurodevelopmental condition is present using first information obtained from one or more first sources of information storing genetic information of a first plurality of individuals in which the neurodevelopmental condition is present;
determining a second number of groupings of the individuals in which the neurodevelopmental condition is present using second information obtained from one or more second sources of information storing biological pathway information of a second plurality of individuals in which the neurodevelopmental condition is present;
determining a third number of groupings of the individuals in which the neurodevelopmental condition is present using third information obtained from one or more third sources of information storing phenotypic information of a third plurality of individuals in which the neurodevelopmental condition is present; and
determining a fourth number of groupings of individuals in which the neurodevelopmental condition is present using fourth information obtained from one or more fourth sources of information storing clinical data of a fourth plurality of individuals in which the neurodevelopmental condition is present.

41. The system of claim 40, wherein at least a portion of the individuals included in a first grouping of the first number of groupings is included in at least one of a second grouping of the second number of groupings, a third grouping of the third number of groupings, or a fourth grouping of the fourth number of groupings.

42. The system of claim 40, wherein the one or more non-transitory computer-readable storage media store additional computer-readable instructions that, when executed by the one or more hardware processors, cause the one or more hardware processors to perform operations comprising:
determining a first level of preservation between the first individuals included in the first grouping and a portion of the second individuals included in a second grouping of the second number of groupings;
determining a second level of preservation between the first individuals included in the first grouping and a portion of the third individuals included in a third grouping of the third number of groupings;
determining a third level of preservation between the first individuals included in the first grouping and a portion of the third individuals included in a third grouping of the third number of groupings; and
determining that at least one of the first level of preservation, the second level of preservation, or the third level of preservation corresponds to a threshold level of preservation.

43. The system of claim 42, wherein:
the one or more non-transitory computer-readable storage media store additional computer-readable instructions that, when executed by the one or more hardware processors, cause the one or more hardware processors to perform operations comprising determining an amount of overlap between the first individuals included in the first grouping and a number of the second individuals included in the second grouping; and
the first level of preservation is based on the amount of overlap in relation to an expected amount of overlap, the expected amount of overlap corresponding to respective amounts of overlap between individuals included in a number of pairs of groupings that are selected in a random or pseudo-random manner.

44. The system of claim 42, wherein:
the one or more non-transitory computer-readable storage media store additional computer-readable instructions that, when executed by the one or more hardware processors, cause the one or more hardware processors to perform operations comprising determining a number of features of the first individuals included in the first grouping that are shared with a number of the second individuals included in the second grouping; and
the first level of preservation is based on the number of features shared between the first individuals included in the first grouping and the number of the second individuals included in the second grouping.

45. The system of any one of claims 42-44, wherein:
the one or more non-transitory computer-readable storage media store additional computer-readable instructions that, when executed by the one or more hardware processors, cause the one or more hardware processors to perform operations comprising determining that the first level of preservation corresponds to the threshold level of preservation; and
at least a portion of the first individuals included in the first grouping and at least a portion of the individuals included in the second grouping are included in the first group of individuals.

46. A method comprising:
obtaining, by a computing system including one or more hardware processors and memory, data indicating measures of expression of a number of genes in response to one or more therapeutics of a plurality of therapeutics;
determining, by the computing system and based on the data, a first gene expression profile for a first therapeutic of the plurality of therapeutics, the first gene expression profile indicating up-regulation of a first group of genes in response to the first therapeutic and down-regulation of a second group of genes in response to the first therapeutic;
determining, by the computing system and based on the data, a second gene expression profile for a second therapeutic of the plurality of therapeutics, the second expression profile indicating up-regulation of a third group of genes in response to the second therapeutic and down-regulation of a fourth group of genes in response to the second therapeutic;
generating, by the computing system, a measure of similarity between the first gene expression profile and the second gene expression profile, the measure of similarity indicating a number of the first group of genes that correspond to a number of the third group of genes and a number of the second group of genes that correspond to a number of the fourth group of genes; and
generating, by the computing system, a gene expression profile distance network, the gene expression profile distance network including a first node that corresponds to the first therapeutic and a second node that corresponds to the second therapeutic with a distance between the first node and the second node in the therapeutics network corresponding to the measure of similarity.

47. The method of claim 46, comprising:
determining, by the computing system, an amount of expression of a gene with respect to a therapeutic based on RNA sequencing information obtained after profiling one or more cell lines with respect to the therapeutic.

48. The method of claim 46 or 47, wherein:
the first group of genes includes from about 50 to 500 first genes having at least a threshold amount of up-regulation in response to the first therapeutic and includes the second group of genes includes from about 50 to 500 second genes having at least a threshold amount of down-regulation in response to the first therapeutic; and
the third group of genes includes from about 50 to 500 third genes having at least the threshold amount of up-regulation in response to the second therapeutic and includes the fourth group of genes includes from about 50 to 500 fourth genes having at least the threshold amount of down-regulation in response to the second therapeutic.

49. The method of any one of claims 46-48, wherein the measure of similarity is determined by:
analyzing the first group of genes with respect to the third group of genes to determine one or more first shared genes that are included in the first group of genes and included in the third group of genes;
analyzing the second group of genes with respect to the fourth group of genes to determine one or more second shared genes that are included in the second group of genes and included in the fourth group of genes;
analyzing the third group of genes with respect to the first group of genes to determine one or more third shared genes that are included in the third group of genes and included in the first group of genes; and
analyzing the fourth group of genes with respect to the second group of genes to determine one or more fourth shared genes that are included in the fourth group of genes and included in the second group of genes.

50. The method of claim 49, comprising:
determining the one or more first shared genes by performing a comparison in relation to a plurality of first pairs of genes with each first pair of genes including one gene selected from the first group of genes and an additional gene selected from the third group of genes; and
determining the one or more second shared genes by performing a comparison in relation to a plurality of second pairs of genes with each second pair of genes including one gene selected from the second group of genes and an additional gene selected from the fourth group of genes.

51. The method of claim 50, comprising:
comparing a first gene selected from the first group of genes with a second gene selected from the third group of genes;
determining that the first gene corresponds to the second gene;
increasing a value of a first measure of similarity based on the first gene corresponding to the second gene;
comparing a third gene selected from the first group of genes with a fourth gene selected from the third group of genes;
determining that the third gene does not correspond to the fourth gene; and
decreasing the value of the first measure of similarity based on a lack of correspondence between the third gene and the fourth gene.

52. The method of claim 51, comprising:
comparing a fifth gene selected from the third group of genes with a sixth gene selected from the first group of genes;
determining that the fifth gene corresponds to the sixth gene;
increasing a value of a second measure of similarity based on the fifth gene corresponding to the sixth gene;
comparing a seventh gene selected from the third group of genes with an eighth gene selected from the first group of genes;
determining that the seventh gene does not correspond to the eighth gene; and
decreasing the value of the second measure of similarity based on a lack of correspondence between the seventh gene and the eighth gene.

53. The method of claim 52, comprising combining the value of the first measure of similarity with the value of the second measure of similarity to determine a value of the measure of similarity between the first gene expression profile and the second gene expression profile.

54. A system comprising:
one or more hardware processors;
one or more non-transitory computer-readable storage media storing computer readable instructions that, when executed by the one or more hardware processors, cause the one or more hardware processors to perform operations comprising:
obtaining data indicating measures of expression of a number of genes in response to one or more therapeutics of a plurality of therapeutics;
determining, based on the data, a first gene expression profile for a first therapeutic of the plurality of therapeutics, the first gene expression profile indicating up-regulation of a first group of genes in response to the first therapeutic and down-regulation of a second group of genes in response to the first therapeutic;
determining, based on the data, a second gene expression profile for a second therapeutic of the plurality of therapeutics, the second gene expression profile indicating up-regulation of a third group of genes in response to the second therapeutic and down-regulation of a fourth group of genes in response to the second therapeutic;
generating a measure of similarity between the first gene expression profile and the second gene expression profile, the measure of similarity indicating a number of the first group of genes that correspond to a number of the third group of genes and a number of the second group of genes that correspond to a number of the fourth group of genes; and
generating a gene expression profile distance network, the gene expression profile distance network including a first node that corresponds to the first therapeutic and a second node that corresponds to the second therapeutic with a distance between the first node and the second node in the therapeutics network corresponding to the measure of similarity.

55. The system of claim 54, wherein:
the one or more non-transitory computer-readable storage media store additional computer readable instructions that, when executed by the one or more hardware processors, cause the one or more hardware processors to perform operations comprising:
generating a ranked list of genes based on respective amounts of up-regulation and respective amounts of down-regulation of the genes in response to the first therapeutic;
determining a first number of genes of the ranked list that have at least a threshold amount of up-regulation; and
determining a second number of genes of the ranked list that have at least a threshold amount of down-regulation; and
the first group of genes included in the first gene expression profile includes the first number of genes and the second group of genes included in the first gene expression profile includes the second number of genes.

56. The system of claim 54, wherein one or more non-transitory computer-readable storage media store additional computer readable instructions that, when executed by the one or more hardware processors, cause the one or more hardware processors to perform operations comprising:
comparing individual genes selected from the first group of genes with individual genes included in the third group of genes;
determining a number of first pairs of shared genes between the individual genes included in the first group of genes and the individual genes included in the third group of genes with each first pair of shared genes including one gene included in the first group of genes and an additional gene included in the third group of genes;
determining a number of first pairs of differing genes between the individual genes included in the first group of genes and the individual genes included in the third group of genes with each first pair of differing genes including a gene included in the first group of genes and another gene included in the third group of genes;
increasing a value of at least a portion of the measure of similarity based on the number of first pairs of shared genes; and
decreasing the value of the at least a portion of the measure of similarity based on the number of first pairs of differing genes.

57. The system of claim 56, wherein one or more non-transitory computer-readable storage media store additional computer readable instructions that, when executed by the one or more hardware processors, cause the one or more hardware processors to perform operations comprising:
comparing individual genes selected from the second group of genes with individual genes included in the fourth group of genes;
determining a number of second pairs of shared genes between the individual genes included in the second group of genes and the individual genes included in the fourth group of genes with each second pair of shared genes including one gene included in the second group of genes and an additional gene included in the fourth group of genes;
determining a number of second pairs of differing genes between the individual genes included in the second group of genes and the individual genes included in the fourth group of genes with each pair of second pair of differing genes including a gene included in the second group of genes and another gene included in the fourth group of genes;
increasing the value of the at least a portion of the measure of similarity based on the number of second pairs of shared genes; and
decreasing the value of the at least a portion of the measure of similarity based on the number of second pairs of shared genes.

58. The system of any one of claims 54-57, wherein:
the data indicating the measures of expression of the number of genes in response to the one or more therapeutics is obtained from a first data source; and
the one or more non-transitory computer-readable storage media store additional computer readable instructions that, when executed by the one or more hardware processors, cause the one or more hardware processors to perform operations comprising:
obtaining additional data indicating additional measures of expression of an additional number of genes in response to one or more additional sources of genetic expression modification that are different from therapeutics;
determining, based on the additional data, a first additional gene expression profile for a first additional source of genetic expression modification, the first additional gene expression profile indicating up-regulation of a first additional group of genes in response to the first additional source of genetic expression modification and down-regulation of a second additional group of genes in response to the first additional source of genetic expression modification;
determining, based on the additional data, a second additional gene expression profile for a second additional source of genetic expression modification, the second additional gene expression profile indicating up-regulation of a third additional group of genes in response to the second additional source of genetic expression modification and down-regulation of a fourth additional group of genes in response to the second additional source of genetic expression modification;
generating an additional measure of similarity between the first additional gene expression profile and the second additional gene expression profile, the additional measure of similarity indicating a number of the first additional group of genes that correspond to a number of the third additional group of genes and a number of the second additional group of genes that correspond to a number of the fourth additional group of genes; and
generating an additional gene expression profile distance network, the additional gene expression profile distance network including a first additional node that corresponds to the first additional source of genetic expression modification and a second additional node that corresponds to the second additional source of genetic expression modification with an additional edge between the first additional node and the second additional node, a length of the additional edge corresponding to the additional measure of similarity.

59. The system of claim 58, wherein the additional source of genetic expression modification includes a biological condition or short hairpin ribonucleic acid (shRNA) molecules.

60. The system of any one of claims 55-59, wherein:
the one or more non-transitory computer-readable storage media store additional computer readable instructions that, when executed by the one or more hardware processors, cause the one or more hardware processors to perform operations comprising:
generating a third gene expression profile indicating an up-regulation of a fifth group of genes in a group of individuals in which a neurodevelopmental condition is present and a down-regulation of a sixth group of genes in the group of individuals;
generating a further measure of similarity between the first gene expression profile and the third gene expression profile, the further measure of similarity indicating a number of the first group of genes that correspond to the sixth group of genes and a number of the second group of genes that correspond to the fifth group of genes;
determining, based on the further measure of similarity, a probability that the first therapeutic is a candidate treatment for the group of individuals in relation to the neurodevelopmental condition.

61. A method comprising:
obtaining, by a computing system including one or more hardware processors and memory, first data indicating measures of expression of a number of genes in response to a therapeutic;
determining, by the computing system and based on the first data, a gene expression profile for the therapeutic, the gene expression profile indicating up-regulation of a first group of genes in response to the therapeutic and down-regulation of a second group of genes in response to the therapeutic;
obtaining, by the computing system, second data including genetic information of a plurality of individuals in which a neurodevelopmental condition is present;
determining, by the computing system and based on the second data, up-regulation of a third group of genes in the plurality of individuals and down-regulation of a fourth group of genes in the plurality of individuals;
determining, by the computing system, a first number of the first group of genes that corresponds to the fourth group of genes and a second number of the second group of genes that corresponds to the third group of genes; and
determining, by the computing system and based on the first number of the first group of genes and the second number of the second group of genes, a probability that the therapeutic is a candidate treatment for the neurodevelopmental condition with respect to the plurality of individuals.

62. The method of claim 6 Implementation 1, wherein the plurality of individuals comprises a subgroup of a population of individuals in which the neurodevelopmental condition is present; and
the method comprising:
obtaining, by the computing system, third data including genetic information of an additional subgroup of the population of individuals in which the neurodevelopmental condition is present;
determining, by the computing system and based on the third data, up-regulation of a fifth group of genes in the additional subgroup and down-regulation of a sixth group of genes in the additional subgroup;
determining, by the computing system, a third number of the first group of genes that corresponds to the sixth group of genes and a fourth number of the second group of genes that corresponds to the fifth group of genes; and
determining, by the computing system and based on the third number of the first group of genes and the fourth number of the second group of genes, an additional probability that the therapeutic is an additional candidate treatment for the neurodevelopmental condition with respect to the additional subgroup of the population.

63. The method of claim 62, wherein the additional probability that the therapeutic is an additional candidate treatment for the neurodevelopmental condition with respect to the additional sub-group of the population is less than the probability that the therapeutic is a candidate treatment for the neurodevelopmental condition with respect to the plurality of individuals.

64. The method of claim 61, wherein at least one gene of the fifth group of genes is different from at least one gene included in the third group of genes and at least one gene of the sixth group of genes is different from at least one gene included in the fourth group of genes.

65. The method of claim 61, comprising:
obtaining third data indicating measures of expression of an additional number of genes in response to an additional therapeutic;
determining, based on the third data, an additional gene expression profile for the additional therapeutic, the additional gene expression profile indicating up-regulation of a fifth group of genes in response to the additional therapeutic and down-regulation of a sixth group of genes in response to the additional therapeutic;
determining, by the computing system, a third number of the fifth group of genes that corresponds to the fourth group of genes and a fourth number of the sixth group of genes that corresponds to the third group of genes; and
determining, by the computing system and based on the third number of the fifth group of genes and the fourth number of the sixth group of genes, an additional probability that the additional therapeutic is an additional candidate treatment for the neurodevelopmental condition with respect to the plurality of individuals.

66. The method of claim 65, comprising:
determining that the probability that the therapeutic is a candidate treatment for the neurodevelopmental condition with respect to the plurality of individuals is at least a threshold probability;
determining that the additional probability that the additional therapeutic is an additional candidate treatment for the neurodevelopmental condition with respect to the plurality of individuals is at least the threshold probability; and
determining that a further candidate treatment for the neurodevelopmental condition with respect to the plurality of individuals includes a combination of the first therapeutic and the second therapeutic.

67. A system comprising
one or more hardware processors;
one or more non-transitory computer-readable storage media storing computer readable instructions that, when executed by the one or more hardware processors, cause the one or more hardware processors to perform operations comprising:
obtaining first data indicating measures of expression of a number of genes in response to a therapeutic;
determining, based on the first data, a gene expression profile for the therapeutic, the gene expression profile indicating up-regulation of a first group of genes in response to the therapeutic and down-regulation of a second group of genes in response to the therapeutic;
obtaining second data including genetic information of a plurality of individuals in which a neurodevelopmental condition is present;
determining, based on the second data, up-regulation of a third group of genes in the plurality of individuals and down-regulation of a fourth group of genes in the plurality of individuals;
determining a first number of the first group of genes that corresponds to the fourth group of genes and a second number of the second group of genes that corresponds to the third group of genes; and
determining, based on the first number of the first group of genes and the second number of the second group of genes, a probability that the therapeutic is a candidate treatment for the neurodevelopmental condition with respect to the plurality of individuals.

68. The system of claim 67, wherein the first group of genes includes from about 50 to 500 genes having at least a threshold amount of up-regulation in response to the therapeutic and the second group of genes includes from about 50 to 500 genes having at least a threshold amount of down-regulation in response to the therapeutic.

69. The system of claim 67 or 68, wherein the one or more non-transitory computer-readable storage media store additional computer readable instructions that, when executed by the one or more hardware processors, cause the one or more hardware processors to perform operations comprising:
determining, based on the second data, a first additional gene expression profile for a first individual included in the plurality of individuals, the first additional gene expression profile indicating up-regulation of a first additional group of genes in the first individual and down-regulation of a second additional group of genes in the first individual;
determining, based on the second data, a second additional gene expression profile for a second individual included in the plurality of individuals, the second additional gene expression profile indicating up-regulation of a third additional group of genes in the second individual and down-regulation of a fourth additional group of genes in the second individual; and
generating a consensus gene expression profile based on at least the first additional gene expression profile and the second additional gene expression profile, the consensus gene expression profile indicating up-regulation of a fifth additional group of genes and down-regulation of a sixth additional group of genes, the fifth additional group of genes including at least a portion of the first additional group of genes and at least a portion of the third additional group of genes and the sixth additional group of genes including at least a portion of the second additional group of genes and at least a portion of the fourth additional group of genes.

70. The system of claim 69, wherein the one or more non-transitory computer-readable storage media store additional computer readable instructions that, when executed by the one or more hardware processors, cause the one or more hardware processors to perform operations comprising:
generating a plurality of additional gene expression profiles for a plurality of individuals, each additional gene expression profile of the plurality of gene expression profiles indicating up-regulation of one or more genes and down-regulation of one or more genes of a respective individual included in the plurality of individuals;
generating a plurality of merged gene expression profiles based on the plurality of additional gene expression profiles, each merged gene expression profile of the plurality of merged gene expression profiles being generated based on a first plurality of up-regulated genes and a first plurality of down-regulated genes of a first additional gene expression profile of the plurality of additional gene expression profiles and on a second plurality of up-regulated genes and a second plurality of down-regulated genes of a second additional gene expression profile of the plurality of additional gene expression profiles; and
determining an overall ranking of genes included in the plurality of additional gene expression profiles based on rankings of the genes in at least a portion of the plurality of merged gene expression profiles, the overall ranking of genes corresponding to the consensus gene expression profile.

71. The system of claim 67 or 68, wherein the one or more non-transitory computer-readable storage media store additional computer readable instructions that, when executed by the one or more hardware processors, cause the one or more hardware processors to perform operations comprising:
determining an additional gene expression profile for the plurality of individuals, the additional gene expression profile including the third group of genes and the fourth group of genes;
analyzing the first group of genes with respect to the fourth group of genes to determine one or more first shared genes that are included in the first group of genes and included in the fourth group of genes;
analyzing the second group of genes with respect to the third group of genes to determine one or more second shared genes that are included in the second group of genes and included in the third group of genes;
analyzing the fourth group of genes with respect to the first group of genes to determine one or more third shared genes that are included in the fourth group of genes and included in the first group of genes;
analyzing the third group of genes with respect to the second group of genes to determine one or more fourth shared genes that are included in the third group of genes and included in the second group of genes; and
determining a measure of similarity between the gene expression profile and the additional gene expression profile based on a number of the one or more first shared genes, a number of the one or more second shared genes, a number of the one or more third shared genes, and a number of the one or more fourth shared genes.

72. The system of claim 71, wherein the one or more non-transitory computer-readable storage media store additional computer readable instructions that, when executed by the one or more hardware processors, cause the one or more hardware processors to perform operations comprising:
determining the one or more first shared genes by performing a comparison in relation to a plurality of first pairs of genes with each first pair of genes including one gene selected from the first group of genes and an additional gene selected from the fourth group of genes; and
determining the one or more second shared genes by performing a comparison in relation to a plurality of second pairs of genes with each second pair of genes including one gene selected from the second group of genes and an additional gene selected from the third group of genes.

73. The system of claim 72, wherein the one or more non-transitory computer-readable storage media store additional computer readable instructions that, when executed by the one or more hardware processors, cause the one or more hardware processors to perform operations comprising:
comparing a first gene selected from the first group of genes with a second gene selected from the fourth group of genes;
determining that the first gene corresponds to the second gene;
increasing a value of a first measure of similarity based on the first gene corresponding to the second gene;
comparing a third gene selected from the first group of genes with a fourth gene selected from the fourth group of genes;
determining that the third gene does not correspond to the fourth gene; and
decreasing the value of the first measure of similarity based on a lack of correspondence between the third gene and the fourth gene.

74. The system of claim 73, wherein the one or more non-transitory computer-readable storage media store additional computer readable instructions that, when executed by the one or more hardware processors, cause the one or more hardware processors to perform operations comprising:
comparing a fifth gene selected from the fourth group of genes with a sixth gene selected from the first group of genes;
determining that the fifth gene corresponds to the sixth gene;
increasing a value of a second measure of similarity based on the fifth gene corresponding to the sixth gene;
comparing a seventh gene selected from the fourth group of genes with an eighth gene selected from the first group of genes;
determining that the seventh gene does not correspond to the eighth gene; and
decreasing the value of the second measure of similarity based on a lack of correspondence between the seventh gene and the eighth gene.

75. The system of claim 74, wherein the one or more non-transitory computer-readable storage media store additional computer readable instructions that, when executed by the one or more hardware processors, cause the one or more hardware processors to perform operations comprising:
combining the value of the first measure of similarity with the value of the second measure of similarity to determine a value of the measure of similarity between the gene expression profile and the additional gene expression profile.

76. A method comprising:
obtaining, by a computing system including one or more hardware processors and memory, genetic data from at least one data source including at least one of co-occurring condition genetic data, phenotype classification genetic data, or biological pathway genetic data;
obtaining, by the computing system, a gene expression profile corresponding to a therapeutic, the gene expression profile indicating a first group of genes that are up-regulated in response to the therapeutic and a second group of genes that are down-regulated in response to the therapeutic;
analyzing, by the computing system, the gene expression profile in relation to the genetic data obtained from the at least one data source to determine one or more genes included in the gene expression profile that have at least a threshold amount of representation in at least one of the co-occurring condition genetic data, the phenotype classification genetic data, or the biological pathway genetic data;
determining, by the computing system, a therapeutic responders profile that includes a plurality of features, at least a portion of the plurality of features corresponding to the one or more genes;
analyzing, by the computing system, information corresponding to a plurality of subgroups of individuals in which a neurodevelopmental condition is present to determine respective profiles corresponding to each of the subgroups, the respective subgroup profiles each indicating features of individuals included in the respective subgroups;
determining, by the computing system, an amount of overlap between the plurality of features included in the therapeutic responders profile and the features of individuals included in the respective subgroup profiles;
determining, by the computing system, a subgroup of the plurality of subgroups that has at least a threshold amount of overlap between the plurality of features included in the therapeutic responders profile and a number of features corresponding to individuals included in the subgroup profile; and
determining, by the computing system, a probability that the therapeutic is a candidate treatment for the neurodevelopmental condition with respect to the subgroup.

77. The method of claim 76, wherein:
the co-occurring condition genetic data is related to a first number of genes that are over-expressed in a group of first individuals in which the neurodevelopmental condition and an additional biological condition are present and a second number of genes that are under-expressed in the group of first individuals in which the neurodevelopmental condition and the additional biological condition are present;
the phenotype classification genetic data indicates one or more genes that correspond to at least one phenotype of a group of second individuals in which the neurodevelopmental condition is present; and
the biological pathway genetic data indicates one or more genes that are expressed in relation to disruption of at least one biological pathway in a group of third individuals in which the neurodevelopmental condition is present.

78. The method of claim 77, wherein at least one first individual included in the group of first individuals is different from at least one second individual included in the group of second individuals and from at least one third individual included in the group of third individuals; and
at least one additional second individual included in the group of second individuals is different from at least one additional third individual included in the group of third individuals.

79. The method of any one of claims 76-78, comprising:
determining a first ranking of a gene included in the gene expression profile based on an amount of up-regulation of the gene in response to the therapeutic or an amount of down-regulation of the gene in response to the therapeutic;
determining that the gene is present in at least one of the co-occurring condition genetic data, phenotype classification genetic data, or biological pathway genetic data; and
generating an additional gene expression profile that includes the gene with the gene having a second ranking in the additional gene expression profile, the second ranking being based on the first ranking and the additional gene expression profile being included in the therapeutic responders profile.

80. The method of claim 79, comprising:
determining that an additional gene included in the gene expression profile is not present in at least one of the co-occurring condition genetic data, phenotype classification genetic data, or biological pathway genetic data; and
determining that the additional gene is to be excluded from the additional gene expression profile.

81. The method of any one of claims 79 or 80, comprising:
generating a further gene expression profile for the subgroup of the plurality of subgroups, the further gene expression profile indicating a third group of genes that are up-regulated in individuals included in the subgroup and a fourth group of genes that are down-regulated in individuals included in the subgroup; and
determining a measure of similarity between the additional gene expression profile included in the therapeutic responders profile and the further gene expression profile for the subgroup, the measure of similarity corresponding to at least a portion of the amount of overlap between the plurality of features included in the therapeutic responders profile and the number of features corresponding to the individuals included in the subgroup.

82. The method of any one of claims 76-81, wherein the therapeutic responders profile includes at least one of a biological pathway that is disrupted in individuals in which the neurodevelopmental condition is present, a co-occurring biological condition that is present in individuals in which the neurodevelopmental condition is present, a phenotype that is related to individuals in which the neurodevelopmental condition is present, levels of analytes present in individuals in which the neurodevelopmental condition is present, or a morphological condition that is present in individuals in which the neurodevelopmental condition is present.

83. The method of any one of claims 76-82, wherein the probability that the therapeutic is a candidate treatment for the neurodevelopmental condition with respect to the subgroup corresponds to a probability that the therapeutic alleviates at least a portion of the symptoms of the subgroup with respect to the neurodevelopmental condition.

84. A system comprising:
one or more hardware processors;
one or more non-transitory computer-readable storage media storing computer readable instructions that, when executed by the one or more hardware processors, cause the one or more hardware processors to perform operations comprising:
obtaining genetic data from at least one data source including at least one of co-occurring condition genetic data, phenotype classification genetic data, or biological pathway genetic data;
obtaining a gene expression profile corresponding to a therapeutic, the gene expression profile indicating a first group of genes that are up-regulated in response to the therapeutic and a second group of genes that are down-regulated in response to the therapeutic;
analyzing the gene expression profile in relation to the genetic data obtained from the at least one data source to determine one or more genes included in the gene expression profile that have at least a threshold amount of representation in at least one of the co-occurring condition genetic data, the phenotype classification genetic data, or the biological pathway genetic data;
determining a therapeutic responders profile that includes a plurality of features, at least a portion of the plurality of features corresponding to the one or more genes;
analyzing information corresponding to a plurality of subgroups of individuals in which a neurodevelopmental condition is present to determine respective subgroup profiles corresponding to each of the subgroups, the respective subgroup profiles each indicating features of individuals included in the respective subgroups;
determining an amount of overlap between the plurality of features included in the therapeutic responders profile and the features of individuals included in the respective subgroup profiles;
determining a subgroup of the plurality of subgroups that has at least a threshold amount of overlap between the plurality of features included in the therapeutic responders profile and a number of features corresponding to individuals included in the subgroup profile; and
determining a probability that the therapeutic is a candidate treatment for the neurodevelopmental condition with respect to the subgroup.

85. The system of claim 84, wherein:
the one or more non-transitory computer-readable storage media store additional computer readable instructions that, when executed by the one or more hardware processors, cause the one or more hardware processors to perform operations comprising:
generating a ranked list of genes based on respective amounts of up-regulation and respective amounts of down-regulation of a plurality of genes in response to the therapeutic;
determining a first number of genes of the ranked list that have at least a threshold amount of up-regulation; and
determining a second number of genes of the ranked list that have at least a threshold amount of down-regulation; and
the first group of genes included in the gene expression profile includes the first number of genes and the second group of genes included in the gene expression profile includes the second number of genes.

86. The system of claim 84 or 85, wherein the one or more non-transitory computer-readable storage media store additional computer readable instructions that, when executed by the one or more hardware processors, cause the one or more hardware processors to perform operations comprising:
determining a first ranking of a gene included in the gene expression profile based on an amount of up-regulation of the gene in response to the therapeutic or an amount of down-regulation of the gene in response to the therapeutic;
determining that the gene is present in at least one of the co-occurring condition genetic data, phenotype classification genetic data, or biological pathway genetic data; and
generating an additional gene expression profile that includes the gene with the gene having a second ranking in the additional gene expression profile, wherein the second ranking is based on the first ranking, the additional gene expression profile is included in the therapeutic responders profile, and the additional gene expression profile indicates up-regulation of a third group of genes that includes at least a portion of the first group of genes and down-regulation of a fourth group of genes that includes at least a portion of the second group of genes.

87. The system of claim 86, wherein:
the information corresponding to the plurality of subgroups of individuals in which the neurodevelopmental condition is present includes genetic information of a plurality of individuals included in the plurality of subgroups;
the one or more non-transitory computer-readable storage media store additional computer readable instructions that, when executed by the one or more hardware processors, cause the one or more hardware processors to perform operations comprising:
determining, based on the genetic information, up-regulation of a fifth group of genes in the plurality of individuals and down-regulation of a sixth group of genes in the plurality of individuals; and
generating a subgroup gene expression profile that includes the fifth group of genes and the sixth group of genes, the subgroup gene expression profile being included in the subgroup profile.

88. The system of claim 87, wherein the one or more non-transitory computer-readable storage media store additional computer readable instructions that, when executed by the one or more hardware processors, cause the one or more hardware processors to perform operations comprising:
comparing individual genes selected from the third group of genes with individual genes included in the sixth group of genes;
determining a number of first pairs of shared genes between the individual genes included in the third group of genes and the individual genes included in the sixth group of genes with each first pair of shared genes including one gene included in the third group of genes and an additional gene included in the sixth group of genes;
determining a number of first pairs of differing genes between the individual genes included in the third group of genes and the individual genes included in the sixth group of genes with each first pair of differing genes including a gene included in the third group of genes and another gene included in the sixth group of genes;
increasing a value of a first measure of similarity based on the number of first pairs of shared genes; and
decreasing the value of the first measure of similarity based on the number of first pairs of differing genes.

89. The system of claim 88, wherein one or more non-transitory computer-readable storage media store additional computer readable instructions that, when executed by the one or more hardware processors, cause the one or more hardware processors to perform operations comprising:
comparing individual genes selected from the fourth group of genes with individual genes included in the fifth group of genes;
determining a number of second pairs of shared genes between the individual genes included in the fourth group of genes and the individual genes included in the fifth group of genes with each second pair of shared genes including one gene included in the fourth group of genes and an additional gene included in the fifth group of genes;
determining a number of second pairs of differing genes between the individual genes included in the fourth group of genes and the individual genes included in the fifth group of genes with each second pair of differing genes including a gene included in the fourth group of genes and another gene included in the fifth group of genes;
increasing the value of a second measure of similarity based on the number of second pairs of shared genes; and
decreasing the value of the second measure of similarity based on the number of second pairs of shared genes.

90. The system of claim 89, wherein one or more non-transitory computer-readable storage media store additional computer readable instructions that, when executed by the one or more hardware processors, cause the one or more hardware processors to perform operations comprising:
combining the first measure of similarity and the second measure of similarity to generate an overall measure of similarity between the additional gene expression profile and the subgroup gene expression profile, wherein the probability that the therapeutic is a candidate treatment for the neurodevelopmental condition with respect to the subgroup is based at least partly on the overall measure of similarity.

91. A system comprising:
one or more hardware processors;
one or more non-transitory computer-readable storage media storing computer readable instructions that, when executed by the one or more hardware processors, cause the one or more hardware processors to perform operations comprising:
obtaining genetic data from at least one data source including at least one of co-occurring condition genetic data, phenotype classification genetic data, or biological pathway genetic data;
obtaining target profile data of a therapeutic and human protein interaction data indicating a group of genes in a network of genes that are regulated in response to the therapeutic and interactions between the group of genes with proteins in a population of humans;
analyzing the target profile data and protein interaction data in relation to the genetic data obtained from the at least one data source to determine one or more genes included in the network of genes that have at least a threshold amount of representation in at least one of the co-occurring condition genetic data, the phenotype classification genetic data, or the biological pathway genetic data;
determining a therapeutic responders profile that includes a plurality of features, at least a portion of the plurality of features corresponding to the one or more genes;
analyzing information corresponding to a plurality of subgroups of individuals in which a neurodevelopmental condition is present to determine respective subgroup profiles corresponding to each of the subgroups, the respective subgroup profiles each indicating features of individuals included in the respective subgroups;
determining an amount of overlap between the plurality of features included in the therapeutic responders profile and the features of individuals included in the respective subgroup profiles;
determining a subgroup of the plurality of subgroups that has at least a threshold amount of overlap between the plurality of features included in the therapeutic responders profile and a number of features corresponding to individuals included in the subgroup profile; and
determining a probability that the therapeutic is a candidate treatment for the neurodevelopmental condition with respect to the subgroup.
